Europäisches Patentamt

European Patent Office

Office européen des brevets

(19)

(11) **EP 1 454 993 A1**

(12) **EUROPEAN PATENT APPLICATION**

(43) Date of publication:
**08.09.2004 Bulletin 2004/37**

(51) Int Cl.7: **C12Q 1/68**, G01N 33/483,
G01N 30/72

(21) Application number: **03004838.3**

(22) Date of filing: **05.03.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK**<br><br>(71) Applicant: **MAX-PLANCK-GESELLSCHAFT ZUR FÖRDERUNG DER WISSENSCHAFTEN E.V.**<br>**14195 Berlin (DE)** | (72) Inventor: **The designation of the inventor has not yet been filed**<br><br>(74) Representative: **VOSSIUS & PARTNER**<br>**Siebertstrasse 4**<br>**81675 München (DE)** |

(54) **Method for identifying the function of a gene**

(57)    Described is a method for determining the function of a gene. This method involves determining the amount of transcript for each of a set of candidate genes in samples taken from different phenotypic and/or genotypic states of an organism and determining the amount of each of a plurality of metabolites in different samples taken from the same states as those mentioned above. Subsequently, the data obtained is analyzed by suitable mathematical methods in order to identify a transcript and metabolites which correlate in the different states, thereby identifying a transcript corresponding to a gene which influences the amount of metabolites in the organism. Furthermore described is a method for identifying a gene which is capable of modifying the amount of a metabolite in an organism and to a method for identifying a metabolite which is capable of modifying the amount of a transcript in an organism. Likewise, uses of the genes and metabolites identified in the aforementioned methods are described.

EP 1 454 993 A1

**SEQUENCE LISTING**

<110> Max-Planck-Gesellschaft zur Förderung der Wissenschaften e.V.

<120> Method for identifying the function of a gene

<130> G 3258 EP

<160> 3

<170> PatentIn version 3.1

<210> 1
<211> 23
<212> DNA
<213> Artificial sequence

<220>
<223> oligonucleotide

<400> 1
gcttccggct cgtatgttgt gtg                                          23


<210> 2
<211> 23
<212> DNA
<213> Artificial sequence

<220>
<223> Oligonucleotide

<400> 2
aaagggggat gtgctgcaag gcg                                          23


<210> 3
<211> 13
<212> DNA
<213> Artificial sequenze

<220>
<223> oligonucleotide

<400> 3
ttcccagtca cga                                                     13


**Description**

The present invention relates to a method for determining the function of a gene. This method involves determining the amount of transcript for each of a set of candidate genes in samples taken from different phenotypic and/or genotypic states of an organism and determining the amount of each of a plurality of metabolites in different samples taken from the same states as those mentioned above. Subsequently, the data obtained is analyzed by suitable mathematical methods in order to identify a transcript and one or more metabolites which correlate in the different states, thereby identifying a transcript corresponding to a gene which influences the amount of these one or more metabolites in the organism. The invention furthermore relates to a method for identifying a gene which is capable of modifying the amount of a metabolite in an organism and to a method for identifying a metabolite which is capable of modifying the amount of a transcript in an organism. Likewise, the present invention relates to uses of the genes and metabolites identified in the aforementioned methods.

Since the advent of genetic engineering, many techniques have become available that allow the purposeful and specific modification of the transcript level of a given gene. This may lead to either an increase or to a decrease of the transcript level in a so-manipulated cell. Corresponding techniques have been described for many biological systems comprising various classes of prokaryotes and eukaryotes. The change in the transcript level may be effected transiently or, such as in the case of corresponding genetically modified organisms, constitutively.

However, until today, it cannot be reliably foreseen what effect the modified transcript level of a given gene has on the phenotype of the organism. The term "phenotype" refers herein to any possible detectable property of an organism, in particular including the amount of a polypeptide or, further downstream in the reaction path of gene expression, the amount of a metabolite in a cell. The uncertainty about the effect of a modification of the transcript level is mainly due to the complexity of the events that contribute to the expression of a certain phenotype which often involves a redundancy of the various gene expression and regulatory mechanisms, in particular when eukaryotes, and there especially higher eukaryotes such as mammals, plants or insects, are considered. For instance, up to now, one cannot reliably predict whether a significant reduction of a transcript, e.g. by antisense or RNA interference approaches, will effectively lead to a decrease of the corresponding protein activity in the cell. However, if already at the protein level the effect of a transcript reduction is uncertain, the more unpredictable is then the outcome of such a measure at the level of metabolites. Thus, when it is for instance the task to increase or decrease the amount of a certain metabolite, such as a nutritionally relevant compound in a plant, the scientists often fail to reach this goal or only achieve a minor success (see for instance DellaPenna, Science 285 (1999), 375-379). Here, the effect of a modified transcription of a gene that is involved in a biochemical pathway is often compensated by feedback control mechanisms or alternative pathways, as it beforehand could not have been foreseen. Thus, those who attempt to reach such goals are normally faced with an enormous extent of trial and error. Such experimental approaches are usually costly and time-consuming since they often involve the production of genetically modified organisms. Thus, there is a need for methods that allow it to more effectively apply the well-established techniques of modifying transcript levels in order to modify the metabolite composition of an organism.

Taking the inverse view, there is likewise a need for effector molecules that are capable of specifically modifying the transcription rate of a gene. This applies particularly to pathogenic conditions and diseases where, for instance due to a mutation, a certain gene is aberrantly over- or underexpressed.

In the post-genomic era, scientists working in medical and biological disciplines are confronted with the need of assigning functions to genes which have been identified in the course of genome sequencing. Based on this and following the aim to more comprehensively understand the regulatory processes in a cell, systems biology has evolved in the recent years (Kitano, 2002; Ideker, 2001; Oltvai, 2002). This field refers to multi-parallel analyses of a multitude of parameters of a given biological system at a range of different molecular levels following a systematic perturbation of the biological system.

The starting point of systems biology can be seen in the genomic research making use of the advanced technologies allowing the sequencing of enkaryotic genomes (see e.g. Arabidopsis and Human Genome Sequencing, 2000 & 2001) and the analysis of the expression level of complete genomes or large proportions thereof (i.e. transcriptomic analyses) (Lockhart, 1996). However, it is becoming increasingly clear that a wide range of post-translational factors bear functional importance in the cell as well. Thus, the development of strategies to allow similar comprehensive studies of the protein and metabolite complements (proteomics (Shevchenko, 1996) and metabolomics (Fiehn, 2000; Roessner, 2001)) of the cell have begun, but still at a relatively early stage. Despite the well-known connectivity between the molecules described by the transcriptomic, proteomic and metabolomic approaches, only a few studies have been described where it was tried to correlate parameters across the various levels (Ideker, 2001; Gygi, 1999; Futcher, 1999). In particular, approaches where transcriptomic data is directly correlated with metabolomic data has to date not yet been described.

In view of the above explanations, it is clear that there is a need for techniques allowing an improved deployment of transcription modification to achieve a deliberate change of metabolite composition in a biological system.

Thus, the technical problem underlying the present invention is the provision of means and methods that render it possible to modify the level of one or more metabolites in a given organism in a more reliable and predictable manner.

This technical problem is solved by the provision of the embodiments as characterized in the claims.

Accordingly, the present invention relates to a method for determining the function of a gene comprising

(a) determining the amount of transcript for each of a set of candidate genes in two or more samples from an organism, wherein the samples correspond to different phenotypic and/or genotypic states of said organism;
(b) determining the amount of each of a plurality of metabolites present in two or more samples corresponding to the same states as in (a);
(c) analysing by suitable mathematical methods the data obtained in steps (a) and (b) in order to identify a transcript and at least one metabolite the amounts of which significantly correlate in the different states,

said transcript corresponding to a gene having a function that influences the amount of said metabolite(s) in said organism.

The method of the invention is based on experiments by which it was surprisingly possible to find significant correlations between the amount of a transcript and the amount of a metabolite when different developmental stages of wild-type potato tubers and transgenic potato tubers are compared (see Example 2). Interestingly, it has been observed that the amount of one transcript may correlate significantly with the amount of more than one metabolite. Likewise, it has been seen that the amount of one metabolite may correlate significantly with the amount of more than one transcript. The observed correlations provide the information that the gene corresponding to the identified transcript may have a function that influences the amount of the one or more metabolites in the organism under investigation. Correspondingly, it can be expected that this gene is a promising target for an intervention that leads to a modified transcript amount of this gene in the corresponding organism if it is intended to modify the amount of said one or more metabolites. Furthermore, on the other hand, it is likewise conceivable that a metabolite the amount of which significantly correlates with the amount of one or more transcripts, or a structurally related compound exercising essentially similar effects, can be an effector molecule useful for modifying the amount of said transcript in the corresponding organism.

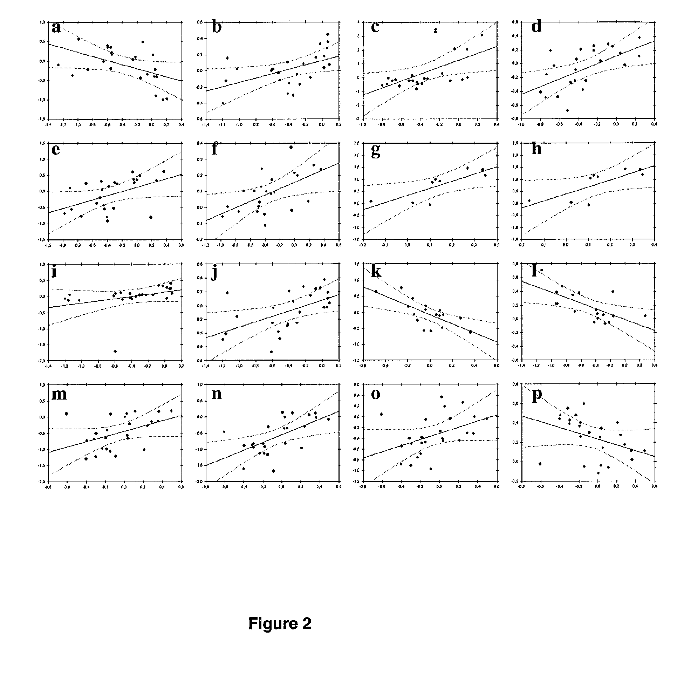

Specifically, in the experiments described in Example 2, 518 out of the 23715 transcript-metabolite pairs analysed exhibited significant correlations, whereby 329 correlations were positive and 189 negative. Some of the observed correlations confirmed interrelationships between gene expression and metabolite level that were already known. This provides the proof that the method of the invention works and gives reasonable results. This refers for instance to the strong negative correlation observed between sucrose and sucrose transporter expression (Figure 2a) that has previously been described by Vaughn (2002). The same holds true for the strong positive correlation between 4-amino butyric acid and glutamate decarboxylase isoform I (Figure 2b) previously described by Facchini (2000). Other observed correlations can at least retrospectively be explained based on the function of the encoded polypeptide. For instance, the metabolite may be a substrate, product or intermediate in a biochemical pathway in which the polypeptide participates. Examples for this are depicted in Figures 2c to 2f. Most interestingly, the analysis carried out according to the method of the invention also provided significant correlations which by no means could have been foreseen. This refers for example to the correlations shown in Figures 2k to 2p referring, e.g. to correlations between nutritionally relevant metabolites and transcription factors (see Example 2). It is believed that such new correlations may have a great potential for biotechnological applications in which it is the goal to modify the metabolite composition by genetic means or where effector compounds are needed for modifying the expression of a specific gene. In particular, the newly identified transcript-metabolite correlations are envisaged to present a powerful tool for the rapid identification of candidate genes which can then be tested by further experimentation for their value regarding applicability. The approach presented herein has not been described before.

By the method of the invention, it is possible to determine the function of a gene. The method involves the above-mentioned steps (a) to (c) by which a significant correlation between the amount of one or more transcripts with the amount of one or more metabolites in different states of an organism is determined. Such a correlation provides the information that, in the organism under investigation, a gene corresponding to the (or a) transcript of this correlation may have the function that it influences the amount of the one or more metabolites to which it correlates.

In connection with the method of the invention, the term "gene" refers to the conventional meaning of this term in the field of molecular genetics. In particular, a gene whose function is determined by applying the method of the invention is a nucleic acid molecule which, under suitable conditions, is transcribed and, if the gene encodes a polypeptide, translated. A "coding sequence" is that part of a gene which encodes an amino acid sequence.

The term "function of a gene" means any possible function that a gene may have as long as this function influences the amount of one or more metabolites in the organism under investigation. Typically the function of the gene will be exerted by the gene product, in most cases a polypeptide, that it encodes. The term "function of a gene" embraces the possibility that, for the gene in question, already a function is known, and that, by applying the method of the invention, a novel function will be revealed. Therefore, the term "determining the function of a gene" may in particular mean determining one of the functions of a gene, preferably an additional function of a gene of which one or more functions are already known. If for example, a gene is known to encode a transcription factor, the novel function may be to influence the amount of a nutritionally relevant or essential metabolite. It may be possible that the known function is directly or indirectly involved in the novel function, but that this causality was hitherto not known. In the given example, this would mean that, for instance, the transcription factor is responsible for the synthesis of an enzyme that participates in the biosynthesis of the metabolite.

The term "influences the amount of said metabolite(s) in said organism" means any kind of causal relationship between the activity of a gene or its gene product and the amount of a metabolite in the organism. Such an influence may for example be a more or less direct influence in that the gene encodes an enzyme which participates in the biosynthesis or the metabolization or degradation of the metabolite. On the other hand, the influence may be indirect such as that the gene regulates the activity or the amount of an enzyme which participates in the biosynthesis or the metabolization or degradation of the metabolite.

It is preferred that the gene of which the function is determined by the method of the invention encodes an enzyme, a regulatory protein, a transport protein or a transcription factor.

The term "set of candidate genes" refers to a plurality of genes the amount of transcript of which is determined in step (a). The method of the invention requires that a selection is made of the genes to be analyzed, thereby taking into account a number of factors. These factors include the availability of sequence information of the genes rendering it feasible to obtain specific and significant data on the transcript amount. It is certainly favorable if it is known that the candidate gene is transcribed in the organism of which the samples are taken. Furthermore, experimental restrictions as to the feasibility to produce suitable probes for the respective gene may play a role. Also, the genes to be analyzed in step (a) may be pre-selected by the individual user according to certain predictions on the gene function or requirements concerning for example the intended use of the gene of which a novel function is sought. For instance, in the experiments underlying the present invention, the candidate genes were selected among genes encoding enzymes involved in the primary metabolism and transcription factors. Among these in turn, genes were selected from available tomato EST libraries. In particular, each candidate gene corresponds to one so-called Tentative Consensus (TC) sequence, each being created by assembling ESTs into virtual transcripts. TCs contain full or partial cDNA sequences (ESTs) obtained by classical methods. TCs contain information on the source library and the abundance of ESTs and in many cases represent full-length transcripts. Alternative splice forms are built into separate TCs. To create TCs, CAP3, a DNA sequence assembly program, was used (Huang, X. and Madan, A. (1999) CAP3: A DNA Sequence Assembly Program. Genome Research, 9: 868-877).

The tomato genes used are annotated as described by Van der Hoeven (Plant Cell 14 (2002), 1441-1456). At least two EST clones were selected for microarray construction for each analyzed candidate gene. This example for selecting candidate genes may be adapted by the individual user of the present invention according to his needs. In particular, it may be recommendable to select more than one, if not more than two or even more than three EST clones or corresponding probe molecules being specific for one candidate gene for constructing a microarray or an equivalent device for analyzing the amount of transcripts in step (a) of the method of the invention. Here the methodologies according to the state of the art, such as described in Aharoni (Plant Mol. Biol. 48 (2002), 99-118), may be applied.

In general the number of genes which are analyzed in step (a) should be as big as possible in order to be able to obtain as many as possible correlations between transcripts and metabolites.

In a preferred embodiment, a set of at least 20, preferably of at least 50, more preferably at least 100, and even more preferred of at least 200 genes is used in step (a).

The term "transcript" refers to the RNA that is produced upon transcription of each candidate gene which may be in particular mRNA or also pre-mRNA, i.e. the primary transcript of a gene or a premature processed form thereof. The "amount of transcript" determined in step (a) is the quantity of the transcript in the sample and may for example be expressed in the form amount per fresh or dry weight of the sample. The amount of transcript depends on several factors, however mainly on the transcription rate of the corresponding gene and on the RNA degradation rate.

In step (a), the transcript amount may be determined by applying any suitable technique available to the person skilled in the art. Preferred are techniques that allow the parallel quantification of a plurality of transcripts, especially if data retrieval can be carried out partially or fully automatically. In the field of transcriptome analysis corresponding suitable techniques have been described which mainly focus on the use of DNA chips or microarrays (see for example Aharoni (loc. cit.), Colebatch, 2002 and Thimm, 2001).

In a preferred embodiment, the determination of the amount of transcript is performed as described in the Examples.

In a preferred embodiment of the method of the invention, for determining the amount of transcripts, probes are used that are homologous with respect to the organism of which the samples are taken.

This means that each probe, at least in the region where it is aimed that hybridization takes place, is essentially complementary to the sequence of the transcript of the respective candidate genes. Preferably, the complementary sequence of the probe is identical with the complement of said transcript over the corresponding stretch.

However, the use of homologous probes is not a mandatory requirement. It is also possible to use heterologous probes, i.e. for instance derived from a different species than that of the organism under investigation. In this case, however, one should take care that each probe reliably hybridizes with the respective transcript.

The amount of transcript of the candidate genes and the amount of metabolites is determined from two or more samples from an organism, wherein the samples correspond to different phenotypic and/or genotypic states of said organism.

The term "organism" refers to any living matter that is capable of gene expression. In particular, "living matter" may be one or more cells, a tissue, an organ or a complete organism such as a plant or an animal. The living matter may be in a naturally occurring form or in a man-made form such as in a cultured form, e.g. cell culture, protoplast culture, tissue culture or the like or in the form of a genetically modified organism. In connection with metabolite determination, the term "organism" also includes the direct environment of the living matter, wherein the "direct environment" is characterized by the presence of a metabolite or a gene product produced by said living matter. This gene product may for example influence the metabolite content in the environment of the cell. The direct environment may for example be the extracellular space around a cell, the apoplast, the cell wall, the interstitial space or a culture medium. Furthermore, the metabolite sample may be taken from a certain part of the organism as for example from certain cellular compartments such as plastids, mitochondria, the nucleus, vacuole etc.

The samples analyzed in steps (a) and (b) are taken from different phenotypic and/or genotypic states of said organism. This is explained by the fact that correlations within the transcript and metabolite composition can only be found if the organism is in different states, whereby these states must be connected with differences in the transcript content and in the metabolite content of the organism. It is the idea behind the present invention that a correlation between a transcript and a metabolite may indicate a causal interrelatedness between the two compounds. Therefore, it is envisaged that, in accordance with the correlation observed, the artificial modification of the amount of one compound may lead to a modification of the amount of the other compound. Thereby, the first compound may be either the transcript or the metabolite.

The term "phenotypic state" refers to differences in the phenotype of the organism under investigation. "Phenotype" means any kind of feature that can be detected and which is not a feature of the genome. Such phenotypic states may for example be visually identified such as a morphological or anatomical difference like they can be observed at different developmental stages. Phenotypic states may likewise manifest themselves by the composition of chemical compounds or the occurrence of a disease. Thus, the phenotypic states may be a healthy state in comparison to one or more pathogenic states, different stages of a pathogenicity or an uninfected versus one or more infected organisms. The term "genotypic state" reflects differences in the genome of the organism. Thus, if the samples are taken from different genotypic states of an organism, the term "organism" specifically refers to organisms according to the definition given above which belong to the same taxonomic unit, but which differ in at least one genetic trait. Specifically, the "taxonomic unit" is a genus, preferably a species, and more preferably an even lower taxonomic rank such as a race, variety, cultivar, strain, isolate, population or the like. Most preferably, the taxonomic rank is an isogenic line with variance in only a limited number, preferably three, more preferably two genetic traits and most preferably one genetic trait, whereby "genetic trait" refers to a chromosomal region, a gene locus or, as it is preferred, to a gene. Typically, differences in the genotypic state can be differences between a wild-type organism and one or more corresponding mutant or transgenic organisms or between different mutant or transgenic organisms. A certain genotypic state may be stable or transient as is the case with transduced or transfected cells for instance containing a plasmid, phage or viral vector. Advantageously, organisms of different genotypic state are analyzed when they are in the same developmental stage.

It is immediately clear that the terms "phenotypic" and "genotypic" states may overlap. In particular, normally a genotypic state, if the differing genetic trait(s) is/are expressed in the organism, lead(s) to a difference in the phenotype.

According to the above explanations, in a preferred embodiment of the method of the invention, the different phenotypic and/or genotypic states are different developmental stages, taxonomic units, wild-type and mutant or transgenic organisms, infected and uninfected states, diseased and healthy states or different stages of a pathogenicity.

For each phenotypic and/or genotypic state of the organism, samples are taken in order to determine the amount of the transcripts and the metabolites in these samples.

The term "sample" encompasses any amount of material taken from the organism that is susceptible to the method of the invention. For instance, a sample can be fresh material such as a tissue explant, a body fluid or an aliquot from a bacterial or cell culture, preferably deprived of the culture medium, that may be directly subjected to extraction. On the other hand, samples may also be stored for a certain time period, preferably in a form that prevents degradation of the transcripts and metabolites in the sample. For this purpose, the sample may be frozen, for instance in liquid nitrogen, or lyophilized.

The samples may be prepared according to methods known to the person skilled in the art and as described in the literature. In particular, the preparation should be carried out in a way that the respective compounds to be analyzed are not degraded during the extraction in order to prevent a falsification of the determination in steps (a) and (b). The samples for transcription analysis may for example be prepared according to procedures described in Logemann (1987). The samples for metabolite analysis may for example be prepared according to procedures described in Roessner (2000).

Advantageously, the sample preparation involves the employment of suitable methods in order to remove detection-disturbing compounds from the transcripts (RNA) and/or the metabolites prior to determining the amounts of said transcripts and/or metabolites in the samples. This refers in particular to detection-disturbing compounds which are carbohydrates or other compounds that may disturb identification and quantification of RNA. Routinely, compounds that may disturb the detection of RNA or metabolites are removed by suitable techniques known to the skilled practitioner if such a removal improves the quality and significance of the detection (i.e. the determination of the amounts of said compounds in the sample). For example, it has been shown that the presence of carbohydrates disturbs the detection of RNA by microarrays and that the removal of the carbohydrates from the sample may significantly improve the quality of the detected signals.

In a preferred embodiment of the method of the invention, the amount of transcripts and the amount of metabolites is each determined from the same sample.

This preferred embodiment is based on a technology described in PCT/EP03/00196 and in Fiehn (Eur. J. Biochem. 270 (2003), 579-588). The method described therein provides data useful for quantitatively analyzing metabolites, proteins and/or RNA in a biological source material, whereby said analysis involves suitable statistical evaluation and correlation analysis on the data obtained. In this method, extracting, identifying and quantifying of at least two compound classes of the group consisting of metabolites, proteins and RNA are each determined from one sample. Accordingly, in the preferred embodiment of the method of the present invention, steps (a) and (b) are carried out by applying the corresponding teachings of PCT/EP03/00196. In a particularly preferred embodiment, steps (a) and (b) are performed by (i) extracting the metabolites from the respective sample with at least one solvent or mixture of solvents; and (ii) extracting the RNA from the remainder of the sample after step (i). Thereby, it is a further option that metabolites may additionally be extracted from the yet undissolved remaining cellular material contained in the sample after (ii). Preferably, extraction is carried out by using a mixture of solvents that comprises at least one highly polar solvent, at least one less polar solvent and at least one lipophilic solvent. Thereby, the use of a mixture of solvents comprising water, methanol and chloroform is particularly preferred. More preferably, this mixture of solvents contains water, methanol and chloroform in the approximate proportion by volume of 1: 2.5: 1. Advantageously, the extraction in step (i) is carried out at a temperature between -60°C and +4°C.

The term "metabolite" refers to any substance within an organism of which a sample useful for applying the method of the invention can be taken and for which techniques for determining the amount are available. According to the invention, nucleic acid molecules are not within the meaning of "metabolite". Preferably, the metabolites addressed by the present invention have a low molecular weight, i.e. for instance not more than 4000 Da, preferably not more than 2000 Da, more preferably not more than 1000 Da. Typically, the metabolites to be analyzed may belong to the following, however non-limiting list of compounds: carbohydrates (e.g. sugars, oligo-and polysaccharides such as polyglucans as for example starch or polyfructans), sugar alcohols, amines, polyamines, amino alcohols, aliphatics, aliphatic alcohols, amino acids, lipids, fatty acids, fatty alcohols, organic acids, organic phosphates, organic or anorganic ions, nucleotides, sugar nucleotides, sterols, terpenes, terpenoids, flavons and flavonoids, glucosides, carotenes, carotenoids, cofactors, ascorbate, tocopherol and vitamins.

In a particularly preferred embodiment, the metabolites to be analyzed comprise sugars, sugars alcohols, organic acids, amino acids, ascorbate, tocopherol, fatty acids, vitamins and/or polyamines.

The number and selection of metabolites analysed in step (b) depends on the question for which kind of metabolites a correlation with transcripts is aimed to be determined. Moreover, this depends on the availability of suitable techniques for determining the amount of the respective metabolite, wherein "determining" means identifying and quantifying.

Accordingly, in a preferred embodiment of the invention, the amount of at least 20, more preferably at least 50, still more preferably at least 100, even more preferably at least 150 and most preferably at least 200 or even at least 300 metabolites is determined in step (b).

The determination of the amount of metabolites of interest can be done according to well-known techniques known in the prior art and familiar to the person skilled in the art. Preferably, techniques are applied that allow the identification and quantification in one step and, advantageously, are suited to record the respective metabolites contained in the sample in a comprehensive manner.

For example, the metabolites may be identified and quantified using gas chromatography/mass spectrometry (GC/MS), liquid chromatography/mass spectrometry (LC/MS), NMR or FT-IR or combinations thereof. Further useful methods include LC/UV, refractory index determination, the use of radioactivity in connection with suitable methods known to the skilled person, thin layer chromatography (TLC), capillary electrophoresis (CE), CE/UV, CE/laser induced fluorescence (LIF), fluorescence detection, electrochemical detection (i.e. colorimetry), direct injection MS, flow injection MS, MS/MS, MS/MS/MS, and further combinations of MS steps (MSn), fourier transform ion mass spectrometry (FT/MS), and gel permeation chromatography (GPC). If appropriate, any of the above methods may be combined.

An exemplary non-biased analysis is described in Fiehn (2000). In this study, of different plant mutants, 326 distinct compounds (ranging from primary polar metabolites to sterols) were detected and relatively quantified, including both identified and non-identified compounds by applying a GC/MS analysis. Another example of a GC/MS analysis that can be applied in the method of the invention has been described by Roessner (2001), who used it for comprehensively studying the metabolism in potato tubers. Alternatively, metabolite data can be obtained by extended chromatographic analysis such as described by Tweeddale (1998) where, after growing wild type and mutant E.coli strains in minimal media and 14C-labelled glucose, 70 metabolites could be separated using two dimensional thin layer chromatography. The relative quantification of metabolites was carried out by radioactive detection.

In step (c), the data obtained in steps (a) and (b) are analyzed by applying suitable mathematical methods in order to identify a transcript and at least one metabolite the amounts of which significantly correlate in the different phenotypic and/or genotypic states.

The term "analyzed by applying suitable mathematical methods" refers to any mathematical analysis method that is suited to further process the quantitative data obtained in steps (a) and (b) in a way that significant correlations between transcripts and metabolites can be ascertained. These data represent the amount of the analyzed compounds present in each sample either in absolute terms (e.g. weight or moles per weight sample) or in relative terms (i.e. normalized to a certain reference quantity). Usually normalized data is used for performing correlation analyses.

Normalization may involve the representation of the amount of an examined compound at each state by setting the figure in relation to one reference value determined at one specific state. Normalization may furthermore involve the correction of background levels and the combination of the transcript and the metabolite data sets into a single data sheet. Corresponding mathematical methods and computer programs are known to the skilled person. Examples include SAS, SPSS, systatR, R and Matlab. As the next step, the statistically pre-processed data may be subjected to a pairwise correlation analysis. Here series of pairs of data points from the analyzed compounds are looked at for correlation, whether positive or negative, for instance by using the Pearson's correlation coefficient.

In a preferred embodiment, the mathematical analysis of the method of the invention furthermore involves network analysis. Network analysis aims at finding out higher order interplays of multiple factors on the basis of pairwise correlation data. By taking several data sets each obtained from one sample, correlations between metabolites and transcripts as well as among these classes of compounds can be analysed in order to derive information about the network regulation of biological systems, e.g. upon genetic or environmental perturbation. The analysis of pairwise correlations in particular between metabolites and transcripts allows to establish links between regulatory and metabolic networks and the computation of general properties such as connectivity for both types of networks.

A comprehensive overview of methods for quantitatively analyzing data obtained according to the method of the invention including principle component analysis, "snapshot analysis", Pearson correlation analysis, mutual information and network analyses can be found in Fiehn (2001).

According to the present invention, a significant correlation between the amount of a transcript and the amount of a metabolite in the different states, whereby the candidate genes may be pre-selected or non-selected, can be determined by a non-parametric Spearman's rank order correlation analyzis. Rank order correlation appears to be preferable to other methods because transcript and metabolite levels may be correlated in a non-linear manner. In particular, in a first step, for all possible transcript and metabolite pairs Spearman correlation coefficients may be calculated. Subsequently, the correlation coefficients may be compared with a Spearman Rank significance table for specific parameters (P = 0.01). This approach has also been taken in the experiments described in Example 2. The value $r_s$ is the result of the Spearman correlation coefficient calculation. The formula for the Rank (Spearman) Correlation Coefficient is

$$r_s = 1 - \frac{6\sum d^2}{n(n^2-1)}$$

In a preferred embodiment, step (c) of the method of the invention comprises the steps

(i) determining transcripts the amount of which differs significantly between the samples of (a); and
(ii) determining metabolites the amount of which differs significantly between the samples of (b);

wherein the data obtained for the transcripts and the metabolites determined in steps (i) and (ii), respectively, are analyzed by suitable mathematical methods in order to identify said transcript and metabolite(s) the amounts of which significantly correlate in the different states.

In this embodiment, first those transcripts and metabolites are determined the amount of which shows significant differences between the analyzed states of the organism. Corresponding statistical analysis methods and applicable software are known to the person skilled in the art and described in the literature. This may for example be done as it is described in Example 1. On the so-pretreated data, the analysis for finding pairwise correlations between transcripts and metabolites may be performed as described above. Preferably, only the transcripts and metabolites showing significant differences as determined in steps (i) and (ii), respectively, may be used for the correlation analysis. This may have the advantage of saving computational and other capacities.

It is the result of the method of the invention to identify a transcript in step (c) that significantly correlates with at least one metabolite in the different states. This finding gives an indication that the gene corresponding to this transcript has a function that influences the amount of said metabolite(s) in said organism. The term "gene corresponding to a transcript" refers to the gene from which the transcript is transcribed. The gene can be identified according to conventional techniques common to the molecular biologist. The term "function that influences the amount of a metabolite" has already been defined further above.

In a further aspect, the present invention relates to a method for identifying a gene which is capable of modifying the amount of a metabolite in an organism comprising steps (a) to (c) of the aforementioned method for determining the function of a gene, wherein said transcript identified in step (c) corresponds to a gene being capable of modifying the amount of said metabolite(s) identified in step (c).

The term "gene capable of modifying the amount of a metabolite in an organism" refers to a gene having the function to influence the amount of a metabolite in the organism as it can be determined by the corresponding method described above. As a consequence, it is contemplated that an exogenously induced alteration of the expression of this gene as compared to the normal wild-type gene expression in the organism under the same developmental and environmental conditions will lead to a significant modification of the amount of said metabolite in the organism as compared to the amount of the metabolite in the corresponding organism with the expression of said gene not being altered.

Thus, when a correlation between a transcript and a metabolite has been revealed by the method of the invention, a gene corresponding to this transcript can be made the target of a specific alteration of its gene expression if it is intended to deliberately modify the amount of the metabolite. In this context, the term "altered gene expression" refers to any measures that lead to an altered amount of the transcript of said gene in the organism. Primarily encompassed are measures that modify the transcription rate or the stability of the transcript RNA. However, if the gene encodes a polypeptide, measures are also encompassed that modify the translation rate, the activity of the encoded gene product or post-translational modifications of the polypeptide resulting in a modified activity of the gene product. Depending on whether the correlation is positive or negative, an increased gene expression may lead to an increase or a decrease of the amount of the metabolite in the organism. Accordingly, a decreased gene expression may lead to a decrease or an increase of the metabolite in the organism. This effect can be employed, for example, in order to produce useful plants having an increased content in a nutritionally valuable metabolite such as a vitamin or having a reduced content in a undesirable metabolite such as a compound being responsible for allergic reactions. Likewise, the effect can be used in gene therapeutical approaches in order to increase or decrease a certain metabolite in a specific tissue or organ. Often it will be necessary that the modification of the particular metabolite is not accompanied by the modification of other metabolites which might evoke undesirable side effects. In such a case, a gene can be selected for modification which does not show significant correlations with other metabolites.

Various methods for exogenously inducing an alteration of the expression of a specific gene are known and described in the literature that can be applied when a gene has been identified that is capable of modifying the amount of a metabolite in an organism.

An increase of gene expression may for example be achieved by over-expressing the corresponding gene product from a gene construct introduced into said organism by applying conventional methods such as those described in Sambrook (2001) and Gassen (1999). However, the state of the art provides further methods for achieving an increased gene expression. For example, the corresponding endogenous gene may be modified at its natural location, e.g. by homologous recombination, for example by positively affecting the promoter activity. Applicable homologous recombination techniques (also known as "in vivo mutagenesis") are known to the person skilled in the art and are described in the literature. One such technique involves the use of a hybrid RNA-DNA oligonucleotide ("chimeroplast") which is introduced into cells by transformation (TIBTECH 15 (1997), 441-447; WO95/15972; Kren, Hepatology 25 (1997), 1462-1468; Cole-Strauss, Science 273 (1996), 1386-1389).

A reduction of gene expression may be achieved by different techniques described in the prior art. These include but are not limited to antisense, ribozyme, co-suppression, RNA interference, expression of dominant negative mutants, antibody expression and in vitro mutagenesis approaches. All of them include the introduction of a suitable nucleic acid molecule into a cell. Such a foreign nucleic acid molecule is present in cells of a correspondingly treated organism, but absent from the cells of the corresponding source organism. Thereby encompassed are nucleic acid molecules, e.g. gene sequences, which differ from the corresponding nucleic acid molecule in the source organism by at least one mutation (substitution, insertion, deletion, etc. of at least one nucleotide), wherein such a mutation inhibits the expression of the affected gene or reduces the activity of the gene product. Furthermore encompassed by the term "foreign" are nucleic acid molecules which are homologous with respect to the source organism but are situated in a different chromosomal location or differ, e.g., by way of a reversed orientation for instance with respect to the promoter. In principle, the nucleic acid molecule to be introduced in accordance with the present embodiment may be of any conceivable origin, e.g. eukaryotic or prokaryotic. It may be from any organism which comprises such molecules. Furthermore, it may be synthetic or derived from naturally occurring molecules by, e.g., modification of its sequence, i.e. it may be a variant or derivative of a naturally occurring molecule. Such variants and derivatives include but are not limited to molecules derived from naturally occurring molecules by addition, deletion, mutation of one or more nucleotides or by recombination. It is, e.g., possible to change the sequence of a naturally occurring molecule so as to match the preferred codon usage of the target organism. It is preferred that the nucleic acid molecule introduced into the organism has to be expressed in order to exert its reducing effect upon gene expression of the target gene. The term "expressed" means that such a nucleic acid molecule is at least transcribed and, for some embodiments, also translated into a protein. Preferred examples of such nucleic acid molecules relate to those embodiments wherein a reduced gene expression is achieved by an antisense, co-suppression, ribozyme or RNA interference effect or by the expression of antibodies or other suitable polypeptides capable of specifically reducing the activity of the encoded gene product or by the expression of a dominant-negative mutant. These methods are further explained in the following.

In particular, gene expression may be reduced by using nucleic acid molecules encoding an antisense RNA or directly by using antisense RNA, said antisense RNA being complementary to transcripts of the gene the expression of which is to be reduced. Thereby, complementarity does not signify that the RNA has to be 100% complementary. A low degree of complementarity may be sufficient as long as it is high enough to inhibit the gene expression. The transcribed RNA is preferably at least 90% and most preferably at least 95% complementary to the transcript of the gene. In order to cause an antisense effect during the transcription the antisense RNA molecules have a length of at least 15 bp, preferably a length of more than 100 bp and most preferably a length or more than 500 bp, however, usually less than 2000 bp, preferably shorter than 1500 bp. For example, for plants, exemplary methods for achieving an antisense effect are described by Müller-Röber (EMBO J. 11 (1992), 1229-1238), Landschütze (EMBO J. 14 (1995), 660-666), D'Aoust (Plant Cell 11 (1999), 2407-2418) and Keller (Plant J. 19 (1999), 131-141). Likewise, an antisense effect may also be achieved by applying a triple-helix approach, whereby a nucleic acid molecule complementary to a region of the respective gene is designed according to the principles for instance laid down in Lee (Nucl. Acids Res. 6 (1979), 3073); Cooney (Science 241 (1998), 456) or Dervan (Science 251 (1991), 1360).

A similar effect as with antisense techniques can be achieved by applying RNA interference (RNAi). Thereby, the formation of double-stranded RNA leads to an inhibition of gene expression in a sequence-specific fashion. More specifically, in RNAi constructs, a sense portion comprising the coding region of the gene to be inactivated (or a part thereof, with or without non-translated region) is followed by a corresponding antisense sequence portion. Between both portions, an intron not necessarily originating from the same gene may be inserted. After transcription, RNAi constructs form typical hairpin structures. The RNAi technique may be carried out as described by Smith (Nature 407 (2000), 319-320), Marx (Science 288 (2000), 1370-1372) or Elbashir (Nature 411 (2001), 428-429).

Furthermore, DNA molecules can also be employed which, during their expression lead to the synthesis of an RNA which reduces the expression of the gene to be inactivated due to a co-suppression effect. The principle of co-suppression as well as the production of corresponding DNA sequences is precisely described, for example, in WO 90/12084. Such DNA molecules preferably encode an RNA having a high degree of homology to transcripts of the target gene. It is, however, not absolutely necessary that the coding RNA is translatable into a protein. The principle of the co-suppression effect is known to the person skilled in the art and is, for example, described in Jorgensen, Trends Biotechnol. 8 (1990), 340-344; Niebel, Curr. Top. Microbiol. Immunol. 197 (1995), 91-103; Flavell, Curr. Top. Microbiol. Immunol. 197 (1995), 43-36; Palaqui and Vaucheret, Plant. Mol. Biol. 29 (1995), 149-159; Vaucheret, Mol. Gen.

Genet. 248 (1995), 311-317; de Borne, Mol. Gen. Genet. 243 (1994), 613-621 and in other sources. Likewise, ribozymes which specifically cleave transcripts of the gene to be inactivated can be used. Ribozymes are catalytically active RNA molecules capable of cleaving RNA molecules at a specific target sequence. There are various classes of ribozymes. For practical applications aiming at the specific cleavage of the transcript of a certain gene, use is preferably made of the group I intron ribozyme type or of ribozymes exhibiting the so-called "hammerhead" motif as a characteristic feature. By means of recombinant DNA techniques, the specific recognition of the target RNA molecule may be modified by altering the sequences flanking the hammerhead motif. By base pairing with sequences in the target molecule, the flanking sequences determine the position at which cleavage of the target molecule takes place. Since the sequence requirements for an efficient cleavage are low, it is in principle possible to develop specific ribozymes for practically each desired RNA molecule. In order to produce nucleic acid molecules encoding a ribozyme which specifically cleaves the transcript of the gene to be inactivated, for example, a DNA sequence encoding a catalytic domain of a ribozyme is bilaterally linked with DNA sequences which are complementary to sequences of the transcript. Sequences encoding the catalytic domain may for example be the catalytic domain of the satellite DNA of the SCMo virus (Davies, Virology 177 (1990), 216-224 and Steinecke, EMBO J. 11 (1992), 1525-1530) or that of the satellite DNA of the TobR virus (Haseloff and Gerlach, Nature 334 (1988), 585-591). The expression of ribozymes in order to decrease the activity of certain proteins in cells is known to the person skilled in the art and is, for example, described in EP-B1 0 321 201. The expression of ribozymes in plant cells is for example described in Feyter (Mol. Gen. Genet. 250 (1996), 329-338).

Furthermore, nucleic acid molecules encoding antibodies specifically recognizing the polypeptide encoded by the gene to be inactivated or specific fragments or epitopes of such a polypeptide can be used for inhibiting the gene expression of said gene. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in Köhler and Milstein (Nature 256 (1975), 495) and Galfré (Meth. Enzymol. 73 (1981) 3), which comprise the fusion of mouse myeloma cells to spleen cells derived from immunized mammals. Furthermore, antibodies or fragments thereof to the aforementioned polypeptide can be obtained by using methods which are described, e.g., in Harlow and Lane "Antibodies, A Laboratory Manual", CSH Press, Cold Spring Harbor, 1988. In plants, expression of antibodies or antibody-like molecules can be achieved by methods well known in the art. These include the expression of, for example, full-size antibodies (Düring, Plant. Mol. Biol. 15 (1990), 281-293; Hiatt, Nature 342 (1989), 469-470; Voss, Mol. Breeding 1 (1995), 39-50), Fab-fragments (De Neve, Transgenic Res. 2 (1993), 227-237), scFvs (Owen, Bio/Technology 10 (1992), 790-794; Zimmermann, Mol. Breeding 4 (1998), 369-379; Tavladoraki, Nature 366 (1993), 469-472; Artsaenko, Plant J. 8 (1995), 745-750) and variable heavy chain domains (Benvenuto, Plant Mol. Biol. 17 (1991), 865-874) have been successfully expressed in tobacco, potato (Schouten, FEBS Lett. 415 (1997), 235-241) or *Arabidopsis,* reaching expression levels as high as 6.8% of the total protein (Fiedler, Immunotechnology 3 (1997), 205-216).

Moreover, also nucleic acid molecules encoding peptides or polypeptides capable of reducing the activity of the polypeptide encoded by the gene to be inactivated other than antibodies can be used in the present context. Examples of suitable peptides or polypeptides that can be constructed in order to achieve the intended purpose can be taken from the prior art and include, for instance, binding proteins such as lectins.

In addition, nucleic acid molecules encoding a mutant form of the polypeptide encoded by the gene to be inactivated can be used to interfere with the activity of the wild-type protein. Such a mutant form preferably has lost its biological activity and may be derived from the corresponding wild-type protein by way of amino acid deletion(s), substitution(s), and/or additions in the amino acid sequence of the protein. Mutant forms of such proteins may show, in addition to the loss of the hydrolytic activity, an increased substrate affinity and/or an elevated stability in the cell, for instance, due to the incorporation of amino acids that stabilize proteins in the cellular environment. These mutant forms may be naturally occurring or, as is preferred, genetically engineered mutants.

It is also possible that the nucleic acid molecule, the presence of which in the genome of an organism leads to a reduction of gene expression, does not require its expression to exert its reducing effect on gene expression. Correspondingly, preferred examples relate to methods wherein this effect is achieved by in vivo mutagenesis or by the insertion of a heterologous DNA sequence in the target gene.

The term "in vivo mutagenesis", relates to methods where the sequence of the gene to be inactivated is modified at its natural chromosomal location such as for instance by techniques applying homologous recombination. This may be achieved by using a hybrid RNA-DNA oligonucleotide ("chimeroplast"), as described above.

The term "insertion of a heterologous DNA sequence" refers to DNA sequences which can be inserted into the target gene via appropriate techniques other than in vivo mutagenesis. The insertion of such a heterologous DNA sequence may be accompanied by other mutations in the target gene such as the deletion, inversion or rearrangement of the sequence located at the insertion site. In connection with preparing transgenic plants, this embodiment includes randomly introducing a heterologous DNA sequence into the respective plant genome, thereby generating a pool, i.e. a plurality, of transgenic plants having a genome into which the heterologous DNA sequence is randomly spread over various chromosomal locations. This generation of transgenic plants is followed by selecting those transgenic plants out of the pool which show the desired genotype, i.e. an inactivating insertion in the target gene and/or the desired phenotype, i.e. a reduced activity of the polypeptide encoded by the target gene and/or a modified amount of the metabolite which correlates with the transcript of said gene.

Suitable heterologous DNA sequences that can be taken for such an approach are described in the literature and include for instance vector sequences capable of self-integration into the host genome or mobile genetic elements. Particularly preferred in this regard are T-DNA or transposons which are well-known to the person skilled in the art from so-called tagging experiments used for randomly knocking out genes in plants. The production of such pools of transgenic plants can for example be carried out as described in Jeon (Plant J. 22 (2000), 561-570) or Parinov (Curr. Op. Biotechnol. 11 (2000), 157-161).

Another example of insertional mutations that may result in gene silencing includes the duplication of promoter sequences which may lead to a methylation and thereby an inactivation of the promoter (Morel, Current Biology 10 (2000), 1591-1594).

Furthermore, it is immediately evident to the person skilled in the art that the above-described approaches, such as antisense, ribozyme, co-suppression, in-vivo mutagenesis, RNAi, expression of antibodies, other suitable peptides or polypeptides or dominant-negative mutants and the insertion of heterologous DNA sequences, can also be used for reducing the expression of a gene that encodes a regulatory protein such as a transcription factor that controls the expression of the gene to be inactivated. It is also evident from the above descriptions any of the above-mentioned approaches can be combined in order an effective reduction of gene expression of the target gene.

In yet another aspect, the present invention refers to a method for identifying a metabolite which is capable of modifying the amount of a transcript in an organism comprising steps (a) to (c) of the aforementioned method for determining the function of a gene, wherein a metabolite identified in step (c) is a candidate for a metabolite being capable of modifying the amount of said transcript identified in step (c).

The term "metabolite capable of modifying the amount of a transcript in an organism" refers to a metabolite the amount of which significantly correlates with said transcript in the different states of the organism as it can be determined by performing steps (a) to (c) of the above-described method for determining the function of a gene. It is thus contemplated that an exogenously induced alteration of the amount of the metabolite as compared to the normal amount of the metabolite in the organism under the same developmental and environmental conditions may lead to a significant modification of the amount of the transcript in the organism as compared to the amount of the transcript in the corresponding organism with the amount of said metabolite not being altered. Thus, when a correlation between a metabolite and a transcript has been revealed by the method of the invention, a metabolite or a thereto structurally related compound can be regarded as a candidate for a compound that may modify the expression of the gene corresponding to this transcript.

The term "candidate" reflects that the metabolite identified in step (c) may not necessarily be causative for the observed differences of the correlated transcript in the different states of the organism under investigation. Thus, it may be required that the identified metabolite be further tested for the property of being capable of modifying the amount of the transcript in an organism. For this purpose, gene expression assays may be conducted according to methods known in the prior art as for instance described in Sambrook (2001) and Gassen (1999). For example, the amount of the respective transcript may be detected for the organism to which a certain amount of the candidate metabolite is added in comparison with a corresponding organism at the same conditions to which no metabolite is added. As already mentioned above, such a candidate may also be a compound which is structurally related to the specific metabolite and which has a similar effect on the transcript level as the metabolite.

It is envisaged that the method of the present embodiment may for example provide novel starting points for developing therapeutically useful agents that may be used for ameliorating an aberrant over- or under-expression of a gene in a patient who suffers from a genetic disease.

Generally, most of the metabolites that occur in nature may be synthesized chemically or by microorganisms or by a combination of these two possibilities and often are commercially available. Correspondingly, modifications to a metabolite compound may be introduced according to methods of organic chemistry and biochemistry known in the art. Compounds being structurally related with a metabolite thus produced may be tested for their activity of modifying the amount of a transcript with which said metabolite correlates according gene expression assays known in the art, as mentioned above.

In this context, an "alteration of the amount of a metabolite" may on the one hand mean an increase of this amount in the organism. This may be achieved by the addition of this metabolite or of a structurally related compound having a similar effect on the transcript level to be modified to the organism. Alternatively, other methods for increasing the amount of a certain metabolite in an organism known in the prior art may be used as well such as measures that indirectly lead to an increase of the metabolite in the organism, e.g. by influencing the biosynthesis or degradation of the metabolite. On the other hand, the "alteration of the amount of a metabolite" may be a reduction of this amount in the organism.

For administering a metabolite or a structurally related compound having a corresponding activity, said metabolite or structurally related compound may be formulated in a composition.

If for example administration is meant for plants the composition may be composed in the form of a plant protection composition, wherein the metabolite or the structurally related compound may be formulated by conventional means commonly used for the application of, for example, herbicides or pesticides. For example, certain additives known to those skilled in the art such as stabilizers or substances which facilitate the uptake by the plant cell, plant tissue or plant may be used as for example harpins, elicitins, salicylic acid (SA), benzol(1,2,3)thiadiazole-7-carbothioic acid (BTH), 2,6-dichloro isonicotinic acid (INA), jasmonic acid (JA) or methyljasmonate.

If for example administration is meant for mammals or corresponding mammalian cells or tissues, the composition may be composed in the form of a pharmaceutical composition and may further comprise a pharmaceutically acceptable carrier and/or diluent. The composition may furthermore contain substances that stabilize or facilitate the uptake of the metabolite or compound by the cells. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods.

These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the subject's size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment.

Compositions may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Often it will be necessary that the modification of the particular transcript level induced by the correlated metabolite is not accompanied by the modification of the amount of other transcripts which might evoke undesired side effects. In such a case, a metabolite can be selected which does not show significant correlations with other transcripts.

Furthermore, the present invention relates to the use of a gene the function of which has been determined by the above-described method for determining the function of a gene, of a nucleic acid molecule comprising the coding sequence of said gene or a fragment or derivative of said coding sequence showing said function or of a polypeptide encoded by said gene or nucleic acid molecule for applying said function. With the method of the invention, it is possible to elucidate gene functions that have not been thought of before. Therefore, the present invention also refers to the use of the genes for applying the function that has been identified by the method of the invention.

In the experiments underlying this invention, several new correlations between transcript level and metabolite level have been revealed. Accordingly, the present invention refers in particularly preferred embodiments to the uses of the genes corresponding to these transcripts for applying the newly revealed functions each of which involves influencing the amount of the respective metabolite, preferably in a plant, especially in potato, more specifically in potato tuber.

In particular, these uses are evident for the person skilled in the art from the following transcript-metabolite correlations (see also Example 2):

(1) a positive correlation of tryptophan with the beta-2chain of tryptophan synthase (Fig. 2c);
(2) a positive correlation of tyrosine with the beta-2 chain of tryptophan synthase (Fig. 2d);
(3) a positive correlation of serine with ornithine carbamoyltransferase (Fig. 2e);
(4) a positive correlation of cysteine with ornithine carbamoyltransferase (Fig. 2f);
(5) a positive correlation of fructose-6-phosphate with aminotransferase (Fig. 2g);
(6) a positive correlation of glucose-6-phosphate with aminotransferase which correlates with both fructose-6-phosphate and glucose-6-phosphate (Fig. 2h);
(7) a positive correlation of spermidine with glutamate decarboxylase isoform I (Fig. 2i);
(8) a positive correlation of tyrosine with glutamate decarboxylase isoform I (Fig. 2j);
(9) a negative correlation of ascorbate with a homologue of the clock gene CONSTANS (Fig. 2k);
(10) a negative correlation of tocopherol with succinyl CoA synthetase (Fig. 2l);
(11) a positive correlation of lysine with the transcription factor WRKY6 (Fig. 2m);
(12) a positive correlation of lysine with S-adenosyl-L-methionine synthetase (Fig. 2n);
(13) a positive correlation of lysine with ornithine carbamoyltransferase (Fig. 2o);
(14) a negative correlation of lysine with caffeoyl-CoA O-methyltransferase (Fig. 2p);
(15) a negative correlation of sucrose with osmotic stress-induced zinc-finger protein;
(16) a positive correlation of 4-aminobutyric acid with osmotic stress-induced zinc-finger protein; and
(17) a positive correlation of tryptophan with osmotic stress-induced zinc-finger protein.

Since most molecular biological applications do not use a gene sequence (in particular if it contains one or more introns), but the coding sequence or fragments or derivatives thereof, the uses of the present inventions are not limited to the genes identified according to the methods of the invention, but also refer to nucleic acid molecules comprising the coding sequence of said gene or a fragment or derivative of said coding sequence showing said function.

The terms "nucleic acid molecule" and "coding sequence of a gene" refer to the conventional meaning of these terms as they are familiar to the skilled person. The term "nucleic acid molecule" furthermore encompasses molecules which, in addition to said coding sequence, comprise additional sequences useful for the intended uses. Such additional nucleotide sequences may in particular be additional coding sequences fused to the aforementioned coding sequence, said additional coding sequence for example encoding a tag facilitating easy purification of the encoded fusion protein, a stabilizing moiety inhibiting degradation of the fusion protein or targeting signal sequences. Furthermore, such additional nucleotide sequences may be expression control sequences operably linked to the coding sequence or vector sequences allowing the propagation of the nucleic acid molecule in a suitable host. Corresponding construction manuals for nucleic acid molecules are known to the skilled person and described in the literature (see e.g. Sambrook (2001) and Gassen (1999)).

The term "fragment of said coding sequence showing said function" refers to fragments of the coding sequence of the gene that has the function of influencing the amount of a metabolite to which the transcript of said gene shows a correlation, whereby the fragment when expressed in a corresponding organism shows a similar, preferably the same effect on the amount of the metabolite as the gene or its entire coding sequence. This effect can be determined by well-known methods known in the art, for example involving the expression of the fragment in the organism or a biological test system derived from this organism (e.g. a cell culture or the like) and measuring the amount of the metabolite therein. This measurement may be compared with one obtained from a corresponding experiment undertaken with the gene or the entire coding sequence, and preferably with a measurement obtained from a control setting, wherein no heterologous nucleic acid molecule is expressed. If the amount of the metabolite upon fragment expression does not significantly deviate from that measured upon expression of the gene or the entire coding sequence, the fragment shows a similar or even the same function as the gene or the entire coding sequence.

The term "derivative of said coding sequence showing said function" refers to nucleotide sequences the complementary strand of which hybridizes with the coding sequence and wherein the derivative when expressed in a corresponding organism shows a similar, preferably the same effect on the amount of the metabolite with which the transcript of the coding sequence correlates as the gene or the coding sequence. This effect can be determined as described above in connection with fragments of the coding sequence.

If the coding sequence encodes a polypeptide, such a derivative may encode a polypeptide which has a homology, that is to say a sequence identity, of at least 30%, preferably of at least 40%, more preferably of at least 50%, even more preferably of at least 60% and particularly preferred of at least 70%, especially preferred of at least 80% and even more preferred of at least 90% to the entire amino acid sequence encoded by the coding sequence.

Moreover, such a derivative may have a homology, that is to say a sequence identity, of at least 40%, preferably of at least 50%, more preferably of at least 60%, even more preferably of more than 65%, in particular of at least 70%, especially preferred of at least 80%, in particular of at least 90% and even more preferred of at least 95% when compared to the coding sequence.

The use of the present embodiment also relates to derivatives the sequence of which deviates from above-described hybridizing or homologous nucleotide sequences due to the degeneracy of the genetic code and which have a similar, preferably the same function as the coding sequence.

In the context of the present invention the term "hybridization" means hybridization under conventional hybridization conditions, preferably under stringent conditions, as for instance described in Sambrook and Russell (2001), Molecular Cloning: A Laboratory Manual, CSH Press, Cold Spring Harbor, NY, USA. In an especially preferred embodiment, the term "hybridization" means that hybridization occurs under the following conditions:

| | |
|---|---|
| Hybridization buffer: | 2 x SSC; 10 x Denhardt solution (Fikoll 400 + PEG + BSA; ratio 1:1:1); 0.1% SDS; 5 mM EDTA; 50 mM Na2HPO4; 250 µg/ml of herring sperm DNA; 50 µg/ml of tRNA; or 0.25 M of sodium phosphate buffer, pH 7.2; 1 mM EDTA 7% SDS |
| Hybridization temperature T | = 60°C |
| Washing buffer: | 2 x SSC; 0.1% SDS |
| Washing temperature T | = 60°C. |

Derivatives which hybridize with the coding sequence of a gene the function of which is identified by applying the method of the invention can for instance be isolated from genomic libraries or cDNA libraries of bacteria, fungi, plants or animals. According to one aspect of the invention, it is preferred that such polynucleotides are from plant origin, particularly preferred from a plant belonging to the dicotyledons, more preferably from the family of Solanaceae. Preferably, the derivative is a variant, preferably an ortholog of said coding sequence. Alternatively, such derivatives can be prepared by genetic engineering or chemical synthesis.

Such hybridizing polynucleotides may be identified and isolated by using a nucleic acid molecule comprising the coding sequence or parts or reverse complements thereof, for instance by hybridization according to standard methods (see for instance Sambrook (2001). Fragments used as hybridization probes can also be synthetic fragments which are prepared by usual synthesis techniques, and the sequence of which is substantially identical with the coding sequence of the gene, a function of which has been determined by the method of the invention.

The molecules hybridizing with said coding sequence comprise fragments, derivatives and allelic variants of the specific coding sequence corresponding to the gene a function of which has been determined by applying the method of the invention.

Preferably, the degree of homology is determined by comparing the respective nucleotide sequence with the coding sequence of the gene a function of which has been identified by applying the method of the invention. When the sequences which are compared do not have the same length, the degree of homology preferably refers to the percentage of nucleotide residues in the shorter sequence which are identical to nucleotide residues in the longer sequence. The degree of homology can be determined conventionally using known computer programs such as the DNAstar program with the ClustalW analysis. This program can be obtained from DNASTAR, Inc., 1228 South Park Street, Madison, WI 53715 or from DNASTAR, Ltd., Abacus House, West Ealing, London W13 OAS UK (support@dnastar.com) and is accessible at the server of the EMBL outstation.

When using the Clustal analysis method to determine whether a particular sequence is, for instance, 80% identical to a reference sequence the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.

Preferably, the degree of homology of the hybridizing polynucleotide is calculated over the complete length of its coding sequence. It is furthermore preferred that such a hybridizing polynucleotide, and in particular the coding sequence comprised therein, has a length of at least 300 nucleotides, preferably at least 500 nucleotides, more preferably of at least 750 nucleotides, even more preferably of at least 1000 nucleotides, particularly preferred of at least 1500 nucleotides and most preferably of at least 2000 nucleotides.

Preferably, sequences hybridizing to the coding sequence of the gene a function of which has been identified by applying the method of the invention comprise a region of homology of at least 90%, preferably of at least 93%, more preferably of at least 95%, still more preferably of at least 98% and particularly preferred of at least 99% identity to an above-described polynucleotide, wherein this region of homology has a length of at least 500 nucleotides, more preferably of at least 750 nucleotides, even more preferably of at least 1000 nucleotides, particularly preferred of at least 1500 nucleotides and most preferably of at least 2000 nucleotides.

Homology, moreover, means that there is a functional and/or structural equivalence between the corresponding polynucleotides or polypeptides encoded thereby. Polynucleotides which are homologous to the above-described molecules and represent derivatives of these molecules are normally variations of these molecules which represent modifications having the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques. Deviations from the above-described polynucleotides may have been produced, e.g., by deletion, substitution, insertion and/or recombination.

The polypeptides encoded by the different variants of the coding sequence of the gene, a function of which has been determined by applying the method of the invention, possess certain characteristics they have in common. These include for instance biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.

In connection with the present use of the invention, the term "polypeptide" refers to any polypeptide encoded by the above-mentioned gene, nucleic acid molecule, coding sequence, fragment or derivative. This polypeptide may, e.g., be a naturally purified product or a product of chemical synthetic procedures or produced by recombinant techniques from a prokaryotic or eukaryotic host (for example, by bacterial, yeast, higher plant, insect and mammalian cells in culture). Depending upon the host employed in a recombinant production procedure, the polypeptide may be glycosylated or non-glycosylated. The polypeptide may also include an initial methionine amino acid residue. It may be further modified to contain additional chemical moieties not normally part of the polypeptide. Those derivatized moieties may, e.g., improve the stability, solubility, the biological half life or absorption of the polypeptide. The moieties may also reduce or eliminate any undesirable side effects of the polypeptide and the like. An overview for these moieties can be found, e.g., in Remington's Pharmaceutical Sciences (18th ed., Mack Publishing Co., Easton, PA (1990)). Polyethylene glycol (PEG) is an example for such a chemical moiety which has been used for the preparation of therapeutic polypeptides. The attachment of PEG to polypeptides has been shown to protect them against proteolysis (Sada et al., J. Fermentation Bioengineering 71 (1991), 137-139). Various methods are available for the attachment of certain PEG moieties to polypeptides (for review see:

Abuchowski et al., in "Enzymes as Drugs"; Holcerberg and Roberts, eds. (1981), 367-383). Generally, PEG molecules are connected to the polypeptide via a reactive group found on the polypeptide. Amino groups, e.g. on lysines or the amino terminus of the polypeptide are convenient for this attachment among others.

In addition, the present invention relates in a further embodiment to the use of a gene identified by the above-described method for identifying a gene which is capable of modifying the amount of a metabolite in an organism, of a nucleic acid molecule comprising the coding sequence of said gene or a fragment or derivative of said coding sequence which is capable of modifying the amount of a metabolite in an organism or of a polypeptide encoded by said gene or nucleic acid molecule for modifying the amount of a metabolite in an organism.

This use is in principle a preferred embodiment of the aforementioned use of a gene, the function of which has been determined by the method for determining the function of a gene, in that the "function" can be seen as the capacity of modifying the amount of a metabolite in an organism. Accordingly, the definitions for "coding sequence", "nucleic acid molecule", "fragment", "derivative" and "polypeptide" given above likewise apply to the present embodiment.

Accordingly, also the preferred embodiments enumerated under (1) to (14), above, as to the uses of the genes corresponding to transcripts for applying the newly revealed functions also apply to the modification of the amount of the respective metabolite, preferably in a plant, especially in potato, more specifically in potato tuber.

Moreover, the present invention relates in a further embodiment to the use of a metabolite identified by the above-described method for identifying a metabolite which is capable of modifying the amount of a transcript in an organism for modifying the amount of a transcript in an organism.

This use is in principle a preferred embodiment of the above-outlined use of a gene, the function of which has been determined by the method for determining the function of a gene, in that the "function" can be seen as the capacity that the amount of the transcript of the gene can be modified by the metabolite.

Accordingly, the correlations enumerated under (1) to (14), above, give rise to preferred embodiments of the present use. In particular, it is conceivable that each of the metabolites mentioned there can be used for modifying the amount of the respective transcript for which the metabolite shows a correlation. These uses will preferably be applicable to plants, especially to potato, more specifically to potato tuber.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-364.

Furthermore, the term "and/or" when occurring herein includes the meaning of "and", "or" and "all or any other combination of the elements connected by said term".

The present invention is further described by reference to the following non-limiting figures, tables and examples.

The Figures and the Tables show:

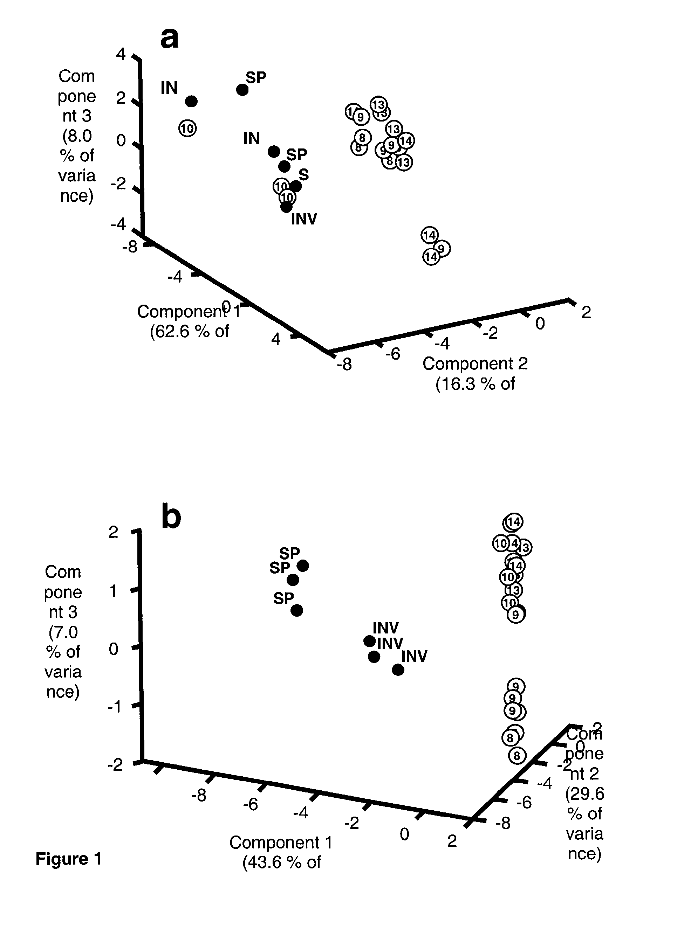

**Figure 1**    is a visualization of the 1$^{st}$ to 3$^{rd}$ components from a principle component analysis (PCA) of gene expression profiles **(a)** and of metabolite profiles **(b)**. The percentage of variance explained by each component is shown in parenthesis. The transgenic lines INV2-30 (INV) and SP 29 (SP), filled circles, and potato tuber after 8, 9, 10, 13 and 14 weeks growth, open circles.

**Figure 2**    depicts correlations between metabolite and transcript levels of the analyzed systems. The correlation between the level of chemically defined metabolites and the level of each selected clone was assessed. All correlations with significant value P = 0.01 when assessed by Spearman are presented on Table 3. Several representative examples are shown (all are plotted using the logarithmic scale, rs values are given in parenthesis): (a) sucrose transporter versus sucrose (rs = -0.52), (b) glutamate decarboxylase versus 4-aminobutyric acid (rs = 0.55); (c) tryptophan synthase b chain 1 versus tryptophan; (rs = 0.61) (d) tryptophan synthase b chain 1 versus tyrosine (rs = 0.65); (e) ornithine carbamoyltransferase versus serine (rs = 0.53); (f) ornithine carbamoyltransferase versus cysteine (rs = 0.54); (g) aminotransferaselike protein versus fructose-6-P (rs = 0.85); (h) aminotransferase-like protein versus glucose-6-P (rs = 0.85); (i) glutamate decarboxylase versus spermidine (rs = 0.64); (j) glutamate decarboxylase versus tyrosine (rs = 0.63);

(k) CONSTANS-like protein versus ascorbate (rs = - 0.72); (I) succinyl-CoA synthetase alpha subunit versus tocopherol (rs = - 0.64); (m) transcription factor WRKY6 versus lysine (rs = 0.51); (n) S- adenosyl-L-methionine synthetase versus lysine (rs = 0.6); (o) ornithine carboxyltransferase versus lysine (rs = 0.54); (p) caffeoyl-CoA O- methyltransferase versus lysine (rs = -0.52).

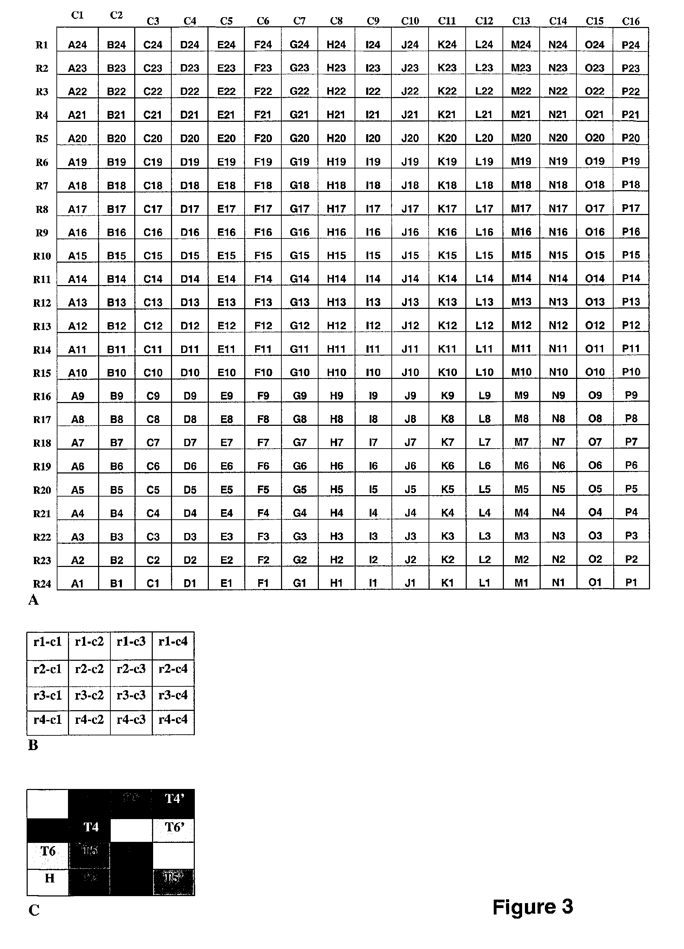

**Figure 3**    displays the spotting scheme applied for the construction of the micro arrays used in the Examples 1 and 2. The filter is organized in 16 x 24 block (Figure 3A).
Description A1 to P24 corresponding to the position on the 384 well-plates.
The orientation is left-bottom since the origin of each plate A1 is located in the left bottom position of each block on the filter.
The description of R1-R24 and C1-C24 corresponding to the primary rows (R) and columns (C) in AIS program.
The secondary grit contain 4 x 4 spots (Figure 3B). Each of the spots represent duplicates of the same clone (Figure C). In secondary grid 3 spots are left empty to quantify the local background.
The control DNA (human gene) is spotted at the position r4-c1.
Each secondary grid contains different clones from 6 different plates (T1-T6) (Figure 3C).
The whole filter consists of 384 secondary grids which adds up to 2112 individual clones represented by the spots.

**Table 1:**    Up and down regulated transcripts in developing wild-type and in transgenic potato tubers
For the developing tubers the results from potato tuber after 8 weeks growth were chosen as the reference for calculating the relative amounts of transcripts in the subsequent stages of development. For the transgenic tubers, the results from wild-type tubers after 10 weeks growth were chosen as the reference.

         a) 9 weeks
         b) 10 weeks
         c) 13 weeks
         d) 14 weeks
         e) SP29 line (SP)
         f) INV30 line (INV2-30)

**Table 2:**    Metabolite levels in potato tubers
The data obtained from the transgenic lines are normalized to the mean response calculated for the wild-type tubes after 10 weeks of growing. For the developing wild-type tubers, 8 weeks grown tubers were used as reference. The values are presented as the mean ±SE of all
independently determined replicates. Those metabolites that significantly differ from the reference are shown in boldface.

         a) Developing wild-type tubers
         b) Transgenic lines

**Table 3:**    Significant correlations between selected transcripts and chemically identified metabolites
Correlations between selected transcripts (identified by reference to the EST clone to which it hybridises, see "EST ID" and "Annotation") and metabolites (see "MB") were calculated using the method of Spearman, taking correlations with significant value P = 0.01 as the threshold of significance. Under "sig 0.001", it is indicated whether a calculated correlation is also significant for p = 0.001, the number 1 meaning significant and 0 non-significant. "NOP" stands for the number of correlated pairs.

**Table 4:**    List of the EST clones spotted onto the microarray used in Examples 1 and 2

The following Examples illustrate the invention:

**Experimental set-up**

1. Plant material

*Solanum tubersosum* L. cv Desiree was obtained from Saatzucht Lange AG (Bad Schwartau, Germany). The generation of the transgenic plant lines SP29 and INV2-30 used in this study has been detailed previously (Sonnewald, 1997; Tretheway, 2001; Roessner, 2001). Plants were handled as described in the literature (Tauberger, 2000; Regierer, 2002). Plants were maintained in tissue culture with a 16-hr.light/8-hr-dark regime on Murashige and Skoog (1962) medium that contained 2% sucrose. In the greenhouse, plants from all lines and wild-type were grown under the same light regime with a minimum of 250 µmol photons m$^{-2}$ sec$^{-1}$ at 22°C. Wild-type tubers were harvested after 8, 9 10, 13 and 14 weeks growing. Transgenic lines were harvested after 10 weeks of development.

## 2. Gene expression analysis

### 2.1 RNA isolation

Total RNA was isolated from 2g fresh weight of tuber tissue using REB isolation buffer (25mM TrisHCl pH.8, 75 mM EDTA, 75 mM NaCl, 1% w/v SDS, 1 M β-MercaptoEtOH) and 10M LiCl2 as described by Logemann et al. (1987).

### 2.2 Microarray construction

Microarrays were constructed on nylon filters as described previously (Thimm, 2001; Colebatch, 2002). More than 2000 tomato clones were collected from the cDNA library constructed in the laboratories of Dr. Steve Tanksley, Cornell University Solanaceae Genome Network, Dr. Greg Martin, Boyce Thompson Institute and Dr. Jim Giovannoni, Boyce Thompson Institute and provided by TIGR - Institute for Genomic Research (Van der Hoeven, Plant Cell 14 (2002), 1441-1456). These ESTs correspond to approximately 1000 tomato genes which are highly homologous to those from potato (Fulton, 2002). In particular, using the web site annotation http://www.tigr.org/tdb/tgi/lgi/ form LeGI Release 7.0 - May 22, 2001, about one thousand interesting TCs (Tentative Consensus) were selected. For each TC, at least two ESTs were picked to the Solanum library, i.e. of each bacterial colony containing a specific EST, an alignot was selected and transferred to a new 96 well plate. (for the complete list of selected clones see Table 4). 96 additional potato clones were added, but the obtained results were not used for this work. The cDNA was amplified by PCR using LacZ-specific primers (forward LacZ1 5' GCTTCCGGCT CGTATGTTGT GTG 3' (SEQ ID NO:1) and reverse LacZ2 5' AAAGGGGGGATGTGCT GCAAGGCG 3' (SEQ ID NO:2)) and Taq polymerase. The PCR products were selectively checked on an agarose gel before spotting. The PCR products were spotted automatically onto the nylon membranes (Biogrid, Biorobotics, Cambridge, UK; Nytran Supercharg, 22.2 x 22.2 cm, Schleicher and Schüll, Dassel, Germany). The Solanum library was spotted six times onto one membrane (for details of the spotting scheme see Figure 3).

### 2.3 Reference hybridization

To normalize the amount of spotted cDNA, a reference hybridization for each filter was carried out using a [$^{33}$P]-labeled PCR product-specific primer (T4 polynucleotide kinase, New England Biolabs, Beverly, MA; [$^{33}$P]ATP, Hartmann Analytic, Braunschweig, Germany; 5' TTCCCAGTCACGA (SEQ ID NO:3)). The filters were hybridized at 5°C overnight and washed for 40 min at 5°C in Ssarc (4xSSC, 7% [v/v] Sarcosyl NL30, and 4 µM EDTA). Filters were exposed o/n on imaging plates and detected with phosphorimager (BAS-1800, Fuji, Tokyo). Radioactivity was removed from the filters by washing two times in SSarc at 65°C for 30 minutes. After every stripping, removing quality was checked by o/n exposition with imaging plates.

### 2.4 Reverse transcriptase labelling reaction

For reverse transcription, 10µg total RNA was used (Superscript II, GibcoBRL, Karlsruhe, Germany; [$^{33}$P] CTP, Hartmann Analytic, Germany). After reverse transcription, RNA was hydrolyzed with NaOH (0.25N) and neutralized with HCl (0.2 N) and sodium phosphorylate buffer (40mM, pH 7.2). Labelling efficiency was controlled by scintillation counting (LS6500, Beckman Munich) after removal of unincorporated oligonucleotides by Sephadex G-50 chromatography (NICK Columns, Amersham Pharmacia). After pre-hybridisation for 2 h at 65°C in Church buffer (7%[w/w] SDS, 1mM EDTA, pH 8.0, and 0.5 M sodium phosphate, pH 7.2) containing salmon sperm DNA (100 ng ml$^{-1}$, Roth, Carl GmbH&Co, Karlsruhe, Germany), filters were hybridised with the labelled cDNA probe at 65°C for 24h. Washing steps were carried out at 65°C for 20 min each with 1xSSC, 0.1% (w/v) SDS, 4mM $Na_2PO_4$ (pH 7.2); 0.2xSSC, 0.1% (w/v) SDS, 4mM $Na_2PO_4$ (pH 7.2); 0.1 SSC, 0.1 % (w/v) SDS, 4mM $Na_2PO_4$ (pH 7.2). The filters were exposed on imaging plates for 16 h and signals were detected using a phosphorimager (BAS-1800 II, Fuiji) followed by stripping for two times for 30 minutes at 80°C (0.1 % [w/v] SDS, 5mM $Na_2PO_4$, pH 7.2). After every stripping, removing quality was checked by the o/n exposition with imaging plates. For transgenic lines and 10 weeks tubers, three repetitions of hybridisation with a newly synthesized and labelled cDNA probe of corresponding RNA were performed. For 8, 9, 13 and 14 weeks old tubers four repetitions were done.

2.5 Data analysis

For data analysis, the signal intensities of the reference and complex hybridisation were quantified using the Arrayvision 5.1 software (Imaging Research Inc., Haverhill, UK). A predefined grid, determining the area of signal quantification, was manually optimised to ensure correct signal recording. The quantified signals, defined as photo-stimulated luminescence mm$^{-2}$, were assigned to the corresponding cDNA clones stored in a suitable database ("Haruspex") (http://www.mpimp-aolm.mp.de/haruspex/index-e.html). The cDNAs on the filter were arranged as 4x4 arrays, each containing six doubly spotted clones, a human gene (desmin) (not used in this study), and an empty field to determine specific local background (LB) (for details see Figure 3). In one membrane, the Solanum library was spotted six times (six block per membrane), and each block was analyzed separately. After each hybridization, six replicated results were obtained and normalized using the Haruspex database. From the available options, replacement by estimating values was used. Additionally, the normalized data was then subjected to Grubbs'test (Grubbs 1969 and Stefansky 1972) in order to detect outliers in the univariate data set. For each clone, the average value for every repetition was calculated. Clones were described as differentially expressed in the tested situation while differences between average tested values and average reference values were two fold and additionally test showed significant change at the level of P 0.05.

3. Metabolite analysis

The preparation and derivatisation of samples for metabolite analysis, and the subsequent operation of the GC-MS and evaluation of the resultant chromatograms and normalization of the results were carried out exactly as described (Roessner, 2001).

3.1 GC-TOF analysis

For GC-TOF analysis, the organic phase was dried and dissolved in 50μl of methoxamine hydrochloride (20mg/mL pyridine) and incubated at 30 °C for 90 min with continuous shaking. Then 80 μL of N-Methyl-N-trimethylsilyltrifluoroacetamid (MSTFA) was added to derivatize polar functional groups at 37°C for 30 min. The derivatized samples were stored at room temperature for 120 min before injection. GC-TOF analysis was performed on a HP 5890 gas chromatograph with standard liners and splitless injection at 230°C injector temperature. The GC was operated at constant flow of 1 mL/min Helium and a 40 m 0.25 mm ID 0.25 μm RTX-5 column with 10 m integrated pre-column. The temperature gradient started at 80°C, was held isocratic for 2 min, and subsequently ramped at 15°C/min to a final temperature of 330°C which was held for 6 min. 20 spectra per second were recorded between m/z 85 to 500. After data acquisition was finished, reference chromatograms were defined that had a maximum of detected peaks over a signal/noise threshold of 20 and used for automated peak identification based on mass spectral comparison to a standard NIST 98. Automated assignments of unique ions for each individual metabolite were taken as default as quantifiers, and manually corrected where necessary. All artifactual peaks caused by column bleeding or phtalates and polysiloxanes derived from MSTFA hydrolysation were removed from the results table. All data were normalized to plant mg FW and to the internal references and log-transformed. t-test, correlation analysis, and variance analysis were performed in Excel 5.

3.2 Two-dimensional liquid chromatography/mass spectrometry.

The dried protein pellet was dissolved in freshly prepared 1 M Urea in 0.05 M Tris buffer pH 7.6. The complex protein mixture was digested with modified trypsin (Böhringer Mannheim) according to the manufacturer's instructions. The tryptic digest was dried down and dissolved in 300 μl water (1% formic acid). Unsoluble material was removed by centrifugation. An aliquot of the digest (~100 μg protein) was injected onto two-dimensional chromatography on a thermofinnigan proteomeX system coupled to an LCQDecaXp ion trap (Thermofinnigan). The chromatographic separation was done according to manufacturer's instructions. After a 12 cycle run the MS/MS spectra were searched against an Arabidopsis thaliana database (downloaded from the TAIR homepage www.arabidopsis.org) using Turbosequest implemented in Bioworks 3.0 (Thermofinnigan). Matches were filtered according to Wolters et al. (2001). Additionally, we used the multiple scoring filter of Bioworks 3.0 with 50 percent ion coverage. For the quantification approach aliquots of the complex tryptic digest of Arabidopsis leaf protein ( 50 μL) were analysed on reversed phase chromatography. Quantification was achieved by integrating peak areas of target peptides representative for proteins. These peak areas were normalised to the sum of internal standard peptides that had been added to the mixture (Chelius et al. (2002), Bondarenko et al. (2002)).

4. Discriminant analysis

Several different data transformations, distance measure and clustering methods were tested on the data set, before using principal component analysis (PCA). PCA was performed independently on the data sets obtained from transcript and metabolite profiling with the software package S-Plus 2000 (Insightfull, Berlin, Germany) using the default weighted covariance estimation function. The data was log10 transformed prior to further analysis and metabolites or ESTs that were not common to all samples were removed.

5. Correlation analysis

Correlation analysis was used to select ESTs which gave highly reproducible results with respect to other ESTs of the consensus sequence from which they were derived. For this purpose the significance threshold was set to P > 0.001. Only ESTs that were above this threshold according to the Spearman method and which appeared reliable during every experiment were selected for subsequent analysis. Table 1 shows the EST clones for which corresponding differentially expressed transcripts were observed. Correlations between selected transcripts and metabolites were calculated using the method of Spearman, taking correlations with significant value P = 0.01.

**Example 1: Separate profiling of transcripts and metabolites of potato tubers at different developmental stages and of transgenic potato tubers**

The parallel analysis of a given biological system is described using two phenotyping technologies, the current technology of metabolic profiling based on GC-MS analysis and gene expression analysis using classical array technology. In attempting these experiments, it was first tried to answer the question what the relative power of the two phenotyping technologies is to discriminate biological systems which either differ in the developmental state or exhibit well characterized transgenic changes.

When these experiments were designed, it was decided to use a plant system, namely the potato tuber system, to evaluate the above questions. The reasons for this were two-fold: first, the potato tuber displays well-defined but nevertheless highly related developmental stages and allows the assessment of a number of well-characterized transgenic situations. Second, the laboratory of the inventors is very well acquainted with the analysis of the potato tubers on both the biochemical and the molecular levels (Fernie, 2002). As a first step, the expression of 1000 genes represented by at least two expressed sequence tags (ESTs) comprising the genes of many transcription factors and a wide variety of biosynthetic genes were analysed on a custom array. After analysing the data sets obtained, 279 ESTs were selected which gave highly reproducible results with respect to the consensus sequence from which they were derived. In order to see whether various developmental stages could be discriminated from one another and from the transcript profiles of transgenic potato tubers ectopically expressing a more efficient pathway of sucrose mobilization (Roessner, 2001), a principal component analysis (PCA) was performed. Table 1 is a presentation of those clones of which the corresponding transcripts are differentially expressed. As can be seen in Figure 1 a, some of the developmental stages can be well distinguished from each other on the basis of their transcript profiles. In this respect, the clear differentiation of tubers harvested after 10 weeks of growth from the other developmental stages is most notable, a fact which may be attributed to the high metabolic activity at this developmental stage. However, it was also observed that the transcript levels of the transgenic tubers could not be discriminated either from each other nor from the corresponding wild-type tuber which was quite surprising. It is important to note that a similar picture was observed following principal component analysis of the entire transcript data set (data not shown). Whilst it is clear that the tuber samples harvested following ten weeks of plant growth were strikingly different from those harvested at other developmental stages, the fact remains that these results suggest that the transcriptional variation during development is greater than that following a relatively severe genetic perturbation of the primary metabolism (Roessner, 2001).

Metabolic profiling was then carried out on samples corresponding to those in the above-described transcript analysis in order to determine the levels of the major metabolites of primary metabolism including sugars, sugar alcohols, organic acids, amino acids as well as the nutritionally important compounds ascorbate and tocopherol. The corresponding metabolite profiles are depicted in Table 2. When a principal component analysis was carried out on the data set obtained from the metabolic profiling studies (Figure 1b), a picture was observed different from that seen upon analysis of the transcript data. In this instance, the transgenic plants clustered completely independently (both with respect to the wild-type control and to one another). With regard to the different developmental stages of the wild-type plants, however, the metabolite complement samples taken after 10 weeks of growth were relatively similar to those taken at other time points.

In conclusion, the discriminatory power of transcript and metabolite profiling approaches are different with metabolite profiling allowing a greater resolution of the different systems studied here. Whether or not this reflects the fact that changes on the transcript level are less pronounced as compared to changes on the metabolite level or merely highlights limitations of the profiling technologies used remains an open question. However, whatever the reason, these results imply that the discrimination of biological systems should be performed at more than one level.

**Example 2: Parallel transcript and metabolite profiling of potato tubers and correlation analysis of the two data sets obtained**

As a further question, it was examined whether the combined analysis of transcript and metabolite profiling data presents a useful approach for the identification of candidate genes that may change the metabolic composition of a given biological system. For this purpose, all the transcript and metabolite data points obtained in the analysis described in Example 1 were run through pairwise correlation analysis in order to determine for each transcript whether it is correlated with any of the metabolites.Out of the 23715 analysed pairs 329 positive and 189 negative correlations were identified. The used significance threshold of $P = 0.01$ in the non-parametric Spearman's rank correlation analysis is a rather conservative estimation of the number of chance correlations. Therefore, the identification of 518 correlations of high statistical significance was a surprising result. A couple of representative correlations is shown in Figure 2 and discussed in detail below, whilst the entire list is given in Table 3.

First, as with any new approach it is important to see whether the data obtained is in agreement with observations made following different, more established experimental strategies. This is clearly the case if one contemplates for instance the strong negative correlation between sucrose and sucrose transporter expression (Figure 2a) and the strong positive correlation between 4-aminobutyric acid and glutamate decarboxylase isoform I (Figure 2b). Both correlations have previously been reported in the literature (Vaughn, 2002; Facchini, 2000), thus providing a confirmation of the validity of the herein presented new approach. Second, many further correlations seem to have a functional basis that can be explained retrospectively. The positive correlations of both tryptophan and tyrosine with the β2chain of tryptophan synthase (Figures 2c, 2d) and ornithine carbamoyltransferase with serine and cysteine (Figures 2e, 2f) are two such examples. Third, although several of the correlations, such as those described above, were predictable, the majority of the correlations obtained was novel and unpredictable since they are not directly related to the biochemical pathway in which the respective gene products participate. Several correlations were identified between transcripts and metabolites of the same or related pathways a fact that may strengthen the interpretation of these linkages. Examples of such instances include aminotransferase which correlates with both fructose-6-phosphate and glucose-6-phosphate (Figures 2g, 2h). These two correlations could not be predicted on the basis of the previous knowledge. But the existence of one of these correlations makes it likely that also the other one exists, as it has been shown in the present studies. These findings may offer hints to the function of the genes involved (the elucidation of which, if carried out by conventional methods, requires a huge amount of research effort). It is also interesting to note that several transcripts correlate with more than one metabolite, such as the aminotransferase mentioned above. Other examples include glutamate decarboxylase isoform I which correlates both with spermidine (Figure 2i) and tyrosine (Figure 2j) and various transcription factors correlating with sucrose, 4-aminobutyric acid and tryptophan. Finally it is exciting to see that nutritionally important metabolites such as ascorbate, tocopherol and lysine were tightly correlated with the expression levels of various enzymes or transcription factors: e.g. ascorbate being negatively correlated with a homologue of the clock gene CONSTANS (Figure 2k), tocopherol being negatively correlated with succinyl CoA synthetase (Figure 2l) and lysine being positively correlated with the transcription factor WRKY6 (Figure 2m), S-adenosyl-L-methionine synthetase (Figure 2n) and ornithine carbamoyltransferase (Figure 2o) and negatively correlated with caffeoyl-CoA O-methyltransferase (Figure 2p). It is believed that these essentially unexpected correlations are of great potential for biotechnological applications in which it is the goal to modify the metabolite composition by genetic means. The approach of linking transcript and metabolite data via pair-wise correlation analysis presents a very powerful tool for the rapid identification of candidate genes, which then have to be tested for their value as regards applicability via further experimentation.

As a conclusion from the afore-described experiments, one has to note that, although, as might have been expected from previous experimental work aimed at comparing the transcript and protein levels (Ideker, 2001; Gygi, 1999; Futcher, 1999) and mathematical studies (TerKuile, 2001), the number of strong metabolite-transcript correlations observed is relatively small, it is conceivable that they allow the generation of clearly verifiable hypotheses. Of particular interest in this regard are the observation of the correlation of genes with the essential amino acid lysine and with the vitamins ascorbate and tocopherol. These linkages define strong candidate genes for the manipulation of the content of these nutritionally important compounds in plants.

**References**

Bondarenko *et al., Analytical Chemistry* 74 (2002), 4741-4749.

Chelius et al., J. *Proteome Res.* 1 (2002), 317-323.

Colebatch, G. *et al.* Novel aspects of symbiotic nitrogen fixation uncovered by transcript profiling with cDNA arrays. *Mol. Plant Microbe. Interact.* 15, 411-420 (2002).

Facchini, P. J., Huber-Allanach, K. L. & Tari L. W. Plant aromatic L-amino acid decarboxylases: evolution, biochemistry, regulation, and metabolic engineering applications. *Phytochemistry* 54, 121-138 (2000).

Fernie, A. R., Willmitzer, L. & Trethewey, R. N. Sucrose to starch: a transition in molecular plant physiology. Trends Plant Sci. 7, 35-42 (2002).

Fiehn, O. *et al.* Metabolite profiling for plant functional genomics. *Na. Biotechnol.* 18, 1157-1161 (2000).

Fiehn O. Metabolomics - the link between genotypes and phenotypes. Plant Mol Biol 48, 155-171 (2001).

Fulton, T. M., Van der Hoeven, R., Eannetta, N.T. & Tanksley, S. D. Identification, analysis, and utilization of conserved ortholog set markers for comparative genomics in higher plants. *Plant Cell* 14, 1457-1467 (2002).

Futcher, B., Latter, G. I., Monardo, P., McLaughlin, C. S. & Garrells, J. I. A sampling of the yeast proteome. *Mol. Cell Biol.* 19, 7357-7368 (1999).

Gassen, H. G. and Schrimpf, G. (1999) Gentechnische Methoden, Spektrum Akademisher Verlag, Gustav Fischer, Heidelberg Berlin

Grubbs, F. Procedures for detecting outlying observations in samples. *Technometrics* 11, 1-21 (1969).

Gygi, S. P., Rochon, Y., Franza, B. R. & Aebersold, R. Correlation between protein and mRNA abundance in yeast. *Mol. Cell BioL* 19, 1720-1730 (1999).

Ideker, T. *et al.* Integrated genomic and proteomic analyses of a systematically perturbed metabolic network. *Science* 292, 929-934 (2001).

Ideker, T., Galitski, T. & Hood, L. A new approach to decoding life: systems biology. *Annu. Rev. Genomics Hum. Genet.* 2, 343-372 (2001).

International Human Genome Sequencing Consortium Initial sequencing and analysis of the human genome. *Nature* 409, 860-921 (2001).

Kitano, H. Systems biology: A brief overview. *Science* 295, 1662-1664 (2002).

Kuiper, H. A., Kleter, G. A., Noteborn, H. P. J. M. & Kok, E. J. Assessment of the food safety issues related to genetically modified foods. *Plant J.* 27, 503-528 (2001).

Lockhart, D. J *et al.* Expression monitoring by hybridisation to high-density oligonucleotide arrays. *Nat. Biotechnol.* 14, 1675-1680 (1996).

Logemann, J., Schell, J., Willmitzer, L. Ann. Biochem. 163, 16-20(1987).

Murashige, T., Skoog, F. Physiol. Plantarum 15, 473-497(1962).

Oltvai, Z. N. & Barabasi, A. L. Systems biology: Lifes complexity pyramid. *Science* 295, 1662-1664 (2002).

Regierer, B. *et al.* Starch content and yield increase as a result of altering adenylate pools in transgenic plants. *Nat. Biotechnol.* 20, 1256-1260 (2002).

Roessner, U. *et al.* Metabolic profiling allows comprehensive phenotyping of genetically or environmentally modified plant systems. *Plant Cell* 13, 11-29 (2001).

Roessner, U. et al., Plant J. 23(1) (2000), 131-142.

Sambrook and Russell (2001) Molecular Cloning: A Laboratory Manual, Cold Spring Harbour Laboratory Press, New York

Shevchenko, A. *et al.* Linking genome and proteome by mass spectrometry: large scale identification of yeast proteins from two-dimensional gels. *Proc. Natl. Acad. Sci., USA* 93, 14440-14445 (1996).

Sonnewald, U. *et al.* Increased potato tuber size resulting from apoplastic expression of a yeast invertase. *Nat. Biotechnol.* 15, 794-797 (1997).

Stefansky, W. Rejecting outliers in factorial designs. Technometrics 14, 469-479 (1972).

Tauberger, E. *et al.* Antisense inhibition of plastidial phosphoglucomutase provides compelling evidence that potato tuber amyloplasts import carbon from the cytosol in the form of glucose-6-phosphate. *Plant J.* 23, 43-53 (2000).

ter Kuile, B. H. & Westerhoff, H. V. Transcriptome meets metabolome: hierarchical and metabolic regulation of the glycolytic pathway. *FEBS Lett.* 500, 169-171 (2001).

The Arabidopsis Genome Initiative Analysis of the genome sequence of the flowering plant Arabidopsis thaliana *Nature* 408, 796-815 (2000).

Thimm, O., Essigmann, B., Kloska, S., Altmann, T. & Buckhout, T. J. Response of arabidopsis to iron deficiency stress as revealed by microarray analysis. *Plant Physiol.* 127, 1030-1043 (2001).

Trethewey, R. N. *et al.* Expression of a bacterial sucrose phosphorylase in potato tubers results in a glucose-independent induction of glycolysis. *Plant Cell Environ.* 24, 357-365 (2001).

Trethewey, R.N., Geigenberger, P., Riedel, K., Hajirezaei, M.R., Sonnewald, U., Stitt, M., Riemeier, J.W., Willmitzer, LPlant J. 15, 109-118. (1998).

Trewavas, A. & Leaver, C. J. Conventional crops are the test of GM prejudice. *Nature* 401, 640-640(1999).

Tweeddale, H., Notley-McRobb, L. and Ferenci, T. Effect of slow growth on metabolism of Escherichia coli, as revealed by global metabolite pool ("Metabolome") analysis. J. Bacteriol. 180, 5109-5116 (1998).

Vaughn, M. W., Harrington, G. N. & Bush D. R. Sucrose-mediated transcriptional regulation of sucrose symporter activity in the phloem. *Proc. Natl. Acad. Sci., USA* 99, 10876-10880 (2002).

## Table 1

### (a)  List of up and down regulated clones in 9 weeks old potato tuber

| ANNOTATION | EST ID | XFOLD |
|---|---|---|
| S-adenosyl-L-methionine synthetase | CTOF3K21 | 0,20 |
| glycogen (starch) synthase precursor {Solanum tuberosum}SP\|Q00775\|UGST_SOLTU GRANULE-BOUND GLYCOGEN | CTOF21A12 | 0,22 |
| Similar to gb\|AF135422 GDP-mannose pyrophosphorylase A (GMPPA) from Homo sapiens.  ESTs gb\|AA712990, | CLET27N17 | 0,27 |
| NADH-ubiquinone oxidoreductase 20 kDa subunit precursor {Solanum tuberosum}SP\|Q43844\|NUKM_SOLTU NAD | CLEG33O5 | 0,33 |
| S-adenosylmethionine:2-demethylmenaquinone methyltransferase-like protein {Arabidopsis thaliana} | CLED30J21 | 0,38 |
| NADH-ubiquinone oxidoreductase 20 kDa subunit precursor {Solanum tuberosum}SP\|Q43844\|NUKM_SOLTU NAD | CLED16D1 | 0,40 |
| transcription factor CRC {Arabidopsis thaliana}GP\|12325076\|gb\|AAG52485.1\|AC018364_3\|AC018364 transc | CLED15E7 | 0,46 |
| S-adenosylmethionine:2-demethylmenaquinone methyltransferase-like protein {Arabidopsis thaliana} | CTOF26K3 | 0,48 |
| transcription factor IIA small subunit {Arabidopsis thaliana}GP\|5051786\|emb\|CAB45079.1\|\|AL078637 tr | CTOE6J10 | 0,49 |
| cytochrome P450, putative {Arabidopsis thaliana}PIR\|F86441\|F86441 probable cytochrome P450 [importe | CTOF3J9 | 0,49 |
| hydroxycinnamoyl-CoA:tyramine N-(hydroxycinnamoyl)transferase {Capsicum annuum} | CLEC4A12 | 0,50 |
| putative C3HC4-type RING zinc finger protein {Arabidopsis thaliana}PIR\|B84813\|B84813 probable RING | CLEC32P10 | 2,00 |
| bHLH transcription factor GBOF-1 {Tulipa gesneriana} | CLEM17A5 | 3,68 |

## (b) List of up and down regulated clones in 10 weeks old potato tuber

| ANNOTATION | EST ID | XFOLD |
|---|---|---|
| caffeoyl-CoA O-methyltransferase 5 {Nicotiana tabacum}GP\|1679853\|emb\|CAB05369.1\|\|Z82982 caffeoyl-Co | CLEZ20I22 | 0,44 |
| "putative ABC transporter; 60211-54925 {Arabidopsis thaliana}PIR\|E96742\|E96742 probable ABC transpor" | CLED21K4 | 0,45 |
| aldose 1-epimerase-like protein {Arabidopsis thaliana} | CLEZ10I24 | 0,46 |
| NADH-dependent glutamate synthase {Arabidopsis thaliana} | CLEC38H15 | 0,47 |
| glutamine synthetase I {Medicago truncatula} | CLEC16M12 | 0,48 |
| URIDYLATE KINASE (EC 2.7.4.-) (UK) (URIDINE MONOPHOSPHATE KINASE) (UMP KINASE) (UMP/CMP KINASE).GP\| | CLEY26B11 | 2,00 |
| putative cytochrome P450 {Solanum chacoense}SP\|P93530\|C7D6_SOLCH CYTOCHROME P450 71D6 (EC 1.14.-.-) | CLED28H13 | 2,02 |
| zinc finger protein-like {Arabidopsis thaliana} | CLEG37C14 | 2,06 |
| NADH-ubiquinone oxidoreductase 20 kDa subunit precursor {Solanum tuberosum}SP\|Q43844\|NUKM_SOLTU NAD | CLEG33O5 | 2,09 |
| tyramine hydroxycinnamoyltransferase {Nicotiana tabacum} | CLEZ9E23 | 2,10 |
| zinc finger protein-like {Arabidopsis thaliana} | CTOF19M22 | 2,11 |
| putative homeodomain transcription factor {Arabidopsis thaliana}PIR\|H84774\|H84774 probable homeodom | CLED30M20 | 2,11 |
| sugar transporter like protein {Arabidopsis thaliana}GP\|2464913\|emb\|CAB16808.1\|\|Z99708 sugar transp | CLPP11N17 | 2,16 |
| transcription factor CRC {Arabidopsis thaliana}GP\|12325076\|gb\|AAG52485.1\|AC018364_3\|AC018364 transc | CLED4O9 | 2,21 |
| putative cytochrome P450 {Solanum chacoense}SP\|P93530\|C7D6_SOLCH CYTOCHROME P450 71D6 (EC 1.14.-.-) | CLED4O17 | 2,27 |
| ALTERNATIVE OXIDASE 1 PRECURSOR (EC 1.-.-.- ).GP\|558054\|gb\|AAC60576.1\|\|S71335 alternative oxidase, A | CLEY14I23 | 2,30 |
| UDP-glucose:salicylic acid glucosyltransferase {Nicotiana tabacum} | CLED25E6 | 2,31 |
| ALTERNATIVE OXIDASE 1 PRECURSOR (EC 1.-.-.- ).GP\|558054\|gb\|AAC60576.1\|\|S71335 alternative oxidase, A | CLEY7C9 | 2,33 |
| lysine-ketoglutarate reductase/saccharopine dehydrogenase bifunctional enzyme {Arabidopsis thaliana} | CLEC6K9 | 2,34 |
| sugar-phosphate isomerase-like protein {Arabidopsis thaliana}PIR\|T47628\|T47628 sugar-phosphate isom | CLEY19P11 | 2,35 |
| S-adenosylmethionine:2-demethylmenaquinone methyltransferase-like protein {Arabidopsis thaliana} | CTOF26K3 | 2,37 |
| "ornithine carbamoyltransferase; OCTase {Canavalia lineata}GP\|12003188\|gb\|AAG43481.1\|AF203688_1\|AF20" | CLED6P9 | 2,38 |
| transcription factor TEIL {Nicotiana tabacum} | CLEG39L11 | 2,38 |
| transcription factor WRKY6 {Arabidopsis thaliana}GP\|12658412\|gb\|AAK01128.1\|AF331713_1\|AF331713 tran | CLEI5A6 | 2,38 |
| PROBABLE VACUOLAR ATP SYNTHASE SUBUNIT F (EC 3.6.1.34) (V-ATPASE F SUBUNIT) (VACUOLAR PROTON PUMP F | CLEX11L22 | 2,38 |
| ATP synthase delta' subunit, mitochondrial precursor {Ipomoea batatas}SP\|Q40089\|ATP4_IPOBA ATP SYNT | CTOF10I1 | 2,39 |
| putative homeodomain transcription factor {Arabidopsis thaliana}PIR\|F84565\|F84565 probable homeodom | CLED21A22 | 2,40 |
| contains similarity to sugar transporters (Pfam: sugar_tr.hmm, score: 395.91) {Arabidopsis thaliana} | CTOD2G24 | 2,41 |

| | | |
|---|---|---|
| cytochrome P450-like protein {Arabidopsis thaliana}GP\|7270932\|emb\|CAB80611.1\|\|AL161595 cytochrome P | CLHT23L11 | 2,41 |
| succinate dehydrogenase flavoprotein alpha subunit {Arabidopsis thaliana}GP\|8843734\|dbj\|BAA97282.1\| | CLEZ9G24 | 2,42 |
| cytochrome p450 lxxia4 {Solanum melongena}SP\|P37117\|C714_SOLME CYTOCHROME P450 71A4 (EC 1.14.-.-) ( | CLED11B9 | 2,43 |
| glycerol-3-phosphate dehydrogenase {Arabidopsis thaliana}PIR\|F84832\|F84832 glycerol-3-phosphate deh | CLES5M24 | 2,44 |
| putative monosaccharide transporter 1 {Petunia x hybrida} | CLPP11G6 | 2,45 |
| sugar transporter like protein {Arabidopsis thaliana}GP\|2464913\|emb\|CAB16808.1\|\|Z99708 sugar transp | CLPP7D3 | 2,46 |
| CCAAT box binding factor/ transcription factor Hap2a {Arabidopsis thaliana}PIR\|T49898\|T49898 CCAAT | CLEG43E15 | 2,46 |
| fructokinase 1 {Arabidopsis thaliana}GP\|13878053\|gb\|AAK44104.1\|AF370289_1\|AF370289 putative fructok | CLPP13M11 | 2,51 |
| 4-hydroxyphenylpyruvate dioxygenase {Solenostemon scutellarioides} | CLEC33J10 | 2,54 |
| delta 1-pyrroline-5-carboxylate synthetase | CTOE2C17 | 2,54 |
| URIDYLATE KINASE (EC 2.7.4.-) (UK) (URIDINE MONOPHOSPHATE KINASE) (UMP KINASE) (UMP/CMP KINASE).GP\| | CLEM3K4 | 2,56 |
| transcription factor WRKY6 {Arabidopsis thaliana}GP\|12658412\|gb\|AAK01128.1\|AF331713_1\|AF331713 tran | CLEC11I13 | 2,58 |
| contains similarity to sugar transporters (Pfam: sugar_tr.hmm, score: 395.91) {Arabidopsis thaliana} | CLPP7N18 | 2,59 |
| Dof zinc finger protein {Arabidopsis thaliana}GP\|9280230\|dbj\|BAB01720.1\|\|AB023045 Dof zinc finger p | CLED28N11 | 2,59 |
| bHLH transcription factor GBOF-1 {Tulipa gesneriana} | CLED22C14 | 2,60 |
| acetylornithine aminotransferase precursor {Alnus glutinosa}SP\|O04866\|ARGD_ALNGL ACETYLORNITHINE AM | CLED24N6 | 2,62 |
| glucosyl transferase {Nicotiana tabacum}GP\|1805359\|dbj\|BAA19155.1\|\|AB000623 glucosyl transferase {N | CLEG32P19 | 2,62 |
| glutamate decarboxylase isozyme 1 {Nicotiana tabacum} | CLPP9O23 | 2,63 |
| putative cytochrome P450 | CLES20P12 | 2,63 |
| glutamate decarboxylase isozyme 1 {Nicotiana tabacum} | CLPP5L24 | 2,67 |
| TRYPTOPHAN SYNTHASE BETA CHAIN 2 PRECURSOR (EC 4.2.1.20).GP\|2792520\|gb\|AAB97087.1\|\|AF042320 tryptop | CLEI13J17 | 2,69 |
| MADS-box transcription factor FBP21 {Petunia x hybrida} | CLEX2O20 | 2,75 |
| cytochrome P450 like_TBP {Nicotiana tabacum}GP\|1545805\|dbj\|BAA10929.1\|\|D64052 cytochrome P450 like_ | CTOF7K11 | 2,75 |
| homeodomain protein | CLED11B1 | 2,78 |
| TRYPTOPHAN SYNTHASE BETA CHAIN 2 PRECURSOR (EC 4.2.1.20).GP\|2792520\|gb\|AAB97087.1\|\|AF042320 tryptop | CLEM4L3 | 2,79 |
| ethylene-responsive transcriptional coactivator | CTOF23I9 | 2,80 |
| NADH-ubiquinone oxidoreductase 20 kDa subunit precursor {Solanum tuberosum}SP\|Q43844\|NUKM_SOLTU NAD | CLED16D1 | 2,85 |
| ALTERNATIVE OXIDASE 1 PRECURSOR (EC 1.-.-.-).GP\|558054\|gb\|AAC60576.1\|\|S71335 alternative oxidase, A | CLPP2C18 | 2,86 |
| alanine aminotransferase {Arabidopsis thaliana} | CLEC29A19 | 2,92 |
| glucosyltransferase-like protein {Arabidopsis thaliana} | CLEC6D16 | 2,93 |
| hydroxycinnamoyl-CoA:tyramine N-(hydroxycinnamoyl)transferase {Capsicum annuum} | CLEC36G5 | 2,97 |

| | | |
|---|---|---|
| SNAP25A protein {Arabidopsis thaliana}GP\|5731763\|emb\|CAB52582.1\|\|X92419 SNAP25A protein {Arabidopsi | CLET10A17 | 2,97 |
| phosphate/phosphoenolpyruvate translocator-like protein {Arabidopsis thaliana} | CLPP8K20 | 3,13 |
| ADP-glucose pyrophosphorylase small subunit | CLEM19M11 | 3,18 |
| homeodomain protein | CTOF6M4 | 3,22 |
| ATP synthase delta' subunit, mitochondrial precursor {Ipomoea batatas}SP\|Q40089\|ATP4_IPOBA ATP SYNT | CLEY15P13 | 3,26 |
| ethylene-responsive transcriptional coactivator | CLEC40C16 | 3,31 |
| putative homeodomain transcription factor {Arabidopsis thaliana}PIR\|H84774\|H84774 probable homeodom | CLED15E8 | 3,33 |
| dTDP-glucose 4-6-dehydratases-like protein {Arabidopsis thaliana}PIR\|T45701\|T45701 dTDP-glucose 4-6 | CLEZ16P14 | 3,33 |
| "putative transcription factor BTF3 (RNA polymerase B transcription factor 3); 26343-27201 {Arabidops" | CLEZ8J8 | 3,33 |
| Similar to acyl carrier protein, mitochondrial precursor (ACP) NADH-ubiquinone oxidoreductase 9.6 KD | CLEZ20E22 | 3,37 |
| putative C3HC4-type RING zinc finger protein {Arabidopsis thaliana}PIR\|B84813\|B84813 probable RING | CLEC32P10 | 3,45 |
| "putative transcription factor BTF3 (RNA polymerase B transcription factor 3); 26343-27201 {Arabidops" | CTOF23N20 | 3,50 |
| flavanone 3-hydroxylase-like protein {Arabidopsis thaliana} | CTOE12M9 | 3,55 |
| glucosyltransferase-like protein {Arabidopsis thaliana} | CLED34H6 | 3,58 |
| MADS-box transcription factor FBP21 {Petunia x hybrida} | CLET8H21 | 3,59 |
| phosphate/phosphoenolpyruvate translocator protein-like {Arabidopsis thaliana} | CLEG41L23 | 3,61 |
| putative homeodomain transcription factor {Arabidopsis thaliana}PIR\|F84565\|F84565 probable homeodom | CLEG35I11 | 3,65 |
| putative C3HC4-type RING zinc finger protein {Arabidopsis thaliana}PIR\|B84813\|B84813 probable RING | CLEX12A9 | 3,72 |
| NADH-cytochrome b5 reductase {Arabidopsis thaliana}GP\|4240118\|dbj\|BAA74838.1\|\|AB007800 NADH-cytochr | CLEY21D5 | 3,73 |
| mas-binding factor MBF2=transcription factor TGA1a homolog {Solanum tuberosum=potatoes, root, Peptid | CLEY24E3 | 3,74 |
| SNAP25A protein {Arabidopsis thaliana}GP\|5731763\|emb\|CAB52582.1\|\|X92419 SNAP25A protein {Arabidopsi | CLEM23I12 | 3,76 |
| serine/threonine-specific protein kinase NAK {Arabidopsis thaliana}PIR\|T48250\|T48250 serine/threoni | CLER2B1 | 3,82 |
| dehydroquinate dehydratase/shikimate:NADP oxidoreductase | CLEG39N19 | 3,86 |
| S-adenosylmethionine:2-demethylmenaquinone methyltransferase-like protein {Arabidopsis thaliana} | CLED30J21 | 3,87 |
| putative NADH-ubiquinone oxireductase {Arabidopsis thaliana}PIR\|C84588\|C84588 probable NADH-ubiquin | CLEG27G1 | 3,90 |
| S-adenosyl-L-methionine synthetase | CTOF10L8 | 3,97 |
| fructose-6-phosphate 2-kinase/fructose-2,6-bisphosphatase {Solanum tuberosum}PIR\|T07016\|T07016 6-ph | CLEI12O14 | 4,04 |
| fructose-6-phosphate 2-kinase/fructose-2,6-bisphosphatase {Solanum tuberosum}PIR\|T07016\|T07016 6-ph | CLEI15M10 | 4,07 |
| Putative UDP-glucose glucosyltransferase {Arabidopsis thaliana}PIR\|H86356\|H86356 probable UDP-gluco | CLEX11K13 | 4,10 |
| phosphate/phosphoenolpyruvate translocator-like protein {Arabidopsis thaliana} | CLPP9C20 | 4,31 |
| w-3 desaturase {Solanum tuberosum}PIR\|T07685\|T07685 omega-3 fatty acid desaturase (EC 1.14.99.-) - | CLEG33A5 | 4,49 |

| | | |
|---|---|---|
| glycogen (starch) synthase precursor {Solanum tuberosum}SP\|Q00775\|UGST_SOLTU GRANULE-BOUND GLYCOGEN | CTOF21A12 | 4,60 |
| glycogen (starch) synthase precursor {Solanum tuberosum}SP\|Q00775\|UGST_SOLTU GRANULE-BOUND GLYCOGEN | CLPT10L12 | 4,68 |
| acetylornithine aminotransferase precursor {Alnus glutinosa}SP\|O04866\|ARGD_ALNGL ACETYLORNITHINE AM | CLEI11O22 | 4,70 |
| CCAAT box binding factor/ transcription factor Hap2a {Arabidopsis thaliana}PIR\|T49898\|T49898 CCAAT | CLEC15K2 | 4,79 |
| HD-Zip protein [Arabidopsis thaliana] | CLEC13O19 | 4,84 |
| UDP-glucose dehydrogenase-like protein {Arabidopsis thaliana}PIR\|T51527\|T51527 UDP-glucose dehydrog | CLEY2E3 | 4,88 |
| ALTERNATIVE OXIDASE 1 PRECURSOR (EC 1.-.-.-).GP\|558054\|gb\|AAC60576.1\|\|S71335 alternative oxidase, A | CLEY14E21 | 4,90 |
| MYB-like DNA-binding protein {Catharanthus roseus} | CTOF22G14 | 4,93 |
| similar to ATPases associated with various cellular activites (Pfam: AAA.hmm, score: 230.91) {Arabid | CLEG8L3 | 4,96 |
| putative cytochrome P450 | CTOE2F5 | 5,01 |
| cytochrome P450 like_TBP {Nicotiana tabacum}GP\|1545805\|dbj\|BAA10929.1\|\|D64052 cytochrome P450 like_ | CLPP10E22 | 5,07 |
| osmotic stress-induced zinc-finger protein {Nicotiana tabacum}PIR\|T01985\|T01985 zinc-finger protein | CLEX4M2 | 5,07 |
| phosphate/phosphoenolpyruvate translocator protein-like {Arabidopsis thaliana} | CLEG30O13 | 5,60 |
| TOM (target of myb1)-like protein {Arabidopsis thaliana}PIR\|T51543\|T51543 TOM (target of myb1)-like | CLHT6E15 | 5,73 |
| osmotic stress-induced zinc-finger protein {Nicotiana tabacum}PIR\|T01985\|T01985 zinc-finger protein | CLEC21H24 | 5,89 |
| TOM (target of myb1)-like protein {Arabidopsis thaliana}PIR\|T51543\|T51543 TOM (target of myb1)-like | CLHT18D23 | 6,01 |
| similar to ATPases associated with various cellular activites (Pfam: AAA.hmm, score: 230.91) {Arabid | CLEG12O3 | 6,47 |
| "putative ABC transporter; 73228-76244 {Arabidopsis thaliana}" | CLEN21M4 | 6,57 |
| ADP-glucose pyrophosphorylase small subunit | CLES16N17 | 6,94 |
| bHLH transcription factor GBOF-1 {Tulipa gesneriana} | CLEM17A5 | 7,00 |
| cytochrome P450-like protein {Arabidopsis thaliana}GP\|7270932\|emb\|CAB80611.1\|\|AL161595 cytochrome P | CLEG12G7 | 7,29 |
| 76 kDa mitochondrial complex I subunit {Solanum tuberosum}SP\|Q43644\|NUAM_SOLTU NADH-UBIQUINONE OXID | CLEG28D24 | 7,32 |
| dehydroquinate dehydratase/shikimate:NADP oxidoreductase | CLEM21P8 | 10,01 |
| 76 kDa mitochondrial complex I subunit {Solanum tuberosum}SP\|Q43644\|NUAM_SOLTU NADH-UBIQUINONE OXID | CLET38C16-1 | 10,16 |

## (c)  List of up and down regulated clones in 13 weeks old potato tuber

| ANNOTATION | EST ID | XFOLD |
|---|---|---|
| cytochrome P450 like_TBP {Nicotiana tabacum}GP\|1545805\|dbj\|BAA10929.1\|\|D64052 cytochrome P450 like_ | CTOF7K11 | 0,16 |
| S-adenosyl-L-methionine synthetase | CTOF3K21 | 0,18 |
| Similar to gb\|AF135422 GDP-mannose pyrophosphorylase A (GMPPA) from Homo sapiens. ESTs gb\|AA712990, | CLET27N17 | 0,25 |
| glycogen (starch) synthase precursor {Solanum tuberosum}SP\|Q00775\|UGST_SOLTU GRANULE-BOUND GLYCOGEN | CTOF21A12 | 0,28 |
| NADH-ubiquinone oxidoreductase 20 kDa subunit precursor {Solanum tuberosum}SP\|Q43844\|NUKM_SOLTU NAD | CLEG33O5 | 0,30 |
| PROBABLE VACUOLAR ATP SYNTHASE SUBUNIT F (EC 3.6.1.34) (V-ATPASE F SUBUNIT) (VACUOLAR PROTON PUMP F | CLEX11L22 | 0,36 |
| Similar to acyl carrier protein, mitochondrial precursor (ACP) NADH-ubiquinone oxidoreductase 9.6 KD | CLED7G23 | 0,45 |
| fructose-6-phosphate 2-kinase/fructose-2,6-bisphosphatase {Solanum tuberosum}PIR\|T07016\|T07016 6-ph | CLEI12O14 | 0,46 |
| Dof zinc finger protein {Arabidopsis thaliana}GP\|9280230\|dbj\|BAB01720.1\|\|AB023045 Dof zinc finger p | CLED28N11 | 0,48 |
| chorismate synthase 1 precursor 3-phosphate phospholyase 1) {Lycopersicon esculentum}SP\|Q42884\|ARC1 | CTOF26D9 | 0,49 |
| UDP-glucose:salicylic acid glucosyltransferase {Nicotiana tabacum} | CLEG1L22 | 2,04 |
| NADH-cytochrome b5 reductase {Arabidopsis thaliana}GP\|4240118\|dbj\|BAA74838.1\|\|AB007800 NADH-cytochr | CLEY21D5 | 2,04 |
| putative C3HC4-type RING zinc finger protein {Arabidopsis thaliana}PIR\|B84813\|B84813 probable RING | CLEC32P10 | 2,25 |
| bHLH transcription factor GBOF-1 {Tulipa gesneriana} | CLEM17A5 | 2,54 |

## (d) List of up and down regulated clones in 14 weeks old potato tuber.

| ANNOTATION | EST ID | XFOLD |
|---|---|---|
| transcription factor WRKY6 {Arabidopsis thaliana}GP\|12658412\|gb\|AAK01128.1\|AF331713_1\|AF331713 tran | CLEI5A6 | 0,22 |
| transcription factor IIA small subunit {Arabidopsis thaliana}GP\|5051786\|emb\|CAB45079.1\|\|AL078637 tr | CTOE6J10 | 0,42 |
| dTDP-glucose 4-6-dehydratases-like protein {Arabidopsis thaliana}PIR\|T45701\|T45701 dTDP-glucose 4-6 | CLEZ16P14 | 0,45 |
| Zn finger protein {Nicotiana tabacum}GP\|1360078\|emb\|CAA66601.1\|\|X97942 Zn finger protein {Nicotiana | CLED15J6 | 0,46 |
| hydroxycinnamoyl-CoA:tyramine N-(hydroxycinnamoyl)transferase {Capsicum annuum} | CLEC4A12 | 0,46 |
| "ornithine carbamoyltransferase; OCTase {Canavalia lineata}GP\|12003188\|gb\|AAG43481.1\|AF203688_1\|AF20" | CLED6P9 | 0,46 |
| alpha-glucosidase {Solanum tuberosum subsp. tuberosum} | CLED6K9 | 0,47 |
| alanine aminotransferase {Arabidopsis thaliana} | CLEG9A20 | 0,48 |
| bHLH transcription factor GBOF-1 {Tulipa gesneriana} | CLEM17A5 | 2,69 |
| cytochrome P450 like_TBP {Nicotiana tabacum}GP\|1545805\|dbj\|BAA10929.1\|\|D64052 cytochrome P450 like_ | CLPP10E22 | 3,41 |

## (e) List of up and down regulated clones in transgenic tubers - line SP29

| ANNOTATION | EST ID | XFOLD |
|---|---|---|
| ADP-glucose pyrophosphorylase small subunit | CLES16N17 | 0,49 |
| UDP-glucose:salicylic acid glucosyltransferase {Nicotiana tabacum} | CLEG1L22 | 2,13 |

## (f) List of up and down regulated clones in transgenic tubers - line INV30

| ANNOTATION | EST ID | XFOLD |
|---|---|---|
| S-adenosyl-L-methionine synthetase | CTOF3K21 | 0,42 |
| Dof zinc finger protein {Arabidopsis thaliana}GP\|9280230\|dbj\|BAB01720.1\|\|AB023045 Dof zinc finger p | CLED28N11 | 0,49 |
| putative cytochrome P450 | CTOE2F5 | 0,49 |
| cytochrome P450 {Arabidopsis thaliana} | CLEI13B24 | 2,16 |
| transcription factor TEIL {Nicotiana tabacum} | CLEG42L9 | 2,20 |
| "putative ABC transporter; 73228-76244 {Arabidopsis thaliana}" | CLEG42N24 | 2,38 |
| UDP-glucose:salicylic acid glucosyltransferase {Nicotiana tabacum} | CLEG1L22 | 3,02 |
| Putative UDP-glucose glucosyltransferase {Arabidopsis thaliana}PIR\|H86356\|H86356 probable UDP-gluco | CLEX13P4 | 3,08 |

## Table 2

### (a)  Potato tuber development

| Metabolite | 9 weeks | SE | 10 weeks | SE | 13 weeks | SE | 14 weeks | SE | 8weeks | SE |
|---|---|---|---|---|---|---|---|---|---|---|
| 5-oxoproline | 0,43 | ± 0,21 | 0,72 | ± 0,18 | 0,43 | ± 0,37 | 0,74 | ± 0,72 | 1,00 | ± 0,28 |
| aconitate | 1,68 | ± 0,09 | 0,78 | ± 0,07 | 0,98 | ± 0,20 | 1,12 | ± 0,27 | 1,00 | ± 0,23 |
| alanine | 0,58 | ± 0,16 | 0,91 | ± 0,35 | **0,24** | **± 0,31** | **0,27** | **± 0,53** | 1,00 | ± 0,20 |
| arginine | 0,87 | ± 0,25 | 0,69 | ± 0,19 | **0,69** | **± 0,07** | 1,19 | ± 0,42 | 1,00 | ± 0,10 |
| asparagine | 1,24 | ± 0,29 | 1,02 | ± 0,37 | 1,79 | ± 0,37 | **2,43** | **± 0,42** | 1,00 | ± 0,12 |
| aspartate | 0,94 | ± 0,08 | **0,66** | **± 0,12** | 0,86 | ± 0,08 | **0,76** | **± 0,04** | 1,00 | ± 0,08 |
| α-tocopherol | 0,75 | ± 0,07 | **0,35** | **± 0,07** | 0,41 | ± 0,08 | 0,80 | ± 0,90 | 1,00 | ± 0,24 |
| β-alanine | **3,28** | **± 0,17** | 2,37 | ± 0,52 | 1,23 | ± 0,12 | 1,65 | ± 0,50 | 1,00 | ± 0,43 |
| γ-caffeate | 1,62 | ± 0,23 | 0,70 | ± 0,27 | **0,56** | **± 0,14** | **0,48** | **± 0,20** | 1,00 | ± 0,05 |
| citramalate | **1,69** | **± 0,09** | 0,63 | ± 0,16 | 1,11 | ± 0,14 | 0,80 | ± 0,26 | 1,00 | ± 0,14 |
| citrate | 1,29 | ± 0,09 | **0,80** | **± 0,06** | 0,93 | ± 0,11 | 0,90 | ± 0,06 | 1,00 | ± 0,05 |
| cysteine | 0,89 | ± 0,05 | 0,73 | ± 0,10 | 0,74 | ± 0,09 | **0,59** | **± 0,21** | 1,00 | ± 0,10 |
| dehydroascorbate | 0,37 | ± 0,51 | 0,37 | ± 0,16 | 1,12 | ± 0,36 | 0,36 | ± 0,49 | 1,00 | ± 0,68 |
| D-isoascorbate | 0,67 | ± 0,22 | 0,91 | ± 0,23 | 0,94 | ± 0,25 | 0,97 | ± 0,41 | 1,00 | ± 0,13 |
| erythritol | 0,90 | ± 0,20 | 0,64 | ± 0,18 | 0,94 | ± 0,27 | 0,98 | ± 0,35 | 1,00 | ± 0,20 |
| FA 16:0 | 0,92 | ± 0,12 | **0,61** | **± 0,12** | 0,89 | ± 0,08 | 0,84 | ± 0,07 | 1,00 | ± 0,06 |
| FA 18:0 | 0,96 | ± 0,12 | **0,62** | **± 0,19** | 0,99 | ± 0,08 | 0,91 | ± 0,08 | 1,00 | ± 0,07 |
| FA 9,12(Z,Z)-18:2 | 0,91 | ± 0,10 | 0,73 | ± 0,13 | **0,74** | **± 0,07** | **0,64** | **± 0,10** | 1,00 | ± 0,07 |
| fructose-6-P | 0,70 | ± 0,16 | 0,50 | ± 0,06 | 0,59 | ± 0,06 | 0,74 | ± 0,32 | 1,00 | ± 0,36 |
| fructose | 0,24 | ± 0,06 | 0,10 | ± 0,24 | 0,10 | ± 0,16 | 0,10 | ± 0,33 | 1,00 | ± 0,46 |
| fucose | 0,73 | ± 0,17 | 0,48 | ± 0,17 | 0,69 | ± 0,17 | 0,83 | ± 0,56 | 1,00 | ± 0,21 |
| fumarate | 0,45 | ± 0,13 | 0,24 | ± 0,08 | 0,24 | ± 0,33 | 0,20 | ± 0,60 | 1,00 | ± 0,58 |
| GABA | 1,34 | ± 0,06 | 1,48 | ± 0,07 | 1,39 | ± 0,12 | 1,51 | ± 0,38 | 1,00 | ± 0,15 |
| galactonate-1,4-lactone | 0,76 | ± 0,14 | **0,21** | **± 0,16** | **0,29** | **± 0,11** | **0,33** | **± 0,83** | 1,00 | ± 0,19 |
| galactose | 0,40 | ± 0,56 | **0,09** | **± 0,26** | **0,12** | **± 0,26** | **0,13** | **± 0,56** | 1,00 | ± 0,15 |
| galacturonate | 0,97 | ± 0,08 | 0,44 | ± 0,30 | 0,54 | ± 0,11 | **0,46** | **± 0,34** | 1,00 | ± 0,18 |
| glucose-6-P | 0,68 | ± 0,22 | 0,51 | ± 0,08 | 0,58 | ± 0,07 | 0,78 | ± 0,37 | 1,00 | ± 0,47 |
| gluconate | 1,17 | ± 0,06 | 0,87 | ± 0,15 | **0,55** | **± 0,11** | **0,54** | **± 0,09** | 1,00 | ± 0,13 |
| glucose | 0,37 | ± 0,46 | 0,06 | ± 0,45 | 0,07 | ± 0,40 | 0,08 | ± 0,53 | 1,00 | ± 0,63 |
| glutamate | 0,91 | ± 0,08 | 0,81 | ± 0,23 | 1,03 | ± 0,05 | **1,23** | **± 0,07** | 1,00 | ± 0,04 |
| glutamine 4 | 0,28 | ± 0,18 | 0,58 | ± 0,07 | 0,34 | ± 0,32 | 0,47 | ± 0,51 | 1,00 | ± 0,39 |
| glycerate | 1,20 | ± 0,15 | 0,97 | ± 0,14 | 1,11 | ± 0,11 | 1,26 | ± 0,16 | 1,00 | ± 0,23 |
| glycerate-3-P | 0,92 | ± 0,22 | 0,93 | ± 0,19 | 0,96 | ± 0,05 | 1,66 | ± 0,16 | 1,00 | ± 0,27 |
| glycerol | 0,88 | ± 0,29 | 0,92 | ± 0,11 | 0,69 | ± 0,21 | 0,88 | ± 0,42 | 1,00 | ± 0,14 |
| glycerol-1-P | 1,08 | ± 0,18 | 0,99 | ± 0,08 | 0,80 | ± 0,07 | 0,72 | ± 0,11 | 1,00 | ± 0,17 |
| glycine | **0,44** | **± 0,10** | **0,48** | **± 0,21** | **0,24** | **± 0,13** | **0,34** | **± 0,49** | 1,00 | ± 0,10 |
| homoserine | 0,75 | ± 0,16 | **0,53** | **± 0,13** | **0,37** | **± 0,25** | **0,32** | **± 0,46** | 1,00 | ± 0,13 |

| | | | | | | | | | |
|---|---|---|---|---|---|---|---|---|---|
| inositol | 1,00 | ± 0,10 | 0,58 | ± 0,12 | 1,16 | ± 0,07 | 1,11 | ± 0,25 | 1,00 | ± 0,22 |
| inositol-1-P | 1,32 | ± 0,05 | 0,75 | ± 0,04 | 1,34 | ± 0,11 | 0,92 | ± 0,27 | 1,00 | ± 0,16 |
| isocitrate | 1,57 | ± 0,05 | 1,10 | ± 0,11 | 1,63 | ± 0,14 | 1,39 | ± 0,29 | 1,00 | ± 0,21 |
| **isoleucine** | **0,30** | **± 0,30** | **0,22** | **± 0,18** | **0,18** | **± 0,15** | **0,23** | **± 0,38** | 1,00 | ± 0,10 |
| L-ascorbate | 1,12 | ± 0,18 | 1,00 | ± 0,12 | 1,13 | ± 0,19 | 0,86 | ± 0,27 | 1,00 | ± 0,07 |
| leucine | 0,57 | ± 0,18 | 0,59 | ± 0,31 | **0,28** | **± 0,15** | **0,26** | **± 0,12** | 1,00 | ± 0,21 |
| **lysine** | **0,45** | **± 0,15** | 0,62 | ± 0,14 | **0,32** | **± 0,11** | **0,44** | **± 0,35** | 1,00 | ± 0,19 |
| malate | 0,63 | ± 0,21 | **0,24** | **± 0,20** | **0,27** | **± 0,19** | **0,21** | **± 0,50** | 1,00 | ± 0,25 |
| maleate | 0,81 | ± 0,29 | 0,54 | ± 0,31 | 0,39 | ± 0,37 | 0,36 | ± 0,70 | 1,00 | ± 0,27 |
| maltose | 1,02 | ± 0,28 | **0,50** | **± 0,13** | **0,36** | **± 0,16** | **0,44** | **± 0,55** | 1,00 | ± 0,14 |
| mannitol | 0,64 | ± 0,04 | **0,46** | **± 0,05** | **0,39** | **± 0,22** | 0,58 | ± 0,38 | 1,00 | ± 0,16 |
| mannose | 0,73 | ± 0,44 | **0,15** | **± 0,18** | **0,17** | **± 0,23** | 0,25 | ± 0,86 | 1,00 | ± 0,29 |
| melezitose | 1,33 | ± 0,04 | 0,87 | ± 0,10 | 1,50 | ± 0,12 | 1,29 | ± 0,28 | 1,00 | ± 0,16 |
| methionine | 0,72 | ± 0,18 | 0,88 | ± 0,25 | 0,62 | ± 0,32 | 0,86 | ± 0,49 | 1,00 | ± 0,13 |
| **ornithine** | **0,51** | **± 0,12** | 0,83 | ± 0,21 | 0,53 | ± 0,25 | 0,66 | ± 0,33 | 1,00 | ± 0,14 |
| phenylalanine | 0,62 | ± 0,15 | 1,05 | ± 0,11 | 0,78 | ± 0,16 | 0,87 | ± 0,33 | 1,00 | ± 0,19 |
| phosphate | 1,48 | ± 0,15 | 0,90 | ± 0,17 | 1,42 | ± 0,07 | 1,31 | ± 0,25 | 1,00 | ± 0,14 |
| proline | 0,29 | ± 0,16 | 0,45 | ± 0,43 | 0,16 | ± 0,32 | 0,15 | ± 0,18 | 1,00 | ± 0,55 |
| **putrescine** | **1,73** | **± 0,09** | 1,43 | ± 0,06 | 1,27 | ± 0,06 | 1,21 | ± 0,21 | 1,00 | ± 0,19 |
| **quinate** | **1,78** | **± 0,09** | 1,18 | ± 0,10 | **0,59** | **± 0,08** | 0,62 | ± 0,56 | 1,00 | ± 0,11 |
| raffinose | 0,59 | ± 0,06 | **0,24** | **± 0,16** | **0,19** | **± 0,14** | **0,22** | **± 0,62** | 1,00 | ± 0,16 |
| saccharate | 1,32 | ± 0,20 | 1,31 | ± 0,09 | 1,11 | ± 0,09 | 1,08 | ± 0,33 | 1,00 | ± 0,12 |
| serine | 1,07 | ± 0,21 | 0,88 | ± 0,41 | 0,51 | ± 0,32 | 0,18 | ± 0,23 | 1,00 | ± 0,37 |
| shikimate | 0,74 | ± 0,11 | 0,79 | ± 0,13 | 0,54 | ± 0,13 | 3,99 | ± 0,80 | 1,00 | ± 0,19 |
| sorbitol/galactitol | 0,67 | ± 0,10 | 0,61 | ± 0,20 | 0,79 | ± 0,13 | 0,96 | ± 0,41 | 1,00 | ± 0,23 |
| spermidine | 0,85 | ± 0,31 | 1,06 | ± 0,09 | 1,05 | ± 0,10 | 1,15 | ± 0,13 | 1,00 | ± 0,04 |
| succinate | 2,01 | ± 0,19 | 0,43 | ± 0,25 | **0,21** | **± 0,36** | **0,10** | **± 1,21** | 1,00 | ± 0,23 |
| sucrose | 1,47 | ± 0,15 | 0,56 | ± 0,24 | 0,65 | ± 0,17 | **0,31** | **± 0,30** | 1,00 | ± 0,17 |
| t-4-HO-proline | 0,50 | ± 0,16 | 0,65 | ± 0,29 | 0,53 | ± 0,26 | 0,42 | ± 1,15 | 1,00 | ± 0,24 |
| threonate | 0,99 | ± 0,05 | 0,75 | ± 0,06 | 0,82 | ± 0,13 | **0,58** | **± 0,15** | 1,00 | ± 0,10 |
| threonine | 1,21 | ± 0,34 | 2,21 | ± 0,50 | 0,75 | ± 0,17 | **0,67** | **± 0,18** | 1,00 | ± 0,11 |
| trehalose | 1,00 | ± 0,28 | 0,46 | ± 0,08 | 0,61 | ± 0,16 | 0,83 | ± 0,32 | 1,00 | ± 0,27 |
| tryptophan | 0,79 | ± 0,30 | 1,51 | ± 0,29 | 0,83 | ± 0,23 | 0,82 | ± 0,23 | 1,00 | ± 0,33 |
| tyramine | 1,09 | ± 0,12 | 0,99 | ± 0,24 | 1,66 | ± 0,24 | 1,06 | ± 0,38 | 1,00 | ± 0,24 |
| tyrosine | 0,55 | ± 0,08 | 1,14 | ± 0,15 | 0,67 | ± 0,15 | 1,33 | ± 0,51 | 1,00 | ± 0,31 |
| **valine** | **0,60** | **± 0,10** | 0,70 | ± 0,17 | **0,27** | **± 0,32** | 0,35 | ± 0,43 | 1,00 | ± 0,04 |
| xylose/arabinose | 0,63 | ± 0,28 | **0,16** | **± 0,36** | **0,09** | **± 0,23** | 0,08 | ± 0,84 | 1,00 | ± 0,21 |

## (b)    Potato transgenic lines

| | INV | SE | SP | SE | 10 | SE |
|---|---|---|---|---|---|---|
| 3PGA | 1,00 | ± 0,00 | 146,67 | ± 0,01 | 1,00 | ± 0,00 |
| 5-oxoproline | 0,68 | ± 0,09 | 0,28 | ± 0,24 | 1,00 | ± 0,07 |
| 6-P-gluconate | 556,67 | ± 0,17 | 206,67 | ± 0,10 | 1,00 | ± 0,00 |
| α-ketoglutarate | 1,00 | ± 0,00 | 4,67 | ± 0,04 | 1,00 | ± 0,00 |
| alanine | 2,08 | ± 0,10 | 1,29 | ± 0,01 | 1,00 | ± 0,12 |
| arginine | 2,11 | ± 0,16 | **3,63** | **± 0,08** | 1,00 | ± 0,10 |
| asparagine | 1,17 | ± 0,06 | **0,10** | **± 0,02** | 1,00 | ± 0,15 |
| aspartate | **1,20** | **± 0,02** | 0,93 | ± 0,03 | 1,00 | ± 0,11 |
| β-alanine | 0,84 | ± 0,05 | 1,00 | ± 0,05 | 1,00 | ± 0,11 |
| citarte | **1,03** | **± 0,06** | 1,32 | ± 0,02 | 1,00 | ± 0,03 |
| cysteine | 2,20 | ± 0,14 | **1,90** | **± 0,08** | 1,00 | ± 0,05 |
| fructose-6-P | 27,00 | ± 0,10 | 10,17 | ± 0,04 | 1,00 | ± 0,16 |
| fructose | 2,55 | ± 0,07 | 1,09 | ± 0,08 | 1,00 | ± 0,26 |
| fumarate | 0,23 | ± 0,06 | 0,11 | ± 0,00 | 1,00 | ± 0,30 |
| GABA | 1,42 | ± 0,05 | 2,09 | ± 0,05 | 1,00 | ± 0,07 |
| galactinol | 1,00 | ± 0,00 | **1,00** | **± 0,00** | 1,00 | ± 0,00 |
| galactose | 0,26 | ± 0,12 | **2,96** | **± 0,16** | 1,00 | ± 0,35 |
| glucose-6-P | 23,23 | ± 0,08 | 13,23 | ± 0,04 | 1,00 | ± 0,10 |
| gluconate | 66,67 | ± 0,10 | 1,00 | ± 0,00 | 1,00 | ± 0,00 |
| glucose | 5,11 | ± 0,11 | 0,01 | ± 0,16 | 1,00 | ± 0,45 |
| glucoronate | 1,00 | ± 0,00 | 1,00 | ± 0,00 | 1,00 | ± 0,00 |
| glutamate | 1,38 | ± 0,02 | 0,93 | ± 0,03 | 1,00 | ± 0,02 |
| glutmine | 0,43 | ± 0,33 | 0,10 | ± 0,20 | 1,00 | ± 0,14 |
| glutarate | 1,00 | ± 0,00 | **1,00** | **± 0,00** | 1,00 | ± 0,00 |
| glycerate | 6,33 | ± 0,06 | **15,33** | **± 0,15** | 1,00 | ± 0,05 |
| glycerol | 1,00 | ± 0,00 | 23,33 | ± 0,08 | 1,00 | ± 0,00 |
| glycine | 0,84 | ± 0,09 | 0,75 | ± 0,06 | 1,00 | ± 0,06 |
| histidine | 1,00 | ± 0,00 | 2083,33 | ± 0,12 | 1,00 | ± 0,00 |
| homocysteine | 1,00 | ± 0,00 | 4,67 | ± 0,14 | 1,00 | ± 0,00 |
| homoglutamine | 1,00 | ± 0,00 | 6,33 | ± 0,16 | 1,00 | ± 0,00 |
| homoserine | 0,50 | ± 0,21 | 0,50 | ± 0,08 | 1,00 | ± 0,10 |
| inositol | 0,21 | ± 0,08 | 0,18 | ± 0,04 | 1,00 | ± 0,14 |
| isocitrate | 0,67 | ± 0,12 | 0,90 | ± 0,02 | 1,00 | ± 0,03 |
| isoleucine | 1,16 | ± 0,14 | 0,93 | ± 0,15 | 1,00 | ± 0,06 |
| isomaltose | 13,33 | ± 0,14 | 1,00 | ± 0,00 | 1,00 | ± 0,00 |
| L-ascorbate | **0,73** | **± 0,33** | **0,68** | **± 0,07** | 1,00 | ± 0,26 |
| leucine | 1,70 | ± 0,17 | **1,21** | **± 0,15** | 1,00 | ± 0,12 |
| lysine | 0,62 | ± 0,19 | 2,22 | ± 0,09 | 1,00 | ± 0,04 |
| malate | 2,05 | ± 0,07 | 1,69 | ± 0,08 | 1,00 | ± 0,02 |
| maltitol | 586,67 | ± 0,14 | 1,00 | ± 0,00 | 1,00 | ± 0,00 |
| maltose | **8043,33** | **± 0,02** | **1,00** | **± 0,00** | 1,00 | ± 0,00 |
| mannitol | 2,94 | ± 0,06 | 1,46 | ± 0,04 | 1,00 | ± 0,04 |
| mannose | 10,18 | ± 0,10 | 0,05 | ± 0,00 | 1,00 | ± 0,30 |
| methionine | **0,84** | **± 0,10** | 1,21 | ± 0,06 | 1,00 | ± 0,05 |
| norleucine | 0,72 | ± 0,16 | **1,32** | **± 0,14** | 1,00 | ± 0,06 |
| norvaline | 0,58 | ± 0,10 | 1,03 | ± 0,07 | 1,00 | ± 0,07 |

| | | | | | | |
|---|---|---|---|---|---|---|
| ornithine | 1,09 | ± 0,15 | **2,29** | **± 0,16** | 1,00 | ± 0,26 |
| oxalate | 1,00 | ± 0,00 | **1776,67** | **± 0,08** | 1,00 | ± 0,00 |
| phenylalanine | 1,77 | ± 0,07 | **1,47** | **± 0,02** | 1,00 | ± 0,07 |
| phosphate | 0,90 | ± 0,05 | 1,40 | ± 0,01 | 1,00 | ± 0,03 |
| phosphoethanol amine | 1,11 | ± 0,07 | 0,61 | ± 0,05 | 1,00 | ± 0,16 |
| proline | **0,82** | **± 0,09** | 0,59 | ± 0,05 | 1,00 | ± 0,03 |
| quinate | 1,42 | ± 0,02 | 1,84 | ± 0,08 | 1,00 | ± 0,08 |
| raffinose | 1,00 | ± 0,00 | 1,00 | ± 0,00 | 1,00 | ± 0,00 |
| serine | 3,12 | ± 0,05 | 1,90 | ± 0,01 | 1,00 | ± 0,09 |
| shikimate | 2,67 | ± 0,00 | 1,56 | ± 0,10 | 1,00 | ± 0,21 |
| spermidine | 2,10 | ± 0,05 | 2,30 | ± 0,06 | 1,00 | ± 0,14 |
| succinate | 2,23 | ± 0,01 | 1,10 | ± 0,07 | 1,00 | ± 0,25 |
| sucrose | 0,11 | ± 0,04 | 0,58 | ± 0,17 | 1,00 | ± 0,21 |
| threonate | 1,67 | ± 0,03 | 1,13 | ± 0,04 | 1,00 | ± 0,17 |
| threonine | 1,05 | ± 0,09 | 1,16 | ± 0,04 | 1,00 | ± 0,08 |
| trehalose | 193,33 | ± 0,14 | 1,00 | ± 0,00 | 1,00 | ± 0,00 |
| tryptophan | 266,67 | ± 0,33 | 1996,67 | ± 0,15 | 1,00 | ± 0,00 |
| tyrosine | 1,68 | ± 0,13 | 1,53 | ± 0,06 | 1,00 | ± 0,16 |
| valine | 0,90 | ± 0,10 | 0,53 | ± 0,06 | 1,00 | ± 0,02 |

## Table 3

| EST ID | ANNOTATION | MB | SPEARMAN | sig0.001 | NOP |
|---|---|---|---|---|---|
| CLEC13O19 | HD-Zip protein [Arabidopsis thaliana] | GABA | 0,5430769 | 0 | 25 |
| CLEC13O19 | HD-Zip protein [Arabidopsis thaliana] | INOSITOL | -0,7853846 | 1 | 25 |
| CLEC13O19 | HD-Zip protein [Arabidopsis thaliana] | SPERMIDINE | 0,5892308 | 0 | 25 |
| CLEC13O19 | HD-Zip protein [Arabidopsis thaliana] | TRYPTOPHAN | 0,5913044 | 0 | 24 |
| CLEC13O19 | HD-Zip protein [Arabidopsis thaliana] | TYROSINE | 0,5638462 | 0 | 25 |
| CLEC15K2 | CCAAT box binding factor/ transcription factor Hap2a {Arabidopsis thaliana}PIR|T49898|T49898 CCAAT | GABA | 0,5923077 | 0 | 25 |
| CLEC15K2 | CCAAT box binding factor/ transcription factor Hap2a {Arabidopsis thaliana}PIR|T49898|T49898 CCAAT | INOSITOL | -0,7946154 | 1 | 25 |
| CLEC15K2 | CCAAT box binding factor/ transcription factor Hap2a {Arabidopsis thaliana}PIR|T49898|T49898 CCAAT | SPERMIDINE | 0,6169231 | 0 | 25 |
| CLEC15K2 | CCAAT box binding factor/ transcription factor Hap2a {Arabidopsis thaliana}PIR|T49898|T49898 CCAAT | TRYPTOPHAN | 0,6321739 | 1 | 24 |
| CLEC15K2 | CCAAT box binding factor/ transcription factor Hap2a {Arabidopsis thaliana}PIR|T49898|T49898 CCAAT | TYROSINE | 0,5476923 | 0 | 25 |
| CLEC21H24 | osmotic stress-induced zinc-finger protein {Nicotiana tabacum}PIR|T01985|T01985 zinc-finger protein | GABA | 0,5176923 | 0 | 25 |
| CLEC21H24 | osmotic stress-induced zinc-finger protein {Nicotiana tabacum}PIR|T01985|T01985 zinc-finger protein | INOSITOL | -0,7461538 | 1 | 25 |
| CLEC21H24 | osmotic stress-induced zinc-finger protein {Nicotiana tabacum}PIR|T01985|T01985 zinc-finger protein | QUINATE | 0,5338461 | 0 | 25 |
| CLEC21H24 | osmotic stress-induced zinc-finger protein {Nicotiana tabacum}PIR|T01985|T01985 zinc-finger protein | SERINE | 0,5053846 | 0 | 25 |
| CLEC21H24 | osmotic stress-induced zinc-finger protein {Nicotiana tabacum}PIR|T01985|T01985 zinc-finger protein | SPERMIDINE | 0,6 | 0 | 25 |
| CLEC21H24 | osmotic stress-induced zinc-finger protein {Nicotiana tabacum}PIR|T01985|T01985 zinc-finger protein | TRYPTOPHAN | 0,6026087 | 0 | 24 |
| CLEC21H24 | osmotic stress-induced zinc-finger protein {Nicotiana tabacum}PIR|T01985|T01985 zinc-finger protein | TYROSINE | 0,5592307 | 0 | 25 |
| CLEC28H13 | homeodomain protein {Malus x domestica} | GABA | 0,5084615 | 0 | 25 |

| CLEC28H13 | homeodomain protein {Malus x domestica} | SPERMIDINE | 0,5938461 | 0 | 25 |
|---|---|---|---|---|---|
| CLEC29A19 | alanine aminotransferase {Arabidopsis thaliana} | INOSITOL | -0,7061539 | 1 | 25 |
| CLEC29A19 | alanine aminotransferase {Arabidopsis thaliana} | SPERMIDINE | 0,5715384 | 0 | 25 |
| CLEC29A19 | alanine aminotransferase {Arabidopsis thaliana} | TRYPTOPHAN | 0,5208696 | 0 | 24 |
| CLEC32P10 | putative C3HC4-type RING zinc finger protein {Arabidopsis thaliana}PIR\|B84813\|B84813 probable RING | GABA | 0,5238461 | 0 | 25 |
| CLEC32P10 | putative C3HC4-type RING zinc finger protein {Arabidopsis thaliana}PIR\|B84813\|B84813 probable RING | INOSITOL | -0,5276923 | 0 | 25 |
| CLEC35G8 | CYTOCHROME P450 83B1 (EC 1.14.-.-).GP\|3164126\|dbj\|BAA28531.1\|\|D78598 cytochrome P450 monooxygenase | INOSITOL | -0,5130769 | 0 | 25 |
| CLEC35G8 | CYTOCHROME P450 83B1 (EC 1.14.-.-).GP\|3164126\|dbj\|BAA28531.1\|\|D78598 cytochrome P450 monooxygenase | SPERMIDINE | 0,5207692 | 0 | 25 |
| CLEC36G5 | hydroxycinnamoyl-CoA:tyramine N-(hydroxycinnamoyl)transferase {Capsicum annuum} | INOSITOL | -0,7607692 | 1 | 25 |
| CLEC36G5 | hydroxycinnamoyl-CoA:tyramine N-(hydroxycinnamoyl)transferase {Capsicum annuum} | MELEZITOSE | -0,6210526 | 0 | 19 |
| CLEC36G5 | hydroxycinnamoyl-CoA:tyramine N-(hydroxycinnamoyl)transferase {Capsicum annuum} | TYROSINE | 0,6007692 | 0 | 25 |
| CLEC38H15 | NADH-dependent glutamate synthase {Arabidopsis thaliana} | TYROSINE | -0,5807692 | 0 | 25 |
| CLEC39M7 | NADH-dependent glutamate synthase {Arabidopsis thaliana} | CITRAMALATE | 0,5754386 | 0 | 19 |
| CLEC40C16 | ethylene-responsive transcriptional coactivator | GABA | 0,5492308 | 0 | 25 |
| CLEC40C16 | ethylene-responsive transcriptional coactivator | INOSITOL | -0,81 | 1 | 25 |
| CLEC40C16 | ethylene-responsive transcriptional coactivator | MELEZITOSE | -0,5929825 | 0 | 19 |
| CLEC40C16 | ethylene-responsive transcriptional coactivator | SPERMIDINE | 0,6330769 | 1 | 25 |
| CLEC40C16 | ethylene-responsive transcriptional coactivator | THREONATE | 0,5338461 | 0 | 25 |
| CLEC40C16 | ethylene-responsive transcriptional coactivator | THREONINE | 0,5539131 | 0 | 24 |
| CLEC40C16 | ethylene-responsive transcriptional coactivator | TRYPTOPHAN | 0,5443478 | 0 | 24 |
| CLEC40C16 | ethylene-responsive transcriptional coactivator | TYROSINE | 0,6223077 | 1 | 25 |
| CLEC4A12 | hydroxycinnamoyl-CoA:tyramine N-(hydroxycinnamoyl)transferase {Capsicum annuum} | CYSTEINE | 0,5385376 | 0 | 23 |
| CLEC4A12 | hydroxycinnamoyl-CoA:tyramine N-(hydroxycinnamoyl)transferase {Capsicum annuum} | INOSITOL | -0,6476923 | 1 | 25 |

| CLEC4A12 | hydroxycinnamoyl-CoA:tyramine N-(hydroxycinnamoyl)transferase {Capsicum annuum} | LYSINE | 0,5092308 | 0 | 25 |
|---|---|---|---|---|---|
| CLEC4A12 | hydroxycinnamoyl-CoA:tyramine N-(hydroxycinnamoyl)transferase {Capsicum annuum} | MELEZITOSE | -0,6210526 | 0 | 19 |
| CLEC4A12 | hydroxycinnamoyl-CoA:tyramine N-(hydroxycinnamoyl)transferase {Capsicum annuum} | TYROSINE | 0,5592307 | 0 | 25 |
| CLEC6D16 | glucosyltransferase-like protein {Arabidopsis thaliana} | INOSITOL | -0,5807692 | 0 | 25 |
| CLEC6K9 | lysine-ketoglutarate reductase/saccharopine dehydrogenase bifunctional enzyme {Arabidopsis thaliana} | INOSITOL | -0,5907692 | 0 | 25 |
| CLEC6K9 | lysine-ketoglutarate reductase/saccharopine dehydrogenase bifunctional enzyme {Arabidopsis thaliana} | SPERMIDINE | 0,5407692 | 0 | 25 |
| CLEC6K9 | lysine-ketoglutarate reductase/saccharopine dehydrogenase bifunctional enzyme {Arabidopsis thaliana} | TYROSINE | 0,5076923 | 0 | 25 |
| CLED11B1 | homeodomain protein | INOSITOL | -0,6515385 | 1 | 25 |
| CLED11B1 | homeodomain protein | SPERMIDINE | 0,6030769 | 0 | 25 |
| CLED11B1 | homeodomain protein | TYROSINE | 0,62 | 1 | 25 |
| CLED11B18 | lysine-ketoglutarate reductase/saccharopine dehydrogenase bifunctional enzyme {Arabidopsis thaliana} | GABA | 0,5669231 | 0 | 25 |
| CLED11B18 | lysine-ketoglutarate reductase/saccharopine dehydrogenase bifunctional enzyme {Arabidopsis thaliana} | INOSITOL | -0,6469231 | 1 | 25 |
| CLED11B18 | lysine-ketoglutarate reductase/saccharopine dehydrogenase bifunctional enzyme {Arabidopsis thaliana} | SPERMIDINE | 0,5830769 | 0 | 25 |
| CLED11B18 | lysine-ketoglutarate reductase/saccharopine dehydrogenase bifunctional enzyme {Arabidopsis thaliana} | THREONATE | 0,5084615 | 0 | 25 |
| CLED11B18 | lysine-ketoglutarate reductase/saccharopine dehydrogenase bifunctional enzyme {Arabidopsis thaliana} | TYROSINE | 0,5184615 | 0 | 25 |
| CLED11B9 | cytochrome p450 lxxia4 {Solanum melongena}SP\|P37117\|C714_SOLME CYTOCHROME P450 71A4 (EC 1.14.-.-) ( | INOSITOL | -0,6653846 | 1 | 25 |
| CLED11B9 | cytochrome p450 lxxia4 {Solanum melongena}SP\|P37117\|C714_SOLME CYTOCHROME P450 71A4 (EC 1.14.-.-) ( | TYROSINE | 0,52 | 0 | 25 |
| CLED15E8 | putative homeodomain transcription factor {Arabidopsis thaliana}PIR\|H84774\|H84774 probable homeodom | GABA | 0,5846154 | 0 | 25 |

| | | | | |
|---|---|---|---|---|---|
| CLED15E8 | putative homeodomain transcription factor {Arabidopsis thaliana}PIR\|H84774\|H84774 probable homeodom | INOSITOL | -0,7115384 | 1 | 25 |
| CLED15E8 | putative homeodomain transcription factor {Arabidopsis thaliana}PIR\|H84774\|H84774 probable homeodom | SPERMIDINE | 0,6315385 | 1 | 25 |
| CLED15E8 | putative homeodomain transcription factor {Arabidopsis thaliana}PIR\|H84774\|H84774 probable homeodom | TRYPTOPHAN | 0,546087 | 0 | 24 |
| CLED15E8 | putative homeodomain transcription factor {Arabidopsis thaliana}PIR\|H84774\|H84774 probable homeodom | TYROSINE | 0,5507692 | 0 | 25 |
| CLED15J6 | Zn finger protein {Nicotiana tabacum}GP\|1360078\|emb\|CAA66601.1\|\|X97942 Zn finger protein {Nicotiana | INOSITOL | -0,5330769 | 0 | 25 |
| CLED16D1 | NADH-ubiquinone oxidoreductase 20 kDa subunit precursor {Solanum tuberosum}SP\|Q43844\|NUKM_SOLTU NAD | INOSITOL | -0,7161539 | 1 | 25 |
| CLED16D1 | NADH-ubiquinone oxidoreductase 20 kDa subunit precursor {Solanum tuberosum}SP\|Q43844\|NUKM_SOLTU NAD | MELEZITOSE | -0,6385965 | 0 | 19 |
| CLED16D1 | NADH-ubiquinone oxidoreductase 20 kDa subunit precursor {Solanum tuberosum}SP\|Q43844\|NUKM_SOLTU NAD | SPERMIDINE | 0,56 | 0 | 25 |
| CLED16D1 | NADH-ubiquinone oxidoreductase 20 kDa subunit precursor {Solanum tuberosum}SP\|Q43844\|NUKM_SOLTU NAD | THREONINE | 0,5391304 | 0 | 24 |
| CLED16D1 | NADH-ubiquinone oxidoreductase 20 kDa subunit precursor {Solanum tuberosum}SP\|Q43844\|NUKM_SOLTU NAD | TYROSINE | 0,6338462 | 1 | 25 |
| CLED21K4 | "putative ABC transporter; 60211-54925 {Arabidopsis thaliana}PIR\|E96742\|E96742 probable ABC transpor" | INOSITOL | 0,6238462 | 1 | 25 |
| CLED21K4 | "putative ABC transporter; 60211-54925 {Arabidopsis thaliana}PIR\|E96742\|E96742 probable ABC transpor" | SPERMIDINE | -0,5253846 | 0 | 25 |
| CLED21K4 | "putative ABC transporter; 60211-54925 {Arabidopsis thaliana}PIR\|E96742\|E96742 probable ABC transpor" | TYROSINE | -0,5892308 | 0 | 25 |
| CLED22C14 | bHLH transcription factor GBOF-1 {Tulipa gesneriana} | GABA | 0,5623077 | 0 | 25 |
| CLED22C14 | bHLH transcription factor GBOF-1 {Tulipa gesneriana} | INOSITOL | -0,53 | 0 | 25 |
| CLED24N6 | acetylornithine aminotransferase precursor {Alnus glutinosa}SP\|O04866\|ARGD_ALNGL ACETYLORNITHINE AM | INOSITOL | -0,7492307 | 1 | 25 |
| CLED24N6 | acetylornithine aminotransferase precursor {Alnus glutinosa}SP\|O04866\|ARGD_ALNGL ACETYLORNITHINE AM | MELEZITOSE | -0,6631579 | 0 | 19 |

EP 1 454 993 A1

EP 1 454 993 A1

| CLED24N6 | acetylornithine aminotransferase precursor {Alnus glutinosa}SP\|O04866\|ARGD_ALNGL ACETYLORNITHINE AM | SPERMIDINE | 0,5492308 | 0 | 25 |
|---|---|---|---|---|---|
| CLED24N6 | acetylornithine aminotransferase precursor {Alnus glutinosa}SP\|O04866\|ARGD_ALNGL ACETYLORNITHINE AM | TRYPTOPHAN | 0,5547826 | 0 | 24 |
| CLED24N6 | acetylornithine aminotransferase precursor {Alnus glutinosa}SP\|O04866\|ARGD_ALNGL ACETYLORNITHINE AM | TYROSINE | 0,5884615 | 0 | 25 |
| CLED25E6 | UDP-glucose:salicylic acid glucosyltransferase {Nicotiana tabacum} | INOSITOL | -0,5915385 | 0 | 25 |
| CLED25E6 | UDP-glucose:salicylic acid glucosyltransferase {Nicotiana tabacum} | SPERMIDINE | 0,5253846 | 0 | 25 |
| CLED28H13 | putative cytochrome P450 {Solanum chacoense}SP\|P93530\|C7D6_SOLCH CYTOCHROME P450 71D6 (EC 1.14.-.-) | INOSITOL | -0,5284615 | 0 | 25 |
| CLED28H13 | putative cytochrome P450 {Solanum chacoense}SP\|P93530\|C7D6_SOLCH CYTOCHROME P450 71D6 (EC 1.14.-.-) | MELEZITOSE | -0,577193 | 0 | 19 |
| CLED28N11 | Dof zinc finger protein {Arabidopsis thaliana}GP\|9280230\|dbj\|BAB01720.1\|\|AB023045 Dof zinc finger p | INOSITOL | -0,6361538 | 1 | 25 |
| CLED28N11 | Dof zinc finger protein {Arabidopsis thaliana}GP\|9280230\|dbj\|BAB01720.1\|\|AB023045 Dof zinc finger p | TYROSINE | 0,5415385 | 0 | 25 |
| CLED30B19 | flavanone 3-hydroxylase-like protein {Arabidopsis thaliana} | INOSITOL | -0,5384616 | 0 | 25 |
| CLED30B19 | flavanone 3-hydroxylase-like protein {Arabidopsis thaliana} | TYROSINE | 0,5107692 | 0 | 25 |
| CLED30J21 | S-adenosylmethionine:2-demethylmenaquinone methyltransferase-like protein {Arabidopsis thaliana} | INOSITOL | -0,6807692 | 1 | 25 |
| CLED30J21 | S-adenosylmethionine:2-demethylmenaquinone methyltransferase-like protein {Arabidopsis thaliana} | SPERMIDINE | 0,6107692 | 0 | 25 |
| CLED30J21 | S-adenosylmethionine:2-demethylmenaquinone methyltransferase-like protein {Arabidopsis thaliana} | SUCROSE | -0,5191304 | 0 | 24 |
| CLED30J21 | S-adenosylmethionine:2-demethylmenaquinone methyltransferase-like protein {Arabidopsis thaliana} | TYROSINE | 0,6738461 | 1 | 25 |
| CLED30M20 | putative homeodomain transcription factor {Arabidopsis thaliana}PIR\|H84774\|H84774 probable homeodom | GABA | 0,5469231 | 0 | 25 |
| CLED30M20 | putative homeodomain transcription factor {Arabidopsis thaliana}PIR\|H84774\|H84774 probable homeodom | INOSITOL | -0,7423077 | 1 | 25 |
| CLED30M20 | putative homeodomain transcription factor {Arabidopsis thaliana}PIR\|H84774\|H84774 probable homeodom | NORLEUCINE | -0,9428571 | 0 | 6 |

| CLED30M20 | putative homeodomain transcription factor {Arabidopsis thaliana}PIR\|H84774\|H84774 probable homeodom | ORNITHINE | -1 | 1 | 6 |
|---|---|---|---|---|---|
| CLED30M20 | putative homeodomain transcription factor {Arabidopsis thaliana}PIR\|H84774\|H84774 probable homeodom | SERINE | 0,5069231 | 0 | 25 |
| CLED30M20 | putative homeodomain transcription factor {Arabidopsis thaliana}PIR\|H84774\|H84774 probable homeodom | SPERMIDINE | 0,6007692 | 0 | 25 |
| CLED30M20 | putative homeodomain transcription factor {Arabidopsis thaliana}PIR\|H84774\|H84774 probable homeodom | THREONATE | 0,5438461 | 0 | 25 |
| CLED30M20 | putative homeodomain transcription factor {Arabidopsis thaliana}PIR\|H84774\|H84774 probable homeodom | TRYPTOPHAN | 0,5191304 | 0 | 24 |
| CLED30M20 | putative homeodomain transcription factor {Arabidopsis thaliana}PIR\|H84774\|H84774 probable homeodom | TYROSINE | 0,6215385 | 1 | 25 |
| CLED34H6 | glucosyltransferase-like protein {Arabidopsis thaliana} | FUCOSE | -0,5807018 | 0 | 19 |
| CLED34H6 | glucosyltransferase-like protein {Arabidopsis thaliana} | GALACTOSE | -0,5846154 | 0 | 25 |
| CLED34H6 | glucosyltransferase-like protein {Arabidopsis thaliana} | INOSITOL | -0,6592308 | 1 | 25 |
| CLED34H6 | glucosyltransferase-like protein {Arabidopsis thaliana} | SPERMIDINE | 0,5346154 | 0 | 25 |
| CLED34H6 | glucosyltransferase-like protein {Arabidopsis thaliana} | TRYPTOPHAN | 0,56 | 0 | 24 |
| CLED34H6 | glucosyltransferase-like protein {Arabidopsis thaliana} | TYROSINE | 0,5176923 | 0 | 25 |
| CLED4O17 | putative cytochrome P450 {Solanum chacoense}SP\|P93530\|C7D6_SOLCH CYTOCHROME P450 71D6 (EC 1.14.-.-) | GABA | 0,5346154 | 0 | 25 |
| CLED4O17 | putative cytochrome P450 {Solanum chacoense}SP\|P93530\|C7D6_SOLCH CYTOCHROME P450 71D6 (EC 1.14.-.-) | INOSITOL | -0,67 | 1 | 25 |
| CLED4O9 | transcription factor CRC {Arabidopsis thaliana}GP\|12325076\|gb\|AAG52485.1\|AC018364_3\|AC018364 transc | INOSITOL | -0,5423077 | 0 | 25 |
| CLED5L18 | "ornithine carbamoyltransferase; OCTase {Canavalia lineata}GP\|12003188\|gb\|AAG43481.1\|AF203688_1\|AF20" | CYSTEINE | 0,5306324 | 0 | 23 |
| CLED5L18 | "ornithine carbamoyltransferase; OCTase {Canavalia lineata}GP\|12003188\|gb\|AAG43481.1\|AF203688_1\|AF20" | FA 16:0 | -0,6305469 | 0 | 18 |
| CLED5L18 | "ornithine carbamoyltransferase; OCTase {Canavalia lineata}GP\|12003188\|gb\|AAG43481.1\|AF203688_1\|AF20" | GABA | 0,5630769 | 0 | 25 |
| CLED5L18 | "ornithine carbamoyltransferase; OCTase {Canavalia lineata}GP\|12003188\|gb\|AAG43481.1\|AF203688_1\|AF20" | INOSITOL | -0,7792308 | 1 | 25 |

| CLED5L18 | "ornithine carbamoyltransferase; OCTase {Canavalia lineata}GP\|12003188\|gb\|AAG43481.1\|AF203688_1\|AF20" | MELEZITOSE | -0,6 | 0 | 19 |
|---|---|---|---|---|---|
| CLED5L18 | "ornithine carbamoyltransferase; OCTase {Canavalia lineata}GP\|12003188\|gb\|AAG43481.1\|AF203688_1\|AF20" | TRYPTOPHAN | 0,7104348 | 1 | 24 |
| CLED5L18 | "ornithine carbamoyltransferase; OCTase {Canavalia lineata}GP\|12003188\|gb\|AAG43481.1\|AF203688_1\|AF20" | TYROSINE | 0,5592307 | 0 | 25 |
| CLED6K9 | alpha-glucosidase {Solanum tuberosum subsp. tuberosum} | THREONATE | 0,5107692 | 0 | 25 |
| CLED6P9 | "ornithine carbamoyltransferase; OCTase {Canavalia lineata}GP\|12003188\|gb\|AAG43481.1\|AF203688_1\|AF20" | ALANINE | 0,5553846 | 0 | 25 |
| CLED6P9 | "ornithine carbamoyltransferase; OCTase {Canavalia lineata}GP\|12003188\|gb\|AAG43481.1\|AF203688_1\|AF20" | CYSTEINE | 0,5375494 | 0 | 23 |
| CLED6P9 | "ornithine carbamoyltransferase; OCTase {Canavalia lineata}GP\|12003188\|gb\|AAG43481.1\|AF203688_1\|AF20" | INOSITOL | -0,8292308 | 1 | 25 |
| CLED6P9 | "ornithine carbamoyltransferase; OCTase {Canavalia lineata}GP\|12003188\|gb\|AAG43481.1\|AF203688_1\|AF20" | LYSINE | 0,5407692 | 0 | 25 |
| CLED6P9 | "ornithine carbamoyltransferase; OCTase {Canavalia lineata}GP\|12003188\|gb\|AAG43481.1\|AF203688_1\|AF20" | MELEZITOSE | -0,7631579 | 1 | 19 |
| CLED6P9 | "ornithine carbamoyltransferase; OCTase {Canavalia lineata}GP\|12003188\|gb\|AAG43481.1\|AF203688_1\|AF20" | SERINE | 0,5323077 | 0 | 25 |
| CLED6P9 | "ornithine carbamoyltransferase; OCTase {Canavalia lineata}GP\|12003188\|gb\|AAG43481.1\|AF203688_1\|AF20" | SPERMIDINE | 0,5515385 | 0 | 25 |
| CLED6P9 | "ornithine carbamoyltransferase; OCTase {Canavalia lineata}GP\|12003188\|gb\|AAG43481.1\|AF203688_1\|AF20" | T-4-HO-PROLINE | 0,6 | 0 | 19 |
| CLED6P9 | "ornithine carbamoyltransferase; OCTase {Canavalia lineata}GP\|12003188\|gb\|AAG43481.1\|AF203688_1\|AF20" | THREONATE | 0,5176923 | 0 | 25 |
| CLED6P9 | "ornithine carbamoyltransferase; OCTase {Canavalia lineata}GP\|12003188\|gb\|AAG43481.1\|AF203688_1\|AF20" | THREONINE | 0,5591304 | 0 | 24 |
| CLED6P9 | "ornithine carbamoyltransferase; OCTase {Canavalia lineata}GP\|12003188\|gb\|AAG43481.1\|AF203688_1\|AF20" | TRYPTOPHAN | 0,5930435 | 0 | 24 |
| CLED6P9 | "ornithine carbamoyltransferase; OCTase {Canavalia lineata}GP\|12003188\|gb\|AAG43481.1\|AF203688_1\|AF20" | TYROSINE | 0,6330769 | 1 | 25 |
| CLED7G23 | Similar to acyl carrier protein, mitochondrial precursor (ACP) NADH-ubiquinone oxidoreductase 9.6 KD | INOSITOL | -0,6430769 | 1 | 25 |

| | | | | |
|---|---|---|---|---|---|
| CLED7G23 | Similar to acyl carrier protein, mitochondrial precursor (ACP) NADH-ubiquinone oxidoreductase 9.6 KD | MELEZITOSE | -0,6157895 | 0 | 19 |
| CLED8G16 | DIACYLGLYCEROL KINASE 1 (EC 2.7.1.107) (DIGLYCERIDE KINASE) (DGK 1) (DAG KINASE 1).GP\|1374772\|dbj\|B | INOSITOL | -0,5869231 | 0 | 25 |
| CLEG12G7 | cytochrome P450-like protein {Arabidopsis thaliana}GP\|7270932\|emb\|CAB80611.1\|\|AL161595 cytochrome P | GABA | 0,5992308 | 0 | 25 |
| CLEG12G7 | cytochrome P450-like protein {Arabidopsis thaliana}GP\|7270932\|emb\|CAB80611.1\|\|AL161595 cytochrome P | INOSITOL | -0,7884616 | 1 | 25 |
| CLEG12G7 | cytochrome P450-like protein {Arabidopsis thaliana}GP\|7270932\|emb\|CAB80611.1\|\|AL161595 cytochrome P | SERINE | 0,5430769 | 0 | 25 |
| CLEG12G7 | cytochrome P450-like protein {Arabidopsis thaliana}GP\|7270932\|emb\|CAB80611.1\|\|AL161595 cytochrome P | SPERMIDINE | 0,62 | 1 | 25 |
| CLEG12G7 | cytochrome P450-like protein {Arabidopsis thaliana}GP\|7270932\|emb\|CAB80611.1\|\|AL161595 cytochrome P | TRYPTOPHAN | 0,6226087 | 0 | 24 |
| CLEG12G7 | cytochrome P450-like protein {Arabidopsis thaliana}GP\|7270932\|emb\|CAB80611.1\|\|AL161595 cytochrome P | TYROSINE | 0,6115385 | 0 | 25 |
| CLEG12O3 | similar to ATPases associated with various cellular activites (Pfam: AAA.hmm, score: 230.91) {Arabid | GABA | 0,5638462 | 0 | 25 |
| CLEG12O3 | similar to ATPases associated with various cellular activites (Pfam: AAA.hmm, score: 230.91) {Arabid | INOSITOL | -0,7407692 | 1 | 25 |
| CLEG12O3 | similar to ATPases associated with various cellular activites (Pfam: AAA.hmm, score: 230.91) {Arabid | SERINE | 0,5238461 | 0 | 25 |
| CLEG12O3 | similar to ATPases associated with various cellular activites (Pfam: AAA.hmm, score: 230.91) {Arabid | SPERMIDINE | 0,6476923 | 1 | 25 |
| CLEG12O3 | similar to ATPases associated with various cellular activites (Pfam: AAA.hmm, score: 230.91) {Arabid | TRYPTOPHAN | 0,5869565 | 0 | 24 |
| CLEG12O3 | similar to ATPases associated with various cellular activites (Pfam: AAA.hmm, score: 230.91) {Arabid | TYROSINE | 0,5746154 | 0 | 25 |
| CLEG16B16 | succinate dehydrogenase flavoprotein alpha subunit {Arabidopsis thaliana}GP\|8843734\|dbj\|BAA97282.1\| | GABA | 0,5776923 | 0 | 25 |
| CLEG16B16 | succinate dehydrogenase flavoprotein alpha subunit {Arabidopsis thaliana}GP\|8843734\|dbj\|BAA97282.1\| | INOSITOL | -0,7607692 | 1 | 25 |

EP 1 454 993 A1

| | | | | | |
|---|---|---|---|---|---|
| CLEG16B16 | succinate dehydrogenase flavoprotein alpha subunit {Arabidopsis thaliana}GP\|8843734\|dbj\|BAA97282.1\| | TYROSINE | 0,6015385 | 0 | 25 |
| CLEG27G1 | putative NADH-ubiquinone oxireductase {Arabidopsis thaliana}PIR\|C84588\|C84588 probable NADH-ubiquin | GABA | 0,6184615 | 1 | 25 |
| CLEG27G1 | putative NADH-ubiquinone oxireductase {Arabidopsis thaliana}PIR\|C84588\|C84588 probable NADH-ubiquin | INOSITOL | -0,7776923 | 1 | 25 |
| CLEG27G1 | putative NADH-ubiquinone oxireductase {Arabidopsis thaliana}PIR\|C84588\|C84588 probable NADH-ubiquin | MALTITOL | 0,9428571 | 0 | 6 |
| CLEG27G1 | putative NADH-ubiquinone oxireductase {Arabidopsis thaliana}PIR\|C84588\|C84588 probable NADH-ubiquin | SERINE | 0,5661538 | 0 | 25 |
| CLEG27G1 | putative NADH-ubiquinone oxireductase {Arabidopsis thaliana}PIR\|C84588\|C84588 probable NADH-ubiquin | SPERMIDINE | 0,6846154 | 1 | 25 |
| CLEG27G1 | putative NADH-ubiquinone oxireductase {Arabidopsis thaliana}PIR\|C84588\|C84588 probable NADH-ubiquin | SUCROSE | -0,5252174 | 0 | 24 |
| CLEG27G1 | putative NADH-ubiquinone oxireductase {Arabidopsis thaliana}PIR\|C84588\|C84588 probable NADH-ubiquin | THREONATE | 0,5207692 | 0 | 25 |
| CLEG27G1 | putative NADH-ubiquinone oxireductase {Arabidopsis thaliana}PIR\|C84588\|C84588 probable NADH-ubiquin | TRYPTOPHAN | 0,6008695 | 0 | 24 |
| CLEG27G1 | putative NADH-ubiquinone oxireductase {Arabidopsis thaliana}PIR\|C84588\|C84588 probable NADH-ubiquin | TYROSINE | 0,6153846 | 0 | 25 |
| CLEG28D24 | 76 kDa mitochondrial complex I subunit {Solanum tuberosum}SP\|Q43644\|NUAM_SOLTU NADH-UBIQUINONE OXID | GABA | 0,5392308 | 0 | 25 |
| CLEG28D24 | 76 kDa mitochondrial complex I subunit {Solanum tuberosum}SP\|Q43644\|NUAM_SOLTU NADH-UBIQUINONE OXID | INOSITOL | -0,6692308 | 1 | 25 |
| CLEG28D24 | 76 kDa mitochondrial complex I subunit {Solanum tuberosum}SP\|Q43644\|NUAM_SOLTU NADH-UBIQUINONE OXID | SERINE | 0,5392308 | 0 | 25 |
| CLEG28D24 | 76 kDa mitochondrial complex I subunit {Solanum tuberosum}SP\|Q43644\|NUAM_SOLTU NADH-UBIQUINONE OXID | SPERMIDINE | 0,5761539 | 0 | 25 |
| CLEG28D24 | 76 kDa mitochondrial complex I subunit {Solanum tuberosum}SP\|Q43644\|NUAM_SOLTU NADH-UBIQUINONE OXID | TRYPTOPHAN | 0,5182609 | 0 | 24 |
| CLEG30O13 | phosphate/phosphoenolpyruvate translocator protein-like {Arabidopsis thaliana} | GABA | 0,5876923 | 0 | 25 |

| | | | | | |
|---|---|---|---|---|---|
| CLEG30O13 | phosphate/phosphoenolpyruvate translocator protein-like {Arabidopsis thaliana} | INOSITOL | -0,7707692 | 1 | 25 |
| CLEG30O13 | phosphate/phosphoenolpyruvate translocator protein-like {Arabidopsis thaliana} | QUINATE | 0,5076923 | 0 | 25 |
| CLEG30O13 | phosphate/phosphoenolpyruvate translocator protein-like {Arabidopsis thaliana} | SPERMIDINE | 0,6084616 | 0 | 25 |
| CLEG30O13 | phosphate/phosphoenolpyruvate translocator protein-like {Arabidopsis thaliana} | TRYPTOPHAN | 0,6147826 | 0 | 24 |
| CLEG32P19 | glucosyl transferase {Nicotiana tabacum}GP|1805359|dbj|BAA19155.1||AB000623 glucosyl transferase {N | GABA | 0,6169231 | 0 | 25 |
| CLEG32P19 | glucosyl transferase {Nicotiana tabacum}GP|1805359|dbj|BAA19155.1||AB000623 glucosyl transferase {N | INOSITOL | -0,5876923 | 0 | 25 |
| CLEG32P19 | glucosyl transferase {Nicotiana tabacum}GP|1805359|dbj|BAA19155.1||AB000623 glucosyl transferase {N | SPERMIDINE | 0,5246154 | 0 | 25 |
| CLEG33A5 | w-3 desaturase {Solanum tuberosum}PIR|T07685|T07685 omega-3 fatty acid desaturase (EC 1.14.99.-) - | GABA | 0,5238461 | 0 | 25 |
| CLEG33A5 | w-3 desaturase {Solanum tuberosum}PIR|T07685|T07685 omega-3 fatty acid desaturase (EC 1.14.99.-) - | INOSITOL | -0,6038461 | 0 | 25 |
| CLEG33O5 | NADH-ubiquinone oxidoreductase 20 kDa subunit precursor {Solanum tuberosum}SP|Q43844|NUKM_SOLTU NAD | INOSITOL | -0,7030769 | 1 | 25 |
| CLEG33O5 | NADH-ubiquinone oxidoreductase 20 kDa subunit precursor {Solanum tuberosum}SP|Q43844|NUKM_SOLTU NAD | SERINE | 0,523077 | 0 | 25 |
| CLEG33O5 | NADH-ubiquinone oxidoreductase 20 kDa subunit precursor {Solanum tuberosum}SP|Q43844|NUKM_SOLTU NAD | SPERMIDINE | 0,5138462 | 0 | 25 |
| CLEG33O5 | NADH-ubiquinone oxidoreductase 20 kDa subunit precursor {Solanum tuberosum}SP|Q43844|NUKM_SOLTU NAD | THREONATE | 0,51 | 0 | 25 |
| CLEG33O5 | NADH-ubiquinone oxidoreductase 20 kDa subunit precursor {Solanum tuberosum}SP|Q43844|NUKM_SOLTU NAD | TYROSINE | 0,6584615 | 1 | 25 |
| CLEG35I11 | putative homeodomain transcription factor {Arabidopsis thaliana}PIR|F84565|F84565 probable homeodom | GABA | 0,5915385 | 0 | 25 |

EP 1 454 993 A1

| CLEG35I11 | putative homeodomain transcription factor {Arabidopsis thaliana}PIR\|F84565\|F84565 probable homeodom | INOSITOL | -0,7892308 | 1 | 25 |
|---|---|---|---|---|---|
| CLEG35I11 | putative homeodomain transcription factor {Arabidopsis thaliana}PIR\|F84565\|F84565 probable homeodom | SPERMIDINE | 0,5415385 | 0 | 25 |
| CLEG35I11 | putative homeodomain transcription factor {Arabidopsis thaliana}PIR\|F84565\|F84565 probable homeodom | TRYPTOPHAN | 0,5634782 | 0 | 24 |
| CLEG35I11 | putative homeodomain transcription factor {Arabidopsis thaliana}PIR\|F84565\|F84565 probable homeodom | TYROSINE | 0,5907692 | 0 | 25 |
| CLEG37C14 | zinc finger protein-like {Arabidopsis thaliana} | GABA | 0,5523077 | 0 | 25 |
| CLEG37C14 | zinc finger protein-like {Arabidopsis thaliana} | INOSITOL | -0,8192308 | 1 | 25 |
| CLEG37C14 | zinc finger protein-like {Arabidopsis thaliana} | MELEZITOSE | -0,654386 | 0 | 19 |
| CLEG37C14 | zinc finger protein-like {Arabidopsis thaliana} | SPERMIDINE | 0,5746154 | 0 | 25 |
| CLEG37C14 | zinc finger protein-like {Arabidopsis thaliana} | TRYPTOPHAN | 0,5721739 | 0 | 24 |
| CLEG37C14 | zinc finger protein-like {Arabidopsis thaliana} | TYROSINE | 0,6707692 | 1 | 25 |
| CLEG39L11 | transcription factor TEIL {Nicotiana tabacum} | INOSITOL | -0,5953846 | 0 | 25 |
| CLEG39N19 | dehydroquinate dehydratase/shikimate:NADP oxidoreductase | GABA | 0,54 | 0 | 25 |
| CLEG39N19 | dehydroquinate dehydratase/shikimate:NADP oxidoreductase | INOSITOL | -0,7023077 | 1 | 25 |
| CLEG39N23 | dTDP-glucose 4-6-dehydratases-like protein {Arabidopsis thaliana}PIR\|T45701\|T45701 dTDP-glucose 4-6 | INOSITOL | -0,5907692 | 0 | 25 |
| CLEG3E11 | glycerol-3-phosphate dehydrogenase {Arabidopsis thaliana}PIR\|F84832\|F84832 glycerol-3-phosphate deh | INOSITOL | -0,5938461 | 0 | 25 |
| CLEG41L23 | phosphate/phosphoenolpyruvate translocator protein-like {Arabidopsis thaliana} | GABA | 0,56 | 0 | 25 |
| CLEG41L23 | phosphate/phosphoenolpyruvate translocator protein-like {Arabidopsis thaliana} | INOSITOL | -0,74 | 1 | 25 |
| CLEG41L23 | phosphate/phosphoenolpyruvate translocator protein-like {Arabidopsis thaliana} | SERINE | 0,5653846 | 0 | 25 |
| CLEG41L23 | phosphate/phosphoenolpyruvate translocator protein-like {Arabidopsis thaliana} | SPERMIDINE | 0,63 | 1 | 25 |
| CLEG41L23 | phosphate/phosphoenolpyruvate translocator protein-like {Arabidopsis thaliana} | TRYPTOPHAN | 0,5660869 | 0 | 24 |
| CLEG41L23 | phosphate/phosphoenolpyruvate translocator protein-like {Arabidopsis thaliana} | TYROSINE | 0,5246154 | 0 | 25 |
| CLEG41P2 | delta 1-pyrroline-5-carboxylate synthetase | 3PGA | -0,9428571 | 0 | 6 |

| | | | | | |
|---|---|---|---|---|---|
| CLEG41P2 | delta 1-pyrroline-5-carboxylate synthetase | A-KETOGLUTARATE | -1 | 1 | 5 |
| CLEG41P2 | delta 1-pyrroline-5-carboxylate synthetase | GABA | 0,5092308 | 0 | 25 |
| CLEG41P2 | delta 1-pyrroline-5-carboxylate synthetase | INOSITOL | -0,7530769 | 1 | 25 |
| CLEG41P2 | delta 1-pyrroline-5-carboxylate synthetase | SPERMIDINE | 0,5492308 | 0 | 25 |
| CLEG41P2 | delta 1-pyrroline-5-carboxylate synthetase | THREONATE | 0,5107692 | 0 | 25 |
| CLEG41P2 | delta 1-pyrroline-5-carboxylate synthetase | TYROSINE | 0,6723077 | 1 | 25 |
| CLEG42N24 | "putative ABC transporter; 73228-76244 {Arabidopsis thaliana}" | INOSITOL | -0,59 | 0 | 25 |
| CLEG43E15 | CCAAT box binding factor/ transcription factor Hap2a {Arabidopsis thaliana}PIR\|T49898\|T49898 CCAAT | GABA | 0,5469231 | 0 | 25 |
| CLEG43E15 | CCAAT box binding factor/ transcription factor Hap2a {Arabidopsis thaliana}PIR\|T49898\|T49898 CCAAT | INOSITOL | -0,5330769 | 0 | 25 |
| CLEG43N9 | sugar-phosphate isomerase-like protein {Arabidopsis thaliana}PIR\|T47628\|T47628 sugar-phosphate isom | INOSITOL | -0,5153846 | 0 | 25 |
| CLEG8L3 | similar to ATPases associated with various cellular activites (Pfam: AAA.hmm, score: 230.91) {Arabid | GABA | 0,6207692 | 1 | 25 |
| CLEG8L3 | similar to ATPases associated with various cellular activites (Pfam: AAA.hmm, score: 230.91) {Arabid | GALACTOSE | -0,5215384 | 0 | 25 |
| CLEG8L3 | similar to ATPases associated with various cellular activites (Pfam: AAA.hmm, score: 230.91) {Arabid | INOSITOL | -0,7284616 | 1 | 25 |
| CLEG8L3 | similar to ATPases associated with various cellular activites (Pfam: AAA.hmm, score: 230.91) {Arabid | SPERMIDINE | 0,6807692 | 1 | 25 |
| CLEG8L3 | similar to ATPases associated with various cellular activites (Pfam: AAA.hmm, score: 230.91) {Arabid | SUCROSE | -0,5478261 | 0 | 24 |
| CLEG8L3 | similar to ATPases associated with various cellular activites (Pfam: AAA.hmm, score: 230.91) {Arabid | TRYPTOPHAN | 0,5669565 | 0 | 24 |
| CLEG8L3 | similar to ATPases associated with various cellular activites (Pfam: AAA.hmm, score: 230.91) {Arabid | TYROSINE | 0,5715384 | 0 | 25 |
| CLEG9A20 | alanine aminotransferase {Arabidopsis thaliana} | GABA | 0,5507692 | 0 | 25 |
| CLEG9A20 | alanine aminotransferase {Arabidopsis thaliana} | INOSITOL | -0,6023077 | 0 | 25 |
| CLEG9A20 | alanine aminotransferase {Arabidopsis thaliana} | SPERMIDINE | 0,5392308 | 0 | 25 |
| CLEG9A20 | alanine aminotransferase {Arabidopsis thaliana} | THREONATE | 0,5561538 | 0 | 25 |

| CLEI11O22 | acetylornithine aminotransferase precursor {Alnus glutinosa}SP\|O04866\|ARGD_ALNGL ACETYLORNITHINE AM | GABA | 0,5930769 | 0 | 25 |
|---|---|---|---|---|---|
| CLEI11O22 | acetylornithine aminotransferase precursor {Alnus glutinosa}SP\|O04866\|ARGD_ALNGL ACETYLORNITHINE AM | INOSITOL | -0,77 | 1 | 25 |
| CLEI11O22 | acetylornithine aminotransferase precursor {Alnus glutinosa}SP\|O04866\|ARGD_ALNGL ACETYLORNITHINE AM | SERINE | 0,52 | 0 | 25 |
| CLEI11O22 | acetylornithine aminotransferase precursor {Alnus glutinosa}SP\|O04866\|ARGD_ALNGL ACETYLORNITHINE AM | SPERMIDINE | 0,6461539 | 1 | 25 |
| CLEI11O22 | acetylornithine aminotransferase precursor {Alnus glutinosa}SP\|O04866\|ARGD_ALNGL ACETYLORNITHINE AM | TRYPTOPHAN | 0,6017391 | 0 | 24 |
| CLEI11O22 | acetylornithine aminotransferase precursor {Alnus glutinosa}SP\|O04866\|ARGD_ALNGL ACETYLORNITHINE AM | TYROSINE | 0,5607693 | 0 | 25 |
| CLEI12O14 | fructose-6-phosphate 2-kinase/fructose-2,6-bisphosphatase {Solanum tuberosum}PIR\|T07016\|T07016 6-ph | GABA | 0,51 | 0 | 25 |
| CLEI12O14 | fructose-6-phosphate 2-kinase/fructose-2,6-bisphosphatase {Solanum tuberosum}PIR\|T07016\|T07016 6-ph | INOSITOL | -0,7807692 | 1 | 25 |
| CLEI12O14 | fructose-6-phosphate 2-kinase/fructose-2,6-bisphosphatase {Solanum tuberosum}PIR\|T07016\|T07016 6-ph | MELEZITOSE | -0,5947368 | 0 | 19 |
| CLEI12O14 | fructose-6-phosphate 2-kinase/fructose-2,6-bisphosphatase {Solanum tuberosum}PIR\|T07016\|T07016 6-ph | SERINE | 0,5692308 | 0 | 25 |
| CLEI12O14 | fructose-6-phosphate 2-kinase/fructose-2,6-bisphosphatase {Solanum tuberosum}PIR\|T07016\|T07016 6-ph | SPERMIDINE | 0,5907692 | 0 | 25 |
| CLEI12O14 | fructose-6-phosphate 2-kinase/fructose-2,6-bisphosphatase {Solanum tuberosum}PIR\|T07016\|T07016 6-ph | TRYPTOPHAN | 0,5469565 | 0 | 24 |
| CLEI12O14 | fructose-6-phosphate 2-kinase/fructose-2,6-bisphosphatase {Solanum tuberosum}PIR\|T07016\|T07016 6-ph | TYROSINE | 0,6484615 | 1 | 25 |
| CLEI13J17 | TRYPTOPHAN SYNTHASE BETA CHAIN 2 PRECURSOR (EC 4.2.1.20).GP\|2792520\|gb\|AAB97087.1\|\|AF042320 tryptop | GABA | 0,6007692 | 0 | 25 |
| CLEI13J17 | TRYPTOPHAN SYNTHASE BETA CHAIN 2 PRECURSOR (EC 4.2.1.20).GP\|2792520\|gb\|AAB97087.1\|\|AF042320 tryptop | INOSITOL | -0,7107692 | 1 | 25 |
| CLEI13J17 | TRYPTOPHAN SYNTHASE BETA CHAIN 2 PRECURSOR (EC 4.2.1.20).GP\|2792520\|gb\|AAB97087.1\|\|AF042320 tryptop | SPERMIDINE | 0,6561539 | 1 | 25 |

| | | | | |
|---|---|---|---|---|
| CLEI13J17 | TRYPTOPHAN SYNTHASE BETA CHAIN 2 PRECURSOR (EC 4.2.1.20).GP\|2792520\|gb\|AAB97087.1\|\|AF042320 tryptop | TYROSINE | 0,5876923 | 0 | 25 |
| CLEI15M10 | fructose-6-phosphate 2-kinase/fructose-2,6-bisphosphatase {Solanum tuberosum}PIR\|T07016\|T07016 6-ph | INOSITOL | -0,7076923 | 1 | 25 |
| CLEI15M10 | fructose-6-phosphate 2-kinase/fructose-2,6-bisphosphatase {Solanum tuberosum}PIR\|T07016\|T07016 6-ph | THREONINE | 0,5286956 | 0 | 24 |
| CLEI15M10 | fructose-6-phosphate 2-kinase/fructose-2,6-bisphosphatase {Solanum tuberosum}PIR\|T07016\|T07016 6-ph | TYROSINE | 0,6469231 | 1 | 25 |
| CLEI5A6 | transcription factor WRKY6 {Arabidopsis thaliana}GP\|12658412\|gb\|AAK01128.1\|AF331713_1\|AF331713 tran | ALANINE | 0,5330769 | 0 | 25 |
| CLEI5A6 | transcription factor WRKY6 {Arabidopsis thaliana}GP\|12658412\|gb\|AAK01128.1\|AF331713_1\|AF331713 tran | INOSITOL | -0,7923077 | 1 | 25 |
| CLEI5A6 | transcription factor WRKY6 {Arabidopsis thaliana}GP\|12658412\|gb\|AAK01128.1\|AF331713_1\|AF331713 tran | LYSINE | 0,5076923 | 0 | 25 |
| CLEI5A6 | transcription factor WRKY6 {Arabidopsis thaliana}GP\|12658412\|gb\|AAK01128.1\|AF331713_1\|AF331713 tran | SERINE | 0,6246154 | 1 | 25 |
| CLEI5A6 | transcription factor WRKY6 {Arabidopsis thaliana}GP\|12658412\|gb\|AAK01128.1\|AF331713_1\|AF331713 tran | SPERMIDINE | 0,6038461 | 0 | 25 |
| CLEI5A6 | transcription factor WRKY6 {Arabidopsis thaliana}GP\|12658412\|gb\|AAK01128.1\|AF331713_1\|AF331713 tran | THREONATE | 0,5107692 | 0 | 25 |
| CLEI5A6 | transcription factor WRKY6 {Arabidopsis thaliana}GP\|12658412\|gb\|AAK01128.1\|AF331713_1\|AF331713 tran | THREONINE | 0,5156522 | 0 | 24 |
| CLEI5A6 | transcription factor WRKY6 {Arabidopsis thaliana}GP\|12658412\|gb\|AAK01128.1\|AF331713_1\|AF331713 tran | TYROSINE | 0,6753846 | 1 | 25 |
| CLEL16O9 | Similar to Populus balsamifera subsp. trichocarpa X Populus deltoides vegetative storage protein. (L | SORBITOL/GALACTITOL | 0,7058824 | 0 | 17 |
| CLEM17A5 | bHLH transcription factor GBOF-1 {Tulipa gesneriana} | 3PGA | 0,9428571 | 0 | 6 |
| CLEM17A5 | bHLH transcription factor GBOF-1 {Tulipa gesneriana} | GABA | 0,5769231 | 0 | 25 |
| CLEM17A5 | bHLH transcription factor GBOF-1 {Tulipa gesneriana} | INOSITOL | -0,6176923 | 0 | 25 |
| CLEM17A5 | bHLH transcription factor GBOF-1 {Tulipa gesneriana} | MANNOSE | -0,5855449 | 0 | 22 |
| CLEM19M11 | ADP-glucose pyrophosphorylase small subunit | INOSITOL | -0,5384616 | 0 | 25 |
| CLEM21P8 | dehydroquinate dehydratase/shikimate:NADP oxidoreductase | INOSITOL | -0,6807692 | 1 | 25 |
| CLEM21P8 | dehydroquinate dehydratase/shikimate:NADP oxidoreductase | SERINE | 0,5269231 | 0 | 25 |
| CLEM21P8 | dehydroquinate dehydratase/shikimate:NADP oxidoreductase | SPERMIDINE | 0,6407692 | 1 | 25 |

EP 1 454 993 A1

| | | | | | |
|---|---|---|---|---|---|
| CLEM21P8 | dehydroquinate dehydratase/shikimate:NADP oxidoreductase | THREONATE | 0,5538462 | 0 | 25 |
| CLEM21P8 | dehydroquinate dehydratase/shikimate:NADP oxidoreductase | THREONINE | 0,5373913 | 0 | 24 |
| CLEM23I12 | SNAP25A protein {Arabidopsis thaliana}GP\|5731763\|emb\|CAB52582.1\|\|X92419 SNAP25A protein {Arabidopsi | GABA | 0,6192307 | 1 | 25 |
| CLEM23I12 | SNAP25A protein {Arabidopsis thaliana}GP\|5731763\|emb\|CAB52582.1\|\|X92419 SNAP25A protein {Arabidopsi | INOSITOL | -0,6284615 | 1 | 25 |
| CLEM23I12 | SNAP25A protein {Arabidopsis thaliana}GP\|5731763\|emb\|CAB52582.1\|\|X92419 SNAP25A protein {Arabidopsi | SPERMIDINE | 0,6623077 | 1 | 25 |
| CLEM23I12 | SNAP25A protein {Arabidopsis thaliana}GP\|5731763\|emb\|CAB52582.1\|\|X92419 SNAP25A protein {Arabidopsi | SUCROSE | -0,5834783 | 0 | 24 |
| CLEM23I12 | SNAP25A protein {Arabidopsis thaliana}GP\|5731763\|emb\|CAB52582.1\|\|X92419 SNAP25A protein {Arabidopsi | TYROSINE | 0,6146154 | 0 | 25 |
| CLEM3K4 | URIDYLATE KINASE (EC 2.7.4.-) (UK) (URIDINE MONOPHOSPHATE KINASE) (UMP KINASE) (UMP/CMP KINASE).GP\| | GABA | 0,5107692 | 0 | 25 |
| CLEM3K4 | URIDYLATE KINASE (EC 2.7.4.-) (UK) (URIDINE MONOPHOSPHATE KINASE) (UMP KINASE) (UMP/CMP KINASE).GP\| | INOSITOL | -0,7507693 | 1 | 25 |
| CLEM3K4 | URIDYLATE KINASE (EC 2.7.4.-) (UK) (URIDINE MONOPHOSPHATE KINASE) (UMP KINASE) (UMP/CMP KINASE).GP\| | SPERMIDINE | 0,5130769 | 0 | 25 |
| CLEM3K4 | URIDYLATE KINASE (EC 2.7.4.-) (UK) (URIDINE MONOPHOSPHATE KINASE) (UMP KINASE) (UMP/CMP KINASE).GP\| | TYROSINE | 0,6261538 | 1 | 25 |
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | ALANINE | 0,5161539 | 0 | 25 |
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | GABA | 0,5569231 | 0 | 25 |
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | INOSITOL | -0,8246154 | 1 | 25 |
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | MELEZITOSE | -0,6087719 | 0 | 19 |

| | | | | | |
|---|---|---|---|---|---|
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | SPERMIDINE | 0,5569231 | 0 | 25 |
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | TRYPTOPHAN | 0,6104348 | 0 | 24 |
| CLEM4L3 | TRYPTOPHAN SYNTHASE BETA CHAIN 2 PRECURSOR (EC 4.2.1.20).GP\|2792520\|gb\|AAB97087.1\|\|AF042320 tryptop | TYROSINE | 0,6469231 | 1 | 25 |
| CLEM8E24 | glucose-6-phosphate 1-dehydrogenase {Solanum tuberosum}SP\|P37830\|G6PD_SOLTU GLUCOSE-6-PHOSPHATE 1-D | TYRAMINE | 0,620227 | 0 | 18 |
| CLEN21M4 | "putative ABC transporter; 73228-76244 {Arabidopsis thaliana}" | GABA | 0,6323077 | 1 | 25 |
| CLEN21M4 | "putative ABC transporter; 73228-76244 {Arabidopsis thaliana}" | INOSITOL | -0,7407692 | 1 | 25 |
| CLEN21M4 | "putative ABC transporter; 73228-76244 {Arabidopsis thaliana}" | SPERMIDINE | 0,6023077 | 0 | 25 |
| CLEN21M4 | "putative ABC transporter; 73228-76244 {Arabidopsis thaliana}" | TRYPTOPHAN | 0,6095652 | 0 | 24 |
| CLER2B1 | serine/threonine-specific protein kinase NAK {Arabidopsis thaliana}PIR\|T48250\|T48250 serine/threoni | INOSITOL | -0,5807692 | 0 | 25 |
| CLES16N17 | ADP-glucose pyrophosphorylase small subunit | INOSITOL | -0,7192308 | 1 | 25 |
| CLES16N17 | ADP-glucose pyrophosphorylase small subunit | SERINE | 0,52 | 0 | 25 |
| CLES16N17 | ADP-glucose pyrophosphorylase small subunit | THREONINE | 0,5434783 | 0 | 24 |
| CLES16N17 | ADP-glucose pyrophosphorylase small subunit | TYROSINE | 0,6161538 | 0 | 25 |
| CLES20P12 | putative cytochrome P450 | INOSITOL | -0,5369231 | 0 | 25 |
| CLES20P12 | putative cytochrome P450 | MELEZITOSE | -0,6 | 0 | 19 |
| CLET10A17 | SNAP25A protein {Arabidopsis thaliana}GP\|5731763\|emb\|CAB52582.1\|\|X92419 SNAP25A protein {Arabidopsi | GABA | 0,5892308 | 0 | 25 |
| CLET10A17 | SNAP25A protein {Arabidopsis thaliana}GP\|5731763\|emb\|CAB52582.1\|\|X92419 SNAP25A protein {Arabidopsi | INOSITOL | -0,7292308 | 1 | 25 |
| CLET10A17 | SNAP25A protein {Arabidopsis thaliana}GP\|5731763\|emb\|CAB52582.1\|\|X92419 SNAP25A protein {Arabidopsi | SPERMIDINE | 0,6638461 | 1 | 25 |
| CLET10A17 | SNAP25A protein {Arabidopsis thaliana}GP\|5731763\|emb\|CAB52582.1\|\|X92419 SNAP25A protein {Arabidopsi | SUCROSE | -0,5313044 | 0 | 24 |

| | | | | | |
|---|---|---|---|---|---|
| CLET10A17 | SNAP25A protein {Arabidopsis thaliana}GP\|5731763\|emb\|CAB52582.1\|\|X92419 SNAP25A protein {Arabidopsi | TRYPTOPHAN | 0,5469565 | 0 | 24 |
| CLET10A17 | SNAP25A protein {Arabidopsis thaliana}GP\|5731763\|emb\|CAB52582.1\|\|X92419 SNAP25A protein {Arabidopsi | TYROSINE | 0,6376923 | 1 | 25 |
| CLET12C14 | MYB-like DNA-binding protein {Catharanthus roseus} | INOSITOL | -0,5492308 | 0 | 25 |
| CLET12C14 | MYB-like DNA-binding protein {Catharanthus roseus} | TYROSINE | 0,5630769 | 0 | 25 |
| CLET1B18 | CYTOCHROME P450 83B1 (EC 1.14.-.-).GP\|3164126\|dbj\|BAA28531.1\|\|D78598 cytochrome P450 monooxygenase | INOSITOL | -0,5192308 | 0 | 25 |
| CLET27N17 | Similar to gb\|AF135422 GDP-mannose pyrophosphorylase A (GMPPA) from Homo sapiens. ESTs gb\|AA712990, | INOSITOL | -0,7669231 | 1 | 25 |
| CLET27N17 | Similar to gb\|AF135422 GDP-mannose pyrophosphorylase A (GMPPA) from Homo sapiens. ESTs gb\|AA712990, | MELEZITOSE | -0,5807018 | 0 | 19 |
| CLET27N17 | Similar to gb\|AF135422 GDP-mannose pyrophosphorylase A (GMPPA) from Homo sapiens. ESTs gb\|AA712990, | SPERMIDINE | 0,5946154 | 0 | 25 |
| CLET27N17 | Similar to gb\|AF135422 GDP-mannose pyrophosphorylase A (GMPPA) from Homo sapiens. ESTs gb\|AA712990, | THREONINE | 0,5156522 | 0 | 24 |
| CLET27N17 | Similar to gb\|AF135422 GDP-mannose pyrophosphorylase A (GMPPA) from Homo sapiens. ESTs gb\|AA712990, | TYROSINE | 0,6815385 | 1 | 25 |
| CLET38C16-1 | 76 kDa mitochondrial complex I subunit {Solanum tuberosum}SP\|Q43644\|NUAM_SOLTU NADH-UBIQUINONE OXID | GABA | 0,5223077 | 0 | 25 |
| CLET38C16-1 | 76 kDa mitochondrial complex I subunit {Solanum tuberosum}SP\|Q43644\|NUAM_SOLTU NADH-UBIQUINONE OXID | GALACTOSE | -0,5384616 | 0 | 25 |
| CLET38C16-1 | 76 kDa mitochondrial complex I subunit {Solanum tuberosum}SP\|Q43644\|NUAM_SOLTU NADH-UBIQUINONE OXID | INOSITOL | -0,6223077 | 1 | 25 |
| CLET38C16-1 | 76 kDa mitochondrial complex I subunit {Solanum tuberosum}SP\|Q43644\|NUAM_SOLTU NADH-UBIQUINONE OXID | SPERMIDINE | 0,5884615 | 0 | 25 |
| CLET8H21 | MADS-box transcription factor FBP21 {Petunia x hybrida} | GABA | 0,5430769 | 0 | 25 |
| CLET8H21 | MADS-box transcription factor FBP21 {Petunia x hybrida} | INOSITOL | -0,6869231 | 1 | 25 |
| CLET8H21 | MADS-box transcription factor FBP21 {Petunia x hybrida} | SPERMIDINE | 0,5669231 | 0 | 25 |
| CLEW18G10 | tyrosine aminotransferase-like protein {Arabidopsis thaliana} | HOMOGLUTAMINE | 1 | 1 | 5 |

| | | | | | |
|---|---|---|---|---|---|
| CLEX11K13 | Putative UDP-glucose glucosyltransferase {Arabidopsis thaliana}PIR\|H86356\|H86356 probable UDP-gluco | GABA | 0,6376923 | 1 | 25 |
| CLEX11K13 | Putative UDP-glucose glucosyltransferase {Arabidopsis thaliana}PIR\|H86356\|H86356 probable UDP-gluco | INOSITOL | -0,7146154 | 1 | 25 |
| CLEX11K13 | Putative UDP-glucose glucosyltransferase {Arabidopsis thaliana}PIR\|H86356\|H86356 probable UDP-gluco | NORLEUCINE | -0,9428571 | 0 | 6 |
| CLEX11K13 | Putative UDP-glucose glucosyltransferase {Arabidopsis thaliana}PIR\|H86356\|H86356 probable UDP-gluco | NORVALINE | -1 | 1 | 6 |
| CLEX11K13 | Putative UDP-glucose glucosyltransferase {Arabidopsis thaliana}PIR\|H86356\|H86356 probable UDP-gluco | SERINE | 0,5130769 | 0 | 25 |
| CLEX11K13 | Putative UDP-glucose glucosyltransferase {Arabidopsis thaliana}PIR\|H86356\|H86356 probable UDP-gluco | SPERMIDINE | 0,6784616 | 1 | 25 |
| CLEX11K13 | Putative UDP-glucose glucosyltransferase {Arabidopsis thaliana}PIR\|H86356\|H86356 probable UDP-gluco | SUCROSE | -0,5669565 | 0 | 24 |
| CLEX11K13 | Putative UDP-glucose glucosyltransferase {Arabidopsis thaliana}PIR\|H86356\|H86356 probable UDP-gluco | THREONATE | 0,51 | 0 | 25 |
| CLEX11K13 | Putative UDP-glucose glucosyltransferase {Arabidopsis thaliana}PIR\|H86356\|H86356 probable UDP-gluco | TRYPTOPHAN | 0,5443478 | 0 | 24 |
| CLEX11K13 | Putative UDP-glucose glucosyltransferase {Arabidopsis thaliana}PIR\|H86356\|H86356 probable UDP-gluco | TYROSINE | 0,5792308 | 0 | 25 |
| CLEX11L22 | PROBABLE VACUOLAR ATP SYNTHASE SUBUNIT F (EC 3.6.1.34) (V-ATPASE F SUBUNIT) (VACUOLAR PROTON PUMP F | INOSITOL | -0,7223077 | 1 | 25 |
| CLEX11L22 | PROBABLE VACUOLAR ATP SYNTHASE SUBUNIT F (EC 3.6.1.34) (V-ATPASE F SUBUNIT) (VACUOLAR PROTON PUMP F | TYROSINE | 0,5707693 | 0 | 25 |
| CLEX11O7 | transaldolase | GALACTOSE | 0,5407692 | 0 | 25 |
| CLEX12A9 | putative C3HC4-type RING zinc finger protein {Arabidopsis thaliana}PIR\|B84813\|B84813 probable RING | GABA | 0,5323077 | 0 | 25 |
| CLEX12A9 | putative C3HC4-type RING zinc finger protein {Arabidopsis thaliana}PIR\|B84813\|B84813 probable RING | INOSITOL | -0,6253846 | 1 | 25 |
| CLEX12A9 | putative C3HC4-type RING zinc finger protein {Arabidopsis thaliana}PIR\|B84813\|B84813 probable RING | SPERMIDINE | 0,5715384 | 0 | 25 |
| CLEX13P4 | Putative UDP-glucose glucosyltransferase {Arabidopsis thaliana}PIR\|H86356\|H86356 probable UDP-gluco | ALANINE | 0,5069231 | 0 | 25 |

EP 1 454 993 A1

| | | | | | |
|---|---|---|---|---|---|
| CLEX13P4 | Putative UDP-glucose glucosyltransferase {Arabidopsis thaliana}PIR\|H86356\|H86356 probable UDP-gluco | INOSITOL | -0,7553846 | 1 | 25 |
| CLEX13P4 | Putative UDP-glucose glucosyltransferase {Arabidopsis thaliana}PIR\|H86356\|H86356 probable UDP-gluco | MELEZITOSE | -0,5982456 | 0 | 19 |
| CLEX13P4 | Putative UDP-glucose glucosyltransferase {Arabidopsis thaliana}PIR\|H86356\|H86356 probable UDP-gluco | NORVALINE | -0,9428571 | 0 | 6 |
| CLEX13P4 | Putative UDP-glucose glucosyltransferase {Arabidopsis thaliana}PIR\|H86356\|H86356 probable UDP-gluco | SPERMIDINE | 0,5538462 | 0 | 25 |
| CLEX13P4 | Putative UDP-glucose glucosyltransferase {Arabidopsis thaliana}PIR\|H86356\|H86356 probable UDP-gluco | THREONATE | 0,5784615 | 0 | 25 |
| CLEX13P4 | Putative UDP-glucose glucosyltransferase {Arabidopsis thaliana}PIR\|H86356\|H86356 probable UDP-gluco | TYROSINE | 0,6669231 | 1 | 25 |
| CLEX14L9 | alanine aminotransferase {Arabidopsis thaliana}GP\|12325273\|gb\|AAG52580.1\|AC016529_11\|AC016529 putat | HISTIDINE | -0,9428571 | 0 | 6 |
| CLEX14L9 | alanine aminotransferase {Arabidopsis thaliana}GP\|12325273\|gb\|AAG52580.1\|AC016529_11\|AC016529 putat | NORVALINE | -0,9428571 | 0 | 6 |
| CLEX1M19 | Similar to gb\|AF135422 GDP-mannose pyrophosphorylase A (GMPPA) from Homo sapiens. ESTs gb\|AA712990, | GABA | 0,5069231 | 0 | 25 |
| CLEX1M19 | Similar to gb\|AF135422 GDP-mannose pyrophosphorylase A (GMPPA) from Homo sapiens. ESTs gb\|AA712990, | INOSITOL | -0,7115384 | 1 | 25 |
| CLEX1M19 | Similar to gb\|AF135422 GDP-mannose pyrophosphorylase A (GMPPA) from Homo sapiens. ESTs gb\|AA712990, | SPERMIDINE | 0,5453846 | 0 | 25 |
| CLEX1M19 | Similar to gb\|AF135422 GDP-mannose pyrophosphorylase A (GMPPA) from Homo sapiens. ESTs gb\|AA712990, | THREONATE | 0,51 | 0 | 25 |
| CLEX1M19 | Similar to gb\|AF135422 GDP-mannose pyrophosphorylase A (GMPPA) from Homo sapiens. ESTs gb\|AA712990, | TYROSINE | 0,6276923 | 1 | 25 |
| CLEX4M2 | osmotic stress-induced zinc-finger protein {Nicotiana tabacum}PIR\|T01985\|T01985 zinc-finger protein | GABA | 0,5192308 | 0 | 25 |
| CLEX4M2 | osmotic stress-induced zinc-finger protein {Nicotiana tabacum}PIR\|T01985\|T01985 zinc-finger protein | INOSITOL | -0,7076923 | 1 | 25 |
| CLEX4M2 | osmotic stress-induced zinc-finger protein {Nicotiana tabacum}PIR\|T01985\|T01985 zinc-finger protein | SPERMIDINE | 0,6207692 | 1 | 25 |

| | | | | |
|---|---|---|---|---|---|
| CLEX4M2 | osmotic stress-induced zinc-finger protein {Nicotiana tabacum}PIR\|T01985\|T01985 zinc-finger protein | SUCROSE | -0,5252174 | 0 | 24 |
| CLEX4M2 | osmotic stress-induced zinc-finger protein {Nicotiana tabacum}PIR\|T01985\|T01985 zinc-finger protein | TYROSINE | 0,6 | 0 | 25 |
| CLEY14E21 | ALTERNATIVE OXIDASE 1 PRECURSOR (EC 1.-.-.-).GP\|558054\|gb\|AAC60576.1\|\|S71335 alternative oxidase, A | GABA | 0,5761539 | 0 | 25 |
| CLEY14E21 | ALTERNATIVE OXIDASE 1 PRECURSOR (EC 1.-.-.-).GP\|558054\|gb\|AAC60576.1\|\|S71335 alternative oxidase, A | INOSITOL | -0,7169231 | 1 | 25 |
| CLEY14E21 | ALTERNATIVE OXIDASE 1 PRECURSOR (EC 1.-.-.-).GP\|558054\|gb\|AAC60576.1\|\|S71335 alternative oxidase, A | SPERMIDINE | 0,6607692 | 1 | 25 |
| CLEY14E21 | ALTERNATIVE OXIDASE 1 PRECURSOR (EC 1.-.-.-).GP\|558054\|gb\|AAC60576.1\|\|S71335 alternative oxidase, A | SUCROSE | -0,5208696 | 0 | 24 |
| CLEY14E21 | ALTERNATIVE OXIDASE 1 PRECURSOR (EC 1.-.-.-).GP\|558054\|gb\|AAC60576.1\|\|S71335 alternative oxidase, A | TRYPTOPHAN | 0,5808696 | 0 | 24 |
| CLEY14E21 | ALTERNATIVE OXIDASE 1 PRECURSOR (EC 1.-.-.-).GP\|558054\|gb\|AAC60576.1\|\|S71335 alternative oxidase, A | TYROSINE | 0,5215384 | 0 | 25 |
| CLEY15E1 | caffeoyl-CoA O-methyltransferase 5 {Nicotiana tabacum}GP\|1679853\|emb\|CAB05369.1\|\|Z82982 caffeoyl-Co | INOSITOL | 0,6846154 | 1 | 25 |
| CLEY15E1 | caffeoyl-CoA O-methyltransferase 5 {Nicotiana tabacum}GP\|1679853\|emb\|CAB05369.1\|\|Z82982 caffeoyl-Co | LYSINE | -0,523077 | 0 | 25 |
| CLEY15E1 | caffeoyl-CoA O-methyltransferase 5 {Nicotiana tabacum}GP\|1679853\|emb\|CAB05369.1\|\|Z82982 caffeoyl-Co | MELEZITOSE | 0,6035088 | 0 | 19 |
| CLEY15E1 | caffeoyl-CoA O-methyltransferase 5 {Nicotiana tabacum}GP\|1679853\|emb\|CAB05369.1\|\|Z82982 caffeoyl-Co | THREONINE | -0,5452174 | 0 | 24 |
| CLEY15E1 | caffeoyl-CoA O-methyltransferase 5 {Nicotiana tabacum}GP\|1679853\|emb\|CAB05369.1\|\|Z82982 caffeoyl-Co | TYROSINE | -0,6915385 | 1 | 25 |
| CLEY15P13 | ATP synthase delta' subunit, mitochondrial precursor {Ipomoea batatas}SP\|Q40089\|ATP4_IPOBA ATP SYNT | INOSITOL | -0,7076923 | 1 | 25 |
| CLEY15P13 | ATP synthase delta' subunit, mitochondrial precursor {Ipomoea batatas}SP\|Q40089\|ATP4_IPOBA ATP SYNT | SERINE | 0,5469231 | 0 | 25 |
| CLEY15P13 | ATP synthase delta' subunit, mitochondrial precursor {Ipomoea batatas}SP\|Q40089\|ATP4_IPOBA ATP SYNT | SPERMIDINE | 0,58 | 0 | 25 |

| | | | | | |
|---|---|---|---|---|---|
| CLEY15P13 | ATP synthase delta' subunit, mitochondrial precursor {Ipomoea batatas}SP\|Q40089\|ATP4_IPOBA ATP SYNT | TYROSINE | 0,6646154 | 1 | 25 |
| CLEY19P11 | sugar-phosphate isomerase-like protein {Arabidopsis thaliana}PIR\|T47628\|T47628 sugar-phosphate isom | INOSITOL | -0,7 | 1 | 25 |
| CLEY19P11 | sugar-phosphate isomerase-like protein {Arabidopsis thaliana}PIR\|T47628\|T47628 sugar-phosphate isom | SPERMIDINE | 0,5476923 | 0 | 25 |
| CLEY19P11 | sugar-phosphate isomerase-like protein {Arabidopsis thaliana}PIR\|T47628\|T47628 sugar-phosphate isom | TYROSINE | 0,6046154 | 0 | 25 |
| CLEY24E3 | mas-binding factor MBF2=transcription factor TGA1a homolog {Solanum tuberosum=potatoes, root, Peptid | GABA | 0,5469231 | 0 | 25 |
| CLEY24E3 | mas-binding factor MBF2=transcription factor TGA1a homolog {Solanum tuberosum=potatoes, root, Peptid | INOSITOL | -0,6369231 | 1 | 25 |
| CLEY24E3 | mas-binding factor MBF2=transcription factor TGA1a homolog {Solanum tuberosum=potatoes, root, Peptid | SPERMIDINE | 0,5169231 | 0 | 25 |
| CLEY24E3 | mas-binding factor MBF2=transcription factor TGA1a homolog {Solanum tuberosum=potatoes, root, Peptid | TYROSINE | 0,5353846 | 0 | 25 |
| CLEY26B11 | URIDYLATE KINASE (EC 2.7.4.-) (UK) (URIDINE MONOPHOSPHATE KINASE) (UMP KINASE) (UMP/CMP KINASE).GP\| | GABA | 0,5061538 | 0 | 25 |
| CLEY26B11 | URIDYLATE KINASE (EC 2.7.4.-) (UK) (URIDINE MONOPHOSPHATE KINASE) (UMP KINASE) (UMP/CMP KINASE).GP\| | INOSITOL | -0,8092307 | 1 | 25 |
| CLEY26B11 | URIDYLATE KINASE (EC 2.7.4.-) (UK) (URIDINE MONOPHOSPHATE KINASE) (UMP KINASE) (UMP/CMP KINASE).GP\| | MELEZITOSE | -0,6315789 | 0 | 19 |
| CLEY26B11 | URIDYLATE KINASE (EC 2.7.4.-) (UK) (URIDINE MONOPHOSPHATE KINASE) (UMP KINASE) (UMP/CMP KINASE).GP\| | SPERMIDINE | 0,5407692 | 0 | 25 |
| CLEY26B11 | URIDYLATE KINASE (EC 2.7.4.-) (UK) (URIDINE MONOPHOSPHATE KINASE) (UMP KINASE) (UMP/CMP KINASE).GP\| | TYROSINE | 0,6523077 | 1 | 25 |
| CLEY2E3 | UDP-glucose dehydrogenase-like protein {Arabidopsis thaliana}PIR\|T51527\|T51527 UDP-glucose dehydrog | GABA | 0,5315385 | 0 | 25 |
| CLEY2E3 | UDP-glucose dehydrogenase-like protein {Arabidopsis thaliana}PIR\|T51527\|T51527 UDP-glucose dehydrog | INOSITOL | -0,6569231 | 1 | 25 |
| CLEY2E3 | UDP-glucose dehydrogenase-like protein {Arabidopsis thaliana}PIR\|T51527\|T51527 UDP-glucose dehydrog | SPERMIDINE | 0,5623077 | 0 | 25 |

EP 1 454 993 A1

| | | | | |
|---|---|---|---|---|---|
| CLEZ16P14 | dTDP-glucose 4-6-dehydratases-like protein {Arabidopsis thaliana}PIR\|T45701\|T45701 dTDP-glucose 4-6 | INOSITOL | -0,7861539 | 1 | 25 |
| CLEZ16P14 | dTDP-glucose 4-6-dehydratases-like protein {Arabidopsis thaliana}PIR\|T45701\|T45701 dTDP-glucose 4-6 | MELEZITOSE | -0,6140351 | 0 | 19 |
| CLEZ16P14 | dTDP-glucose 4-6-dehydratases-like protein {Arabidopsis thaliana}PIR\|T45701\|T45701 dTDP-glucose 4-6 | SERINE | 0,5623077 | 0 | 25 |
| CLEZ16P14 | dTDP-glucose 4-6-dehydratases-like protein {Arabidopsis thaliana}PIR\|T45701\|T45701 dTDP-glucose 4-6 | SPERMIDINE | 0,6 | 0 | 25 |
| CLEZ16P14 | dTDP-glucose 4-6-dehydratases-like protein {Arabidopsis thaliana}PIR\|T45701\|T45701 dTDP-glucose 4-6 | THREONINE | 0,5278261 | 0 | 24 |
| CLEZ16P14 | dTDP-glucose 4-6-dehydratases-like protein {Arabidopsis thaliana}PIR\|T45701\|T45701 dTDP-glucose 4-6 | TRYPTOPHAN | 0,5365217 | 0 | 24 |
| CLEZ16P14 | dTDP-glucose 4-6-dehydratases-like protein {Arabidopsis thaliana}PIR\|T45701\|T45701 dTDP-glucose 4-6 | TYROSINE | 0,6453846 | 1 | 25 |
| CLEZ17K21 | contains similarity to RING zinc finger protein~gene_id:MBD2.14 {Arabidopsis thaliana} | INOSITOL | -0,6253846 | 1 | 25 |
| CLEZ17K21 | contains similarity to RING zinc finger protein~gene_id:MBD2.14 {Arabidopsis thaliana} | MELEZITOSE | -0,5894737 | 0 | 19 |
| CLEZ17K21 | contains similarity to RING zinc finger protein~gene_id:MBD2.14 {Arabidopsis thaliana} | TYROSINE | 0,5907692 | 0 | 25 |
| CLEZ20E22 | Similar to acyl carrier protein, mitochondrial precursor (ACP) NADH-ubiquinone oxidoreductase 9.6 KD | GABA | 0,5469231 | 0 | 25 |
| CLEZ20E22 | Similar to acyl carrier protein, mitochondrial precursor (ACP) NADH-ubiquinone oxidoreductase 9.6 KD | INOSITOL | -0,73 | 1 | 25 |
| CLEZ20E22 | Similar to acyl carrier protein, mitochondrial precursor (ACP) NADH-ubiquinone oxidoreductase 9.6 KD | SPERMIDINE | 0,5784615 | 0 | 25 |
| CLEZ20E22 | Similar to acyl carrier protein, mitochondrial precursor (ACP) NADH-ubiquinone oxidoreductase 9.6 KD | TYROSINE | 0,6161538 | 0 | 25 |
| CLEZ20I22 | caffeoyl-CoA O-methyltransferase 5 {Nicotiana tabacum}GP\|1679853\|emb\|CAB05369.1\|\|Z82982 caffeoyl-Co | GLYCERATE | -0,5643478 | 0 | 24 |
| CLEZ20I22 | caffeoyl-CoA O-methyltransferase 5 {Nicotiana tabacum}GP\|1679853\|emb\|CAB05369.1\|\|Z82982 caffeoyl-Co | INOSITOL | 0,7376923 | 1 | 25 |

| | | | | | |
|---|---|---|---|---|---|
| CLEZ20I22 | caffeoyl-CoA O-methyltransferase 5 {Nicotiana tabacum}GP\|1679853\|emb\|CAB05369.1\|\|Z82982 caffeoyl-Co | SPERMIDINE | -0,56 | 0 | 25 |
| CLEZ20I22 | caffeoyl-CoA O-methyltransferase 5 {Nicotiana tabacum}GP\|1679853\|emb\|CAB05369.1\|\|Z82982 caffeoyl-Co | SUCROSE | 0,5834783 | 0 | 24 |
| CLEZ20I22 | caffeoyl-CoA O-methyltransferase 5 {Nicotiana tabacum}GP\|1679853\|emb\|CAB05369.1\|\|Z82982 caffeoyl-Co | TRYPTOPHAN | -0,5695652 | 0 | 24 |
| CLEZ20I22 | caffeoyl-CoA O-methyltransferase 5 {Nicotiana tabacum}GP\|1679853\|emb\|CAB05369.1\|\|Z82982 caffeoyl-Co | TYROSINE | -0,6776923 | 1 | 25 |
| CLEZ8J8 | "putative transcription factor BTF3 (RNA polymerase B transcription factor 3); 26343-27201 {Arabidops" | INOSITOL | -0,7307692 | 1 | 25 |
| CLEZ8J8 | "putative transcription factor BTF3 (RNA polymerase B transcription factor 3); 26343-27201 {Arabidops" | THREONINE | 0,5321739 | 0 | 24 |
| CLEZ8J8 | "putative transcription factor BTF3 (RNA polymerase B transcription factor 3); 26343-27201 {Arabidops" | TYROSINE | 0,6807692 | 1 | 25 |
| CLEZ9G24 | succinate dehydrogenase flavoprotein alpha subunit {Arabidopsis thaliana}GP\|8843734\|dbj\|BAA97282.1\| | GABA | 0,5692308 | 0 | 25 |
| CLEZ9G24 | succinate dehydrogenase flavoprotein alpha subunit {Arabidopsis thaliana}GP\|8843734\|dbj\|BAA97282.1\| | INOSITOL | -0,7292308 | 1 | 25 |
| CLEZ9G24 | succinate dehydrogenase flavoprotein alpha subunit {Arabidopsis thaliana}GP\|8843734\|dbj\|BAA97282.1\| | SERINE | 0,5738462 | 0 | 25 |
| CLEZ9G24 | succinate dehydrogenase flavoprotein alpha subunit {Arabidopsis thaliana}GP\|8843734\|dbj\|BAA97282.1\| | SPERMIDINE | 0,6384615 | 1 | 25 |
| CLEZ9G24 | succinate dehydrogenase flavoprotein alpha subunit {Arabidopsis thaliana}GP\|8843734\|dbj\|BAA97282.1\| | THREONATE | 0,5207692 | 0 | 25 |
| CLEZ9G24 | succinate dehydrogenase flavoprotein alpha subunit {Arabidopsis thaliana}GP\|8843734\|dbj\|BAA97282.1\| | TYROSINE | 0,5923077 | 0 | 25 |
| CLHT18D23 | TOM (target of myb1)-like protein {Arabidopsis thaliana}PIR\|T51543\|T51543 TOM (target of myb1)-like | INOSITOL | -0,6846154 | 1 | 25 |
| CLHT18D23 | TOM (target of myb1)-like protein {Arabidopsis thaliana}PIR\|T51543\|T51543 TOM (target of myb1)-like | SERINE | 0,5130769 | 0 | 25 |
| CLHT18D23 | TOM (target of myb1)-like protein {Arabidopsis thaliana}PIR\|T51543\|T51543 TOM (target of myb1)-like | SPERMIDINE | 0,64 | 1 | 25 |

| CLHT18D23 | TOM (target of myb1)-like protein {Arabidopsis thaliana}PIR\|T51543\|T51543 TOM (target of myb1)-like | TYROSINE | 0,5407692 | 0 | 25 |
|---|---|---|---|---|---|
| CLHT23L11 | cytochrome P450-like protein {Arabidopsis thaliana}GP\|7270932\|emb\|CAB80611.1\|\|AL161595 cytochrome P | INOSITOL | -0,6053846 | 0 | 25 |
| CLHT31P20 | glycolate oxidase {Arabidopsis thaliana} | INOSITOL | -0,5346154 | 0 | 25 |
| CLHT31P20 | glycolate oxidase {Arabidopsis thaliana} | TYROSINE | 0,5192308 | 0 | 25 |
| CLHT6C11 | w-3 desaturase {Solanum tuberosum}PIR\|T07685\|T07685 omega-3 fatty acid desaturase (EC 1.14.99.-) - | INOSITOL | -0,75 | 1 | 25 |
| CLHT6C11 | w-3 desaturase {Solanum tuberosum}PIR\|T07685\|T07685 omega-3 fatty acid desaturase (EC 1.14.99.-) - | LYSINE | 0,5915385 | 0 | 25 |
| CLHT6C11 | w-3 desaturase {Solanum tuberosum}PIR\|T07685\|T07685 omega-3 fatty acid desaturase (EC 1.14.99.-) - | MELEZITOSE | -0,6070175 | 0 | 19 |
| CLHT6C11 | w-3 desaturase {Solanum tuberosum}PIR\|T07685\|T07685 omega-3 fatty acid desaturase (EC 1.14.99.-) - | SPERMIDINE | 0,5407692 | 0 | 25 |
| CLHT6C11 | w-3 desaturase {Solanum tuberosum}PIR\|T07685\|T07685 omega-3 fatty acid desaturase (EC 1.14.99.-) - | TYROSINE | 0,6907693 | 1 | 25 |
| CLHT6E15 | TOM (target of myb1)-like protein {Arabidopsis thaliana}PIR\|T51543\|T51543 TOM (target of myb1)-like | GABA | 0,5215384 | 0 | 25 |
| CLHT6E15 | TOM (target of myb1)-like protein {Arabidopsis thaliana}PIR\|T51543\|T51543 TOM (target of myb1)-like | INOSITOL | -0,7238461 | 1 | 25 |
| CLHT6E15 | TOM (target of myb1)-like protein {Arabidopsis thaliana}PIR\|T51543\|T51543 TOM (target of myb1)-like | SERINE | 0,5638462 | 0 | 25 |
| CLHT6E15 | TOM (target of myb1)-like protein {Arabidopsis thaliana}PIR\|T51543\|T51543 TOM (target of myb1)-like | SPERMIDINE | 0,64 | 1 | 25 |
| CLHT6E15 | TOM (target of myb1)-like protein {Arabidopsis thaliana}PIR\|T51543\|T51543 TOM (target of myb1)-like | THREONINE | 0,5165218 | 0 | 24 |
| CLHT6E15 | TOM (target of myb1)-like protein {Arabidopsis thaliana}PIR\|T51543\|T51543 TOM (target of myb1)-like | TYROSINE | 0,6069231 | 0 | 25 |
| CLPP11G6 | putative monosaccharide transporter 1 {Petunia x hybrida} | INOSITOL | -0,5330769 | 0 | 25 |
| CLPP11N17 | sugar transporter like protein {Arabidopsis thaliana}GP\|2464913\|emb\|CAB16808.1\|\|Z99708 sugar transp | INOSITOL | -0,5861539 | 0 | 25 |
| CLPP11N17 | sugar transporter like protein {Arabidopsis thaliana}GP\|2464913\|emb\|CAB16808.1\|\|Z99708 sugar transp | MELEZITOSE | -0,5842105 | 0 | 19 |

EP 1 454 993 A1

| | | | | | |
|---|---|---|---|---|---|
| CLPP13C23 | fructokinase 1 {Arabidopsis thaliana}GP|13878053|gb|AAK44104.1|AF370289_1|AF370289 putative fructok | INOSITOL | -0,5376923 | 0 | 25 |
| CLPP13M11 | fructokinase 1 {Arabidopsis thaliana}GP|13878053|gb|AAK44104.1|AF370289_1|AF370289 putative fructok | GABA | 0,5292308 | 0 | 25 |
| CLPP13M11 | fructokinase 1 {Arabidopsis thaliana}GP|13878053|gb|AAK44104.1|AF370289_1|AF370289 putative fructok | INOSITOL | -0,5638462 | 0 | 25 |
| CLPP2C18 | ALTERNATIVE OXIDASE 1 PRECURSOR (EC 1.-.-.-).GP|558054|gb|AAC60576.1||S71335 alternative oxidase, A | GABA | 0,6276923 | 1 | 25 |
| CLPP2C18 | ALTERNATIVE OXIDASE 1 PRECURSOR (EC 1.-.-.-).GP|558054|gb|AAC60576.1||S71335 alternative oxidase, A | INOSITOL | -0,7538462 | 1 | 25 |
| CLPP2C18 | ALTERNATIVE OXIDASE 1 PRECURSOR (EC 1.-.-.-).GP|558054|gb|AAC60576.1||S71335 alternative oxidase, A | SPERMIDINE | 0,65 | 1 | 25 |
| CLPP2C18 | ALTERNATIVE OXIDASE 1 PRECURSOR (EC 1.-.-.-).GP|558054|gb|AAC60576.1||S71335 alternative oxidase, A | TRYPTOPHAN | 0,526087 | 0 | 24 |
| CLPP2C18 | ALTERNATIVE OXIDASE 1 PRECURSOR (EC 1.-.-.-).GP|558054|gb|AAC60576.1||S71335 alternative oxidase, A | TYROSINE | 0,5646154 | 0 | 25 |
| CLPP5L24 | glutamate decarboxylase isozyme 1 {Nicotiana tabacum} | GABA | 0,5592307 | 0 | 25 |
| CLPP5L24 | glutamate decarboxylase isozyme 1 {Nicotiana tabacum} | INOSITOL | -0,7623077 | 1 | 25 |
| CLPP5L24 | glutamate decarboxylase isozyme 1 {Nicotiana tabacum} | MELEZITOSE | -0,6140351 | 0 | 19 |
| CLPP5L24 | glutamate decarboxylase isozyme 1 {Nicotiana tabacum} | SPERMIDINE | 0,6376923 | 1 | 25 |
| CLPP5L24 | glutamate decarboxylase isozyme 1 {Nicotiana tabacum} | THREONINE | 0,5156522 | 0 | 24 |
| CLPP5L24 | glutamate decarboxylase isozyme 1 {Nicotiana tabacum} | TRYPTOPHAN | 0,5878261 | 0 | 24 |
| CLPP5L24 | glutamate decarboxylase isozyme 1 {Nicotiana tabacum} | TYROSINE | 0,6284615 | 1 | 25 |
| CLPP8K20 | phosphate/phosphoenolpyruvate translocator-like protein {Arabidopsis thaliana} | INOSITOL | -0,6984615 | 1 | 25 |
| CLPP9C20 | phosphate/phosphoenolpyruvate translocator-like protein {Arabidopsis thaliana} | GABA | 0,5584615 | 0 | 25 |
| CLPP9C20 | phosphate/phosphoenolpyruvate translocator-like protein {Arabidopsis thaliana} | INOSITOL | -0,6769231 | 1 | 25 |
| CLPP9C20 | phosphate/phosphoenolpyruvate translocator-like protein {Arabidopsis thaliana} | SPERMIDINE | 0,5523077 | 0 | 25 |

| CLPP9O23 | glutamate decarboxylase isozyme 1 {Nicotiana tabacum} | GABA | 0,6446154 | 1 | 25 |
|---|---|---|---|---|---|
| CLPP9O23 | glutamate decarboxylase isozyme 1 {Nicotiana tabacum} | INOSITOL | -0,6515385 | 1 | 25 |
| CLPP9O23 | glutamate decarboxylase isozyme 1 {Nicotiana tabacum} | SPERMIDINE | 0,6115385 | 0 | 25 |
| CLPP9O23 | glutamate decarboxylase isozyme 1 {Nicotiana tabacum} | TYROSINE | 0,5169231 | 0 | 25 |
| CLPT10L12 | glycogen (starch) synthase precursor {Solanum tuberosum}SP\|Q00775\|UGST_SOLTU GRANULE-BOUND GLYCOGEN | GABA | 0,5923077 | 0 | 25 |
| CLPT10L12 | glycogen (starch) synthase precursor {Solanum tuberosum}SP\|Q00775\|UGST_SOLTU GRANULE-BOUND GLYCOGEN | INOSITOL | -0,7446154 | 1 | 25 |
| CLPT10L12 | glycogen (starch) synthase precursor {Solanum tuberosum}SP\|Q00775\|UGST_SOLTU GRANULE-BOUND GLYCOGEN | NORVALINE | -0,9428571 | 0 | 6 |
| CLPT10L12 | glycogen (starch) synthase precursor {Solanum tuberosum}SP\|Q00775\|UGST_SOLTU GRANULE-BOUND GLYCOGEN | ORNITHINE | -0,9428571 | 0 | 6 |
| CLPT10L12 | glycogen (starch) synthase precursor {Solanum tuberosum}SP\|Q00775\|UGST_SOLTU GRANULE-BOUND GLYCOGEN | SPERMIDINE | 0,5930769 | 0 | 25 |
| CLPT10L12 | glycogen (starch) synthase precursor {Solanum tuberosum}SP\|Q00775\|UGST_SOLTU GRANULE-BOUND GLYCOGEN | SUCROSE | -0,5582609 | 0 | 24 |
| CLPT10L12 | glycogen (starch) synthase precursor {Solanum tuberosum}SP\|Q00775\|UGST_SOLTU GRANULE-BOUND GLYCOGEN | TRYPTOPHAN | 0,5391304 | 0 | 24 |
| CLPT10L12 | glycogen (starch) synthase precursor {Solanum tuberosum}SP\|Q00775\|UGST_SOLTU GRANULE-BOUND GLYCOGEN | TYROSINE | 0,6007692 | 0 | 25 |
| CLPT11O16 | putative ripening-related bZIP protein {Vitis vinifera} | SORBITOL/GALACTITOL | 0,627451 | 0 | 17 |
| CLPT5N10 | aldose 1-epimerase-like protein {Arabidopsis thaliana} | SORBITOL/GALACTITOL | 0,6519608 | 0 | 17 |
| CLPT8B12 | ARF GAP-like zinc finger-containing protein ZiGA4 {Arabidopsis thaliana} | INOSITOL | -0,5107692 | 0 | 25 |
| CTOA19J6 | cytochrome p450 lxxia4 {Solanum melongena}SP\|P37117\|C714_SOLME CYTOCHROME P450 71A4 (EC 1.14.-.-) ( | INOSITOL | -0,5915385 | 0 | 25 |
| CTOA19J6 | cytochrome p450 lxxia4 {Solanum melongena}SP\|P37117\|C714_SOLME CYTOCHROME P450 71A4 (EC 1.14.-.-) ( | TYROSINE | 0,5215384 | 0 | 25 |
| CTOE12M9 | flavanone 3-hydroxylase-like protein {Arabidopsis thaliana} | GABA | 0,5192308 | 0 | 25 |
| CTOE12M9 | flavanone 3-hydroxylase-like protein {Arabidopsis thaliana} | INOSITOL | -0,6238462 | 1 | 25 |

EP 1 454 993 A1

| CTOE12M9 | flavanone 3-hydroxylase-like protein {Arabidopsis thaliana} | MANNOSE | -0,608131 | 0 | 22 |
|---|---|---|---|---|---|
| CTOE17L19 | PROBABLE VACUOLAR ATP SYNTHASE SUBUNIT F (EC 3.6.1.34) (V-ATPASE F SUBUNIT) (VACUOLAR PROTON PUMP F | GABA | 0,5215384 | 0 | 25 |
| CTOE17L19 | PROBABLE VACUOLAR ATP SYNTHASE SUBUNIT F (EC 3.6.1.34) (V-ATPASE F SUBUNIT) (VACUOLAR PROTON PUMP F | GALACTOSE | -0,52 | 0 | 25 |
| CTOE17L19 | PROBABLE VACUOLAR ATP SYNTHASE SUBUNIT F (EC 3.6.1.34) (V-ATPASE F SUBUNIT) (VACUOLAR PROTON PUMP F | INOSITOL | -0,6061538 | 0 | 25 |
| CTOE17L19 | PROBABLE VACUOLAR ATP SYNTHASE SUBUNIT F (EC 3.6.1.34) (V-ATPASE F SUBUNIT) (VACUOLAR PROTON PUMP F | SPERMIDINE | 0,6846154 | 1 | 25 |
| CTOE17L19 | PROBABLE VACUOLAR ATP SYNTHASE SUBUNIT F (EC 3.6.1.34) (V-ATPASE F SUBUNIT) (VACUOLAR PROTON PUMP F | SUCROSE | -0,5173913 | 0 | 24 |
| CTOE17L19 | PROBABLE VACUOLAR ATP SYNTHASE SUBUNIT F (EC 3.6.1.34) (V-ATPASE F SUBUNIT) (VACUOLAR PROTON PUMP F | TYROSINE | 0,56 | 0 | 25 |
| CTOE2C17 | delta 1-pyrroline-5-carboxylate synthetase | INOSITOL | -0,5315385 | 0 | 25 |
| CTOE2C17 | delta 1-pyrroline-5-carboxylate synthetase | MANNOSE | -0,5448899 | 0 | 22 |
| CTOE2F5 | putative cytochrome P450 | GABA | 0,5407692 | 0 | 25 |
| CTOE2F5 | putative cytochrome P450 | INOSITOL | -0,6776923 | 1 | 25 |
| CTOE2F5 | putative cytochrome P450 | SPERMIDINE | 0,6046154 | 0 | 25 |
| CTOE2F5 | putative cytochrome P450 | TRYPTOPHAN | 0,5226087 | 0 | 24 |
| CTOE2F5 | putative cytochrome P450 | TYROSINE | 0,5546154 | 0 | 25 |
| CTOE6J10 | transcription factor IIA small subunit {Arabidopsis thaliana}GP\|5051786\|emb\|CAB45079.1\|\|AL078637 tr | INOSITOL | -0,5969231 | 0 | 25 |
| CTOE6J10 | transcription factor IIA small subunit {Arabidopsis thaliana}GP\|5051786\|emb\|CAB45079.1\|\|AL078637 tr | SPERMIDINE | 0,5830769 | 0 | 25 |
| CTOE6J10 | transcription factor IIA small subunit {Arabidopsis thaliana}GP\|5051786\|emb\|CAB45079.1\|\|AL078637 tr | THREONATE | 0,5576923 | 0 | 25 |
| CTOF10I1 | ATP synthase delta' subunit, mitochondrial precursor {Ipomoea batatas}SP\|Q40089\|ATP4_IPOBA ATP SYNT | INOSITOL | -0,74 | 1 | 25 |
| CTOF10I1 | ATP synthase delta' subunit, mitochondrial precursor {Ipomoea batatas}SP\|Q40089\|ATP4_IPOBA ATP SYNT | MELEZITOSE | -0,5807018 | 0 | 19 |
| CTOF10I1 | ATP synthase delta' subunit, mitochondrial precursor {Ipomoea batatas}SP\|Q40089\|ATP4_IPOBA ATP SYNT | SERINE | 0,5107692 | 0 | 25 |
| CTOF10I1 | ATP synthase delta' subunit, mitochondrial precursor {Ipomoea batatas}SP\|Q40089\|ATP4_IPOBA ATP SYNT | SPERMIDINE | 0,6292308 | 1 | 25 |

| CTOF10I1 | ATP synthase delta' subunit, mitochondrial precursor {Ipomoea batatas}SP\|Q40089\|ATP4_IPOBA ATP SYNT | TYROSINE | 0,6846154 | 1 | 25 |
|---|---|---|---|---|---|
| CTOF10L8 | S-adenosyl-L-methionine synthetase | INOSITOL | -0,6415384 | 1 | 25 |
| CTOF10L8 | S-adenosyl-L-methionine synthetase | SPERMIDINE | 0,6230769 | 1 | 25 |
| CTOF10L8 | S-adenosyl-L-methionine synthetase | SUCROSE | -0,5252174 | 0 | 24 |
| CTOF10L8 | S-adenosyl-L-methionine synthetase | TYROSINE | 0,6030769 | 0 | 25 |
| CTOF14N18 | small zinc finger-like protein | INOSITOL | -0,5153846 | 0 | 25 |
| CTOF14N18 | small zinc finger-like protein | TYROSINE | 0,5407692 | 0 | 25 |
| CTOF19M22 | zinc finger protein-like {Arabidopsis thaliana} | INOSITOL | -0,6023077 | 0 | 25 |
| CTOF19M22 | zinc finger protein-like {Arabidopsis thaliana} | SPERMIDINE | 0,5561538 | 0 | 25 |
| CTOF19M22 | zinc finger protein-like {Arabidopsis thaliana} | TYROSINE | 0,5707693 | 0 | 25 |
| CTOF21A12 | glycogen (starch) synthase precursor {Solanum tuberosum}SP\|Q00775\|UGST_SOLTU GRANULE-BOUND GLYCOGEN | INOSITOL | -0,6869231 | 1 | 25 |
| CTOF21A12 | glycogen (starch) synthase precursor {Solanum tuberosum}SP\|Q00775\|UGST_SOLTU GRANULE-BOUND GLYCOGEN | TYROSINE | 0,6715385 | 1 | 25 |
| CTOF22G14 | MYB-like DNA-binding protein {Catharanthus roseus} | GABA | 0,5807692 | 0 | 25 |
| CTOF22G14 | MYB-like DNA-binding protein {Catharanthus roseus} | INOSITOL | -0,7461538 | 1 | 25 |
| CTOF22G14 | MYB-like DNA-binding protein {Catharanthus roseus} | SPERMIDINE | 0,6292308 | 1 | 25 |
| CTOF22G14 | MYB-like DNA-binding protein {Catharanthus roseus} | TYROSINE | 0,6146154 | 0 | 25 |
| CTOF23N20 | "putative transcription factor BTF3 (RNA polymerase B transcription factor 3); 26343-27201 {Arabidops" | INOSITOL | -0,7276923 | 1 | 25 |
| CTOF23N20 | "putative transcription factor BTF3 (RNA polymerase B transcription factor 3); 26343-27201 {Arabidops" | TYROSINE | 0,7007692 | 1 | 25 |
| CTOF25A17 | small zinc finger-like protein | MALTITOL | 0,9428571 | 0 | 6 |
| CTOF26K3 | S-adenosylmethionine:2-demethylmenaquinone methyltransferase-like protein {Arabidopsis thaliana} | INOSITOL | -0,5338461 | 0 | 25 |
| CTOF26K3 | S-adenosylmethionine:2-demethylmenaquinone methyltransferase-like protein {Arabidopsis thaliana} | SPERMIDINE | 0,5161539 | 0 | 25 |
| CTOF26K3 | S-adenosylmethionine:2-demethylmenaquinone methyltransferase-like protein {Arabidopsis thaliana} | TYROSINE | 0,5476923 | 0 | 25 |
| CTOF3J14 | nucleotide diphosphate kinase Ia {Arabidopsis thaliana}GP\|6065740\|emb\|CAB58230.1\|\|AJ012758 nucleoti | INOSITOL | -0,5192308 | 0 | 25 |
| CTOF3K21 | S-adenosyl-L-methionine synthetase | INOSITOL | -0,6761538 | 1 | 25 |
| CTOF3K21 | S-adenosyl-L-methionine synthetase | LYSINE | 0,6038461 | 0 | 25 |
| CTOF3K21 | S-adenosyl-L-methionine synthetase | TYROSINE | 0,66 | 1 | 25 |

EP 1 454 993 A1

| CTOF6M4 | homeodomain protein | INOSITOL | -0,7223077 | 1 | 25 |
|---------|---------------------|----------|-----------|---|-----|
| CTOF6M4 | homeodomain protein | SERINE | 0,5592307 | 0 | 25 |
| CTOF6M4 | homeodomain protein | SPERMIDINE | 0,6046154 | 0 | 25 |
| CTOF6M4 | homeodomain protein | THREONINE | 0,5304348 | 0 | 24 |
| CTOF6M4 | homeodomain protein | TYROSINE | 0,6284615 | 1 | 25 |
| CTOF7K11 | cytochrome P450 like_TBP {Nicotiana tabacum}GP\|1545805\|dbj\|BAA10929.1\|\|D64052 cytochrome P450 like_ | GLYCEROL | 0,5474308 | 0 | 23 |
| CTOF7K11 | cytochrome P450 like_TBP {Nicotiana tabacum}GP\|1545805\|dbj\|BAA10929.1\|\|D64052 cytochrome P450 like_ | INOSITOL | -0,5107692 | 0 | 25 |
| CTOF7K11 | cytochrome P450 like_TBP {Nicotiana tabacum}GP\|1545805\|dbj\|BAA10929.1\|\|D64052 cytochrome P450 like_ | THREONINE | 0,5252174 | 0 | 24 |
| CTOF7K11 | cytochrome P450 like_TBP {Nicotiana tabacum}GP\|1545805\|dbj\|BAA10929.1\|\|D64052 cytochrome P450 like_ | TYROSINE | 0,5446154 | 0 | 25 |

| Table 4<br><br>Description | TC | Accession No. | Clone ID | Position on the 94-well plate | Position on the 384 wells-plate | Position on the filter (primary rows) | Position on the filter (primary columns) | Position position on secondary grid | Position for replicates on secondary grid |
|---|---|---|---|---|---|---|---|---|---|
| hexose transporter^^hexose transporter protein^^pathogenesis-related protein P4^^pathogenesis-related protein PR1a (P4) | TC71791 | AW040775 | cLET10J17 | XIC3 | T3F5 | R20 | C6 | r2-c1 | r4-c3 |
| 33kDa precursor protein of oxygen-evolving complex | TC71796 | AW093398 | cLET24B18 | XIF6 | T3L11 | R14 | C12 | r2-c1 | r4-c3 |
| ADP-glucose pyrophosphorylase small subunit | TC71797 | BF050665 | cLEM19M11 | VIIH8 | T2P15 | R10 | C16 | r4-c2 | r1-c3 |
| utamine synthetase | TC71798 | AW626325 | cLEZ19B18 | XIVF2 | T4K4 | R21 | C11 | r2-c2 | r1-c4 |
| cytochrome P450 like_TBP {Nicotiana tabacum}GP\|1545805\|dbj\|BAA10929.1\|\|D64052 cytochrome P450 like_ | TC71805 | BG124938 | cTOF7K11 | XXF12 | T5L24 | R1 | C12 | r3-c2 | r4-c4 |
| fructose-bisphosphate aldolase {Persea americana} | TC71818 | AW931478 | cLEZ7L12 | XC3 | T3E6 | R19 | C5 | r2-c1 | r4-c3 |
| plastidic aldolase NPALDP1 {Nicotiana paniculata} | TC71821 | BG125770 | cTOF9F8 | XXIE8 | T6I15 | R12 | C9 | r3-c1 | r2-c4 |
| glutamate decarboxylase {Petunia hybrida}SP\|Q07346\|DCE_PETHY GLUTAMATE DECARBOXYLASE (EC 4.1.1.15) | TC71841 | AW622545 | cLEX15F21 | XIIIC11 | T4E21 | R4 | C5 | r2-c2 | r1-c4 |
| phenylalanine ammonia lyase | TC71847 | AI777201 | cLER20L9 | XB6 | T3C12 | R13 | C3 | r2-c1 | r4-c3 |
| glutamate decarboxylase isozyme 1 {Nicotiana tabacum} | TC71868 | BG137392 | cLPP5L24 | XVF1 | T4L1 | R24 | C12 | r2-c2 | r1-c4 |
| plastidic aldolase {Nicotiana paniculata} | TC71875 | AI781396 | cLES15A20 | XE1 | T3I2 | R23 | C9 | r2-c1 | r4-c3 |
| fructose-bisphosphate aldolase {Persea americana} | TC71877 | AW041348 | cLET13N6 | XID1 | T3H1 | R24 | C8 | r2-c1 | r4-c3 |
| ADENINE PHOSPHORIBOSYLTRANSFERASE 1 (EC 2.4.2.7) (APRT).GP\|16164\|emb\|CAA41497.1\|\|X58640 adenine ph | TC71878 | AW092817 | cLET22M4 | XIF3 | T3L5 | R20 | C12 | r2-c1 | r4-c3 |
| ribulose 1,5-bisphosphate carboxylase/oxygenase small subunit^^ribulose 1,5-bisphosphate carboxylase/oxyenase | TC71908 | AW096400 | cLET38B9 | XXH6 | T5P12 | R13 | C16 | r3-c2 | r4-c4 |
| ribulose-1,5-bisphosphate carboxylase, small subunit precursor^^ribulose 1,5-bisphosphate carboxylase/oxygenase^^ribulose-1,5-bisphophate carboxylase/ oxygenase small subunit | TC71912 | AI776560 | cLER19C5 | IXH12 | T3O23 | R2 | C15 | r2-c1 | r4-c3 |
| phosphoribosyl diphosphate synthase {Arabidopsis | TC71917 | AI489108 | cLED18D1 | IIIB6 | T1D11 | R14 | C4 | r1-c2 | r3-c3 |

EP 1 454 993 A1

| | | | | | | | | | |
|---|---|---|---|---|---|---|---|---|---|
| thaliana}GP\|4512664\|gb\|AAD21718.1\|\|AC006931 putati | | | 3 | | | | | | |
| glutamate dehydrogenase | TC71919 | AI782814 | cLES20J18 | XG9 | T3M18 | R7 | C13 | r2-c1 | r4-c3 |
| ribulose bisphosphate carboxylase small subunit 1 precursor {Lycopersicon esculentum}SP\|P08706\|RBS1 | TC71926 | BG124240 | cTOF4F21 | XXE4 | T5J8 | R17 | C10 | r3-c2 | r4-c4 |
| plastidic aldolase NPALDP1 {Nicotiana paniculata} | TC71931 | AW399700 | cLPT8B16 | XXID3 | T6G5 | R20 | C7 | r3-c1 | r2-c4 |
| spermine synthase 1 {Datura stramonium}SP\|Q96556\|SPE1_DATST SPERMIDINE SYNTHASE 1 (EC 2.5.1.16) (PU | TC71984 | AI779341 | cLES7H6 | XIA8 | T3B15 | R12 | C2 | r2-c1 | r4-c3 |
| glutamine synthetase {Lycopersicon esculentum} | TC71994 | BF098043 | cLEW25C9 | XIIG7 | T3N14 | R11 | C14 | r2-c1 | r4-c3 |
| glutamate decarboxylase isozyme 1 {Nicotiana tabacum} | TC71998 | AW625683 | cLEZ16B13 | XIVD10 | T4G20 | R5 | C7 | r2-c2 | r1-c4 |
| beta-fructosidase | TC72004 | AW222371 | cLEN7H24 | IXD5 | T3G9 | R16 | C7 | r2-c1 | r4-c3 |
| acid invertase, AI {EC 3.2.1.26} [Lycopersicon esculentum=tomatoes, cv. Super First, fruits, Peptide, 636 aa]^^vacuolar invertase precursor^^beta-fructofuranosidase | TC72005 | BF112978 | cLEG43E2 | VIG1 | T2M2 | R23 | C13 | r4-c2 | r1-c3 |
| beta-fructofuranosidase precursor {Lycopersicon esculentum}SP\|P29000\|INVA_LYCES ACID BETA-FRUCTOFUR | TC72006 | BE431852 | cLEG4K9 | VIH1 | T2O2 | R23 | C15 | r4-c2 | r1-c3 |
| beta-fructofuranosidase precursor {Lycopersicon esculentum}SP\|P29000\|INVA_LYCES ACID BETA-FRUCTOFUR | TC72007 | BE437004 | cLEG35M1 | VIC8 | T2E16 | R9 | C5 | r4-c2 | r1-c3 |
| enolase | TC72015 | BG131734 | cTOE5I3 | XXIA10 | T6A19 | R6 | C1 | r3-c1 | r2-c4 |
| threonine deaminase | TC72016 | AI488726 | cLED13N11 | IIH9 | T1O18 | R7 | C15 | r1-c2 | r3-c3 |
| ubiquinol--cytochrome-c reductase (EC 1.10.2.2) Rieske iron-sulfur protein - potato | TC72021 | AW037813 | cLET3D18 | XXIH9 | T6O17 | R8 | C15 | r3-c1 | r2-c4 |
| polyphenol oxidase precursor | TC72054 | AW650785 | cLEI14K20 | VIIC3 | T2F5 | R20 | C6 | r4-c2 | r1-c3 |
| polyphenol oxidase precursor | TC72055 | BG133913 | cTOE14D12 | XVIIIC4 | T5E8 | R17 | C5 | r3-c2 | r4-c4 |
| polyphenol oxidase precursor | TC72056 | AW217970 | cTOD6A1 | XVIF2 | T4L4 | R21 | C12 | r2-c2 | r1-c4 |
| polyphenoloxidase, P2 [Lycopersicon esculentum=tomatoes, cv Tiny Tim LA154, flowers, Peptide Chloroplast, 587 aa]^^polyphenol oxidase precursor | TC72057 | BG129916 | cTOF28D12 | XXIH4 | T6O7 | R18 | C15 | r3-c1 | r2-c4 |
| vacuolar ATP synthase subunit b isoform 1 subunit) | TC72080 | BE451002 | cLEC16G9 | XIVA1 | T4A2 | R23 | C1 | r2-c2 | r1-c4 |

| | | | | | | | | |
|---|---|---|---|---|---|---|---|---|
| {Gossypium hirsutum}SP\|Q43432\|VAT1_GOSHI VACUOLA | | | | | | | | |
| cytochrome p450 lxxvia2 {Solanum melongena}SP\|P37122\|C762_SOLME CYTOCHROME P450 76A2 (EC 1.14.-.-) | TC72085 | AI779703 | cLES8J16 | XIA12 | T3B23 | R2 | C2 | r2-c1 | r4-c3 |
| ethylene-responsive methionine synthase | TC72099 | AW649298 | cLEI7F6 | VIIF9 | T2L17 | R8 | C12 | r4-c2 | r1-c3 |
| cinnamic acid 4-hydroxylase {Capsicum annuum} | TC72100 | AW218565 | cLEZ9N15 | XIVA5 | T4A10 | R15 | C1 | r2-c2 | r1-c4 |
| cinnamic acid 4-hydroxylase {Capsicum chinense} | TC72101 | AI484136 | cLER1C5 | XA3 | T3A6 | R19 | C1 | r2-c1 | r4-c3 |
| glyceraldehyde 3-phosphate dehydrogenase b precursor, chloroplast {Pisum sativum}SP\|P12859\|G3PB_PEA | TC72118 | BG128691 | cTOF21D16 | XIXE1 | T5J1 | R24 | C10 | r3-c2 | r4-c4 |
| glyceraldehyde 3-phosphate dehydrogenase b precursor, chloroplast {Pisum sativum}SP\|P12859\|G3PB_PEA | TC72119 | AI782763 | cLES20B4 | XXIH6 | T6O11 | R14 | C15 | r3-c1 | r2-c4 |
| 4-hydroxyphenylpyruvate dioxygenase {Solenostemon scutellarioides} | TC72120 | AI896928 | cLEC24E23 | IF6 | T1K11 | R14 | C11 | r1-c2 | r3-c3 |
| 4-hydroxyphenylpyruvate dioxygenase {Solenostemon scutellarioides} | TC72121 | BG126577 | cTOF12B4 | XVIIIH2 | T5O4 | R21 | C15 | r3-c2 | r4-c4 |
| obtusifoliol 14-alpha-demethylase {Triticum aestivum}SP\|P93596\|CP51_WHEAT CYTOCHROME P450 51 (EC 1. | TC72126 | BG125458 | cTOF8P9 | XXIH12 | T6O23 | R2 | C15 | r3-c1 | r2-c4 |
| fructokinase 1 {Arabidopsis thaliana}GP\|13878053\|gb\|AAK44104.1\|AF370289_1\|AF370289 putative fructok | TC72131 | BG139438 | cLPP13M11 | XVD8 | T4H15 | R12 | C8 | r2-c2 | r1-c4 |
| beta-glucosidase {Arabidopsis thaliana} | TC72139 | BG127001 | cTOF14G15 | XXIB1 | T6C1 | R24 | C3 | r3-c1 | r2-c4 |
| AP2 domain containing protein {Prunus armeniaca} | TC72156 | AW615838 | cTOA17G2 | XVID9 | T4H18 | R7 | C8 | r2-c2 | r1-c4 |
| leucine zipper-containing protein AT103 {Arabidopsis thaliana}PIR\|T47754\|T47754 leucine zipper-cont | TC72159 | AI776440 | cLER18B9 | IXH3 | T3O5 | R20 | C15 | r2-c1 | r4-c3 |
| proline oxidase precursor {Arabidopsis thaliana} | TC72165 | BG127967 | cTOF18H21 | XIXB12 | T5D23 | R2 | C4 | r3-c2 | r4-c4 |
| homeobox | TC72179 | BF050472 | cLEM18A14 | VA8 | T2A15 | R12 | C1 | r4-c2 | r1-c3 |
| cytosolic aconitase {Nicotiana tabacum} | TC72186 | BG135767 | cTOE23F23 | XVG12 | T4N23 | R2 | C14 | r2-c2 | r1-c4 |
| cytosolic aconitase {Nicotiana tabacum} | TC72187 | BG127318 | cTOF16I5 | XIXA9 | T5B17 | R8 | C2 | r3-c2 | r4-c4 |
| hypothetical Cys-3-His zinc finger protein {Arabidopsis thaliana}GP\|6598933\|gb\|AAF18728.1\|AC018721_ | TC72194 | AW625359 | cLEZ12O7 | XIVD4 | T4G8 | R17 | C7 | r2-c2 | r1-c4 |
| aminotransferase-like protein {Arabidopsis thaliana} | TC72199 | AW626087 | cLEZ18C16 | VB1 | T2C1 | R24 | C3 | r4-c2 | r1-c3 |
| PROBABLE VACUOLAR ATP SYNTHASE | TC72206 | AW223863 | cLEN13L16 | VIIIH8 | T2P16 | R9 | C16 | r4-c2 | r1-c3 |

| Description | TC | Accession | Clone 1 | Clone 2 | Clone 3 | R | C | Coord 1 | Coord 2 |
|---|---|---|---|---|---|---|---|---|---|
| SUBUNIT D 2 (EC 3.6.1.34) (V-ATPASE D SUBUNIT 2) (VACUOLAR PROTON PUM | TC72213 | AI772692 | cLER3F19 | IB8 | T1C15 | R12 | C3 | r1-c2 | r3-c3 |
| malate dehydrogenase, glyoxysomal precursor {Citrullus vulgaris}EGAD|130842|139627 glyoxysomal mala | TC72222 | AW616121 | cLHT6C11 | XVC9 | T4F17 | R8 | C6 | r2-c2 | r1-c4 |
| w-3 desaturase {Solanum tuberosum}PIR|T07685|T07685 omega-3 fatty acid desaturase (EC 1.14.99.-) - | TC72225 | AI773341 | cLER6A4 | XC4 | T3E8 | R17 | C5 | r2-c1 | r4-c3 |
| ATP synthase gamma subunit, mitochondrial precursor {Ipomoea batatas}SP|P26360|ATP3_IPOBA ATP SYNTH | TC72228 | BG130597 | cTOF31F13 | XXC4 | T5F8 | R17 | C6 | r3-c2 | r4-c4 |
| nucleoside diphosphate kinase | TC72235 | BG129590 | cTOF25C23 | XIXG12 | T5N23 | R2 | C14 | r3-c2 | r4-c4 |
| glutamine synthetase | TC72279 | cLEC37G5 | cLEC37G5 | XXIF11 | T6K21 | R4 | C11 | r3-c1 | r2-c4 |
| sulfite reductase {Nicotiana tabacum}GP|3721540|dbj|BAA33531.1||D83583 Sulfite Reductase {Nicotiana | TC72280 | BE449781 | cLEY14I23 | XIIIG7 | T4M13 | R12 | C13 | r2-c2 | r1-c4 |
| ALTERNATIVE OXIDASE 1 PRECURSOR (EC 1.-.-.-).GP|558054|gb|AAC60576.1||S71335 alternative oxidase, A | TC72286 | BG123332 | cTOF1J9 | XIXD6 | T5H11 | R14 | C8 | r3-c2 | r4-c4 |
| homologous to glucosyltransferases | TC72288 | AI778489 | cLES5B19 | XVB10 | T4D19 | R6 | C4 | r2-c2 | r1-c4 |
| CYTOCHROME P450 81E1 (EC 1.14.-.-) (ISOFLAVONE 2'-HYDROXYLASE) (P450 91A4) (CYP GE-3).GP|2443348|db | TC72291 | AW094482 | cLET28L16 | XIG6 | T3N11 | R14 | C14 | r2-c1 | r4-c3 |
| UDP-GLUCOSE 4-EPIMERASE (EC 5.1.3.2) (GALACTOWALDENASE) (UDP- GALACTOSE 4-EPIMERASE).GP|8698725|gb| | TC72292 | AW626005 | cLEZ17N10 | XIVE5 | T4I10 | R15 | C9 | r2-c2 | r1-c4 |
| transaldolase | TC72300 | BG134485 | cTOE16O12 | XVIIIH1 | T5O1 | R24 | C15 | r3-c2 | r4-c4 |
| transcription factor {Vicia faba}GP|2104681|emb|CAA66481.1||X97907 transcription factor {Vicia faba | TC72313 | BF113609 | cLEY21L1 | XIVB10 | T4C20 | R5 | C3 | r2-c2 | r1-c4 |
| putative CONSTANS-like B-box zinc finger protein {Arabidopsis thaliana}PIR|A84720|A84720 hypothetic | TC72317 | BF113184 | cLEG43F12 | VIG2 | T2M4 | R21 | C13 | r4-c2 | r1-c3 |
| glucose-6-phosphate 1-dehydrogenase {Solanum tuberosum}SP|P37830|G6PD_SOLTU GLUCOSE-6-PHOSPHATE 1-D | TC72318 | AW223852 | cLEN13J18 | VIIIH6 | T2P12 | R13 | C16 | r4-c2 | r1-c3 |
| glucose-6-phosphate 1-dehydrogenase {Solanum tuberosum}SP|P37830|G6PD_SOLTU GLUCOSE-6- | | | | | | | | | |

| PHOSPHATE 1-D | | | | | | | | |
|---|---|---|---|---|---|---|---|---|
| pyruvate kinase-like protein {Arabidopsis thaliana}PIR\|T47556\|T47556 pyruvate kinase-like protein - | TC72325 | AW220370 | cLEX10B10 | XIIIA5 | T4A9 | R16 | C1 | r2-c2 | r1-c4 |
| beta-amylase {Prunus armeniaca} | TC72330 | AW223795 | cLEN13N5 | VIIIH9 | T2P18 | R7 | C16 | r4-c2 | r1-c3 |
| glucose-6-phosphate isomerase, cytosolic 1 (gpi) (phosphoglucose isomerase) (pgi) (phosphohexose iso | TC72335 | AI779723 | cLES8N20 | XIB4 | T3D7 | R18 | C4 | r2-c1 | r4-c3 |
| AP2 domain-containing transcription factor {Nicotiana tabacum} | TC72337 | BG134369 | cTOE16K11 | XXIA7 | T6A13 | R12 | C1 | r3-c1 | r2-c4 |
| pyruvate dehydrogenase E1 beta subunit isoform 1 {Zea mays} | TC72349 | AW154894 | cLEW1C3 | XIIF1 | T3L2 | R23 | C12 | r2-c1 | r4-c3 |
| fructose-1,6-bisphosphatase, cytosolic bisphosphate 1-phosphohydrolase) (fbpase) (cy-f1) {Solanum tu | TC72350 | BF098452 | cLEW27E8 | XIIH5 | T3P10 | R15 | C16 | r2-c1 | r4-c3 |
| fructose-1,6-bisphosphatase, cytosolic bisphosphate 1-phosphohydrolase) (fbpase) (cy-f1) {Solanum tu | TC72351 | AW040771 | cLET10L5 | XIC5 | T3F9 | R16 | C5 | r2-c1 | r4-c3 |
| isopentenyl diphosphate isomerase 1 {Nicotiana tabacum} | TC72352 | BG129273 | cTOF23H8 | XXIG9 | T6M17 | R8 | C13 | r3-c1 | r2-c4 |
| putative glucose regulated repressor protein {Arabidopsis thaliana}PIR\|A84649\|A84649 probable gluco | TC72370 | BG128772 | cTOF22E10 | XIXE10 | T5J19 | R6 | C10 | r3-c2 | r4-c4 |
| pyruvate dehydrogenase E1 alpha subunit {Arabidopsis thaliana} | TC72372 | BE354897 | cTOC14F12 | XVIIC11 | T5E21 | R4 | C5 | r3-c2 | r4-c4 |
| PYROPHOSPHATE--FRUCTOSE 6-PHOSPHATE 1-PHOSPHOTRANSFERASE BETA SUBUNIT (EC 2.7.1.90) (PFP) (6-PHOSPHO | TC72375 | BG125076 | cTOF7H5 | XXF10 | T5L20 | R5 | C12 | r3-c2 | r4-c4 |
| transketolase, chloroplast precursor {Solanum tuberosum}SP\|Q43848\|TKTC_SOLTU TRANSKETOLASE, CHLOROP | TC72376 | AW398784 | cLPT4F22 | XVIA8 | T4B16 | R9 | C2 | r2-c2 | r1-c4 |
| glutamyl-tRNA synthetase {Arabidopsis thaliana}PIR\|T52043\|T52043 probable glutamate--tRNA ligase (E | TC72377 | AI781426 | cLES15G16 | XE3 | T3I6 | R19 | C9 | r2-c1 | r4-c3 |
| URIDYLATE KINASE (EC 2.7.4.-) (UK) (URIDINE MONOPHOSPHATE KINASE) (UMP KINASE) (UMP/CMP KINASE).GP\| | TC72400 | BF114322 | cLEY26B11 | XIVC4 | T4E8 | R17 | C5 | r2-c2 | r1-c4 |
| asparagine synthetase {Triphysaria versicolor}GP\|2429282\|gb\|AAD05034.1\|\|AF014056 asparagine synthet | TC72402 | AW625684 | cLEZ16B17 | XIVD11 | T4G22 | R3 | C7 | r2-c2 | r1-c4 |

| | | | | | | | | | |
|---|---|---|---|---|---|---|---|---|---|
| cytochrome p450 lxxii hydroxylase) (ge10h) {Catharanthus roseus}SP\|Q05047\|CP72_CATRO CYTOCHROME P45 | TC72403 | BE434310 | cLEG16E11 | VE10 | T2I19 | R6 | C9 | r4-c2 | r1-c3 |
| cytochrome P450 {Arabidopsis thaliana} | TC72404 | AI485298 | cLED5C5 | IVA11 | T1B22 | R3 | C2 | r1-c2 | r3-c3 |
| VACUOLAR ATP SYNTHASE SUBUNIT C (EC 3.6.1.34) (V-ATPASE C SUBUNIT) (VACUOLAR PROTON PUMP C SUBUNIT). | TC72410 | BE451373 | cLEY18A9 | XIVA9 | T4A18 | R7 | C1 | r2-c2 | r1-c4 |
| phosphoenolpyruvate carboxylase 2 | TC72426 | BE458917 | cLEM5A15 | IVF8 | T1L16 | R9 | C12 | r1-c2 | r3-c3 |
| lipoxygenase^^loxc homologue | TC72430 | AW222576 | cLEN8L9 | IXD9 | T3G17 | R8 | C7 | r2-c1 | r4-c3 |
| lipoxygenase^^loxc homologue | TC72431 | AI778045 | cLES3F16 | XH3 | T3O6 | R19 | C15 | r2-c1 | r4-c3 |
| succinyl-CoA synthetase, alpha subunit {Arabidopsis thaliana} | TC72435 | AW222820 | cLEN9B13 | IXD10 | T3G19 | R6 | C7 | r2-c1 | r4-c3 |
| VACUOLAR ATP SYNTHASE SUBUNIT D (EC 3.6.1.34) (V-ATPASE D SUBUNIT) (VACUOLAR PROTON PUMP D SUBUNIT). | TC72437 | AW929411 | cTOC8F2 | XVIIE12 | T5I23 | R2 | C9 | r3-c2 | r4-c4 |
| NADPH-cytochrome P450 oxidoreductase (EC 1.-.-.-) - common tobacco | TC72444 | AW441648 | cLEN17N14 | IVH4 | T1P8 | R17 | C16 | r1-c2 | r3-c3 |
| ATP synthase beta subunit | TC72461 | AW617859 | cLHT24H4 | XXIA4 | T6A7 | R18 | C1 | r3-c1 | r2-c4 |
| putative sulfite oxidase {Arabidopsis thaliana}GP\|6513940\|gb\|AAF14844.1\|AC011664_26\|AC011664 sulfit | TC72463 | AW621692 | cLEX13E13 | XIIIC2 | T4E3 | R22 | C5 | r2-c2 | r1-c4 |
| putative homeodomain transcription factor {Arabidopsis thaliana}PIR\|F84565\|F84565 probable homeodom | TC72471 | AI488166 | cLED21A22 | IIIC3 | T1F5 | R20 | C6 | r1-c2 | r3-c3 |
| NADP-dependent glyceraldehyde-3-phosphate dehydrogenase (non-phosphorylating glyceraldehyde 3-phosph | TC72481 | AW649868 | cLEI9L22 | VIIG7 | T2N13 | R12 | C14 | r4-c2 | r1-c3 |
| WRKY transcription factor Nt-SubD48 {Nicotiana tabacum} | TC72483 | BF050338 | cLEM17M14 | VIIH2 | T2P3 | R22 | C16 | r4-c2 | r1-c3 |
| putative anthocyanin 5-aromatic acyltransferase {Arabidopsis thaliana}PIR\|G84823\|G84823 probable an | TC72484 | AW096580 | cLET39A17 | XXIF5 | T6K9 | R16 | C11 | r3-c1 | r2-c4 |
| pyruvate dehydrogenase E1 beta subunit isoform 2 {Zea mays} | TC72498 | AW222942 | cLEN9N12 | IXE3 | T3I5 | R20 | C9 | r2-c1 | r4-c3 |
| S-adenosylmethionine decarboxylase {Nicotiana tabacum}PIR\|T01934\|T01934 adenosylmethionine decarbox | TC72506 | BF051123 | cLEM21L9 | VF9 | T2K17 | R8 | C11 | r4-c2 | r1-c3 |
| NADH-UBIQUINONE OXIDOREDUCTASE 24 KDA SUBUNIT PRECURSOR (EC 1.6.5.3) (EC | TC72512 | AW622611 | cLEX15D4 | XIE4 | T3J7 | R18 | C10 | r2-c1 | r4-c3 |

67

EP 1 454 993 A1

| 1.6.99.3).GP\|7269018\|emb\|C | | | | | | | | |
|---|---|---|---|---|---|---|---|---|---|
| phosphate/phosphoenolpyruvate translocator precursor {Nicotiana tabacum}GP\|1778145\|gb\|AAB40648.1\|\|U | TC72515 | BG643700 | cTOF32E22 | XXC10 | T5F20 | R5 | C6 | r3-c2 | r4-c4 |
| putative glucosyltransferase {Arabidopsis thaliana}PIR\|H84870\|H84870 probable glucosyltransferase [ | TC72520 | AW625702 | cLEZ16H1 | XIVE1 | T4I2 | R23 | C9 | r2-c2 | r1-c4 |
| pyruvate dehydrogenase | TC72539 | BG643832 | cTOF33A1 | XXC12 | T5F24 | R1 | C6 | r3-c2 | r4-c4 |
| zinc finger transcription factor-like protein {Arabidopsis thaliana}PIR\|T49899\|T49899 zinc finger t | TC72540 | AW221733 | cLEN3B6 | XXH10 | T5P20 | R5 | C16 | r3-c2 | r4-c4 |
| flavanone 3-hydroxylase-like protein {Arabidopsis thaliana} | TC72542 | AI897731 | cLED30B19 | IIIE12 | T1J23 | R2 | C10 | r1-c2 | r3-c3 |
| gamma-glutamylcysteine synthetase | TC72560 | BG125088 | cTOF7J11 | XXIC2 | T6E3 | R22 | C5 | r3-c1 | r2-c4 |
| transcription initiation factor iib (tfiib) {Glycine max}SP\|P48513\|TF2B_SOYBN TRANSCRIPTION INITIAT | TC72566 | BF050934 | cLEM21C17 | VIIIA1 | T2B2 | R23 | C2 | r4-c2 | r1-c3 |
| dihydroxy-acid dehydratase {Arabidopsis thaliana} | TC72576 | BG130088 | cTOF29C14 | XXA11 | T5B22 | R3 | C2 | r3-c2 | r4-c4 |
| arginine methyltransferase (pam1) {Arabidopsis thaliana}GP\|7269850\|emb\|CAB79709.1\|\|AL161575 arginin | TC72613 | AI781196 | cLES14F12 | XD11 | T3G22 | R3 | C7 | r2-c1 | r4-c3 |
| N-hydroxycinnamoyl/benzoyltransferase {Ipomoea batatas} | TC72632 | AI898930 | cLED36K11 | IIIH2 | T1P3 | R22 | C16 | r1-c2 | r3-c3 |
| similar to ATPases associated with various cellular activites (Pfam: AAA.hmm, score: 230.91) {Arabid | TC72650 | BF051653 | cLEM23D22 | VIIIB9 | T2D18 | R7 | C4 | r4-c2 | r1-c3 |
| putative monosaccharide transporter 1 {Petunia x hybrida} | TC72655 | BG138866 | cLPP11G6 | XVD3 | T4H5 | R20 | C8 | r2-c2 | r1-c4 |
| 78 kDa glucose regulated protein homolog 5 precursor (grp 78-5) (immunoglobulin heavy subunit bindin | TC72660 | AW622750 | cTOB4O12 | XVIIB3 | T5C5 | R20 | C3 | r3-c2 | r4-c4 |
| threonine synthase {Solanum tuberosum} | TC72666 | AW651453 | cLEI16P15 | VIID3 | T2H5 | R20 | C8 | r4-c2 | r1-c3 |
| CONSTANS-like B-box zinc finger protein-like {Arabidopsis thaliana} | TC72668 | BG129699 | cTOF27L4 | XXA6 | T5B12 | R13 | C2 | r3-c2 | r4-c4 |
| Identical to ribose-phosphate pyrophosphokinase 2 (phosphoribosyl pyrophosphate synthetase 2) (PRSII | TC72677 | AW223107 | cLEN10D3 | VIIIG1 | T2N2 | R23 | C14 | r4-c2 | r1-c3 |
| NADH dehydrogenase {Solanum tuberosum}GP\|668987\|emb\|CAA59063.1\|\|X84320 NADH dehydrogenase {Solanum | TC72678 | BG643904 | cTOF33O15 | XXD3 | T5H8 | R17 | C8 | r3-c2 | r4-c4 |
| Similar to gb\|Z84386 anthranilate N-hydroxycinnamoyl/benzoyltransferase from Dianthus | TC72703 | AW737648 | cTOD3J14 | XVIIG11 | T5M21 | R4 | C13 | r3-c2 | r4-c4 |

| | | | | | | | | | |
|---|---|---|---|---|---|---|---|---|---|
| caryophyllus. | | | | | | | | | |
| putative H+-transporting ATPase {Oryza sativa} | TC72705 | AW220712 | cLEF2G23 | IA12 | T1A23 | R2 | C1 | r1-c2 | r3-c3 |
| Similar to Populus balsamifera subsp. trichocarpa X Populus deltoides vegetative storage protein. (L | TC72708 | AI778197 | cLES4A22 | XH5 | T3O10 | R15 | C15 | r2-c1 | r4-c3 |
| cytochrome P450-dependent fatty acid hydroxylase {Vicia sativa} | TC72710 | AW442394 | cLEN22J6 | VA9 | T2A17 | R8 | C1 | r4-c2 | r1-c3 |
| anthocyanidin 3-O-glucosyltransferase {Petunia x hybrida} | TC72713 | AW945115 | cTOB13F3 | IA2 | T1A3 | R22 | C1 | r1-c2 | r3-c3 |
| cytochrome p450 lxxia4 {Solanum melongena}SP\|P37117\|C714_SOLME CYTOCHROME P450 71A4 (EC 1.14.-.-) ( | TC72718 | AW223657 | cLEN12N5 | VIIIG12 | T2N24 | R1 | C14 | r4-c2 | r1-c3 |
| cytochrome p450 lxxia4 {Solanum melongena}SP\|P37117\|C714_SOLME CYTOCHROME P450 71A4 (EC 1.14.-.-) ( | TC72719 | BE353651 | cTOA19J6 | XVIE4 | T4J8 | R17 | C10 | r2-c2 | r1-c4 |
| phosphoenolpyruvate carboxykinase {Flaveria pringlei} | TC72722 | AW030343 | cLEC16F9 | ID9 | T1G17 | R8 | C7 | r1-c2 | r3-c3 |
| phosphoribosyl pyrophosphate synthase {Spinacia oleracea} | TC72727 | BG124244 | cTOF4H7 | XXE6 | T5J12 | R13 | C10 | r3-c2 | r4-c4 |
| dehydroquinate dehydratase/shikimate:NADP oxidoreductase | TC72770 | BE461692 | cLEG39N19 | VIE9 | T2I18 | R7 | C9 | r4-c2 | r1-c3 |
| AP2 domain containing protein {Prunus armeniaca} | TC72775 | AW093577 | cLET25E20 | XIG2 | T3N3 | R22 | C14 | r2-c1 | r4-c3 |
| putative phosphate/phosphoenolpyruvate translocator {Arabidopsis thaliana} | TC72794 | AW037415 | cLET5A1 | XXIE7 | T6I13 | R12 | C9 | r3-c1 | r2-c4 |
| SNAP25A protein {Arabidopsis thaliana}GP\|5731763\|emb\|CAB52582.1\|\|X92419 SNAP25A protein {Arabidopsi | TC72817 | BF051551 | cLEM23I12 | VIIIB10 | T2D20 | R5 | C4 | r4-c2 | r1-c3 |
| heat shock transcription factor like protein {Arabidopsis thaliana}GP\|2244754\|emb\|CAB10177.1\|\|Z9733 | TC72821 | BF096782 | cLEW17M17 | IVF1 | T1L2 | R23 | C12 | r1-c2 | r3-c3 |
| ADP-glucose pyrophosphorylase large subunit 1 | TC72843 | AI781523 | cLES16E9 | XE9 | T3I18 | R7 | C9 | r2-c1 | r4-c3 |
| putative methylmalonate semi-aldehyde dehydrogenase {Arabidopsis thaliana}PIR\|H84514\|H84514 hypothe | TC72868 | AW224041 | cLEN14F7 | IXA1 | T3A1 | R24 | C1 | r2-c1 | r4-c3 |
| flavonol 3-o-glucosyltransferase 6 {Manihot esculenta}SP\|Q40288\|UFO6_MANES FLAVONOL 3-O-GLUCOSYLTRA | TC72874 | AW036019 | cLEC21A15 | IE7 | T1I13 | R12 | C9 | r1-c2 | r3-c3 |
| Identical to gene ZW10 from Arabidopsis thaliana gb\|AB028195 and is a member of the Phosphoglycerate | TC72889 | BG127811 | cTOF18M15 | XXIIA6 | T6A12 | R13 | C1 | r3-c1 | r2-c4 |
| coproporphyrinogen iii oxidase precursor (coproporphyrinogenase) (coprogen oxidase) {Nicotiana | TC72896 | AW931818 | cTOF4P23 | IVH2 | T1P4 | R21 | C16 | r1-c2 | r3-c3 |

| | | | | | | | | |
|---|---|---|---|---|---|---|---|---|---|
| tabac | | | | | | | | |
| zinc finger protein-like {Arabidopsis thaliana}GP\|5006473\|gb\|AAD37511.1\|AF139098_1\|AF139098 putativ | TC72898 | AI486585 | cLED6E7 | IVB8 | T1D16 | R9 | C4 | r1-c2 | r3-c3 |
| caffeoyl-CoA O-methyltransferase {Nicotiana tabacum}GP\|1103487\|emb\|CAA91228.1\|\|Z56282 caffeoyl-CoA | TC72904 | AI895091 | cLEC6D10 | XIIF2 | T3L4 | R21 | C12 | r2-c1 | r4-c3 |
| phosphoglycerate mutase {Solanum tuberosum} | TC72922 | AW648487 | cLEI4B6 | XIIF7 | T3L14 | R11 | C12 | r2-c1 | r4-c3 |
| acetyl-CoA C-acetyltransferase {Arabidopsis thaliana} | TC72945 | AW928977 | cTOC4K4 | XVIID11 | T5G21 | R4 | C7 | r3-c2 | r4-c4 |
| acetyl-CoA C-acetyltransferase {Arabidopsis thaliana} | TC72946 | AW398370 | cLPT6I1 | XVIB10 | T4D20 | R5 | C4 | r2-c2 | r1-c4 |
| putative NADH-ubiquinone oxireductase {Arabidopsis thaliana}PIR\|C84588\|C84588 probable NADH-ubiquin | TC72951 | AW621179 | cLEX11E19 | XIIIB2 | T4C3 | R22 | C3 | r2-c2 | r1-c4 |
| acetyl-CoA C-acetyltransferase {Arabidopsis thaliana} | TC72956 | AW621890 | cLEX13L9 | XXIF6 | T6K11 | R14 | C11 | r3-c1 | r2-c4 |
| malonyl-CoA:ACP transacylase {Perilla frutescens} | TC72961 | BE450922 | cLEY15N21 | XIIIH8 | T4O15 | R12 | C15 | r2-c2 | r1-c4 |
| N-carbamyl-L-amino acid amidohydrolase-like protein {Arabidopsis thaliana} | TC72977 | AW442132 | cLEN21O18 | IXC3 | T3E5 | R20 | C5 | r2-c1 | r4-c3 |
| uracil phosphoribosyltransferase {Nicotiana tabacum}SP\|P93394\|UPP_TOBAC URACIL PHOSPHORIBOSYLTRANSF | TC72985 | BG124328 | cTOF4N12 | XXE7 | T5J14 | R11 | C10 | r3-c2 | r4-c4 |
| heat stress transcription factor A3 {Lycopersicon peruvianum} | TC72992 | BG642641 | cTOF25C4 | XIXH1 | T5P1 | R24 | C16 | r3-c2 | r4-c4 |
| invertase-like protein {Arabidopsis thaliana}GP\|7270437\|emb\|CAB80203.1\|\|AL161586 invertase-like pro | TC73000 | AI485121 | cLED7B14 | VC7 | T2E13 | R12 | C5 | r4-c2 | r1-c3 |
| contains similarity to acyl-CoA thioesterase~gene_id:K23F3.9 {Arabidopsis thaliana} | TC73001 | BE459589 | cLEM7B1 | VIIE9 | T2J17 | R8 | C10 | r4-c2 | r1-c3 |
| acetylornithine aminotransferase precursor {Alnus glutinosa}SP\|O04866\|ARGD_ALNGL ACETYLORNITHINE AM | TC73014 | AI490283 | cLED24N6 | IIID1 | T1H1 | R24 | C8 | r1-c2 | r3-c3 |
| glucose-6-phosphate isomerase {Spinacia oleracea}PIR\|T09153\|T09153 glucose-6-phosphate isomerase (E | TC73016 | AW399786 | cLPT10F14 | VC2 | T2E3 | R22 | C5 | r4-c2 | r1-c3 |
| hydroxypyruvate reductase {Bruguiera gymnorhiza} | TC73027 | AI487051 | cLED9G3 | IVC12 | T1F24 | R1 | C6 | r1-c2 | r3-c3 |
| PROTEIN-L-ISOASPARTATE O-METHYLTRANSFERASE (EC 2.1.1.77) (PROTEIN-BETA-ASPARTATE METHYLTRANSFERASE) | TC73037 | AI776861 | cLER20E3 | XB1 | T3C2 | R23 | C3 | r2-c1 | r4-c3 |

70

| | | | | | | | | |
|---|---|---|---|---|---|---|---|---|---|
| putative anthocyanidin-3-glucoside rhamnosyltransferase {Arabidopsis thaliana}PIR\|D84614\|D84614 hyp | TC73055 | BG123229 | cTOF1C4 | XIXC12 | T5F23 | R2 | C6 | r3-c2 | r4-c4 |
| putative RING-H2 zinc finger protein ATL6 {Arabidopsis thaliana} | TC73059 | AW399786 | cLEG24D12 | VB2 | T2C3 | R22 | C3 | r4-c2 | r1-c3 |
| adenosine kinase {Arabidopsis thaliana}GP\|7378610\|emb\|CAB83286.1\|\|AL162751 adenosine kinase-like pr | TC73160 | BE462073 | cTOA11E17 | XXIG5 | T6M9 | R16 | C13 | r3-c1 | r2-c4 |
| putative PHD-type zinc finger protein {Arabidopsis thaliana}PIR\|A84437\|A84437 probable PHD-type zin | TC73164 | BF051010 | cLEM21E20 | XXIB6 | T6C11 | R14 | C3 | r3-c1 | r2-c4 |
| quinolinate phosphoribosyltransferase {Nicotiana tabacum} | TC73169 | BG130955 | cTOE2C9 | XVIF8 | T4L16 | R9 | C12 | r2-c2 | r1-c4 |
| 5-enolpyruvylshikimate-3-phosphate synthase precursor (EC 2.5.1.19) | TC73179 | BG126520 | cTOF12F23 | XVIIIH3 | T5O6 | R19 | C15 | r3-c2 | r4-c4 |
| transcription factor | TC73183 | AW218738 | cLEX1C5 | XIIID5 | T4G9 | R16 | C7 | r2-c2 | r1-c4 |
| Dof zinc finger protein {Arabidopsis thaliana}GP\|9280230\|dbj\|BAB01720.1\|\|AB023045 Dof zinc finger p | TC73204 | AI771243 | cLED28N11 | IIIE4 | T1J7 | R18 | C10 | r1-c2 | r3-c3 |
| aldose 1-epimerase-like protein {Arabidopsis thaliana}PIR\|T07719\|T07719 aldose 1-epimerase homolog | TC73210 | BE461978 | cLEG40O8 | VIF1 | T2K2 | R23 | C11 | r4-c2 | r1-c3 |
| glucosyl transferase {Nicotiana tabacum}GP\|1805359\|dbj\|BAA19155.1\|\|AB000623 glucosyl transferase {N | TC73225 | AI483731 | cLED23J21 | XXID2 | T6G3 | R22 | C7 | r3-c1 | r2-c4 |
| unnamed protein product {unidentified} □GP\|2462931\|emb\|CAB06082.1\|\|Z83833 UDP-glucose:sterol glucosyl | TC73255 | AW615991 | cTOA17D2 | XVID8 | T4H16 | R9 | C8 | r2-c2 | r1-c4 |
| homogentisate 1,2-dioxygenase | TC73262 | AW092200 | cLET17L20 | XID10 | T3H19 | R6 | C8 | r2-c1 | r4-c3 |
| putative aspartate aminotransferase; 38163-36256 {Arabidopsis thaliana}PIR\|C96835\|C96835 hypothetic | TC73280 | BG133934 | cTOE14H6 | XVIIIC6 | T5E12 | R13 | C5 | r3-c2 | r4-c4 |
| ATP synthase alpha chain {Vigna radiata} | TC73289 | AW031503 | cLEC40G14 | IID5 | T1G10 | R15 | C7 | r1-c2 | r3-c3 |
| MYB-like DNA-binding protein {Catharanthus roseus} | TC73317 | BG128786 | cTOF22G14 | XIXE12 | T5J23 | R2 | C10 | r3-c2 | r4-c4 |
| CYTOCHROME P450 90A1 (EC 1.14.-.-).GP\|853719\|emb\|CAA60793.1\|\|X87367 CYP90 protein {Arabidopsis thal | TC73326 | BE449595 | cLHT32I3 | XVC8 | T4F15 | R12 | C6 | r2-c2 | r1-c4 |

| | | | | | | | | | |
|---|---|---|---|---|---|---|---|---|---|
| vsf-1^^transcription factor VSF-1 | TC73332 | AW094595 | cLET29E6 | XIG9 | T3N17 | R8 | C14 | r2-c1 | r4-c3 |
| ATP synthase alpha subunit {Nicotiana tabacum}SP\|P00823\|ATPA_TOBAC ATP SYNTHASE ALPHA CHAIN (EC 3.6 | TC73341 | BF112918 | cLEG43G11 | VIG3 | T2M6 | R19 | C13 | r4-c2 | r1-c3 |
| isoflavone reductase homolog {Solanum tuberosum}SP\|P52578\|IFRH_SOLTU ISOFLAVONE REDUCTASE HOMOLOG ( | TC73357 | BG125717 | cTOF9H3 | XXG9 | T5N18 | R7 | C14 | r3-c2 | r4-c4 |
| fumarase {Solanum tuberosum}GP\|1488652\|emb\|CAA62817.1\|X91615 fumarase {Solanum tuberosum}PIR\|T073 | TC73367 | BF112535 | cLEG41L11 | VC9 | T2E17 | R8 | C5 | r4-c2 | r1-c3 |
| ornithine carbamoyltransferase; OCTase {Canavalia lineata}GP\|12003188\|gb\|AAG43481.1\|AF203688_1\|AF 20 | TC73370 | AI486970 | cLED6P9 | IVC2 | T1F4 | R21 | C6 | r1-c2 | r3-c3 |
| N-glyceraldehyde-2-phosphotransferase-like {Arabidopsis thaliana} | TC73374 | BG630730 | cLEL4E1 | XXIG4 | T6M7 | R18 | C13 | r3-c1 | r2-c4 |
| putative cinnamyl alcohol dehydrogenase {Malus x domestica}PIR\|T16995\|T16995 probable cinnamyl-alco | TC73375 | BE451381 | cLEY18C9 | XIVA10 | T4A20 | R5 | C1 | r2-c2 | r1-c4 |
| putative anthocyanidin-3-glucoside rhamnosyltransferase {Arabidopsis thaliana}PIR\|D84614\|D84614 hyp | TC73422 | AW441205 | cLEN4F1 | IXC10 | T3E19 | R6 | C5 | r2-c1 | r4-c3 |
| serine/threonine-specific protein kinase NAK {Arabidopsis thaliana}PIR\|T48250\|T48250 serine/threoni | TC73426 | AI772369 | cLER2B1 | XXIA11 | T6A21 | R4 | C1 | r3-c1 | r2-c4 |
| putative threonine dehydratase/deaminase {Oryza sativa} | TC73457 | BE449339 | cLPT23P6 | XVB11 | T4D21 | R4 | C4 | r2-c2 | r1-c4 |
| pyruvate dehydrogenase kinase {Arabidopsis thaliana} | TC73458 | BE459347 | cLEM6D21 | IVH6 | T1P12 | R13 | C16 | r1-c2 | r3-c3 |
| Cytochrome P450-like protein {Arabidopsis thaliana}GP\|7270098\|emb\|CAB79912.1\|AL161580 Cytochrome P | TC73461 | AW929893 | cTOC8A14 | IVE10 | T1J20 | R5 | C10 | r1-c2 | r3-c3 |
| putative arginine methyltransferase {Arabidopsis thaliana} | TC73476 | AI899735 | cLES20J21 | IA9 | T1A17 | R8 | C1 | r1-c2 | r3-c3 |
| glutamine synthetase {Nicotiana tabacum}GP\|1419094\|emb\|CAA65173.1\|X95932 glutamine synthetase {Nic | TC73479 | AW621882 | cLEX13J15 | XIIIC3 | T4E5 | R20 | C5 | r2-c2 | r1-c4 |
| glucosyl transferase {Nicotiana tabacum}GP\|1805359\|dbj\|BAA19155.1\|AB000623 glucosyl transferase {N | TC73487 | AI896066 | cLEC13B18 | IC8 | T1E15 | R12 | C5 | r1-c2 | r3-c3 |

| | | | | | | | | | |
|---|---|---|---|---|---|---|---|---|---|
| putative RING zinc finger protein; 36546-35989 {Arabidopsis thaliana}GP|12323975|gb|AAG51946.1|AC01 | TC73490 | AW223952 | cLEN14A6 | VIIIH11 | T2P22 | R3 | C16 | r4-c2 | r1-c3 |
| cytochrome P450 {Nicotiana tabacum} | TC73493 | AW616568 | cLHT11B6 | XIVH2 | T4O4 | R21 | C15 | r2-c2 | r1-c4 |
| PHOSPHOGLUCOMUTASE, CYTOPLASMIC (EC 5.4.2.2) (GLUCOSE PHOSPHOMUTASE) (PGM).GP|8250624|emb|CAB93681. | TC73495 | BG132150 | cTOE6J5 | XVIIC1 | T5E1 | R24 | C5 | r3-c2 | r4-c4 |
| starch phosphorylase (AA 1-966) {Solanum tuberosum} | TC73506 | AW738720 | cTOD8G8 | XVIIIB3 | T5C6 | R19 | C3 | r3-c2 | r4-c4 |
| alpha-glucan phosphorylase, l isozyme 1 precursor (starch phosphorylase 1-1) {Solanum tuberosum}SP| | TC73507 | BG642630 | cTOF25A4 | XIXG10 | T5N19 | R6 | C14 | r3-c2 | r4-c4 |
| acetyl-coA dehydrogenase, putative {Arabidopsis thaliana} | TC73523 | BG127274 | cTOF14P14 | XVIIF10 | T5K19 | R6 | C11 | r3-c2 | r4-c4 |
| l-asparaginase (l-asparagine amidohydrolase) {Arabidopsis thaliana} | TC73526 | BG123680 | cTOF2D8 | XXB6 | T5D12 | R13 | C4 | r3-c2 | r4-c4 |
| PROBABLE PHOSPHORIBOSYLFORMYLGLYCINAMIDINE SYNTHASE, CHLOROPLAST PRECURSOR (EC 6.3.5.3) (FGAM SYNTHA | TC73527 | AW622713 | cTOB4I2 | XVIIB2 | T5C3 | R22 | C3 | r3-c2 | r4-c4 |
| Putative phospholipid cytidylyltransferase {Oryza sativa} | TC73548 | BF096825 | cLEW17K4 | XIID10 | T3H20 | R5 | C8 | r2-c1 | r4-c3 |
| 2-oxoglutarate/malate translocator {Arabidopsis thaliana} | TC73585 | BG643918 | cTOF33D5 | XXD1 | T5H2 | R23 | C8 | r3-c2 | r4-c4 |
| putative ripening-related bZIP protein {Vitis vinifera} | TC73588 | AW222150 | cLEN7I11 | XIIIA2 | T4A3 | R22 | C1 | r2-c2 | r1-c4 |
| ethylene-responsive transcriptional coactivator | TC73593 | AW031517 | cLEC40C16 | IID4 | T1G8 | R17 | C7 | r1-c2 | r3-c3 |
| PHOSPHOGLUCOMUTASE, CYTOPLASMIC (EC 5.4.2.2) (GLUCOSE PHOSPHOMUTASE) (PGM).GP|8250624|emb|CAB93681. | TC73595 | BE458969 | cLEM5K5 | XXIG11 | T6M21 | R4 | C13 | r3-c1 | r2-c4 |
| putative transcripton factor {Nostoc sp. PCC 7120} | TC73599 | AW945043 | cTOB12P12 | XVIH3 | T4P6 | R19 | C16 | r2-c2 | r1-c4 |
| microsomal oleate desaturase {Arachis ipaensis} | TC73604 | AI775627 | cLER16G21 | XXIC3 | T6E5 | R20 | C5 | r3-c1 | r2-c4 |
| pyruvate dehydrogenase e1 component, alpha subunit precursor {Solanum tuberosum}SP|P52903|ODPA_SOLT | TC73607 | AI782695 | cLES20G10 | XG6 | T3M12 | R13 | C13 | r2-c1 | r4-c3 |
| enoyl-ACP reductase {Nicotiana tabacum}GP|2204236|emb|CAA74176.1||Y13861 enoyl-ACP reductase {Nicot | TC73615 | AW399701 | cLPT8B18 | IVD10 | T1H20 | R5 | C8 | r1-c2 | r3-c3 |
| putative dehydroquinase shikimate dehydrogenase | TC73630 | BF096277 | cLEW11I15 | XIID7 | T3H14 | R11 | C8 | r2-c1 | r4-c3 |

| Description | TC | Accession | Clone | | BAC | | | | |
|---|---|---|---|---|---|---|---|---|---|
| {Arabidopsis thaliana} | | | | | | | | | |
| myb-related transcription factor {Lycopersicon esculentum} PIR|T07393|T07393 myb-related transcripti {Arabidopsis thaliana} | TC73643 | AI487918 | cLED10D18 | XXIF1 | T6K1 | R24 | C11 | r3-c1 | r2-c4 |
| bZIP transcriptional activator RSG, putative {Arabidopsis thaliana}GP|12321383|gb|AAG50761.1|AC0791 | TC73646 | AW616861 | cLHT17P21 | XIIID4 | T4G7 | R18 | C7 | r2-c2 | r1-c4 |
| putative zinc finger protein {Oryza sativa} | TC73652 | AI897679 | cLED30G19 | IIIF2 | T1L3 | R22 | C12 | r1-c2 | r3-c3 |
| pyruvate kinase (EC 2.7.1.40) A, chloroplast - common tobacco | TC73662 | AW625105 | cLEZ10G1 | XIVC11 | T4E22 | R3 | C5 | r2-c2 | r1-c4 |
| 6-phosphogluconate dehydrogenase, putative; 13029-14489 {Arabidopsis thaliana} | TC73678 | AW624047 | cTOB13N24 | XVIH10 | T4P20 | R5 | C16 | r2-c2 | r1-c4 |
| zinc-finger protein, putative; 7043-7771 {Arabidopsis thaliana} PIR|H86450|H86450 probable zinc-fing | TC73679 | BF050885 | cLEM19J4 | VIIIC3 | T2F6 | R19 | C6 | r4-c2 | r1-c3 |
| ornithine aminotransferase {Arabidopsis thaliana} | TC73702 | AW222058 | cLEN6D8 | IXC12 | T3E23 | R2 | C5 | r2-c1 | r4-c3 |
| pyrophosphate-dependent phosphofructo-1-kinase {Arabidopsis thaliana}GP|7269478|emb|CAB79482.1||AL1 | TC73706 | BE433184 | cLEG12J15 | XIIB5 | T3D10 | R15 | C4 | r2-c1 | r4-c3 |
| sugar-phosphate isomerase-like protein {Arabidopsis thaliana}PIR|T47628|T47628 sugar-phosphate isom | TC73722 | BE451590 | cLEY19P11 | XIVB3 | T4C6 | R19 | C3 | r2-c2 | r1-c4 |
| cytochrome P450, putative {Arabidopsis thaliana} | TC73737 | BE463315 | cTOC12H5 | XVIIC6 | T5E11 | R14 | C5 | r3-c2 | r4-c4 |
| RING-H2 finger protein RHF2a {Arabidopsis thaliana}GP|13374859|emb|CAC34493.1||AL589883 RING-H2 fin | TC73753 | AW617482 | cLHT23O11 | XIIIA3 | T4A5 | R20 | C1 | r2-c2 | r1-c4 |
| Contains similarity to gb|AF136530 transcriptional regulator from Zea mays. {Arabidopsis thaliana}P | TC73763 | AW980011 | cLEC19M9 | XIIF10 | T3L20 | R5 | C12 | r2-c1 | r4-c3 |
| glucose-6-phosphate 1-dehydrogenase {Solanum tuberosum} | TC73842 | BE451514 | cLEY19C1 | XIVA12 | T4A24 | R1 | C1 | r2-c2 | r1-c4 |
| putative pyrophosphate--fructose-6-phosphate 1-phosphotransferase {Arabidopsis thaliana}PIR|B84613| | TC73845 | AW033973 | cLEC38A6 | IIC6 | T1E12 | R13 | C5 | r1-c2 | r3-c3 |
| putative glucosyltransferase {Arabidopsis thaliana}PIR|E84680|E84680 probable glucosyltransferase [ | TC73904 | AW035536 | cLEC39G15 | IB10 | T1C19 | R6 | C3 | r1-c2 | r3-c3 |
| acetyl-CoA synthetase {Solanum tuberosum} | TC73927 | BG130097 | cTOF29E8 | XXA12 | T5B24 | R1 | C2 | r3-c2 | r4-c4 |
| Knotted 1 (TKn1) | TC73929 | AW648828 | cLEI6C19 | IB11 | T1C21 | R4 | C3 | r1-c2 | r3-c3 |
| homeotic protein BEL1 homolog {Arabidopsis thaliana} | TC73945 | AI780410 | cLES11N9 | XD6 | T3G12 | R13 | C7 | r2-c1 | r4-c3 |
| flavanone 3-hydroxylase-like protein {Arabidopsis thaliana} | TC73949 | BE450984 | cLEY16C7 | XIIIH12 | T4O23 | R2 | C15 | r2-c2 | r1-c4 |

| | | | | | | | | | |
|---|---|---|---|---|---|---|---|---|---|
| thaliana} | | | | | | | | | |
| putative cinnamoyl CoA reductase {Arabidopsis thaliana}PIR\|C84630\|C84630 probable cinnamoyl CoA red | TC73957 | AW616521 | cLHT11H15 | XIVH5 | T4O10 | R15 | C15 | r2-c2 | r1-c4 |
| glucosyl transferase {Nicotiana tabacum}GP\|1805359\|dbj\|BAA19155.1\|\|AB000623 glucosyl transferase {N | TC73986 | BE434399 | cLEG16L10 | VF2 | T2K3 | R22 | C11 | r4-c2 | r1-c3 |
| helicase-like transcription factor-like protein {Arabidopsis thaliana} | TC73997 | BG124688 | cTOF6I11 | XXF2 | T5L4 | R21 | C12 | r3-c2 | r4-c4 |
| pyruvate dehydrogenase E1 beta subunit isoform 1 {Zea mays} | TC74002 | AI771828 | cLED38G13 | IIIH7 | T1P13 | R12 | C16 | r1-c2 | r3-c3 |
| fructose-6-phosphate 2-kinase/fructose-2,6-bisphosphatase {Solanum tuberosum}PIR\|T07016\|T07016 6-ph | TC74004 | BF112964 | cLEG43O19 | VIG7 | T2M14 | R11 | C13 | r4-c2 | r1-c3 |
| 1,4-alpha-glucan branching enzyme {Solanum tuberosum}☐GP\|1621012\|emb\|CAA70038.1\|\|Y08786 1,4-alpha-gl | TC74010 | AW929962 | cLEF40A9 | IVE9 | T1J18 | R7 | C10 | r1-c2 | r3-c3 |
| 76 kDa mitochondrial complex I subunit {Solanum tuberosum}SP\|Q43644\|NUAM_SOLTU NADH-UBIQUINONE OXID | TC74019 | BG124963 | cTOF7O21 | XXG2 | T5N4 | R21 | C14 | r3-c2 | r4-c4 |
| Similar to ATP-citrate-lyase {Arabidopsis thaliana}PIR\|F96633\|F96633 hypothetical protein F8A5.32 [ | TC74060 | BF050945 | cLEM21G15 | XXID9 | T6G17 | R8 | C7 | r3-c1 | r2-c4 |
| putative anthocyanidin-3-glucoside rhamnosyltransferase {Arabidopsis thaliana}PIR\|D84614\|D84614 hyp | TC74086 | AW647635 | cLEI10B9 | VIIA8 | T2B15 | R12 | C2 | r4-c2 | r1-c3 |
| floral homeotic protein pmads 2 {Petunia hybrida}SP\|Q07474\|MAD2_PETHY FLORAL HOMEOTIC PROTEIN PMADS | TC74087 | BG627196 | cLEL16I15 | XXIC4 | T6E7 | R18 | C5 | r3-c1 | r2-c4 |
| CYTOCHROME P450 71D10 (EC 1.14.-.-).GP\|2739000\|gb\|AAB94588.1\|\|AF022459 CYP71D10p {Glycine max}PIR\| | TC74103 | AW218370 | cLEZ7L22 | XIVB2 | T4C4 | R21 | C3 | r2-c2 | r1-c4 |
| putative CONSTANS-like B-box zinc finger protein {Arabidopsis thaliana}PIR\|G84920\|G84920 hypothetic | TC74105 | BG129734 | cTOF28C7 | XXA8 | T5B16 | R9 | C2 | r3-c2 | r4-c4 |
| Similar to gb\|X90982 phosphoenolpyruvate carboxylase (ppc1) from Solanum tuberosum. {Arabidopsis tha | TC74116 | AI490644 | cLED24K5 | VIIIC12 | T2F24 | R1 | C6 | r4-c2 | r1-c3 |
| putative lipoxygenase {Arabidopsis | TC74124 | BE353443 | cTOA19O1 | VA11 | T2A21 | R4 | C1 | r4-c2 | r1-c3 |

| | | | | | | | | |
|---|---|---|---|---|---|---|---|---|---|
| thaliana}PIR\|B96699\|B96699 probable lipoxygenase F12B7.11 [impor | | | 1 | | | | | |
| putative beta-amylase {Arabidopsis thaliana}GP\|5302810\|emb\|CAB46051.1\|\|Z97342 putative beta-amylase | TC74131 | BG642863 | cTOF25L12 | XIXH2 | T5P3 | R22 | C16 | r3-c2 | r4-c4 |
| UDP-glucose:protein transglucosylase {Solanum tuberosum} | TC74136 | BE353792 | cTOD6G2 | VA3 | T2A5 | R20 | C1 | r4-c2 | r1-c3 |
| hydroxymethyltransferase {Arabidopsis thaliana}GP\|2244749\|emb\|CAB10172.1\|\|Z97335 hydroxymethyltrans | TC74141 | BE449811 | cLEY14O1 9 | XIIIG11 | T4M21 | R4 | C13 | r2-c2 | r1-c4 |
| hexose transporter {Nicotiana tabacum} | TC74146 | BG642646 | cTOF25C18 | XIXG11 | T5N21 | R4 | C14 | r3-c2 | r4-c4 |
| alpha-glucosidase {Solanum tuberosum subsp. tuberosum} | TC74149 | BE437180 | cLEG35B1 6 | XVIA11 | T4B22 | R3 | C2 | r2-c2 | r1-c4 |
| ATP synthase delta' subunit, mitochondrial precursor {Ipomoea batatas}SP\|Q40089\|ATP4_IPOBA ATP SYNT | TC74166 | BF176603 | cLEZ20N15 | XIVG3 | T4M6 | R19 | C13 | r2-c2 | r1-c4 |
| knotted1-like homeobox protein {Malus domestica}SP\|O04136\|HKL3_MALDO HOMEOBOX PROTEIN KNOTTED-1 LIK | TC74178 | BG127568 | cTOF17B16 | XIXA11 | T5B21 | R4 | C2 | r3-c2 | r4-c4 |
| GLYCINE DEHYDROGENASE [DECARBOXYLATING], MITOCHONDRIAL PRECURSOR (EC 1.4.4.2) (GLYCINE DECARBOXYLASE | TC74186 | BG139081 | cLPP12A23 | XVD5 | T4H9 | R16 | C8 | r2-c2 | r1-c4 |
| contains similarity to CONSTANS homologs~gene_id:MIF21.14 {Arabidopsis thaliana} | TC74187 | AW037464 | cLET4F4 | XIIF4 | T3L8 | R17 | C12 | r2-c1 | r4-c3 |
| chalcone synthase | TC74227 | BG628623 | cLEL22P8 | XXIF4 | T6K7 | R18 | C11 | r3-c1 | r2-c4 |
| ADP-glucose pyrophosphorylase large subunit | TC74234 | AI776884 | cLER20I1 | XB2 | T3C4 | R21 | C3 | r2-c1 | r4-c3 |
| aldose 1-epimerase-like protein {Arabidopsis thaliana} | TC74239 | AW223542 | cLEN12C2 2 | VIIIG8 | T2N16 | R9 | C14 | r4-c2 | r1-c3 |
| 3-phoshoshikimate 1-carboxyvinyltransferase precursor (5-enolpyruvylshikimate-3-phosphate synthase) | TC74249 | AI780056 | cLES10E5 | XC12 | T3E24 | R1 | C5 | r2-c1 | r4-c3 |
| CYTOCHROME P450 98A2 (EC 1.14.-.- ).GP\|2738998\|gb\|AAB94587.1\|\|AF022458 CYP98A2p {Glycine max}PIR\|T0 | TC74259 | BE451618 | cLEY20I21 | XIVB4 | T4C8 | R17 | C3 | r2-c2 | r1-c4 |
| zinc finger protein {Oryza sativa}PIR\|JE0113\|JE0113 zinc-finger protein S3574 [imported] - rice | TC74290 | AI777848 | cLES3A24 | XH1 | T3O2 | R23 | C15 | r2-c1 | r4-c3 |

76

| | | | | | | | | | |
|---|---|---|---|---|---|---|---|---|---|
| Chain A, Glycolate Oxidase (E.C.1.1.3.15) Mutant With Tyr 24 Replaced By Phe (Y24f)GP\|999543\|pdb\|1G | TC74300 | AW441496 | cLEN17M12 | IXB1 | T3C1 | R24 | C3 | r2-c1 | r4-c3 |
| RING finger-like protein {Arabidopsis thaliana}PIR\|T47605\|T47605 RING finger-like protein - Arabido | TC74308 | AW220427 | cLEX10L12 | XIIIA8 | T4A15 | R12 | C1 | r2-c2 | r1-c4 |
| Contains similarity to acyl-CoA thioesterase from Streptomyces coelicolor A3(2) gb\|AL163641. EST gb | TC74331 | BG629827 | cLEL30C6 | XXIE1 | T6I1 | R24 | C9 | r3-c1 | r2-c4 |
| putative bZIP transcription factor {Arabidopsis thaliana}PIR\|G84831\|G84831 probable bZIP transcript | TC74347 | BG644072 | cTOF34C15 | XXD5 | T5H10 | R15 | C8 | r3-c2 | r4-c4 |
| hyoscyamine 6-dioxygenase hydroxylase, putative {Arabidopsis thaliana}PIR\|G86472\|G86472 probable hy | TC74351 | AW033695 | cLEC29O19 | IH2 | T1O3 | R22 | C15 | r1-c2 | r3-c3 |
| leucine zipper transcription factor TGA2.1 {Nicotiana tabacum}SP\|O24160\|TG21_TOBAC TGACG-SEQUENCE S | TC74356 | BG129128 | cTOF23K20 | XIXG1 | T5N1 | R24 | C14 | r3-c2 | r4-c4 |
| succinate dehydrogenase flavoprotein alpha subunit {Arabidopsis thaliana}GP\|8843734\|dbj\|BAA97282.1\| | TC74365 | AW398787 | cLPT4H10 | XVIA9 | T4B18 | R7 | C2 | r2-c2 | r1-c4 |
| cytochrome P450 {Arabidopsis thaliana} | TC74397 | AW617140 | cLHT21D12 | XVB8 | T4D15 | R12 | C4 | r2-c2 | r1-c4 |
| N-hydroxycinnamoyl/benzoyltransferase-like protein {Arabidopsis thaliana} | TC74426 | AW617365 | cLHT22D16 | XVB1 | T4D1 | R24 | C4 | r2-c2 | r1-c4 |
| phosphoribosyl pyrophosphate synthase isozyme 4 {Spinacia oleracea} | TC74427 | AI485769 | cLED4K18 | IVA6 | T1B12 | R13 | C2 | r1-c2 | r3-c3 |
| (+)-DELTA-CADINENE SYNTHASE ISOZYME A (EC 4.6.1.11) (D-CADINENE SYNTHASE).GP\|1217956\|emb\|CAA65289.1 | TC74429 | AW035405 | cLEC38O24 | IIC10 | T1E20 | R5 | C5 | r1-c2 | r3-c3 |
| contains similarity to apoptosis antagonizing transcription factor~gene_id:MFB13.10 {Arabidopsis tha | TC74441 | AW036113 | cLEE1M7 | IVD8 | T1H16 | R9 | C8 | r1-c2 | r3-c3 |
| putative sugar transporter; member of major facilitative superfamily; integral membrane protein {Bet | TC74458 | AI777293 | cLER20L10 | XB4 | T3C8 | R17 | C3 | r2-c1 | r4-c3 |
| 4-alpha-glucanotransferase precursor (disproportionating enzyme) (d-enzyme) {Solanum tuberosum}SP\|Q | TC74463 | AW737543 | cTOD3F15 | XVIIG8 | T5M15 | R12 | C13 | r3-c2 | r4-c4 |
| NADH glutamate dehydrogenase {Nicotiana plumbaginifolia}SP\|O04937\|DHEA_NICPL GLUTAMATE DEHYDROGENAS | TC74481 | AW930356 | cLEF43J5 | XXIIA9 | T6A18 | R7 | C1 | r3-c1 | r2-c4 |
| bZIP protein {Arabidopsis thaliana}PIR\|T49227\|T49227 | TC74487 | AW216659 | cLET39D19 | IIF9 | T1K18 | R7 | C11 | r1-c2 | r3-c3 |

| | | | | | | | | | |
|---|---|---|---|---|---|---|---|---|---|
| bZIP protein - Arabidopsis thaliana | | | | | | | | | |
| zinc-finger protein, putative; 7043-7771 {Arabidopsis thaliana}PIR|H86450|H86450 probable zinc-fing | TC74525 | BG643132 | cTOF26N17 | XIXH11 | T5P21 | R4 | C16 | r3-c2 | r4-c4 |
| cytochrome P450 {Helianthus tuberosus} | TC74527 | AI776121 | cLER17B3 | IXG12 | T3M23 | R2 | C13 | r2-c1 | r4-c3 |
| fructose-bisphosphate aldolase, cytoplasmic isozyme 1 {Pisum sativum}SP|P46256|ALF1_PEA FRUCTOSE-BI | TC74553 | AW929679 | cTOC10G23 | XVIIC3 | T5E5 | R20 | C5 | r3-c2 | r4-c4 |
| transcriptional adaptor ADA2b {Arabidopsis thaliana} | TC74560 | BE459241 | cLEM5P22 | IVH7 | T1P14 | R11 | C16 | r1-c2 | r3-c3 |
| putative oxalyl-CoA decarboxylase {Oryza sativa} | TC74622 | AW094043 | cLET27G5 | XXIE11 | T6I21 | R4 | C9 | r3-c1 | r2-c4 |
| putative UDP-N-acetylglucosamine--N-acetylmuramyl-(pentapeptide)-pyrophosphoryl-undecaprenol N-acety | TC74633 | BF051710 | cLEM24A9 | VIIIB12 | T2D24 | R1 | C4 | r4-c2 | r1-c3 |
| G-Box binding protein 2 {Catharanthus roseus} | TC74645 | BG643085 | cTOF26F11 | XIXH5 | T5P9 | R16 | C16 | r3-c2 | r4-c4 |
| putative indole-3-glycerol phosphate synthase {Arabidopsis thaliana}PIR|B84457|B84457 probable indo | TC74653 | AW648549 | cLEI4P2 | VIIE8 | T2J15 | R12 | C10 | r4-c2 | r1-c3 |
| contains similarity to nucleotide sugar epimerases {Arabidopsis thaliana}GP|7267098|emb|CAB80769.1| | TC74661 | BG126144 | cTOF11K17 | XVIIIG12 | T5M24 | R1 | C13 | r3-c2 | r4-c4 |
| contains similarity to ATPases associated with various cellular activities (Pfam: AAA.hmm, score: 15 | TC74673 | BG123268 | cTOF1K10 | XIXD7 | T5H13 | R12 | C8 | r3-c2 | r4-c4 |
| putative dihydrolipoamide succinyltransferase {Arabidopsis thaliana}GP|7269544|emb|CAB79546.1||AL16 | TC74697 | BE353150 | cLEZ17N3 | XIVE6 | T4I12 | R13 | C9 | r2-c2 | r1-c4 |
| auxin-induced basic helix-loop-helix transcription factor, putative {Arabidopsis thaliana}GP|123213 | TC74751 | AW650619 | cLEI13H3 | VIIB10 | T2D19 | R6 | C4 | r4-c2 | r1-c3 |
| Myb-related transcription factor-like protein {Arabidopsis thaliana} | TC74758 | BF097751 | cLEW23B13 | XIIG2 | T3N4 | R21 | C14 | r2-c1 | r4-c3 |
| contains similarity to enolase-phosphatase~gene_id:K19P17.1 {Arabidopsis thaliana} | TC74779 | AW034046 | cLEC37M7 | IIC5 | T1E10 | R15 | C5 | r1-c2 | r3-c3 |
| formyl transferase, putative {Arabidopsis thaliana}PIR|H96690|H96690 probable formyl transferase F2 | TC74804 | BG132111 | cTOE6O24 | XVIIG2 | T5M3 | R22 | C13 | r3-c2 | r4-c4 |
| immediate-early salicylate-induced glucosyltransferase {Nicotiana tabacum}GP|1685005|gb|AAB36653.1| | TC74808 | AW624795 | cLEZ8O11 | XIVG8 | T4M16 | R9 | C13 | r2-c2 | r1-c4 |
| putative para-aminobenzoate synthase and glutamine amidotransferase, a bifunctional enzyme {Arabidop | TC74821 | AW223881 | cLEN13P22 | VIIIH10 | T2P20 | R5 | C16 | r4-c2 | r1-c3 |
| alpha-glucosidase {Solanum tuberosum subsp. tuberosum} | TC74846 | AI775531 | cLER15F22 | IXF8 | T3K15 | R12 | C11 | r2-c1 | r4-c3 |

78

| | | | | | | | | | |
|---|---|---|---|---|---|---|---|---|---|
| MADS-box transcription factor FBP4 {Petunia x hybrida} | TC74865 | AW441864 | cLEN18D10 | IXB6 | T3C11 | R14 | C3 | r2-c1 | r4-c3 |
| NAD-dependent isocitrate dehydrogenase {Nicotiana tabacum} | TC74889 | BG123588 | cTOF2M10 | XXB9 | T5D18 | R7 | C4 | r3-c2 | r4-c4 |
| cytochrome p450 lxxviia1 {Solanum melongena}SP\|P37123\|C771_SOLME CYTOCHROME P450 77A1 (EC 1.14.-.-) | TC74928 | AW092327 | cLET19H11 | XIE1 | T3J1 | R24 | C10 | r2-c1 | r4-c3 |
| threonine deaminase {Nicotiana attenuata} | TC74935 | AW934272 | cLEF58K10 | VC10 | T2E19 | R6 | C5 | r4-c2 | r1-c3 |
| putative fatty acid desaturase/cytochrome b5 fusion protein {Arabidopsis thaliana}PIR\|A84900\|A84900 | TC74979 | AI484221 | cLES1E11 | XG3 | T3M6 | R19 | C13 | r2-c1 | r4-c3 |
| sugar transporter like protein {Arabidopsis thaliana}GP\|2464913\|emb\|CAB16808.1\|\|Z99708 sugar transp | TC74988 | AI777669 | cLES2O22 | XG12 | T3M24 | R1 | C13 | r2-c1 | r4-c3 |
| putative UDP-glucose:glycoprotein glucosyltransferase; 101200-91134 {Arabidopsis thaliana}PIR\|G9673 | TC75003 | AW030751 | cLEC25H7 | IF10 | T1K19 | R6 | C11 | r1-c2 | r3-c3 |
| putative nucleotide-sugar transporter {Arabidopsis thaliana}PIR\|E84509\|E84509 probable vanadate res | TC75014 | AW219952 | cLEX6M9 | XIIIF4 | T4K7 | R18 | C11 | r2-c2 | r1-c4 |
| Putative UDP-glucose glucosyltransferase {Arabidopsis thaliana}PIR\|H86356\|H86356 probable UDP-gluco | TC75032 | AW621210 | cLEX11K13 | XIIIB4 | T4C7 | R18 | C3 | r2-c2 | r1-c4 |
| RING-H2 finger protein RHF2a {Arabidopsis thaliana}GP\|13374859\|emb\|CAC34493.1\|\|AL589883 RING-H2 fin | TC75038 | BE434274 | cLEG15L16 | VE8 | T2I15 | R12 | C9 | r4-c2 | r1-c3 |
| reticuline oxidase-like protein {Arabidopsis thaliana}GP\|7268879\|emb\|CAB79083.1\|\|AL161553 reticulin | TC75058 | AI771577 | cLED30I10 | IIIF4 | T1L7 | R18 | C12 | r1-c2 | r3-c3 |
| zinc finger protein-like {Arabidopsis thaliana} | TC75079 | BE461025 | cLEG37C14 | VID8 | T2G16 | R9 | C7 | r4-c2 | r1-c3 |
| contains similarity to ATPases associated with various cellular activities (Pfam: AAA.hmm, score: 15 | TC75096 | AI489887 | cLED15P20 | IIIA6 | T1B11 | R14 | C2 | r1-c2 | r3-c3 |
| contains similarity to phosphoenolpyruvate synthase (ppsA) (GB:AE001056) {Arabidopsis thaliana}PIR\| | TC75102 | BE433957 | cLEG9G8 | VIIA5 | T2B9 | R16 | C2 | r4-c2 | r1-c3 |
| cytochrome P450 {Arabidopsis thaliana} | TC75118 | AI895885 | cLEC10K8 | IC1 | T1E1 | R24 | C5 | r1-c2 | r3-c3 |
| Similar to yeast general negative regulator of transcription subunit 1 {Arabidopsis thaliana}PIR\|G8 | TC75146 | AI895013 | cLEC6F5 | IIE6 | T1I12 | R13 | C9 | r1-c2 | r3-c3 |
| putative cytochrome P450 {Oryza sativa}GP\|11761120\|dbj\|BAB19110.1\|\|AP002839 putative cytochrome P45 | TC75173 | BG135463 | cTOE22L17 | XVIIIE6 | T5I12 | R13 | C9 | r3-c2 | r4-c4 |

79

| | | | | | | | | |
|---|---|---|---|---|---|---|---|---|---|
| nitrite reductase {Capsicum annuum} | TC75187 | BE451364 | cLEY17P12 | XIVA6 | T4A12 | R13 | C1 | r2-c2 | r1-c4 |
| tryptophan synthase beta chain {Arabidopsis thaliana} | TC75190 | AW625162 | cLEZ11G16 | XIVD1 | T4G2 | R23 | C7 | r2-c2 | r1-c4 |
| Contains a weak similarity to chalcone--flavonone isomerase from Pueraria lobata GP|Q43056 and conta | TC75202 | BE432201 | cLEG6D8 | VIH4 | T2O8 | R17 | C15 | r4-c2 | r1-c3 |
| similar to ATPases associated with various cellular activites (Pfam: AAA.hmm, score: 230.91) {Arabid | TC75211 | BE433089 | cLEG12O3 | VE1 | T2I1 | R24 | C9 | r4-c2 | r1-c3 |
| UTP-glucose glucosyltransferase {Arabidopsis thaliana} | TC75218 | BE432301 | cLEG7K10 | VIH9 | T2O18 | R7 | C15 | r4-c2 | r1-c3 |
| PROBABLE VACUOLAR ATP SYNTHASE SUBUNIT H (EC 3.6.1.34) (V-ATPASE H SUBUNIT) (VACUOLAR PROTON PUMP H | TC75225 | AW624381 | cTOB15B2 1 | XVIH11 | T4P22 | R3 | C16 | r2-c2 | r1-c4 |
| flavanone 3-hydroxylase-like protein {Arabidopsis thaliana} | TC75238 | BF050330 | cLEM17K2 2 | VIIG12 | T2N23 | R2 | C14 | r4-c2 | r1-c3 |
| isoflavone reductase-like protein {Arabidopsis thaliana}GP|7270404|emb|CAB80171.1||AL161585 isoflav | TC75240 | BF050320 | cLEM17I14 | VIIG11 | T2N21 | R4 | C14 | r4-c2 | r1-c3 |
| cystathionine beta-lyase {Solanum tuberosum} | TC75245 | BE460087 | cLEM8N14 | VIIIF9 | T2L18 | R7 | C12 | r4-c2 | r1-c3 |
| putative acyl-CoA synthetase; 62297-59022 {Arabidopsis thaliana}PIR|D96805|D96805 probable acyl-CoA | TC75253 | BE441105 | cLEM1O7 | VIIH11 | T2P21 | R4 | C16 | r4-c2 | r1-c3 |
| PHOSPHOGLUCOMUTASE, CHLOROPLAST PRECURSOR (EC 5.4.2.2) (GLUCOSE PHOSPHOMUTASE) (PGM).GP|8250622|emb | TC75272 | BE458570 | cLEM2F21 | VIIID2 | T2H4 | R21 | C8 | r4-c2 | r1-c3 |
| putative cytochrome P450 {Solanum chacoense}SP|P93531|C7D7_SOLCH CYTOCHROME P450 71D7 (EC 1.14.-.-) | TC75279 | AW616136 | cLHT6I5 | XVD1 | T4H1 | R24 | C8 | r2-c2 | r1-c4 |
| hydroxymethyltransferase {Arabidopsis thaliana}GP|2244749|emb|CAB10172.1||Z97335 hydroxymethyltrans | TC75281 | AW738537 | cTOD7L23 | XVIIIA9 | T5A18 | R7 | C1 | r3-c2 | r4-c4 |
| cytochrome P450, putative {Arabidopsis thaliana}PIR|F86441|F86441 probable cytochrome P450 importe | TC75284 | AW651509 | cLEI16L8 | VIID2 | T2H3 | R22 | C8 | r4-c2 | r1-c3 |
| HD-Zip protein {Arabidopsis thaliana}GP|3132474|gb|AAC16263.1||AC003096 homeodomain transcription f | TC75348 | AW035070 | cLEC13O1 9 | ID3 | T1G5 | R20 | C7 | r1-c2 | r3-c3 |
| putative bZIP transcription factor {Arabidopsis thaliana}PIR|G84831|G84831 probable bZIP transcript | TC75368 | AW617711 | cLHT24E20 | XVB7 | T4D13 | R12 | C4 | r2-c2 | r1-c4 |
| putative phosphate/phosphoenolpyruvate translocator | TC75371 | AW617772 | cLHT24B1 | XVB4 | T4D7 | R18 | C4 | r2-c2 | r1-c4 |

| | | | | | | | | | |
|---|---|---|---|---|---|---|---|---|---|
| protein {Arabidopsis thaliana}PIR|D84649|D84649 | | | | | | | | | |
| myb-like protein {Arabidopsis thaliana}PIR|T48253|T48253 myb-like protein - Arabidopsis thaliana | TC75389 | AW040511 | cLET8M17 | XIID1 | T3H2 | R23 | C8 | r2-c1 | r4-c3 |
| putative glucosyltransferase {Arabidopsis thaliana}PIR|H84786|H84786 probable glucosyltransferase [ | TC75393 | AW623996 | cTOB13F6 | XVIH5 | T4P10 | R15 | C16 | r2-c2 | r1-c4 |
| tyrosine decarboxylase {Papaver somniferum}SP|P54771|TYD5_PAPSO TYROSINE/DOPA DECARBOXYLASE 5 [INCL | TC75404 | BE449397 | cLHT31K16 | XVC2 | T4F3 | R22 | C6 | r2-c2 | r1-c4 |
| Similar to gb|U44028 transcription factor CKC from Arabidopsis thaliana and contains two PF|00847 AP | TC75426 | AW030921 | cLEC5O13 | IIE3 | T1I6 | R19 | C9 | r1-c2 | r3-c3 |
| bZIP transcription factor 6 {Phaseolus vulgaris} | TC75472 | AW930815 | cLEF41I7 | IVF5 | T1L10 | R15 | C12 | r1-c2 | r3-c3 |
| HEAT SHOCK FACTOR PROTEIN 7 (HSF 7) (HEAT SHOCK TRANSCRIPTION FACTOR 7) (HSTF 7).GP|4539457|emb|CAB | TC75476 | AW931176 | cLEF43B2 | IVG1 | T1N2 | R23 | C14 | r1-c2 | r3-c3 |
| phosphoenolpyruvate carboxylase 1 {Gossypium hirsutum}GP|2266947|gb|AAB80714.1||AF008939 phosphoeno | TC75495 | BG130811 | cTOE1I10 | XVIIID9 | T5G18 | R7 | C7 | r3-c2 | r4-c4 |
| CYP82C1p {Glycine max}PIR|T05942|T05942 cytochrome P450 82C1 - soybean | TC75545 | BG132070 | cTOE6A12 | XVIIIF12 | T5K24 | R1 | C11 | r3-c2 | r4-c4 |
| glucose-6-phosphate isomerase {Spinacia oleracea}PIR|T09153|T09153 glucose-6-phosphate isomerase (E | TC75577 | AW222686 | cLEN9E7 | IXE1 | T3I1 | R24 | C9 | r2-c1 | r4-c3 |
| cytochrome P450 {Solanum tuberosum} | TC75602 | BF176441 | cLEZ20B1 | XIVF10 | T4K20 | R5 | C11 | r2-c2 | r1-c4 |
| acetyl-CoA synthetase-like protein {Arabidopsis thaliana} | TC75606 | AW092581 | cLET20N2 | XIF1 | T3L1 | R24 | C12 | r2-c1 | r4-c3 |
| Similar to ribokinase {Arabidopsis thaliana}PIR|F86307|F86307 hypothetical protein AAD50017.1 [impo | TC75622 | AW929172 | cTOC6B19 | XVIIE4 | T5I7 | R18 | C9 | r3-c2 | r4-c4 |
| 3-phosphoshikimate 1-carboxyvinyltransferase precursor (5-enolpyruvylshikimate-3-phosphate synthase) | TC75646 | BG129236 | cTOF23B2 | XIXF7 | T5L13 | R12 | C12 | r3-c2 | r4-c4 |
| TRYPTOPHAN SYNTHASE BETA CHAIN 2 PRECURSOR (EC 4.2.1.20).GP|2792520|gb|AAB97087.1||AF042320 tryptop | TC75671 | AW031813 | cLEC39L1 | IID1 | T1G2 | R23 | C7 | r1-c2 | r3-c3 |

81

| | | | | | | | | | |
|---|---|---|---|---|---|---|---|---|---|
| cinnamoyl CoA reductase-like protein {Arabidopsis thaliana}PIR\|T48643\|T48643 cinnamoyl CoA reductas | TC75675 | BE463117 | cTOC11G16 | XVIIC5 | T5E9 | R16 | C5 | r3-c2 | r4-c4 |
| nucleotide diphosphate kinase Ia {Arabidopsis thaliana}GP\|6065740\|emb\|CAB58230.1\|AJ012758 nucleoti | TC75705 | AW096444 | cLET38J7 | XIG12 | T3N23 | R2 | C14 | r2-c1 | r4-c3 |
| diacylglycerol kinase ATDGK1 homolog {Arabidopsis thaliana}GP\|6562306\|emb\|CAB62604.1\|AL133421 diac | TC75708 | BF112916 | cLEG43G7 | VIG4 | T2M8 | R17 | C13 | r4-c2 | r1-c3 |
| adenosine kinase {Arabidopsis thaliana}GP\|7378610\|emb\|CAB83286.1\|AL162751 adenosine kinase-like pr | TC75729 | AW091866 | cLET16L10 | IVE3 | T1J6 | R19 | C10 | r1-c2 | r3-c3 |
| bZIP protein {Arabidopsis thaliana}PIR\|T49227\|T49227 bZIP protein - Arabidopsis thaliana | TC75747 | BF112845 | cLEG42H1 | XIH3 | T3P5 | R20 | C16 | r2-c1 | r4-c3 |
| transcription factor like protein {Arabidopsis thaliana}GP\|2244999\|emb\|CAB10419.1\|Z97341 transcrip | TC75876 | AI484729 | cLED3D21 | IIIH12 | T1P23 | R2 | C16 | r1-c2 | r3-c3 |
| putative RING zinc finger protein {Arabidopsis thaliana}GP\|6682260\|gb\|AAF23312.1\|AC016661_37\|A C0166 | TC75892 | AW039871 | cLET13H5 | XIC10 | T3F19 | R6 | C6 | r2-c1 | r4-c3 |
| polyneuridine aldehyde esterase, putative; 10297-12282 {Arabidopsis thaliana} | TC75906 | BG133780 | cTOE14I21 | XVIIIC7 | T5E14 | R11 | C5 | r3-c2 | r4-c4 |
| glucose 6 phosphate/phosphate translocator-like protein {Arabidopsis thaliana}PIR\|T51467\|T51467 glu | TC75908 | BG133380 | cTOE12O11 | XVIIIC1 | T5E2 | R23 | C5 | r3-c2 | r4-c4 |
| leucine zipper transcription factor {Solanum tuberosum}GP\|575418\|emb\|CAA57894.1\|X82544 leucine zip | TC75947 | AW224429 | cLEW2G3 | XIIH8 | T3P16 | R9 | C16 | r2-c1 | r4-c3 |
| zinc finger protein-like {Arabidopsis thaliana} | TC75949 | AW618814 | cLPT17I15 | XVH4 | T4P7 | R18 | C16 | r2-c2 | r1-c4 |
| Strong similarity to the TATA binding protein-associated factor from A. thaliana gb\|Y13673. ESTs gb | TC76006 | AI781503 | cLES16A15 | IIG1 | T1M2 | R23 | C13 | r1-c2 | r3-c3 |
| contains similarity to transcription regulator~gene_id:MRG7.19 {Arabidopsis thaliana} | TC76014 | AW030833 | cLEC22E16 | IE11 | T1I21 | R4 | C9 | r1-c2 | r3-c3 |
| putative beta-amylase {Oryza sativa}GP\|13489165\|gb\|AAK27799.1\|AC022457_2\|AC 022457 putative beta-amy | TC76034 | AW735936 | cTOA5B10 | XVIE11 | T4J22 | R3 | C10 | r2-c2 | r1-c4 |
| aspartate carbamoyltransferase-poj | TC76045 | AI773785 | cLER8M9 | XC10 | T3E20 | R5 | C5 | r2-c1 | r4-c3 |
| phosphate/phosphoenolpyruvate translocator protein-like {Arabidopsis thaliana} | TC76052 | BE431693 | cLEG30O13 | VIA5 | T2A10 | R15 | C1 | r4-c2 | r1-c3 |
| bZIP transcriptional activator RSG {Nicotiana tabacum} | TC76055 | BE435933 | cLEG29D2 | VH10 | T2O19 | R6 | C15 | r4-c2 | r1-c3 |

82

| | | | 3 | | | | | | |
|---|---|---|---|---|---|---|---|---|---|
| soluble starch (bacterial glycogen) synthase {Solanum tuberosum}SP|P93568|UGS2_SOLTU SOLUBLE GLYCOG | TC76060 | BE434991 | cLEG24J21 | VG7 | T2M13 | R12 | C13 | r4-c2 | r1-c3 |
| RING zinc finger protein-like {Arabidopsis thaliana} | TC76080 | AW031045 | cLEC13K8 | ID1 | T1G1 | R24 | C7 | r1-c2 | r3-c3 |
| lipoxygenase {Zantedeschia aethiopica} | TC76090 | AI896460 | cLEC15G13 | ID6 | T1G11 | R14 | C7 | r1-c2 | r3-c3 |
| 13-lipoxygenase {Solanum tuberosum}GP|1495802|emb|CAA65268.1||X96405 13-lipoxygenase {Solanum tuber | TC76097 | AI773255 | cLER5L12 | XC2 | T3E4 | R21 | C5 | r2-c1 | r4-c3 |
| phosphoribosylanthranilate isomerase {Arabidopsis thaliana} | TC76109 | AI485822 | cLED4C4 | XIE9 | T3J17 | R8 | C10 | r2-c1 | r4-c3 |
| glycine hydroxymethyltransferase (EC 2.1.2.1)-like protein {Arabidopsis thaliana}GP|7270156|emb|CAB | TC76133 | AI782600 | cLES20I3 | XG8 | T3M16 | R9 | C13 | r2-c1 | r4-c3 |
| aldose 1-epimerase-like protein {Arabidopsis thaliana} | TC76138 | AW399079 | cLPT5N10 | XVIB4 | T4D8 | R17 | C4 | r2-c2 | r1-c4 |
| Contains a bZIP transcription factor PF|00170 domain. ESTs gb|R30400, gb|AA650964, gb|AI994521 come | TC76171 | BE353406 | cTOA19E21 | XVIE3 | T4J6 | R19 | C10 | r2-c2 | r1-c4 |
| branched-chain alpha-keto acid decarboxylase E1 beta subunit {Arabidopsis thaliana}PIR|D96597|D9659 | TC76174 | BG131126 | cTOE2L1 | XVIIIF3 | T5K6 | R19 | C11 | r3-c2 | r4-c4 |
| HYDROXYMETHYLGLUTARYL-COA SYNTHASE (EC 4.1.3.5) (HMG-COA SYNTHASE) (3-HYDROXY-3-METHYLGLUTARYL COENZ | TC76206 | BG134552 | cTOE16B16 | XD1 | T3G2 | R23 | C7 | r2-c1 | r4-c3 |
| PROBABLE (S)-2-HYDROXY-ACID OXIDASE, PEROXISOMAL 2 (EC 1.1.3.15) (GLYCOLATE OXIDASE 2) (GOX 2) (SHOR | TC76210 | BF051567 | cLEM23B19 | VIIIB8 | T2D16 | R9 | C4 | r4-c2 | r1-c3 |
| glycolate oxidase | TC76211 | cLES15G14 | cLES15G14 | XVG8 | T4N15 | R12 | C14 | r2-c2 | r1-c4 |
| s-adenosylmethionine decarboxylase proenzyme (induced stolen tip protein tub13) {Solanum tuberosum} | TC76213 | BE436856 | cLEG34M14 | XXIH8 | T6O15 | R12 | C15 | r3-c1 | r2-c4 |
| S-adenosyl-L-methionine synthetase | TC76214 | BG129352 | cTOF24G21 | IVG5 | T1N10 | R15 | C14 | r1-c2 | r3-c3 |
| lipoxygenase | TC76225 | AW222704 | cLEN9G21 | IXE2 | T3I3 | R22 | C9 | r2-c1 | r4-c3 |
| lipoxygenase (LOX) | TC76226 | BE435005 | cLEG24P7 | XXH11 | T5P22 | R3 | C16 | r3-c2 | r4-c4 |
| Contains PF|00249 Myb-like DNA-binding domain. EST gb|Z18152 comes from this gene. {Arabidopsis tha | TC76257 | AW442417 | cLEN22N18 | IXC5 | T3E9 | R16 | C5 | r2-c1 | r4-c3 |
| UDP-glucose pyrophosphorylase precursor {Solanum tuberosum}PIR|JX0128|XNPOU UTP--glucose-1-phosphat | TC76266 | BE458654 | cLEM2P4 | VIIID4 | T2H8 | R17 | C8 | r4-c2 | r1-c3 |

83

| | | | | | | | | | |
|---|---|---|---|---|---|---|---|---|---|
| hydroxycinnamoyl-CoA:tyramine N-hydroxycinnamoyltransferase {Capsicum annuum} | TC76267 | AW035768 | cLEC36G5 | IIB11 | T1C22 | R3 | C3 | r1-c2 | r3-c3 |
| hydroxycinnamoyl-CoA:tyramine N-hydroxycinnamoyltransferase {Capsicum annuum} | TC76268 | AI775981 | cLER17K13 | IXH2 | T3O3 | R22 | C15 | r2-c1 | r4-c3 |
| hydroxycinnamoyl-CoA:tyramine N-hydroxycinnamoyltransferase {Capsicum annuum} | TC76269 | AI777112 | cLER20I22 | IIF11 | T1K22 | R3 | C11 | r1-c2 | r3-c3 |
| hydroxycinnamoyl-CoA:tyramine N-hydroxycinnamoyltransferase {Capsicum annuum} | TC76270 | AI774541 | cLER12J23 | IXE7 | T3I13 | R12 | C9 | r2-c1 | r4-c3 |
| tyramine hydroxycinnamoyltransferase {Nicotiana tabacum} | TC76271 | AW624965 | cLEZ9E23 | XIVG10 | T4M20 | R5 | C13 | r2-c2 | r1-c4 |
| tyramine hydroxycinnamoyltransferase {Nicotiana tabacum} | TC76272 | AW221079 | cLEF3P9 | IVE7 | T1J14 | R11 | C10 | r1-c2 | r3-c3 |
| putative transcription factor BTF3 (RNA polymerase B transcription factor 3); 26343-27201 {Arabidops | TC76286 | AW624910 | cLEZ8I8 | XIVG7 | T4M14 | R11 | C13 | r2-c2 | r1-c4 |
| putative transcription factor BTF3 (RNA polymerase B transcription factor 3); 26343-27201 {Arabidops | TC76287 | AI778696 | cLES6I17 | XIA4 | T3B7 | R18 | C2 | r2-c1 | r4-c3 |
| chloroplast triose phosphate translocator precursor (ctpt) (e29) {Solanum tuberosum}SP|P29463|CPTR | TC76288 | BG123350 | cTOF1N9 | XIXD8 | T5H15 | R12 | C8 | r3-c2 | r4-c4 |
| chloroplast triose phosphate translocator precursor (ctpt) (e29) {Solanum tuberosum}SP|P29463|CPTR | TC76289 | AW096697 | cLET39J4 | XIF7 | T3L13 | R12 | C12 | r2-c1 | r4-c3 |
| anthocyanin 5-O-glucosyltransferase {Petunia x hybrida} | TC76292 | AW220874 | cLEF2D12 | IVE5 | T1J10 | R15 | C10 | r1-c2 | r3-c3 |
| phosphoglycerate kinase, cytosolic {Nicotiana tabacum}SP|Q42962|PGKY_TOBAC PHOSPHOGLYCERATE KINASE, | TC76300 | BG123532 | cTOF2O21 | XIF8 | T3L15 | R12 | C12 | r2-c1 | r4-c3 |
| homeobox 1 protein^^class II knotted-like homeodomain protein | TC76313 | BG126987 | cTOF14E11 | XIXA2 | T5B3 | R22 | C2 | r3-c2 | r4-c4 |
| chorismate synthase 1 | TC76324 | BE436253 | cLEG31O19 | VIA11 | T2A22 | R3 | C1 | r4-c2 | r1-c3 |
| chorismate synthase 1 precursor 3-phosphate phospholyase 1) {Lycopersicon esculentum}SP|Q42884|ARC1 | TC76325 | BG128364 | cTOF20K8 | XIXD11 | T5H21 | R4 | C8 | r3-c2 | r4-c4 |
| chorismate synthase 1 precursor 3-phosphate phospholyase 1) {Lycopersicon esculentum}SP|Q42884|ARC1 | TC76326 | AI490294 | cLED24N12 | IIIC12 | T1F23 | R2 | C6 | r1-c2 | r3-c3 |
| cytosolic NADP-malic enzyme^^malate dehydrogenase | TC76336 | AI484269 | cLES1A11 | XG2 | T3M4 | R21 | C13 | r2-c1 | r4-c3 |
| ATP synthase gamma subunit, chloroplast precursor | TC76348 | BE458757 | cLEM4B17 | VIIID6 | T2H12 | R13 | C8 | r4-c2 | r1-c3 |

| | | | | | | | | | |
|---|---|---|---|---|---|---|---|---|---|
| {Nicotiana tabacum}SP\|P29790\|ATPG_TOBAC ATP SYNTH | | | | | | | | | |
| zinc-finger protein {Petunia x hybrida}GP\|439493\|dbj\|BAA05079.1\|\|D26086 zinc-finger protein {Petuni | TC76350 | AI898415 | cLED33G4 | IIIG2 | T1N3 | R22 | C14 | r1-c2 | r3-c3 |
| fructokinase | TC76354 | AW224797 | cLEY6M13 | XIVC8 | T4E16 | R9 | C5 | r2-c2 | r1-c4 |
| ATP synthase beta subunit, mitochondrial precursor {Nicotiana plumbaginifolia}SP\|P17614\|ATP2_NICPL | TC76359 | AW738375 | cTOD7I21 | IB1 | T1C1 | R24 | C3 | r1-c2 | r3-c3 |
| spermidine synthase | TC76360 | AI778176 | cLES4M11 | XH8 | T3O16 | R9 | C15 | r2-c1 | r4-c3 |
| cDNA~Strawberry pyruvate decarboxylase {Fragaria x ananassa}GP\|10121330\|gb\|AAG13131.1\|AF193791_1\|AF | TC76372 | BF051258 | cLEM22E9 | VIIIB1 | T2D2 | R23 | C4 | r4-c2 | r1-c3 |
| oxoglutarate malate translocator {Solanum tuberosum}GP\|1486472\|emb\|CAA68164.1\|\|X99853 oxoglutarate | TC76379 | BG125227 | cTOF8C1 | XXIIA5 | T6A10 | R15 | C1 | r3-c1 | r2-c4 |
| aspartate aminotransferase glyoxysomal isozyme AAT1 precursor {Glycine max}PIR\|T06136\|T06136 aspart | TC76380 | AW648048 | cLEI3C6 | VIID9 | T2H17 | R8 | C8 | r4-c2 | r1-c3 |
| putative bZIP DNA-binding protein {Capsicum chinense} | TC76385 | BE431986 | cLEG4N17 | XXIC8 | T6E15 | R12 | C5 | r3-c1 | r2-c4 |
| ATP synthase B' subunit precursor {Spinacia oleracea}SP\|P31853\|ATPX_SPIOL ATP SYNTHASE B' CHAIN PRE | TC76386 | AI782445 | cLES19K17 | XG1 | T3M2 | R23 | C13 | r2-c1 | r4-c3 |
| ATP synthase beta chain precursor (subunit II) {Arabidopsis thaliana}GP\|2864617\|emb\|CAA16964.1\|\|AL0 | TC76387 | BG127384 | cTOF16G12 | XIXA8 | T5B15 | R12 | C2 | r3-c2 | r4-c4 |
| homeodomain-leucine zipper protein 57 {Glycine max} | TC76389 | AI490100 | cLED22O23 | IIIC8 | T1F15 | R12 | C6 | r1-c2 | r3-c3 |
| VACUOLAR ATP SYNTHASE SUBUNIT G 1 (EC 3.6.1.34) (V-ATPASE G SUBUNIT 1) (VACUOLAR PROTON PUMP G SUBUN | TC76393 | AW626384 | cLEZ19P6 | XIVF6 | T4K12 | R13 | C11 | r2-c2 | r1-c4 |
| cytochrome p450 lxxii hydroxylase) (ge10h) {Catharanthus roseus}SP\|Q05047\|CP72_CATRO CYTOCHROME P45 | TC76404 | BE432714 | cLEG10A1 | VD3 | T2G5 | R20 | C7 | r4-c2 | r1-c3 |
| nucleoside diphosphate kinase {Pisum sativum} | TC76406 | AA824969 | CT252 | XVIC11 | T4F22 | R3 | C6 | r2-c2 | r1-c4 |
| proline oxidase precursor {Arabidopsis thaliana} | TC76411 | AI489806 | cLED15D13 | IIH12 | T1O24 | R1 | C15 | r1-c2 | r3-c3 |
| UDP-glucose dehydrogenase {Glycine | TC76434 | BG134455 | cTOE16I24 | XXH8 | T5P16 | R9 | C16 | r3-c2 | r4-c4 |

EP 1 454 993 A1

| | | | | | | | | | |
|---|---|---|---|---|---|---|---|---|---|
| max}SP\|Q96558\|UGDH_SOYBN UDP-GLUCOSE 6-DEHYDROGENASE (EC 1.1.1.2 | | | | | | | | | |
| UDP-glucose dehydrogenase {Arabidopsis thaliana} | TC76435 | AW399489 | cLPT7B4 | XVIB3 | T4D6 | R19 | C4 | r2-c2 | r1-c4 |
| UDP-glucose dehydrogenase-like protein {Arabidopsis thaliana}PIR\|T51527\|T51527 UDP-glucose dehydrog | TC76436 | BE344437 | cLEY2E3 | XIVC6 | T4E12 | R13 | C5 | r2-c2 | r1-c4 |
| lipoxygenase {Solanum tuberosum}GP\|1407705\|gb\|AAB67865.1\|\|U60202 lipoxygenase {Solanum tuberosum}P | TC76460 | AW091732 | cLET15B12 | XID4 | T3H7 | R18 | C8 | r2-c1 | r4-c3 |
| 3-ketoacyl-CoA thiolase {Arabidopsis thaliana}GP\|2981618\|dbj\|BAA25249.1\|\|AB008855 3-ketoacyl-CoA th | TC76465 | AI774269 | cLER12I19 | IXE6 | T3I11 | R14 | C9 | r2-c1 | r4-c3 |
| alanine aminotransferase {Arabidopsis thaliana}GP\|12325273\|gb\|AAG52580.1\|AC016529_11\|AC016529 putat | TC76487 | AW622240 | cLEX14L9 | XVIF1 | T4L2 | R23 | C12 | r2-c2 | r1-c4 |
| malate synthase, glyoxysomal {Cucumis sativus}SP\|P08216\|MASY_CUCSA MALATE SYNTHASE, GLYOXYSOMAL (EC | TC76489 | AW649182 | cLEI7K2 | VIIG1 | T2N1 | R24 | C14 | r4-c2 | r1-c3 |
| putative 6-phosphogluconolactonase {Arabidopsis thaliana} | TC76497 | AI484635 | cLED2C20 | IIIE6 | T1J11 | R14 | C10 | r1-c2 | r3-c3 |
| 6-phosphogluconolactonase-like protein {Arabidopsis thaliana} | TC76498 | BE436461 | cLEG32O16 | VIB2 | T2C4 | R21 | C3 | r4-c2 | r1-c3 |
| putative cinnamyl alcohol dehydrogenase {Malus x domestica}PIR\|T16995\|T16995 probable cinnamyl-alco | TC76501 | AI776221 | cLER17H12 | IIIC1 | T1F1 | R24 | C6 | r1-c2 | r3-c3 |
| pyrophosphate--fructose 6-phosphate 1-phosphotransferase alpha subunit (pfp) (6-phosphofructokinase | TC76514 | AW035056 | cLEC21E3 | XVH7 | T4P13 | R12 | C16 | r2-c2 | r1-c4 |
| pyrophosphate--fructose 6-phosphate 1-phosphotransferase alpha subunit (pfp) (6-phosphofructokinase | TC76515 | AW649519 | cLEI8D13 | VIIG2 | T2N3 | R22 | C14 | r4-c2 | r1-c3 |
| triosephosphate isomerase chloroplast precursor {Spinacia oleracea}SP\|P48496\|TPIC_SPIOL TRIOSEPHOSP | TC76528 | AW094307 | cLET28M15 | XIG7 | T3N13 | R12 | C14 | r2-c1 | r4-c3 |
| MALATE DEHYDROGENASE [NADP], CHLOROPLAST PRECURSOR (EC 1.1.1.82) (NADP-MDH).GP\|2827076\|gb\|AAB99753. | TC76554 | AI776445 | cLER18D3 | IXH4 | T3O7 | R18 | C15 | r2-c1 | r4-c3 |
| succinyl-CoA-ligase beta subunit {Arabidopsis thaliana}GP\|6598664\|gb\|AAD25643.2\|AC007109_1\|AC | TC76558 | AI777206 | cLER20L19 | XB5 | T3C10 | R15 | C3 | r2-c1 | r4-c3 |

86

007109

| | | | | | | | | | | |
|---|---|---|---|---|---|---|---|---|---|---|
| caffeoyl-CoA O-methyltransferase 5 {Nicotiana tabacum}GP|1679853|emb|CAB05369.1||Z82982 caffeoyl-Co | TC76559 | BF176405 | cLEZ20I22 | XIVF12 | T4K24 | R1 | C11 | r2-c2 | r1-c4 |
| fructokinase, putative {Arabidopsis thaliana}GP|12324405|gb|AAG52172.1|AC020665_17| AC020665 fructok | TC76564 | AW650710 | cLEI13J20 | VIIC1 | T2F1 | R24 | C6 | r4-c2 | r1-c3 |
| transcription factor TEIL {Nicotiana tabacum} | TC76569 | AI781500 | cLES15H21 | XE4 | T3I8 | R17 | C9 | r2-c1 | r4-c3 |
| putative phospholipid cytidylyltransferase {Arabidopsis thaliana}PIR|H84807|H84807 probable phospho | TC76594 | AW030340 | cLEC16F23 | IVE12 | T1J24 | R1 | C10 | r1-c2 | r3-c3 |
| S-adenosylmethionine:2-demethylmenaquinone methyltransferase-like protein {Arabidopsis thaliana} | TC76601 | AI897772 | cLED30J21 | IIIF5 | T1L9 | R16 | C12 | r1-c2 | r3-c3 |
| fructose-1,6-bisphosphatase precursor {Solanum tuberosum} | TC76605 | BG124012 | cTOF3J20 | XXD11 | T5H22 | R3 | C8 | r3-c2 | r4-c4 |
| Similar to gb|D86180 phosphoribosylanthranilate transferase from Pisum sativum and contains 2 PF|001 | TC76611 | AW039366 | cLET9I8 | XXIA8 | T6A15 | R12 | C1 | r3-c1 | r2-c4 |
| VACUOLAR ATP SYNTHASE 16 KDA PROTEOLIPID SUBUNIT (EC 3.6.1.34) (V- ATPASE 16 KDA PROTEOLIPID SUBUNIT | TC76632 | AI778869 | cLES6M24 | IXC9 | T3E17 | R8 | C5 | r2-c1 | r4-c3 |
| glutamate 1-semialdehyde 2,1-aminomutase | TC76649 | BG128388 | cTOF20B1 | XIXD10 | T5H19 | R6 | C8 | r3-c2 | r4-c4 |
| FRUCTOSE-1,6-BISPHOSPHATASE, CHLOROPLAST PRECURSOR (EC 3.1.3.11) (D- FRUCTOSE-1,6-BISPHOSPHATE 1-PHOS | TC76659 | BG642995 | cTOF26E6 | XVIIE1 | T5I1 | R24 | C9 | r3-c2 | r4-c4 |
| Similar to transaldolase {Arabidopsis thaliana}PIR|D86257|D86257 hypothetical protein [imported] - | TC76672 | BG127427 | cTOF16B1 | VA12 | T2A23 | R2 | C1 | r4-c2 | r1-c3 |
| CYP94A1 {Vicia sativa}PIR|T08014|T08014 cytochrome P450 CYP94A1 - spring vetch | TC76678 | BE458460 | cLEM2K13 | VIIID3 | T2H6 | R19 | C8 | r4-c2 | r1-c3 |
| 2-isopropylmalate synthase {Lycopersicon pennellii}SP|O04973|LU1A_LYCPN 2- ISOPROPYLMALATE SYNTHASE | TC76694 | AW441463 | cLEN17E12 | IXA11 | T3A21 | R4 | C1 | r2-c1 | r4-c3 |
| putative cinnamoyl-CoA reductase {Arabidopsis thaliana}PIR|D84747|D84747 probable cinnamoyl-CoA red | TC76707 | AW093204 | cLET24C11 | XIF9 | T3L17 | R8 | C12 | r2-c1 | r4-c3 |
| putative nucleotide-sugar dehydratase {Arabidopsis thaliana}PIR|T00419|T00419 dTDP-glucose 4-6-dehy | TC76708 | AI483494 | cLED24I2 | IIIC11 | T1F21 | R4 | C6 | r1-c2 | r3-c3 |
| zinc finger protein {Arabidopsis thaliana} | TC76725 | AI782757 | cLES20O10 | XXIH3 | T6O5 | R20 | C15 | r3-c1 | r2-c4 |

| | | | | | | | | | |
|---|---|---|---|---|---|---|---|---|---|
| transcription factor Hap5a-like protein {Arabidopsis thaliana} | TC76772 | AI778935 | cLES6J13 | IB7 | T1C13 | R12 | C3 | r1-c2 | r3-c3 |
| cytochrome c oxidase subunit 6b-1 {Oryza sativa}GP\|9967277\|dbj\|BAB12338.1\|\|AB047975 cytochrome c ox | TC76784 | AI776716 | cLER19H17 | XXIG7 | T6M13 | R12 | C13 | r3-c1 | r2-c4 |
| beta-fructofuranosidase (invertase)^^beta-fructosidase^^beta fructosidase | TC76785 | AI489825 | cLED15G4 | IIIA4 | T1B7 | R18 | C2 | r1-c2 | r3-c3 |
| ATP synthase delta subunit, chloroplast precursor {Nicotiana tabacum}SP\|P32980\|ATPD_TOBAC ATP SYNTH | TC76808 | AI772236 | cLER2G15 | XB7 | T3C14 | R11 | C3 | r2-c1 | r4-c3 |
| aminotransferase-like protein {Arabidopsis thaliana} | TC76819 | BG130515 | cTOF30H11 | XXB10 | T5D20 | R5 | C4 | r3-c2 | r4-c4 |
| contains similarity to C2H2-type zinc finger protein~gene_id:MOK16.6 {Arabidopsis thaliana} | TC76821 | AI778334 | cLES5A19 | IVF2 | T1L4 | R21 | C12 | r1-c2 | r3-c3 |
| NADH-cytochrome b5 reductase {Arabidopsis thaliana}GP\|4240118\|dbj\|BAA74838.1\|\|AB007800 NADH-cytochr | TC76827 | BF113582 | cLEY21D5 | XIVB7 | T4C14 | R11 | C3 | r2-c2 | r1-c4 |
| lipoxygenase | TC76842 | BF050599 | cLEM18P7 | VIIH3 | T2P5 | R20 | C16 | r4-c2 | r1-c3 |
| Similar to dTDP-D-glucose 4,6-dehydratase {Arabidopsis thaliana}PIR\|C96814\|C96814 hypothetical prot | TC76851 | BF051373 | cLEM22N13 | IVF12 | T1L24 | R1 | C12 | r1-c2 | r3-c3 |
| UDP-glucose:salicylic acid glucosyltransferase {Nicotiana tabacum} | TC76866 | AI490565 | cLED25E6 | IIID5 | T1H9 | R16 | C8 | r1-c2 | r3-c3 |
| putative cytochrome P450 {Arabidopsis thaliana}GP\|13877669\|gb\|AAK43912.1\|AF370593_1\|AF370593 putati | TC76887 | AW223851 | cLEN13J16 | VIIIH5 | T2P10 | R15 | C16 | r4-c2 | r1-c3 |
| contains similarity to shikimate kinase precursor~gene_id:MDJ14.24 {Arabidopsis thaliana} | TC76889 | AI781256 | cLES14N4 | XD12 | T3G24 | R1 | C7 | r2-c1 | r4-c3 |
| contains similarity to RING zinc finger protein~gene_id:MBD2.14 {Arabidopsis thaliana} | TC76902 | BG129834 | cTOF28B1 | XXA7 | T5B14 | R11 | C2 | r3-c2 | r4-c4 |
| alpha-glucan phosphorylase, h isozyme phosphorylase h) {Solanum tuberosum}SP\|P32811\|PHSH_SOLTU ALPH | TC76936 | AW093686 | cLET25F14 | XIG3 | T3N5 | R20 | C14 | r2-c1 | r4-c3 |
| CTP:phosphocholine cytidylyltransferase {Brassica napus}GP\|1416514\|dbj\|BAA09644.1\|\|D63168 CTP:phosp | TC76939 | BE458926 | cLEM5C9 | XVIA2 | T4B4 | R21 | C2 | r2-c2 | r1-c4 |
| citrate synthase {Nicotiana | TC76947 | AI781385 | cLES15O17 | XE5 | T3I10 | R15 | C9 | r2-c1 | r4-c3 |

88

| | | | | | | | | | |
|---|---|---|---|---|---|---|---|---|---|
| tabacum}EGAD\|126596\|143593 citrate synthase {Nicotiana tabacum}GP\|2300 | | | | | | | | | |
| nucleoside diphosphate kinase II precursor {Spinacia oleracea}SP\|Q01402\|NDK2_SPIOL NUCLEOSIDE DIPHO | TC76957 | BG123962 | cTOF3P9 | XXIIA1 | T6A2 | R23 | C1 | r3-c1 | r2-c4 |
| delta 1-pyrroline-5-carboxylate synthetase | TC76960 | BF112629 | cLEG41P2 | XXIG8 | T6M15 | R12 | C13 | r3-c1 | r2-c4 |
| ferritin subunit cowpea2 precursor {Vigna unguiculata}PIR\|T08124\|T08124 ferritin 2 precursor - cowp | TC76962 | BE460085 | cLEM8N10 | VIIIF8 | T2L16 | R9 | C12 | r4-c2 | r1-c3 |
| tomato invertase inhibitor | TC76975 | AW039904 | cLET13B14 | VA4 | T2A7 | R18 | C1 | r4-c2 | r1-c3 |
| chalcone--flavanone isomerase a {Petunia hybrida}SP\|P11650\|CFIA_PETHY CHALCONE-- FLAVONONE ISOMERASE | TC76987 | AI485187 | cLED5C15 | IVA10 | T1B20 | R5 | C2 | r1-c2 | r3-c3 |
| 3-ketoacyl-CoA thiolase {Arabidopsis thaliana}GP\|2981618\|dbj\|BAA25249.1\|\|AB008855 3- ketoacyl-CoA th | TC76988 | AI781985 | cLES17D18 | XXID11 | T6G21 | R4 | C7 | r3-c1 | r2-c4 |
| chalcone synthase | TC77000 | BG128271 | cTOF19H11 | XIXC6 | T5F11 | R14 | C6 | r3-c2 | r4-c4 |
| contains similarity to diaminopimelate decarboxylase~gene_id:MLN21.17 {Arabidopsis thaliana} | TC77005 | AW224158 | cLEN16M11 | IXA9 | T3A17 | R8 | C1 | r2-c1 | r4-c3 |
| glyceraldehyde 3-phosphate dehydrogenase | TC77013 | AI779651 | cLES8P13 | XXIC12 | T6E23 | R2 | C5 | r3-c1 | r2-c4 |
| aspartate aminotransferase {Medicago sativa}□GP\|777387\|gb\|AAB46611.1\|\|L25335 aspartate aminotransfer | TC77015 | BE463395 | cTOC12P16 | XVIIC10 | T5E19 | R6 | C5 | r3-c2 | r4-c4 |
| Contains similarity to a putative 6- phosphogluconolactonase T1G12.6 GP\|6553917 from Arabidopsis thal | TC77016 | AW399260 | cLPT6D18 | XVIB8 | T4D16 | R9 | C4 | r2-c2 | r1-c4 |
| WRKY transcription factor Nt-SubD48 {Nicotiana tabacum} | TC77024 | BG626344 | cLEL11P19 | XXIB3 | T6C5 | R20 | C3 | r3-c1 | r2-c4 |
| glutamate decarboxylase isozyme 4 {Nicotiana tabacum} | TC77052 | AW030971 | cLEC5E1 | IID12 | T1G24 | R1 | C7 | r1-c2 | r3-c3 |
| D-3-PHOSPHOGLYCERATE DEHYDROGENASE PRECURSOR (EC 1.1.1.95) (PGDH).GP\|2189964\|dbj\|BAA20405.1\|\|AB0032 | TC77066 | AW625643 | cLEZ16I8 | XXH2 | T5P4 | R21 | C16 | r3-c2 | r4-c4 |
| putative glucosyl transferase {Arabidopsis thaliana}PIR\|H84784\|H84784 probable glucosyl | TC77071 | BE432435 | cLEG8C10 | VIE4 | T2I8 | R17 | C9 | r4-c2 | r1-c3 |

89

| | | | | | | | | | |
|---|---|---|---|---|---|---|---|---|---|
| transferase | | | | | | | | | |
| 11S globulin precursor {Sesamum indicum} | TC77083 | AI489377 | cLED17A13 | IVD6 | T1H12 | R13 | C8 | r1-c2 | r3-c3 |
| Putative ABC transporter {Arabidopsis thaliana}PIR\|H96622\|H96622 probable ABC transporter F23H11.19 | TC77089 | AW222251 | cLEN7O6 | IVH10 | T1P20 | R5 | C16 | r1-c2 | r3-c3 |
| putative fatty acid desaturase {Arabidopsis thaliana}GP\|4325341\|gb\|AAD17340.1\|\|AF128393 similar to | TC77096 | AI772259 | cLER2K17 | XB8 | T3C16 | R9 | C3 | r2-c1 | r4-c3 |
| S-adenosyl-L-methionine:salicylic acid carboxyl methyltransferase {Stephanotis floribunda} | TC77118 | AI487371 | cLED13G8 | IIH5 | T1O10 | R15 | C15 | r1-c2 | r3-c3 |
| putative hydroxymethylglutaryl-CoA lyase protein {Arabidopsis thaliana}GP\|13194812\|gb\|AAK15568.1\|AF | TC77127 | AW222487 | cLEN8G12 | IXD8 | T3G15 | R12 | C7 | r2-c1 | r4-c3 |
| PYRROLINE-5-CARBOXYLATE REDUCTASE (EC 1.5.1.2) (P5CR) (P5C REDUCTASE).GP\|1928962\|gb\|AAC14482.1\|\|U92 | TC77132 | AI485470 | cLED4L3 | IVA7 | T1B14 | R11 | C2 | r1-c2 | r3-c3 |
| putative transcription factor {Oryza sativa}GP\|12328532\|dbj\|BAB21190.1\|\|AP002909 putative transcrip | TC77133 | BG124550 | cTOF5G18 | XXIA12 | T6A23 | R2 | C1 | r3-c1 | r2-c4 |
| S-adenosylmethionine:2-demethylmenaquinone methyltransferase-like {Arabidopsis thaliana} | TC77161 | BG129691 | cTOF27J12 | XXA3 | T5B6 | R19 | C2 | r3-c2 | r4-c4 |
| UDP rhamnose: anthocyanidin-3-glucoside rhamnosyltransferase {Petunia x hybrida}PIR\|S36655\|S36655 U | TC77166 | BF051960 | cLEM24D18 | VIIIC2 | T2F4 | R21 | C6 | r4-c2 | r1-c3 |
| phosphoenolpyruvate carboxylase kinase | TC77168 | BG129025 | cTOF23I5 | XIXF11 | T5L21 | R4 | C12 | r3-c2 | r4-c4 |
| putative aspartate aminotransferase {Arabidopsis thaliana}PIR\|E84610\|E84610 probable aspartate amin | TC77175 | AW738611 | cTOD7N10 | XVIIIA10 | T5A20 | R5 | C1 | r3-c2 | r4-c4 |
| ADP-glucose pyrophosphorylase large subunit | TC77179 | AI782268 | cLES18L13 | XF10 | T3K20 | R5 | C11 | r2-c1 | r4-c3 |
| threonine synthase {Solanum tuberosum} | TC77196 | BG127194 | cTOF14P1 | XIXA7 | T5B13 | R12 | C2 | r3-c2 | r4-c4 |
| tryptophan synthase beta chain {Arabidopsis thaliana} | TC77225 | BG135503 | cTOE22D24 | XVIIIE4 | T5I8 | R17 | C9 | r3-c2 | r4-c4 |
| putative C3HC4-type RING zinc finger/ankyrin protein {Arabidopsis thaliana}PIR\|E84689\|E84689 probab | TC77229 | BG126573 | cTOF12P19 | VA10 | T2A19 | R6 | C1 | r4-c2 | r1-c3 |
| UMP/CMP kinase like protein {Arabidopsis thaliana}GP\|7269379\|emb\|CAB81339.1\|\|AL161563 UMP/CMP kinas | TC77240 | AI486780 | cLED11D6 | IIG9 | T1M18 | R7 | C13 | r1-c2 | r3-c3 |

| | | | | | | | | | |
|---|---|---|---|---|---|---|---|---|---|
| PROBABLE UDP-GLUCOSE 4-EPIMERASE AT4G23920 (EC 5.1.3.2) (GALACTOWALDENASE) (UDP-GALACTOSE 4-EPIMERAS | TC77242 | AW649490 | cLEI8K18 | VIIG3 | T2N5 | R20 | C14 | r4-c2 | r1-c3 |
| putative zinc finger protein {Oryza sativa} | TC77251 | AW929282 | cTOC7K15 | VD1 | T2G1 | R24 | C7 | r4-c2 | r1-c3 |
| fructose-1,6-biphosphatase | TC77264 | BG128077 | cTOF18N18 | IVH11 | T1P22 | R3 | C16 | r1-c2 | r3-c3 |
| shikimate kinase precursor | TC77265 | AW738270 | cTOD6F10 | XVIIIA2 | T5A4 | R21 | C1 | r3-c2 | r4-c4 |
| shikimate kinase precursor {Lycopersicon esculentum}SP|Q00497|AROK_LYCES SHIKIMATE KINASE PRECURSOR | TC77266 | BF113149 | cLEG43N23 | VIG5 | T2M10 | R15 | C13 | r4-c2 | r1-c3 |
| putative sugar transporter | TC77279 | AW928958 | cTOC4G10 | XVIID9 | T5G17 | R8 | C7 | r3-c2 | r4-c4 |
| S-adenosyl-L-methionine:salicylic acid carboxyl methyltransferase {Atropa belladonna} | TC77297 | AW928688 | cTOC2F6 | XVIID5 | T5G9 | R16 | C7 | r3-c2 | r4-c4 |
| G-box binding protein | TC77305 | AW737133 | cTOD2M3 | XVIIG5 | T5M9 | R16 | C13 | r3-c2 | r4-c4 |
| unnamed protein product {unidentified}GP|6683619|dbj|BAA89269.1||AB025250 ATP phosphoribosyl transf | TC77310 | BG643583 | cTOF31O17 | XXC8 | T5F16 | R9 | C6 | r3-c2 | r4-c4 |
| putative NADH dehydrogenase (ubiquinone oxidoreductase) {Arabidopsis thaliana}PIR|T02486|T02486 hyp | TC77318 | AI486928 | cLED6L17 | IVB11 | T1D22 | R3 | C4 | r1-c2 | r3-c3 |
| contains similarity to transcription regulator~gene_id:MRG7.19 {Arabidopsis thaliana} | TC77334 | BG643422 | cTOF27H23 | XXA2 | T5B4 | R21 | C2 | r3-c2 | r4-c4 |
| G-box binding protein | TC77337 | cLEN12J21 | cLEN12J21 | IVE2 | T1J4 | R21 | C10 | r1-c2 | r3-c3 |
| NADH dehydrogenase {Solanum tuberosum}SP|P80269|NUIM_SOLTU NADH-UBIQUINONE OXIDOREDUCTASE 23 KDA SU | TC77343 | AI490708 | cLEI15H12 | IA11 | T1A21 | R4 | C1 | r1-c2 | r3-c3 |
| phosphatidylserine decarboxylase {Arabidopsis thaliana} | TC77346 | AW616501 | cLHT11D21 | XIVH3 | T4O6 | R19 | C15 | r2-c2 | r1-c4 |
| bHLH transcription factor JAF13 {Petunia x hybrida} | TC77374 | AI490451 | cLED21H12 | XXIG1 | T6M1 | R24 | C13 | r3-c1 | r2-c4 |
| nitrite reductase {Capsicum annuum} | TC77375 | BE449918 | cLEY11I6 | XIIIF12 | T4K23 | R2 | C11 | r2-c2 | r1-c4 |
| putative aminotransferase; 101422-99564 {Arabidopsis thaliana}PIR|D96806|D96806 probable aminotrans | TC77383 | AW040370 | cLET3A15 | XIH7 | T3P13 | R12 | C16 | r2-c1 | r4-c3 |
| VACUOLAR ATP SYNTHASE 16 KDA PROTEOLIPID SUBUNIT (EC 3.6.1.34) (V- ATPASE 16 KDA PROTEOLIPID SUBUNIT | TC77384 | BG128906 | cTOF22P23 | XIXF5 | T5L9 | R16 | C12 | r3-c2 | r4-c4 |
| cytochrome c oxidase subunit Vb precursor-like protein | TC77386 | BG125994 | cTOF10J17 | XVIIIG7 | T5M14 | R11 | C13 | r3-c2 | r4-c4 |

| | | | | | | | | | |
|---|---|---|---|---|---|---|---|---|---|
| {Arabidopsis thaliana} | | | | | | | | | |
| PROBABLE RIBOSE 5-PHOSPHATE ISOMERASE (EC 5.3.1.6) (PHOSPHORIBOISOMERASE).GP\|4262236\|gb\|AAD1 4529.1\| | TC77390 | AW623788 | cTOB13K7 | XVIH8 | T4P16 | R9 | C16 | r2-c2 | r1-c4 |
| phosphoenolpyruvate carboxylase 1 | TC77403 | AI781699 | cLES16H4 | XE10 | T3I20 | R5 | C9 | r2-c1 | r4-c3 |
| nitrite reductase {Capsicum annuum} | TC77408 | AW219345 | cLEX4I13 | XIIIE7 | T4I13 | R12 | C9 | r2-c2 | r1-c4 |
| hypothetical protein {Arabidopsis thaliana}GP\|3281856\|emb\|CAA19751.1\|\|AL031004 Transcription factor | TC77438 | AW649316 | cLEI7H24 | XIVB1 | T4C2 | R23 | C3 | r2-c2 | r1-c4 |
| putative glucose-6-phosphate/phosphate-tranlocat or {Oryza sativa} | TC77448 | AW034294 | cLEC37H6 | IVE6 | T1J12 | R13 | C10 | r1-c2 | r3-c3 |
| uroporphyrinogen decarboxylase precursor {Nicotiana tabacum}SP\|Q42967\|DCUP_TOBAC UROPORPHYRINOGEN D | TC77455 | AW929502 | cTOC9I7 | XVIIF8 | T5K15 | R12 | C11 | r3-c2 | r4-c4 |
| putative strictosidine synthase; 35901-37889 {Arabidopsis thaliana}PIR\|A96768\|A96768 protein strict | TC77457 | BG126446 | cTOF12I4 | XVIIIH6 | T5O12 | R13 | C15 | r3-c2 | r4-c4 |
| putative aminotransferase {Arabidopsis thaliana} | TC77480 | AI897400 | cLED27I4 | IIIH8 | T1P15 | R12 | C16 | r1-c2 | r3-c3 |
| TRANSCRIPTION INITIATION FACTOR TFIID 100 KDA SUBUNIT (TAFII-100) (TAFII100).GP\|1932938\|gb\|AAC51215 | TC77486 | BE435714 | cLEG28M1 2 | VH8 | T2O15 | R12 | C15 | r4-c2 | r1-c3 |
| putative glucosyl transferase {Arabidopsis thaliana}PIR\|C84784\|C84784 probable glucosyl transferase | TC77491 | AI487582 | cLED9F15 | IVC10 | T1F20 | R5 | C6 | r1-c2 | r3-c3 |
| glucose-6-phosphate 1-dehydrogenase {Solanum tuberosum}SP\|P37830\|G6PD_SOLTU GLUCOSE-6-PHOSPHATE 1-D | TC77502 | BE431600 | cLEG27K2 1 | XIIIE12 | T4I23 | R2 | C9 | r2-c2 | r1-c4 |
| omega-6 fatty acid desaturase {Sesamum indicum} | TC77526 | AI895164 | cLEC6P16 | IH12 | T1O23 | R2 | C15 | r1-c2 | r3-c3 |
| putative C3HC4-type RING zinc finger protein {Arabidopsis thaliana}PIR\|T02413\|T02413 probable RING | TC77528 | AW223270 | cLEN11E11 | VIIIG4 | T2N8 | R17 | C14 | r4-c2 | r1-c3 |
| Alfin-1 {Medicago sativa}PIR\|T09646\|T09646 probable zinc finger protein - alfalfa (fragment) | TC77531 | AI895907 | cLEC10O8 | XXID4 | T6G7 | R18 | C7 | r3-c1 | r2-c4 |
| putative ABC transporter; 66585-65723 {Arabidopsis thaliana}PIR\|C96702\|C96702 probable ABC transpor | TC77538 | AW621599 | cLEX12D2 0 | XIIIB10 | T4C19 | R6 | C3 | r2-c2 | r1-c4 |
| phosphoribosyl pyrophosphate synthase isozyme 3 | TC77539 | AI776625 | cLER19A6 | IXH10 | T3O19 | R6 | C15 | r2-c1 | r4-c3 |

| {Spinacia oleracea} | | | | | | | | | |
|---|---|---|---|---|---|---|---|---|---|
| TRYPTOPHAN SYNTHASE BETA CHAIN 2 PRECURSOR (EC 4.2.1.20).GP\|2792520\|gb\|AAB97087.1\|\|AF042320 tryptop | TC77555 | AI488733 | cLED13J19 | IIH7 | T1O14 | R11 | C15 | r1-c2 | r3-c3 |
| indole-3-glycerol phosphate synthase {Arabidopsis thaliana} | TC77561 | AW033644 | cLEC29B10 | IG9 | T1M17 | R8 | C13 | r1-c2 | r3-c3 |
| cytochrome P450 {Solanum tuberosum} | TC77562 | AW648014 | cLEI3K17 | VIID11 | T2H21 | R4 | C8 | r4-c2 | r1-c3 |
| acetyl-CoA carboxylase {Medicago sativa}GP\|495725\|gb\|AAB42144.1\|\|L25042 acetyl-CoA carboxylase {Med | TC77572 | AW040576 | cLET7G16 | XIIC2 | T3F4 | R21 | C6 | r2-c1 | r4-c3 |
| 3-isopropylmalate dehydratase, small subunit {Arabidopsis thaliana}PIR\|H84861\|H84861 3-isopropylmal | TC77576 | BG131961 | cTOE5J4 | XXIIA8 | T6A16 | R9 | C1 | r3-c1 | r2-c4 |
| MADS-box transcription factor FBP22 {Petunia x hybrida} | TC77597 | AI486684 | cLED11H1 8 | IIG11 | T1M22 | R3 | C13 | r1-c2 | r3-c3 |
| probable UDP-glucuronosyltransferase (EC 2.4.1.-) - garden pea | TC77599 | AI898207 | cLED32E12 | IIIF12 | T1L23 | R2 | C12 | r1-c2 | r3-c3 |
| NADH dehydrogenase {Solanum tuberosum}GP\|639834\|emb\|CAA58823.1\|\|X83999 NADH dehydrogenase {Solanum | TC77602 | BG124296 | cTOF4H14 | XXE5 | T5J10 | R15 | C10 | r3-c2 | r4-c4 |
| phosphoglucomutase-like protein {Arabidopsis thaliana}PIR\|T51457\|T51457 phosphoglucomutase-like pro | TC77603 | AW038594 | cLET7O17 | XIIC5 | T3F10 | R15 | C6 | r2-c1 | r4-c3 |
| biotin-binding protein^^biotin-containing subunit of methylcrotonyl-CoA carboxylase | TC77606 | BG128249 | cTOF19D1 | XIXC4 | T5F7 | R18 | C6 | r3-c2 | r4-c4 |
| ATP:citrate lyase {Capsicum annuum} | TC77626 | BG127685 | cTOF17F3 | XIXB2 | T5D3 | R22 | C4 | r3-c2 | r4-c4 |
| putative cytochrome P450 {Solanum chacoense}SP\|P93530\|C7D6_SOLCH CYTOCHROME P450 71D6 (EC 1.14.-.-) | TC77648 | AI485818 | cLED4O17 | IVA8 | T1B16 | R9 | C2 | r1-c2 | r3-c3 |
| cytochrome c oxidase subunit 6b {Oryza sativa}GP\|9967162\|dbj\|BAB12275.1\|\|AB047923 cytochrome c oxid | TC77660 | AI775016 | cLER14G1 | IXF4 | T3K7 | R18 | C11 | r2-c1 | r4-c3 |
| glycerol-3-phosphate acyltransferase {Cucumis sativus}SP\|Q39639\|PLSB_CUCSA GLYCEROL-3-PHOSPHATE ACY | TC77666 | BE449429 | cLHT31D3 | XVC1 | T4F1 | R24 | C6 | r2-c2 | r1-c4 |
| S-adenosyl-L-methionine:salicylic acid carboxyl | TC77684 | BE354257 | cTOD9B6 | XVIIIB4 | T5C8 | R17 | C3 | r3-c2 | r4-c4 |

| | | | | | | | | | |
|---|---|---|---|---|---|---|---|---|---|
| methyltransferase-like protein {Arabidopsis thaliana | | | | | | | | | |
| 3-deoxy-D-arabino-heptulosonate 7-phosphate synthase {Morinda citrifolia} | TC77693 | AW091589 | cLET15J4 | XVIE5 | T4J10 | R15 | C10 | r2-c2 | r1-c4 |
| serine hydroxymethyltransferase, mitochondrial precursor (serine methylase) (glycine hydroxymethyltr | TC77724 | BG126939 | cTOF13L12 | XVIIIH10 | T5O20 | R5 | C15 | r3-c2 | r4-c4 |
| putative sugar transporter {Arabidopsis thaliana} | TC77728 | AI490207 | cLER1M15 | XA9 | T3A18 | R7 | C1 | r2-c1 | r4-c3 |
| alpha-glucan phosphorylase, l isozyme 2 precursor (starch phosphorylase l-2) {Solanum tuberosum}SP| | TC77734 | BG644107 | cTOF34I23 | XXD6 | T5H12 | R13 | C8 | r3-c2 | r4-c4 |
| HB2 homeodomain protein {Populus tremula x Populus tremuloides} | TC77747 | AW945094 | cTOB16M6 | XVIIA7 | T5A13 | R12 | C1 | r3-c2 | r4-c4 |
| Strong similarity to gb|U61231 cytochrome P450 from Arabidopsis thaliana and is a member of the PF|0 | TC77757 | BG125277 | cTOF8K13 | XXG6 | T5N12 | R13 | C14 | r3-c2 | r4-c4 |
| succinyl-CoA synthetase, alpha subunit {Arabidopsis thaliana} | TC77763 | BE451337 | cLEY17J6 | XIVA4 | T4A8 | R17 | C1 | r2-c2 | r1-c4 |
| NADH dehydrogenase {Solanum tuberosum}GP|639834|emb|CAA58823.1||X83999 NADH dehydrogenase {Solanum | TC77792 | AW623479 | cTOB10F18 | XVIF12 | T4L24 | R1 | C12 | r2-c2 | r1-c4 |
| glucose-6-phosphate/phosphate-translocator precursor {Pisum sativum}PIR|T06254|T06254 glucose-6-pho | TC77793 | AW399232 | cLPT6N15 | XVIB12 | T4D24 | R1 | C4 | r2-c2 | r1-c4 |
| pyrophosphate--fructose 6-phosphate 1-phosphotransferase alpha subunit (pfp) (6-phosphofructokinase | TC77808 | BG643516 | cTOF31A1 | XXC1 | T5F2 | R23 | C6 | r3-c2 | r4-c4 |
| adenosine kinase {Arabidopsis thaliana}GP|7378610|emb|CAB83286.1||AL162751 adenosine kinase-like pr | TC77810 | BE450032 | cLEY11D12 | XIIIF9 | T4K17 | R8 | C11 | r2-c2 | r1-c4 |
| sucrose transporter {Lycopersicon esculentum} | TC77814 | AW650849 | cLEI14L5 | VB3 | T2C5 | R20 | C3 | r4-c2 | r1-c3 |
| transketolase 1 {Capsicum annuum}PIR|T09541|T09541 transketolase (EC 2.2.1.1) TKT1 precursor, chlor | TC77838 | AI780217 | cLES11I1 | XIVA3 | T4A6 | R19 | C1 | r2-c2 | r1-c4 |
| transcription factor inhibitor I kappa B homolog {Arabidopsis thaliana}GP|1773295|gb|AAC49611.1||U7 | TC77842 | AW160234 | cLPT1I9 | XIID11 | T3H22 | R3 | C8 | r2-c1 | r4-c3 |
| aspartate-semialdehyde dehydrogenase precursor {Arabidopsis thaliana} | TC77844 | BG134476 | cTOE16M18 | XVIIID1 | T5G2 | R23 | C7 | r3-c22 | r4-c4 |
| putative flavonol 3-O-glucosyltransferase {Arabidopsis thaliana}PIR|F84618|F84618 probable flavonol | TC77847 | BG127210 | cTOF14B18 | XIXA1 | T5B1 | R24 | C2 | r3-c2 | r4-c4 |
| ornithine carbamoyltransferase; OCTase {Canavalia lineata}GP|12003188|gb|AAG43481.1|AF203688_1|AF | TC77862 | AW222161 | cLEN7K19 | IXD7 | T3G13 | R12 | C7 | r2-c1 | r4-c3 |

| 20 | | | | | | | | | |
|---|---|---|---|---|---|---|---|---|---|
| NADH-dependent glutamate synthase {Medicago sativa} | TC77867 | AW616998 | cLHT19G12 | XVA9 | T4B17 | R8 | C2 | r2-c2 | r1-c4 |
| contains similarity to transcription regulator~gene_id:MRG7.19 {Arabidopsis thaliana} | TC77873 | BG124285 | cTOF4F12 | XXE2 | T5J4 | R21 | C10 | r3-c2 | r4-c4 |
| contains similarity to C2H2-type zinc finger protein~gene_id:MOK16.6 {Arabidopsis thaliana} | TC77881 | BG123144 | cTOF1C1 | XIXC11 | T5F21 | R4 | C6 | r3-c2 | r4-c4 |
| putative zinc finger protein {Oryza sativa} | TC77907 | AI782007 | cLES17H16 | XF3 | T3K6 | R19 | C11 | r2-c1 | r4-c3 |
| putative hydroxymethyltransferase; 49598-47322 {Arabidopsis thaliana}PIR\|F86484\|F86484 probable hyd | TC77914 | AW220191 | cLEX8O6 | XIIIF7 | T4K13 | R12 | C11 | r2-c2 | r1-c4 |
| deoxyuridine triphosphatase, dUTPase, P18 {EC 3.6.1.23} [tomatoes, Tint Tim cultivar LA154, Peptide, 169 aa] | TC77923 | AI487253 | cLED11P16 | IIH3 | T1O6 | R19 | C15 | r1-c2 | r3-c3 |
| cytochrome P450, putative {Arabidopsis thaliana}PIR\|F86441\|F86441 probable cytochrome P450 importe | TC77959 | BG123929 | cTOF3J9 | XXD12 | T5H24 | R1 | C8 | r3-c2 | r4-c4 |
| NAD-malate dehydrogenase {Nicotiana tabacum} | TC77971 | AW648430 | cLEI4F3 | VIIE7 | T2J13 | R12 | C10 | r4-c2 | r1-c3 |
| aspartate aminotransferase {Panicum miliaceum}GP\|20597\|emb\|CAA45022.1\|\|X63428 aspartate aminotransf | TC77973 | AI484604 | cLED2E7 | IIIE7 | T1J13 | R12 | C10 | r1-c2 | r3-c3 |
| phosphoenolpyruvate carboxylase {Nicotiana tabacum}SP\|P27154\|CAPP_TOBAC PHOSPHOENOLPYRUVATE CARBOXY | TC77975 | AI897765 | cLED30H23 | IIIF3 | T1L5 | R20 | C12 | r1-c2 | r3-c3 |
| Contains similarity to DNA-binding protein MYB1 from Petroselinum crispum GP\|7488946 and contains MY | TC77983 | BG127613 | cTOF17E20 | XIXB1 | T5D1 | R24 | C4 | r3-c2 | r4-c4 |
| phosphatidylinositol 4-kinase {Solanum tuberosum} | TC78010 | AI782370 | cLES18N16 | XF11 | T3K22 | R3 | C11 | r2-c1 | r4-c3 |
| L-allo-threonine aldolase homolog F22O13.11 - Arabidopsis thaliana | TC78023 | AW737305 | cTOD3I3 | XVIIG10 | T5M19 | R6 | C13 | r3-c2 | r4-c4 |
| argininosuccinate synthase-like protein {Arabidopsis thaliana}GP\|7269334\|emb\|CAB79393.1\|\|AL161562 a | TC78033 | BE458353 | cLEM1H12 | VIIH9 | T2P17 | R8 | C16 | r4-c2 | r1-c3 |
| hyoscyamine 6-dioxygenase hydroxylase, putative {Arabidopsis thaliana}PIR\|G86472\|G86472 probable hy | TC78043 | BE436223 | cLEG31I23 | VIA8 | T2A16 | R9 | C1 | r4-c2 | r1-c3 |
| SNF2 subfamily global transcription activator {Arabidopsis thaliana}PIR\|G84897\|G84897 hypothetical | TC78051 | BF097336 | cLEW20A10 | XIIF12 | T3L24 | R1 | C12 | r2-c1 | r4-c3 |

| | | | | | | | | | |
|---|---|---|---|---|---|---|---|---|---|
| CYTOCHROME P450 98A2 (EC 1.14.-.-).GP\|2738998\|gb\|AAB94587.1\|\|AF022458 CYP98A2p {Glycine max}PIR\|T0 | TC78082 | AW738012 | cTOD4P2 | XVIIH7 | T5O13 | R12 | C15 | r3-c2 | r4-c4 |
| branched-chain amino acid aminotransferase {Solanum tuberosum} | TC78133 | AW034300 | cLEC37H2 | IVD9 | T1H18 | R7 | C8 | r1-c2 | r3-c3 |
| geranylgeranyl pyrophosphate synthase-related protein {Arabidopsis thaliana}GP\|7270829\|emb\|CAB80510 | TC78147 | AI779636 | cLES8N7 | XIB5 | T3D9 | R16 | C4 | r2-c1 | r4-c3 |
| putative phosphatidylserine decarboxylase {Arabidopsis thaliana}GP\|7269448\|emb\|CAB79452.1\|\|AL161564 | TC78160 | AI776470 | cLER18H17 | IXH6 | T3O11 | R14 | C15 | r2-c1 | r4-c3 |
| cytochrome P450 {Catharanthus roseus}PIR\|T09999\|T09999 cytochrome P450 - Madagascar periwinkle | TC78161 | AW160266 | cLPT1K21 | XVH9 | T4P17 | R8 | C16 | r2-c2 | r1-c4 |
| CYTOCHROME P450 71A9 (EC 1.14.-.-) (P450 CP1).GP\|3334659\|emb\|CAA71513.1\|\|Y10489 putative cytochrome | TC78170 | AI489371 | cLED16B17 | IIIA7 | T1B13 | R12 | C2 | r1-c2 | r3-c3 |
| bA554C12.1 (RBX1 or ROC1 (ring-box or ring finger protein 1)) {Homo sapiens}GP\|4769004\|gb\|AAD29715. | TC78191 | BF114334 | cLEY26D17 | XXIIA7 | T6A14 | R11 | C1 | r3-c1 | r2-c4 |
| alpha amylase precursor {Cuscuta reflexa}GP\|458456\|gb\|AAA16513.1\|\|U06754 alpha amylase precursor {C | TC78197 | BE436573 | cLEG33K23 | VIB10 | T2C20 | R5 | C3 | r4-c2 | r1-c3 |
| putative strictosidine synthase-like {Arabidopsis thaliana} | TC78210 | BG629491 | cLEL27O9 | XXIG10 | T6M19 | R6 | C13 | r3-c1 | r2-c4 |
| malate dehydrogenase, mitochondrial precursor {Citrullus vulgaris}EGAD\|148462\|158380 hypothetical p | TC78217 | BG643772 | cTOF32F13 | XXC11 | T5F22 | R3 | C6 | r3-c2 | r4-c4 |
| flavanone 3-hydroxylase {Citrus sinensis} | TC78218 | BG628989 | cLEL24M13 | XXIF8 | T6K15 | R12 | C11 | r3-c1 | r2-c4 |
| Contains similarity to gb\|Y13720 Hap2a transcription factor from Arabidopsis thaliana.PIR\|A86430\|A8 | TC78236 | BF114407 | cLEY26J2 | XIVC5 | T4E10 | R15 | C5 | r2-c2 | r1-c4 |
| GALACTOKINASE (EC 2.7.1.6) (GALACTOSE KINASE).GP\|12322687\|gb\|AAG51339.1\|AC020580_19 AC020580 galact | TC78245 | AW648708 | cLEI5C16 | VIIE11 | T2J21 | R4 | C10 | r4-c2 | r1-c3 |
| DIACYLGLYCEROL KINASE 1 (EC 2.7.1.107) (DIGLYCERIDE KINASE) (DGK 1) (DAG KINASE 1).GP\|1374772\|dbj\|B | TC78269 | AI489194 | cLED17O10 | IIIB4 | T1D7 | R18 | C4 | r1-c2 | r3-c3 |
| myb-related transcription factor LBM1 {Nicotiana tabacum} | TC78270 | AW032347 | cLEC35G10 | IIB4 | T1C8 | R17 | C3 | r1-c2 | r3-c3 |

| | | | | | | | | |
|---|---|---|---|---|---|---|---|---|---|
| transcription factor {Vicia faba}GP\|2104679\|emb\|CAA66480.1\|\|X97906 transcription factor {Vicia faba | TC78324 | BG127573 | cTOF17D4 | XIXA12 | T5B23 | R2 | C2 | r3-c2 | r4-c4 |
| alpha-glucan phosphorylase, h isozyme phosphorylase h) {Solanum tuberosum}SP\|P32811\|PHSH_SOLTU ALPH | TC78327 | AW616810 | cLHT17D11 | XVA4 | T4B7 | R18 | C2 | r2-c2 | r1-c4 |
| isocitrate dehydrogenase (NAD+) {Solanum tuberosum} | TC78346 | AW441655 | cLEN17P4 | IXB5 | T3C9 | R16 | C3 | r2-c1 | r4-c3 |
| NADH dehydrogenase (ubiquinone) (EC 1.6.5.3) chain nad9 - wheat mitochondrion | TC78368 | AI771898 | cLED38E16 | IIIH6 | T1P11 | R14 | C16 | r1-c2 | r3-c3 |
| Zn finger protein {Nicotiana tabacum}GP\|1360086\|emb\|CAA66605.1\|\|X97946 Zn finger protein {Nicotiana | TC78369 | BG130539 | cTOF30L15 | XXB12 | T5D24 | R1 | C4 | r3-c2 | r4-c4 |
| flavonoid 3',5'-hydroxylase-like; cytochrome P450 {Arabidopsis thaliana} | TC78380 | AW033148 | cLEC25H6 | IF9 | T1K17 | R8 | C11 | r1-c2 | r3-c3 |
| geranylgeranyl pyrophosphate synthetase precursor (ggpp synthetase) (dimethylallyltransferase {Capsi | TC78381 | BE461170 | cLEG37B6 | VID6 | T2G12 | R13 | C7 | r4-c2 | r1-c3 |
| MYB-like DNA-binding domain protein {Gossypium hirsutum}PIR\|T09745\|T09745 myb-related protein - upl | TC78385 | BG123392 | cTOF1F20 | XIXD2 | T5H3 | R22 | C8 | r3-c2 | r4-c4 |
| homeobox 2 protein | TC78390 | AI897000 | cLED26E19 | IIID8 | T1H15 | R12 | C8 | r1-c2 | r3-c3 |
| Contains similarity to dTPD-D-glucose-4,6-dehydratase from Sphingomonas sp.S88 gb\|U51197 and contain | TC78391 | BE353784 | cTOD6E6 | XXIF3 | T6K5 | R20 | C11 | r3-c1 | r2-c4 |
| fructokinase | TC78393 | BE433756 | cLEG20H18 | VG3 | T2M5 | R20 | C13 | r4-c2 | r1-c3 |
| fructose-6-phosphate 2-kinase/fructose-2,6-bisphosphatase {Solanum tuberosum}PIR\|T07016\|T07016 6-ph | TC78403 | AW031877 | cLEC38I8 | IIC8 | T1E16 | R9 | C5 | r1-c2 | r3-c3 |
| cytochrome P450 {Nicotiana tabacum}GP\|1171579\|emb\|CAA64635.1\|\|X95342 cytochrome P450 {Nicotiana tab | TC78431 | AW034715 | cLEC32H2 | IH10 | T1O19 | R6 | C15 | r1-c2 | r3-c3 |
| MADS-box transcription factor FBP21 {Petunia x hybrida} | TC78439 | AW219022 | cLEX2O20 | XIIID10 | T4G19 | R6 | C7 | r2-c2 | r1-c4 |
| 76 kDa mitochondrial complex I subunit {Solanum tuberosum}SP\|Q43644\|NUAM_SOLTU NADH-UBIQUINONE OXID | TC78448 | AW096331 | cLET38C16 | XXH5 | T5P10 | R15 | C16 | r3-c2 | r4-c4 |
| contains similarity to SNF2/RAD54 family (RAD26 subfamily) transcription-repair coupling factor~gene | TC78453 | AW647754 | cLEI2K21 | IVE11 | T1J22 | R3 | C10 | r1-c2 | r3-c3 |

| | | | | | | | | | |
|---|---|---|---|---|---|---|---|---|---|
| homeodomain protein {Malus x domestica} | TC78458 | AW033434 | cLEC28H13 | IG4 | T1M7 | R18 | C13 | r1-c2 | r3-c3 |
| transcription regulator Sir2-like protein {Arabidopsis thaliana}GP\|12006420\|gb\|AAG44850.1\|AF283757_ | TC78492 | BF050336 | cLEM17M10 | VIIH1 | T2P1 | R24 | C16 | r4-c2 | r1-c3 |
| pyrophosphate-dependent phosphofructokinase beta subunit {Citrus x paradisi} | TC78498 | AW980091 | cLEW10K9 | XIID3 | T3H6 | R19 | C8 | r2-c1 | r4-c3 |
| CYP83D1p {Glycine max}PIR\|T05940\|T05940 cytochrome P450 83D1p - soybean (fragment) | TC78508 | BG128761 | cTOF22C6 | XIXE8 | T5J15 | R12 | C10 | r3-c2 | r4-c4 |
| Putative UDP-glucose:sterol glucosyltransferase {Arabidopsis thaliana}PIR\|D96499\|D96499 probable UD | TC78531 | BE433289 | cLEG13A17 | VE2 | T2I3 | R22 | C9 | r4-c2 | r1-c3 |
| contains similarity to cyclopropane fatty acid synthase~gene_id:MEE5.5 {Arabidopsis thaliana} | TC78544 | AW399701 | cLPT8B18 | VB5 | T2C9 | R16 | C3 | r4-c2 | r1-c3 |
| zinc finger and C2 domain protein {Arabidopsis thaliana} | TC78550 | AW220607 | cLEF1F13 | IVE4 | T1J8 | R17 | C10 | r1-c2 | r3-c3 |
| cinnamoyl CoA reductase-like protein {Arabidopsis thaliana}PIR\|T48643\|T48643 cinnamoyl CoA reductas | TC78570 | BE433400 | cLEG13O12 | VE5 | T2I9 | R16 | C9 | r4-c2 | r1-c3 |
| tyrosine aminotransferase-like protein {Arabidopsis thaliana} | TC78598 | AW034806 | cLEC32E16 | IH9 | T1O17 | R8 | C15 | r1-c2 | r3-c3 |
| omega-3 fatty acid desaturase, endoplasmic reticulum {Nicotiana tabacum}SP\|P48626\|FD3E_TOBAC OMEGA- | TC78627 | BG626797 | cLEL14E18 | XXIB10 | T6C19 | R6 | C3 | r3-c1 | r2-c4 |
| anthranilate phosphoribosyltransferase-like protein {Arabidopsis thaliana}PIR\|T46010\|T46010 anthran | TC78632 | AW625356 | cLEZ12M21 | XIVD3 | T4G6 | R19 | C7 | r2-c2 | r1-c4 |
| putative folylpolyglutamate synthetase {Oryza sativa} | TC78676 | BF050762 | cLEM19B11 | VIIH4 | T2P7 | R18 | C16 | r4-c2 | r1-c3 |
| transcription factor IIA large subunit {Arabidopsis thaliana}PIR\|T51333\|T51333 transcription factor | TC78698 | BG140116 | cLPP16A16 | XVD12 | T4H23 | R2 | C8 | r2-c2 | r1-c4 |
| putative alpha-amylase; 60344-64829 {Arabidopsis thaliana}PIR\|E96720\|E96720 probable alpha-amylase | TC78700 | AI775160 | cLER14P7 | VIIIG9 | T2N18 | R7 | C14 | r4-c2 | r1-c3 |
| polyneuridine aldehyde esterase {Rauvolfia serpentina} | TC78712 | AW224223 | cLEN16B20 | IXA7 | T3A13 | R12 | C1 | r2-c1 | r4-c3 |
| 2S seed albumin-1 large subunit [Lycopersicon esculentum] | TC78715 | AW930900 | cLEF42G23 | IVF9 | T1L18 | R7 | C12 | r1-c2 | r3-c3 |
| Strong similarity to F19I3.8 GP\|3033381 putative UDP-galactose-4-epimerase from Arabidopsis thaliana | TC78747 | BF098180 | cLEW26C4 | XIIG11 | T3N22 | R3 | C14 | r2-c1 | r4-c3 |
| Similar to gb\|AF135422 GDP-mannose | TC78749 | AW218839 | cLEX1M19 | XIIID7 | T4G13 | R12 | C7 | r2-c2 | r1-c4 |

| | | | | | | | | | |
|---|---|---|---|---|---|---|---|---|---|
| pyrophosphorylase A (GMPPA) from Homo sapiens. ESTs gb\|AA712990, | | | | | | | | | |
| heat stress transcription factor 8 | TC78884 | AW933448 | cLEF55G15 | VB10 | T2C19 | R6 | C3 | r4-c2 | r1-c3 |
| pyruvate kinase, cytosolic isozyme {Nicotiana tabacum}SP\|Q42954\|KPYC_TOBAC PYRUVATE KINASE, CYTOSOL | TC78918 | BE436523 | cLEG33A19 | VIB4 | T2C8 | R17 | C3 | r4-c2 | r1-c3 |
| 2-oxoglutarate/malate translocator precursor-like protein {Arabidopsis thaliana}PIR\|T49900\|T49900 2 | TC78921 | BG124992 | cTOF7G4 | XXF8 | T5L16 | R9 | C12 | r3-c2 | r4-c4 |
| cytochrome P450 {Solanum tuberosum} | TC78950 | BG132478 | cTOE7P21 | XIIE8 | T3J16 | R9 | C10 | r2-c1 | r4-c3 |
| CYTOCHROME P450 97B2 (EC 1.14.-.-).GP\|2738996\|gb\|AAB94586.1\|\|AF022457 CYP97B2p {Glycine max}PIR\|T0 | TC78953 | AI482612 | cLEB1M6 | IA8 | T1A15 | R12 | C1 | r1-c2 | r3-c3 |
| alpha-glucosidase {Solanum tuberosum}PIR\|T07391\|T07391 probable alpha-glucosidase (EC 3.2.1.20) - p | TC78967 | AW441540 | cLEN17F21 | IXA12 | T3A23 | R2 | C1 | r2-c1 | r4-c3 |
| threonine synthase {Solanum tuberosum} | TC78978 | AI781552 | cLES16I21 | XE11 | T3I22 | R3 | C9 | r2-c1 | r4-c3 |
| NADH dehydrogenase subunit | TC79000 | BG642939 | cTOF26I17 | XIXH7 | T5P13 | R12 | C16 | r3-c22 | r4-c4 |
| RING-H2 finger protein RHF2a {Arabidopsis thaliana}GP\|13374859\|emb\|CAC34493.1\|\|AL589883 RING-H2 fin | TC79012 | AW031373 | cLEC40A4 | IID3 | T1G6 | R19 | C7 | r1-c2 | r3-c3 |
| putative C3HC4-type RING zinc finger/ankyrin protein {Arabidopsis thaliana}PIR\|E84689\|E84689 probab | TC79013 | BG130531 | cTOF30J21 | XXB11 | T5D22 | R3 | C4 | r3-c2 | r4-c4 |
| Dof zinc finger protein {Solanum tuberosum} | TC79103 | AW033619 | cLEC30H5 | IH5 | T1O9 | R16 | C15 | r1-c2 | r3-c3 |
| glucose acyltransferase {Lycopersicon pennellii} | TC79131 | AI778151 | cLES4I7 | XH6 | T3O12 | R13 | C15 | r2-c2 | r4-c3 |
| tyrosine decarboxylase {Arabidopsis thaliana} | TC79134 | AW029702 | cLEC28B17 | IG2 | T1M3 | R22 | C13 | r1-c2 | r3-c3 |
| aldose-1-epimerase-like protein {Nicotiana tabacum}PIR\|T01933\|T01933 probable aldose 1-epimerase (E | TC79135 | AI778167 | cLES4K17 | XH7 | T3O14 | R11 | C15 | r2-c1 | r4-c3 |
| putative ABC transporter {Arabidopsis thaliana}GP\|4115931\|gb\|AAD03441.1\|\|AF118223 contains similari | TC79147 | AW033127 | cLEC25J19 | IF11 | T1K21 | R4 | C11 | r1-c2 | r3-c3 |
| polyneuridine aldehyde esterase {Rauvolfia serpentina} | TC79199 | BF097870 | cLEW24G19 | XIIG6 | T3N12 | R13 | C14 | r2-c1 | r4-c3 |
| glycogen (starch) synthase precursor {Solanum tuberosum}SP\|Q00775\|UGST_SOLTU GRANULE-BOUND GLYCOGEN | TC79234 | BG128517 | cTOF21A12 | XIXD12 | T5H23 | R2 | C8 | r3-c2 | r4-c4 |
| alpha glucosidase-like protein {Arabidopsis thaliana} | TC79238 | BG128749 | cTOF21P16 | XIXE6 | T5J11 | R14 | C10 | r3-c2 | r4-c4 |

| | | | | | | | | | |
|---|---|---|---|---|---|---|---|---|---|
| transcription factor TEIL {Nicotiana tabacum} | TC79239 | BE461679 | cLEG39L11 | VIE8 | T2I16 | R9 | C9 | r4-c2 | r1-c3 |
| neutral invertase, putative {Arabidopsis thaliana}GP\|12324537\|gb\|AAG52223.1\|AC021665_6\|A C021665 put | TC79243 | BE436756 | cLEG34I5 | VIC3 | T2E6 | R19 | C5 | r4-c2 | r1-c3 |
| putative alpha-amylase; 60344-64829 {Arabidopsis thaliana}PIR\|E96720\|E96720 probable alpha-amylase | TC79253 | BE460819 | cLEG36H1 5 | VIC12 | T2E24 | R1 | C5 | r4-c2 | r1-c3 |
| HOMEOBOX-LEUCINE ZIPPER PROTEIN HAT7 (HD-ZIP PROTEIN 7) (HD-ZIP PROTEIN ATHB-3).GP\|549891\|gb\|AAA569 | TC79277 | AW932596 | cLEF49E14 | VA2 | T2A3 | R22 | C1 | r4-c2 | r1-c3 |
| Similar to UTP-glucose glucosyltransferases {Arabidopsis thaliana}PIR\|G86144\|G86144 hypothetical pr | TC79292 | AI894979 | cLEC6M20 | IIE11 | T1I22 | R3 | C9 | r1-c2 | r3-c3 |
| CYP83D1p {Glycine max}PIR\|T05940\|T05940 cytochrome P450 83D1p - soybean (fragment) | TC79302 | AI895470 | cLEC7L16 | IIF2 | T1K4 | R21 | C11 | r1-c2 | r3-c3 |
| mas-binding factor MBF2=transcription factor TGA1a homolog {Solanum tuberosum=potatoes, root, Peptid | TC79327 | BF114052 | cLEY24E3 | XIVC2 | T4E4 | R21 | C5 | r2-c2 | r1-c4 |
| cytochrome P450 {Arabidopsis thaliana} | TC79360 | AI489348 | cLED16E20 | IIIA9 | T1B17 | R8 | C2 | r1-c2 | r3-c3 |
| invertase inhibitor homolog {Nicotiana tabacum}PIR\|T03396\|T03396 invertase inhibitor homolog - comm | TC79368 | BG135878 | cTOE23N1 0 | XVIIIE10 | T5I20 | R5 | C9 | r3-c2 | r4-c4 |
| putative cytochrome P450 {Arabidopsis thaliana}GP\|13877661\|gb\|AAK43908.1\|AF370589_1\|A F370589 putati | TC79382 | BE432001 | cLEG4D22 | VIG11 | T2M22 | R3 | C13 | r4-c2 | r1-c3 |
| triosephosphate isomerase, cytosolic {Coptis japonica}SP\|P21820\|TPIS_COPJA TRIOSEPHOSPHATE ISOMERAS | TC79385 | BE431756 | cLEG1N4 | VG1 | T2M22 | R3 | C13 | r4-c2 | r1-c3 |
| putative ABC transporter; 60211-54925 {Arabidopsis thaliana}PIR\|E96742\|E96742 probable ABC transpor | TC79386 | AI488143 | cLED21K1 1 | IIIC4 | T2M1 | R24 | C13 | r4-c2 | r1-c3 |
| tyrosine aminotransferase-like protein {Arabidopsis thaliana} | TC79388 | AI487927 | cLEN6G15 | IXD1 | T1F7 | R18 | C6 | r1-c2 | r3-c3 |
| fructose-6-phosphate 2-kinase/fructose-2,6-bisphosphatase {Solanum tuberosum}PIR\|T07016\|T07016 6-ph | TC79403 | AW650251 | cLEI12O14 | VIIB3 | T3G1 | R24 | C7 | r2-c1 | r4-c3 |
| putative glycerol-3-phosphate dehydrogenase {Arabidopsis thaliana} | TC79406 | AW650191 | cLEI12C14 | VIIB1 | T2D1 | R24 | C4 | r4-c2 | r1-c3 |
| putative cytochrome P450 {Oryza sativa} | TC79461 | BE354362 | cTOD10I12 | XVIIF9 | T5K17 | R8 | C11 | r3-c2 | r4-c4 |
| cytochrome P450 {Arabidopsis thaliana} | TC79463 | AW650677 | cLEI13B24 | VIIB5 | T2D9 | R16 | C4 | r4-c2 | r1-c3 |

100

| Description | TC# | GenBank | Clone | ID | BAC | R | C | map1 | map2 |
|---|---|---|---|---|---|---|---|---|---|
| anthranilate synthase alpha subunit {Catharanthus roseus} | TC79471 | AW651095 | cLEI15B17 | VIIC5 | T2F9 | R16 | C6 | r4-c2 | r1-c3 |
| homeodomain protein {Malus x domestica} | TC79485 | AW623494 | cTOB10J8 | XVIF10 | T4L20 | R5 | C12 | r2-c2 | r1-c4 |
| ferredoxin–nitrite reductase (EC 1.7.7.1) nir-3 - common tobacco (fragment) | TC79488 | AW039265 | cLET8L21 | XIIC12 | T3F24 | R1 | C6 | r2-c1 | r4-c3 |
| putative sugar transporter {Arabidopsis thaliana} | TC79538 | BE458971 | cLEM5K9 | VIIIE1 | T2J2 | R23 | C10 | r4-c2 | r1-c3 |
| transcription factor-like protein {Arabidopsis thaliana} | TC79544 | AW624307 | cTOB15C3 | XVIH12 | T4P24 | R1 | C16 | r2-c2 | r1-c4 |
| Similar to UTP-glucose glucosyltransferases {Arabidopsis thaliana}PIR|G86144|G86144 hypothetical pr | TC79554 | AW945064 | cTOB16E10 | XVIIA4 | T5A7 | R18 | C1 | r3-c2 | r4-c4 |
| RING finger protein {Arabidopsis thaliana}GP|4689366|gb|AAD27870.1|AF134155_1|AF134155 RING finger | TC79564 | AW034559 | cLEC24E2 | IF5 | T1K9 | R16 | C11 | r1-c2 | r3-c3 |
| hexokinase | TC79603 | AW649867 | cLEI9L20 | VIIG6 | T2N11 | R14 | C14 | r4-c2 | r1-c3 |
| starch-branching enzyme-like protein {Arabidopsis thaliana} | TC79638 | BG130892 | cTOE1P21 | XVIIID12 | T5G24 | R1 | C7 | r3-c2 | r4-c4 |
| formyltransferase purU homolog {Arabidopsis thaliana}GP|2245095|emb|CAB10517.1||Z97343 formyltransf | TC79642 | AW944789 | cTOB12M15 | XVIG12 | T4N24 | R1 | C14 | r2-c2 | r1-c4 |
| putative heat shock transcription factor {Arabidopsis thaliana}PIR|T02609|T02609 probable heat shoc | TC79646 | AW930998 | cLEF42K16 | IVF10 | T1L20 | R5 | C12 | r1-c2 | r3-c3 |
| Identical to A. thaliana Myb-like protein (gb|D58424).{Arabidopsis thaliana}PIR|F86231|F86231 hypo | TC79650 | BG130692 | cTOE1A13 | XVIIID7 | T5G14 | R11 | C7 | r3-c2 | r4-c4 |
| nucleoside diphosphate kinase {Pisum sativum} | TC79659 | BG127408 | cTOF16M2 | XIXA10 | T5B19 | R6 | C2 | r3-c2 | r4-c4 |
| putative C3HC4-type RING zinc finger/ankyrin protein {Arabidopsis thaliana}PIR|E84689|E84689 probab | TC79668 | BG131674 | cTOE4L22 | XVIIIF9 | T5K18 | R7 | C11 | r3-c2 | r4-c4 |
| isopentenyl diphosphate isomerase 1 {Nicotiana tabacum} | TC79669 | BG131971 | cTOE5L2 | XVIIIF11 | T5K22 | R3 | C11 | r3-c2 | r4-c4 |
| vacuolar ATP synthase catalytic subunit a kDa subunit) {Daucus carota};SP|P09469|VATA_DAUCA VACUOLAR | TC79686 | BG131561 | cTOE4F15 | XVIIIF7 | T5K14 | R11 | C11 | r3-c2 | r4-c4 |
| zinc-finger-like protein {Arabidopsis thaliana}PIR|T45654|T45654 zinc-finger-like protein - Arabido | TC79692 | BG132621 | cTOE8I16 | XVIIG4 | T5M8 | R17 | C13 | r3-c2 | r4-c4 |
| phaseolin G-box binding protein PG1 {Phaseolus vulgaris}GP|1142619|gb|AAB00686.1||U18348 phaseolin | TC79707 | AW037806 | cLET3D22 | XIH8 | T3P15 | R12 | C16 | r2-c1 | r4-c3 |

| | | | | | | | | |
|---|---|---|---|---|---|---|---|---|---|
| ALTERNATIVE OXIDASE 1 PRECURSOR (EC 1.-.--).GP\|558054\|gb\|AAC60576.1\|\|S71335 alternative oxidase, A | TC79757 | BE344500 | cLEY7C9 | XIVC9 | T4E18 | R7 | C5 | r2-c2 | r1-c4 |
| dTDP-glucose 4-6-dehydratase-like protein {Arabidopsis thaliana}PIR\|T45892\|T45892 dTDP-glucose 4-6- | TC79798 | AW035990 | cLEC33N6 | IIA6 | T1A12 | R13 | C1 | r1-c2 | r3-c3 |
| flavonol 3-o-glucosyltransferase 6 {Manihot esculenta}SP\|Q40288\|UFO6_MANES FLAVONOL 3-O-GLUCOSYLTRA | TC79809 | AI895028 | cLEC6H13 | IIE7 | T1I14 | R11 | C9 | r1-c2 | r3-c3 |
| soluble starch (bacterial glycogen) synthase {Solanum tuberosum}SP\|P93568\|UGS2_SOLTU SOLUBLE GLYCOG | TC79837 | AW929169 | cTOC4P12 | XVIID12 | T5G23 | R2 | C7 | r3-c2 | r4-c4 |
| lipoxygenase {Lycopersicon esculentum}GP\|1654138\|gb\|AAB65766.1\|\|U37839 lipoxygenase {Lycopersicon e | TC79855 | AW929070 | cTOC4A9 | XVIID8 | T5G15 | R12 | C7 | r3-c2 | r4-c4 |
| alpha-glucosidase {Solanum tuberosum}PIR\|T07391\|T07391 probable alpha-glucosidase (EC 3.2.1.20) - p | TC79865 | AW093459 | cLET24N10 | XIF11 | T3L21 | R4 | C12 | r2-c1 | r4-c3 |
| putative tyrosine decarboxylase {Arabidopsis thaliana}PIR\|A84588\|A84588 probable tyrosine decarboxy | TC79868 | BF112371 | cLEG41M3 | VIF5 | T2K10 | R15 | C11 | r4-c2 | r1-c3 |
| CYTOCHROME P450 83B1 (EC 1.14.-.-).GP\|3164126\|dbj\|BAA28531.1\|\|D78598 cytochrome P450 monooxygenase | TC79908 | AW032343 | cLEC35G8 | IIB6 | T1C12 | R13 | C3 | r1-c2 | r3-c3 |
| lipoxygenase {Solanum tuberosum}GP\|1117793\|gb\|AAD09202.1\|\|U24232 lipoxygenase {Solanum tuberosum}P | TC79919 | BF052088 | cLEM25F11 | VIIIC9 | T2F18 | R7 | C6 | r4-c2 | r1-c3 |
| putative ABC transporter; 73228-76244 {Arabidopsis thaliana} | TC79941 | BF113041 | cLEG42N24 | VIF10 | T2K20 | R5 | C11 | r4-c2 | r1-c3 |
| cytochrome p450 lxxviia2 {Solanum melongena}SP\|P37124\|C772_SOLME CYTOCHROME P450 77A2 (EC 1.14.-.-) | TC79994 | AI779493 | cLES8M5 | XIB3 | T3D5 | R20 | C4 | r2-c1 | r4-c3 |
| ARF GAP-like zinc finger-containing protein ZiGA4 {Arabidopsis thaliana} | TC80002 | BG137080 | cLPP4P19 | XVE12 | T4J23 | R2 | C10 | r2-c2 | r1-c4 |
| auxin-induced basic helix-loop-helix transcription factor, putative {Arabidopsis thaliana}GP\|123213 | TC80007 | BG125296 | cTOF8O13 | XXG7 | T5N14 | R11 | C14 | r3-c2 | r4-c4 |
| contains similarity to limonene | TC80009 | BG125098 | cTOF7L9 | XXG1 | T5N2 | R23 | C14 | r3-c2 | r4-c4 |

| | | | | | | | | | |
|---|---|---|---|---|---|---|---|---|---|
| cyclase~gene_id:K15O15.2 {Arabidopsis thaliana} | | | | | | | | | |
| CYTOCHROME P450 90A1 (EC 1.14.-.-).GP\|853719\|emb\|CAA60793.1\|\|X87367 CYP90 protein {Arabidopsis thal | TC80029 | BG124599 | cTOF5B9 | XXE9 | T5J18 | R7 | C10 | r3-c2 | r4-c4 |
| CYTOCHROME P450 84A1 (FERULATE-5-HYDROXYLASE) (EC 1.14.-.-) (F5H).GP\|1488255\|gb\|AAC49389.1\|\|U38416 | TC80030 | BG124625 | cTOF5F19 | XXE12 | T5J24 | R1 | C10 | r3-c2 | r4-c4 |
| transcription factor RUSH-1alpha isolog; 18684-24052 {Arabidopsis thaliana}PIR\|A86245\|A86245 hypoth | TC80033 | AI485822 | cLED4C4 | XIE9 | T3J17 | R8 | C10 | r2-c1 | r4-c3 |
| FLAVONOID 3',5'-HYDROXYLASE (EC 1.14.-.-) (F3'5'H) (CYTOCHROME P450 75A4).GP\|1620009\|dbj\|BAA12735.1 | TC80085 | BG134684 | cTOE17J5 | XVIIID5 | T5G10 | R15 | C7 | r3-c2 | r4-c4 |
| 76 kDa mitochondrial complex I subunit {Solanum tuberosum}SP\|Q43644\|NUAM_SOLTU NADH-UBIQUINONE OXID | TC80110 | AW618774 | cLPT16O5 | XVH1 | T4P1 | R24 | C16 | r2-c2 | r1-c4 |
| ABC transporter homolog {Populus nigra} | TC80115 | BF096360 | cLEW11G2 | XIID6 | T3H12 | R13 | C8 | r2-c1 | r4-c3 |
| Contains similarity to 12S seed storage globulin precursor GP\|134919. ESTs gb\|T13642, gb\|T21684 and | TC80151 | BG127001 | cTOF14G15 | XXIB2 | T6C3 | R22 | C3 | r3-c1 | r2-c4 |
| decarboxylase like protein {Arabidopsis thaliana}GP\|2245025\|emb\|CAB10445.1\|\|Z97341 decarboxylase li | TC80187 | AW038810 | cLET5H11 | XIIB8 | T3D16 | R9 | C4 | r2-c1 | r4-c3 |
| aldose-1-epimerase-like protein {Nicotiana tabacum}PIR\|T01933\|T01933 probable aldose 1-epimerase (E | TC80210 | AW041617 | cLET9L20 | XIID2 | T3H4 | R21 | C8 | r2-c1 | r4-c3 |
| ATP synthase alpha subunit, mitochondrial {Nicotiana plumbaginifolia}SP\|P05495\|ATP0_NICPL ATP SYNTH | TC80243 | BE436279 | cLEG31G12 | VIA6 | T2A12 | R13 | C1 | r4-c2 | r1-c3 |
| cytochrome p450 lxxia2 {Solanum melongena}SP\|P37118\|C712_SOLME CYTOCHROME P450 71A2 (EC 1.14.-.-) ( | TC80252 | AI896171 | cLEC14E8 | ID4 | T1G7 | R18 | C7 | r1-c2 | r3-c3 |
| CYTOCHROME P450 71A22 (EC 1.14.-.-).GP\|4678357\|emb\|CAB41167.1\|\|AL049659 cytochrome P450-like protei | TC80253 | AI896229 | cLEC14O18 | ID5 | T1G9 | R16 | C7 | r1-c2 | r3-c3 |
| putative dehydroquinase shikimate dehydrogenase {Arabidopsis thaliana} | TC80290 | AW443373 | cLET43P18 | XIIA8 | T3B16 | R9 | C2 | r2-c1 | r4-c3 |
| homogentisate 1,2-dioxygenase {Lycopersicon esculentum} | TC80293 | AW160297 | cLPT1O3 | XVIA1 | T4B2 | R23 | C2 | r2-c2 | r1-c4 |

103

| | | | | | | | | | |
|---|---|---|---|---|---|---|---|---|---|
| general negative transcription regulator-like {Arabidopsis thaliana} | TC80302 | AW398527 | cLPT2M6 | XVIA5 | T4B10 | R15 | C2 | r2-c2 | r1-c4 |
| putative transcriptional co-activator {Arabidopsis thaliana}GP\|3513735\|gb\|AAC33951.1\|\|AF080118 cont | TC80320 | AW040559 | cLET7I18 | XIIC3 | T3F6 | R19 | C6 | r2-c1 | r4-c3 |
| putative cytochrome P450 | TC80358 | AI782844 | cLES20P12 | XG11 | T3M22 | R3 | C13 | r2-c1 | r4-c3 |
| phospho-2-dehydro-3-deoxyheptonate aldolase | TC80361 | AW738073 | cTOD5K17 | XXIIA3 | T6A6 | R19 | C1 | r3-c1 | r2-c4 |
| glyceraldehyde 3-phosphate dehydrogenase a precursor, chloroplast {Nicotiana tabacum}SP\|P09043\|G3PA | TC80364 | AW039138 | cLET8H23 | XIIC10 | T3F20 | R5 | C6 | r2-c1 | r4-c3 |
| aminomethyltransferase precursor system t protein) {Solanum tuberosum}SP\|P54260\|GCST_SOLTU AMINOMET | TC80365 | AW040935 | cLET7O18 | XIIC6 | T3F12 | R13 | C6 | r2-c1 | r4-c3 |
| glyceraldehyde 3-phosphate dehydrogenase a precursor, chloroplast {Nicotiana tabacum}SP\|P09043\|G3PA | TC80366 | BE459022 | cLEM5E2 | VIIID11 | T2H22 | R3 | C8 | r4-c2 | r1-c3 |
| hydroxymethyltransferase {Arabidopsis thaliana}GP\|2244749\|emb\|CAB10172.1\|\|Z97335 hydroxymethyltrans | TC80391 | AW219880 | cLEX6G15 | XIIA7 | T3B14 | R11 | C2 | r2-c1 | r4-c3 |
| hydroxymethyltransferase {Arabidopsis thaliana}GP\|2244749\|emb\|CAB10172.1\|\|Z97335 hydroxymethyltrans | TC80394 | BG123319 | cTOF1F23 | XIXD3 | T5H5 | R20 | C8 | r3-c2 | r4-c4 |
| putative fructose-bisphosphate aldolase, plastidic form {Arabidopsis thaliana}GP\|11762176\|gb\|AAG403 | TC80401 | AI777043 | cLEC1C17 | IE1 | T1I1 | R24 | C9 | r1-c2 | r3-c3 |
| ATP synthase delta' subunit, mitochondrial precursor {Ipomoea batatas}SP\|Q40089\|ATP4_IPOBA ATP SYNT | TC80407 | BG125855 | cTOF10I1 | XVIIIG6 | T5M12 | R13 | C13 | r3-c2 | r4-c4 |
| glyceraldehyde 3-phosphate dehydrogenase | TC80408 | BF098524 | cLEW27D4 | XIIA5 | T3B10 | R15 | C2 | r2-c1 | r4-c3 |
| S-adenosyl-L-methionine synthetase | TC80422 | AW038211 | cLET1P4 | IVE8 | T1J16 | R9 | C10 | r1-c2 | r3-c3 |
| S-adenosyl-L-methionine synthetase | TC80423 | BG126068 | cTOF10L8 | XVIIIG9 | T5M18 | R7 | C13 | r3-c2 | r4-c4 |
| S-adenosylmethionine synthase 3 {Lycopersicon esculentum}SP\|P43282\|METM_LYCES S-ADENOSYLMETHIONINE | TC80424 | BG130132 | cTOF29K10 | XXB1 | T5D2 | R23 | C4 | r3-c2 | r4-c4 |
| cystathionine gamma-synthase isoform 1 {Solanum tuberosum} | TC80427 | AW738500 | cTOD7D11 | XVID5 | T4H10 | R15 | C8 | r2-c2 | r1-c4 |
| acetyl-CoA acyltransferase {Cucumis sativus}GP\|393707\|emb\|CAA47926.1\|\|X67696 acetyl-CoA acyltransf | TC80431 | AW621464 | cLEX12E11 | VA6 | T2A11 | R14 | C1 | r4-c2 | r1-c3 |
| xylose isomerase {Hordeum vulgare}SP\|Q40082\|XYLA_HORVU XYLOSE | TC80432 | AW034312 | cLEC33C6 | XVIG4 | T4N8 | R17 | C14 | r2-c2 | r1-c4 |

| ISOMERASE (EC 5.3.1.5).GP|1296809|em | | | | | | | | | |
|---|---|---|---|---|---|---|---|---|---|
| cytochrome P450 {Arabidopsis thaliana} | TC80453 | BE436610 | cLEG33D2 1 | VIB6 | T2C12 | R13 | C3 | r4-c2 | r1-c3 |
| Contains a PF|00175 Oxidoreductase FAD/NADH-binding domain. ESTs gb|H76345 and gb|AA651465 come fro | TC80456 | AI781810 | cLES17M1 5 | XF5 | T3K10 | R15 | C11 | r2-c1 | r4-c3 |
| caffeoyl-coenzymeA O-methyltransferase {Nicotiana tabacum}GP|1574946|gb|AAC49913.1||U38612 caffeoyl | TC80468 | AI484326 | cLES1I19 | XG4 | T3M8 | R17 | C13 | r2-c1 | r4-c3 |
| homologous to GATA-binding transcription factors {Arabidopsis thaliana}GP|7288001|emb|CAB81839.1||A | TC80486 | AW030365 | cLEC20O1 4 | IE6 | T1I11 | R14 | C9 | r1-c2 | r3-c3 |
| NADP-malic enzyme^^malate dehydrogenase | TC80499 | AW616575 | cLHT11B2 4 | XIVH1 | T4O2 | R23 | C15 | r2-c2 | r1-c4 |
| MADS box transcription factor-like {Arabidopsis thaliana} | TC80500 | BE431840 | cLEG4G17 | XXIB5 | T6C9 | R16 | C3 | r3-c1 | r2-c4 |
| triosephosphate isomerase, cytosolic {Petunia hybrida}SP|P48495|TPIS_PETHY TRIOSEPHOSPHATE ISOMERAS | TC80531 | BE433931 | cLEG9A4 | XVIF4 | T4L8 | R17 | C12 | r2-c2 | r1-c4 |
| putative homeodomain transcription factor {Arabidopsis thaliana}PIR|H84774|H84774 probable homeodom | TC80540 | AI771596 | cLED30M2 0 | IIIF9 | T1L17 | R8 | C12 | r1-c2 | r3-c3 |
| malate dehydrogenase {Nicotiana tabacum} | TC80550 | BF051399 | cLET13A12 | VIIIB2 | T2D4 | R21 | C4 | r4-c2 | r1-c3 |
| bZIP DNA-binding protein | TC80553 | BE460942 | cLEG37C1 | VID7 | T2G14 | R11 | C7 | r4-c2 | r1-c3 |
| lysine-ketoglutarate reductase/saccharopine dehydrogenase bifunctional enzyme {Arabidopsis thaliana} | TC80556 | AI486763 | cLED11B1 8 | IIG7 | T1M14 | R11 | C13 | r1-c2 | r3-c3 |
| phosphoglycerate kinase precursor {Solanum tuberosum}PIR|T07014|T07014 phosphoglycerate kinase (EC | TC80567 | BE353948 | cTOD8H1 | XXH1 | T5P2 | R23 | C16 | r3-c2 | r4-c4 |
| proton pump interactor {Arabidopsis thaliana}GP|7269604|emb|CAB81400.1||AL161571 proton pump intera | TC80570 | AI780385 | cLES11L9 | XD4 | T3G8 | R17 | C7 | r2-c1 | r4-c3 |
| malate dehydrogenase {Glycine max} | TC80572 | AW039846 | cLET13N23 | XIC12 | T3F23 | R2 | C6 | r2-c1 | r4-c3 |
| NADH-ubiquinone oxidoreductase 20 kDa subunit precursor {Solanum tuberosum}SP|Q43844|NUKM_SOLTU NAD | TC80576 | AI489373 | cLED16D1 | IIIA8 | T1B15 | R12 | C2 | r1-c2 | r3-c3 |
| MADS-box transcription factor FBP5 {Petunia x hybrida} | TC80582 | AI487071 | cLED9G21 | IVC11 | T1F22 | R3 | C6 | r1-c2 | r3-c3 |

EP 1 454 993 A1

| | | | | | | | | | |
|---|---|---|---|---|---|---|---|---|---|
| serine hydroxymethyltransferase, mitochondrial precursor {Solanum tuberosum}SP\|P50433\|GLYM_SOLTU SE | TC80593 | AW092318 | cLET19D5 | XXID5 | T6G9 | R16 | C7 | r3-c1 | r2-c4 |
| serine hydroxymethyltransferase, mitochondrial precursor {Solanum tuberosum}SP\|P50433\|GLYM_SOLTU SE | TC80594 | AI773651 | cLER7B24 | XVIA12 | T4B24 | R1 | C2 | r2-c2 | r1-c4 |
| homeodomain protein | TC80595 | BG124777 | cTOF6M4 | XXF4 | T5L8 | R17 | C12 | r3-c2 | r4-c4 |
| homeodomain leucine-zipper protein ATHB13 {Arabidopsis thaliana}GP\|12325190\|gb\|AAG52541.1\|AC013289 | TC80599 | AI898456 | cLED34E15 | IIIG5 | T1N9 | R16 | C14 | r1-c2 | r3-c3 |
| spermidine synthase {Arabidopsis thaliana} | TC80606 | AW441877 | cLEN18F20 | XXIB12 | T6C23 | R2 | C3 | r3-c1 | r2-c4 |
| putative ATP synthase {Arabidopsis thaliana}PIR\|B84606\|B84606 probable ATP synthase [imported] - Ar | TC80612 | BG130784 | cTOE1C12 | XVIIID8 | T5G16 | R9 | C7 | r3-c2 | r4-c4 |
| dTDP-glucose 4-6-dehydratases-like protein {Arabidopsis thaliana}PIR\|T45701\|T45701 dTDP-glucose 4-6 | TC80616 | AW625812 | cLEZ16P14 | XIVE3 | T4I6 | R19 | C9 | r2-c2 | r1-c4 |
| homology to pyroxidal-5'-phosphate-dependant glutamate decarboxylases; putative start codon | TC80620 | BE433185 | cLEG12J17 | IVH8 | T1P16 | R9 | C16 | r1-c2 | r3-c3 |
| glutamate decarboxylase {Lycopersicon esculentum}SP\|P54767\|DCE_LYCES GLUTAMATE DECARBOXYLASE (EC 4. | TC80621 | BE434187 | cLEG15D21 | VE6 | T2I11 | R14 | C9 | r4-c2 | r1-c3 |
| osmotic stress-induced zinc-finger protein {Nicotiana tabacum}PIR\|T01985\|T01985 zinc-finger protein | TC80630 | AW219530 | cLEX4M2 | XIIIE8 | T4I15 | R12 | C9 | r2-c2 | r1-c4 |
| ferritin subunit cowpea2 precursor {Vigna unguiculata}PIR\|T08124\|T08124 ferritin 2 precursor - cowp | TC80669 | BG129323 | cTOF24A17 | XIXG5 | T5N9 | R16 | C14 | r3-c2 | r4-c4 |
| contains similarity to NADH dehydrogenase chain CI-18~gene_id:K9I9.16 {Arabidopsis thaliana} | TC80670 | AW219844 | cLEX6E3 | XIIIE10 | T4I19 | R6 | C9 | r2-c2 | r1-c4 |
| succinate dehydrogenase | TC80679 | BE432138 | cLEG6G20 | XXIG12 | T6M23 | R2 | C13 | r3-c1 | r2-c4 |
| putative glycine decarboxylase p-protein | TC80681 | BE459806 | cLEM8I5 | XXIG2 | T6M3 | R22 | C13 | r3-c1 | r2-c4 |
| S-ADENOSYLMETHIONINE DECARBOXYLASE PROENZYME (EC 4.1.1.50) (ADOMETDC) (SAMDC).GP\|1498080\|gb\|AAC0461 | TC80692 | AI781600 | cLES16D11 | XE7 | T3I14 | R11 | C9 | r2-c1 | r4-c3 |
| CONSTANS-like protein 2 {Malus x domestica} | TC80693 | AI771970 | cLER1E6 | XA4 | T3A8 | R17 | C1 | r2-c1 | r4-c3 |
| pyruvate kinase (EC 2.7.1.40), cytosolic - potato | TC80694 | AW650674 | cLEI13B16 | VIIB4 | T2D7 | R18 | C4 | r4-c2 | r1-c3 |
| ATP synthase delta subunit, mitochondrial precursor | TC80699 | AW625595 | cLEZ15N21 | XIVD7 | T4G14 | R11 | C7 | r2-c2 | r1-c4 |

| (oligomycin sensitivity conferral protein) (oscp | | | | | | | | |
|---|---|---|---|---|---|---|---|---|
| Tetrafunctional protein of glyoxysomal fatty acid beta-oxidation {Brassica napus}PIR\|T08017\|T08017 | TC80702 | BE461193 | cLEG37F20 | XXID8 | T6G15 | R12 | C7 | r3-c1 | r2-c4 |
| PROBABLE VACUOLAR ATP SYNTHASE SUBUNIT H (EC 3.6.1.34) (V-ATPASE H SUBUNIT) (VACUOLAR PROTON PUMP H | TC80710 | AI490156 | cLER1C17 | XA2 | T3A4 | R21 | C1 | r2-c1 | r4-c3 |
| Strong similarity to gb\|L34684 inosine monophosphate dehydrogenase (IMPDH) from Arabidopsis thaliana | TC80721 | AW621167 | cLEX11C19 | XIIIA12 | T4A23 | R2 | C1 | r2-c2 | r1-c4 |
| phosphate/phosphoenolpyruvate translocator-like protein {Arabidopsis thaliana} | TC80730 | BG138135 | cLPP8K20 | XXIF12 | T6K23 | R2 | C11 | r3-c1 | r2-c4 |
| 3-isopropylmalate dehydrogenase precursor dehydrogenase) (imdh) (3-ipm-dh) {Solanum tuberosum}SP\|P2 | TC80800 | BF176554 | cLEZ20F11 | XXIC9 | T6E17 | R8 | C5 | r3-c1 | r2-c4 |
| sucrose transporter | TC80801 | AW218181 | cLEZ1K7 | XIVF8 | T4K16 | R9 | C11 | r2-c2 | r1-c4 |
| citrate synthase, glyoxysomal precursor {Cucurbita maxima}SP\|P49299\|CYSZ_CUCMA CITRATE SYNTHASE, GL | TC80803 | AW032595 | cLEC16J7 | XXIA6 | T6A11 | R14 | C1 | r3-c1 | r2-c4 |
| UDP-glucose:protein transglucosylase {Solanum tuberosum} | TC80818 | BF097146 | cLEW19H11 | XIIE5 | T3J10 | R15 | C10 | r2-c1 | r4-c3 |
| delta-12 fatty acid desaturase {Borago officinalis} | TC80824 | AW618696 | cLPT14B17 | XVG5 | T4N9 | R16 | C14 | r2-c2 | r1-c4 |
| enoyl-ACP reductase {Petunia x hybrida} | TC80834 | BF096267 | cLEW11G11 | XXIA9 | T6A17 | R8 | C1 | r3-c1 | r2-c4 |
| Similar to acyl carrier protein, mitochondrial precursor (ACP) NADH-ubiquinone oxidoreductase 9.6 KD | TC80836 | BF176381 | cLEZ20E22 | XXIC7 | T6E13 | R12 | C5 | r3-c1 | r2-c4 |
| omega-3 fatty acid desaturase, endoplasmic reticulum {Nicotiana tabacum}SP\|P48626\|FD3E_TOBAC OMEGA- | TC80843 | BG139666 | cLPP13J11 | XVD7 | T4H13 | R12 | C8 | r2-c2 | r1-c4 |
| UDP-glucose glucosyltransferase {Arabidopsis thaliana}GP\|9392679\|gb\|AAF87256.1\|AC068562_3\|AC068562 | TC80847 | AW035637 | cLEC39F24 | IIC12 | T1E24 | R1 | C5 | r1-c2 | r3-c3 |
| isocitrate dehydrogenase (NADP+) {Solanum tuberosum}PIR\|T07402\|T07402 probable isocitrate dehydroge | TC80851 | AI778998 | cLES6F20 | XIA3 | T3B5 | R20 | C2 | r2-c1 | r4-c3 |
| zinc finger protein {Pisum sativum}PIR\|T48868\|T48868 zinc finger protein [imported] - garden pea | TC80860 | BG138281 | cLPP8P2 | XVF4 | T4L7 | R18 | C12 | r2-c2 | r1-c4 |
| putative ABC transporter ATPase; 10053-12032 {Arabidopsis thaliana} | TC80867 | AW625248 | cLEZ13O13 | XIVD5 | T4G10 | R15 | C7 | r2-c2 | r1-c4 |

| | | | | | | | | | |
|---|---|---|---|---|---|---|---|---|---|
| transcriptional regulator, putative; 35498-34111 {Arabidopsis thaliana}PIR\|H96576\|H96576 hypothetic | TC80874 | BF051020 | cLEM21G1 8 | VC1 | T2E1 | R24 | C5 | r4-c2 | r1-c3 |
| legumin-like protein {Arabidopsis thaliana}PIR\|H84687\|H84687 legumin-like protein [imported] - Arab | TC80885 | AW224340 | cLEN16H1 2 | XIVB5 | T4C10 | R15 | C3 | r2-c2 | r1-c4 |
| ATP:citrate lyase {Capsicum annuum} | TC80889 | AI488015 | cLED19E8 | IIIB10 | T1D19 | R6 | C4 | r1-c2 | r3-c3 |
| hyoscyamine 6-dioxygenase hydroxylase) {Hyoscyamus niger}SP\|P24397\|HY6H_HYONI HYOSCYAMINE 6-DIOXYGE | TC80893 | AW650608 | cLEI13D23 | VIIB8 | T2D15 | R12 | C4 | r4-c2 | r1-c3 |
| RING-H2 finger protein RHF2a {Arabidopsis thaliana}GP\|13374859\|emb\|CAC34493.1\|\|AL589883 RING-H2 fin | TC80911 | BE433031 | cLEG11N1 9 | XIIIA1 | T4A1 | R24 | C1 | r2-c2 | r1-c4 |
| putative GDP-mannose pyrophosphorylase; 64911-67597 {Arabidopsis thaliana}PIR\|G96778\|G96778 hypothe | TC80933 | AI898042 | cLED31D1 9 | IIIF10 | T1L19 | R6 | C12 | r1-c2 | r3-c3 |
| glucosyltransferase-like protein {Arabidopsis thaliana} | TC80941 | AI895094 | cLEC6D16 | IIE5 | T1I10 | R15 | C9 | r1-c2 | r3-c3 |
| putative caffeoyl-CoA O-methyltransferase {Arabidopsis thaliana} | TC80956 | BE459901 | cLEM8I18 | XVIE1 | T4J2 | R23 | C10 | r2-c2 | r1-c4 |
| fatty acid elongase-like protein (cer2-like) {Arabidopsis thaliana}GP\|7268088\|emb\|CAB78426.1\|\|AL161 | TC80962 | AW616717 | cLHT12O2 3 | XVA3 | T4B5 | R20 | C2 | r2-c2 | r1-c4 |
| TOM (target of myb1)-like protein {Arabidopsis thaliana}PIR\|T51543\|T51543 TOM (target of myb1)-like | TC80976 | AW616128 | cLHT6E15 | XVC10 | T4F19 | R6 | C6 | r2-c2 | r1-c4 |
| heat shock transcription factor-like protein {Arabidopsis thaliana} | TC80978 | AW223910 | cLEN14G3 | IXA2 | T3A3 | R22 | C1 | r2-c1 | r4-c3 |
| Similar to ATP-citrate-lyase {Arabidopsis thaliana}PIR\|F86227\|F86227 hypothetical protein [imported | TC80979 | BE450968 | cLEY15N1 2 | XIIIH7 | T4O13 | R12 | C15 | r2-c2 | r1-c4 |
| putative NADH-ubiquinone oxireductase {Arabidopsis thaliana}PIR\|C84588\|C84588 probable NADH-ubiquin | TC80984 | AI772183 | cLER1N12 | XA10 | T3A20 | R5 | C1 | r2-c1 | r4-c3 |
| putative deoxycytidylate deaminase {Cicer arietinum} | TC80988 | AI774647 | cLER12P6 | IXE10 | T3I19 | R6 | C9 | r2-c1 | r4-c3 |
| putative RING finger protein; 84236-82024 {Arabidopsis thaliana}PIR\|A96829\|A96829 probable RING fin | TC81001 | AW223819 | cLEN13D4 | VIIIH3 | T2P6 | R19 | C16 | r4-c2 | r1-c3 |
| putative RING finger protein; 84236-82024 {Arabidopsis thaliana}PIR\|A96829\|A96829 probable RING fin | TC81002 | BG126984 | cTOF14E5 | XIXA3 | T5B5 | R20 | C2 | r3-c2 | r4-c4 |

| | | | | | | | | | |
|---|---|---|---|---|---|---|---|---|---|
| RING finger-like protein {Arabidopsis thaliana}PIR\|T47595\|T47595 RING finger protein T12E18.50 - Ar | TC81013 | AW035087 | cLEC13K19 | IC12 | T1E23 | R2 | C5 | r1-c2 | r3-c3 |
| mevalonate diphosphate decarboxylase {Arabidopsis thaliana}GP\|3250736\|emb\|CAA76803.1\|\|Y17593 mevalo | TC81015 | AW443810 | cTOF7H21 | XIIB2 | T3D4 | R21 | C4 | r2-c1 | r4-c3 |
| aspartate aminotransferase {Oryza sativa}PIR\|JC5125\|JC5125 aspartate transaminase (EC 2.6.1.1) prec | TC81020 | BG134029 | cTOE15G21 | XXIF7 | T6K13 | R12 | C11 | r3-c1 | r2-c4 |
| NADP-dependent isocitrate dehydrogenase-like protein | TC81025 | BG130056 | cTOF29M15 | XXB3 | T5D4 | R21 | C4 | r3-c2 | r4-c4 |
| Strong similarity to F19I3.8 GP\|3033381 putative UDP-galactose-4-epimerase from Arabidopsis thaliana | TC81034 | BE460513 | cLEG31L11 | VIA10 | T2A20 | R5 | C1 | r4-c2 | r1-c3 |
| glucosyltransferase-like protein {Arabidopsis thaliana} | TC81042 | AW220225 | cLEX9F14 | XVIIF3 | T5K5 | R20 | C11 | r3-c2 | r4-c4 |
| omega-6 fatty acid desaturase, chloroplast precursor {Brassica napus}SP\|P48627\|FD6C_BRANA OMEGA-6 F | TC81045 | AI773855 | cLER8K22 | XC8 | T3E16 | R9 | C5 | r2-c1 | r4-c3 |
| acyl-ACP thioesterase {Garcinia mangostana} | TC81091 | AI778618 | cLES5J24 | XH11 | T3O22 | R3 | C15 | r2-c1 | r4-c3 |
| ATP synthase a subunit precursor {Nicotiana tabacum}SP\|P06288\|ATPI_TOBAC ATP SYNTHASE A CHAIN PRECU | TC81098 | AI483512 | cLED25C6 | IIID3 | T1H5 | R20 | C8 | r1-c2 | r3-c3 |
| 3-isopropylmalate dehydrogenase precursor dehydrogenase) (imdh) (3-ipm-dh) {Brassica napus}SP\|P2910 | TC81104 | AW455243 | cLEX10G16 | XIIIA7 | T4A13 | R12 | C1 | r2-c2 | r1-c4 |
| anthranilate N-benzoyltransferase {Arabidopsis thaliana} | TC81105 | AW030112 | cLEC20N19 | IE5 | T1I9 | R16 | C9 | r1-c2 | r3-c3 |
| cytochrome c oxidase subunit Vb precursor-like protein {Arabidopsis thaliana} | TC81111 | AI776318 | cLER18I21 | IXH7 | T3O13 | R12 | C15 | r2-c1 | r4-c3 |
| UMP synthase {Nicotiana plumbaginifolia} | TC81117 | AW626116 | cLEZ18K8 | XIVE12 | T4I24 | R1 | C9 | r2-c2 | r1-c4 |
| anthocyanin 5-O-glucosyltransferase {Petunia x hybrida} | TC81118 | BE449685 | cLEY11E5 | XIIIF11 | T4K21 | R4 | C11 | r2-c2 | r1-c4 |
| uridine kinase-like protein {Arabidopsis thaliana} | TC81123 | AW929478 | cTOC9E1 | XVIIF5 | T5K9 | R16 | C11 | r3-c2 | r4-c4 |
| putative homeodomain transcription factor {Arabidopsis thaliana}PIR\|H84774\|H84774 probable homeodom | TC81124 | AW093664 | cLET25B16 | XIG1 | T3N1 | R24 | C14 | r2-c1 | r4-c3 |
| glucose-regulated protein 78 | TC81129 | AW040053 | cLET19M12 | XIE2 | T3J3 | R22 | C10 | r2-c1 | r4-c3 |
| contains similarity to acyl-CoA | TC81143 | BE450029 | cLEY11D6 | XIIIF10 | T4K19 | R6 | C11 | r2-c2 | r1-c4 |

109

EP 1 454 993 A1

| | | | | | | | | | |
|---|---|---|---|---|---|---|---|---|---|
| thioesterase~gene_id:K23F3.9 {Arabidopsis thaliana} | | | | | | | | | |
| HOMEOBOX-LEUCINE ZIPPER PROTEIN HAT5 (HD-ZIP PROTEIN 5) (HD-ZIP PROTEIN ATHB-1).□GP|16329|emb|CAA416 | TC81154 | AW220361 | cLEX10P11 | XIIIA11 | T4A21 | R4 | C1 | r2-c2 | r1-c4 |
| phosphoenolpyruvate carboxylase 1 {Gossypium hirsutum}GP|2266947|gb|AAB80714.1||AF008939 phosphoeno | TC81155 | AW223731 | cLEN12P6 | VIIIH1 | T2P2 | R23 | C16 | r4-c2 | r1-c3 |
| transcription factor CRC {Arabidopsis thaliana}GP|12325076|gb|AAG52485.1|AC018364_3|AC018364 transc | TC81170 | AI489150 | cLED15E7 | IIIA2 | T1B3 | R22 | C2 | r1-c2 | r3-c3 |
| putative glucose regulated repressor protein {Arabidopsis thaliana}PIR|A84649|A84649 probable gluco | TC81176 | AW617956 | cLPT11F3 | XVF10 | T4L19 | R6 | C12 | r2-c2 | r1-c4 |
| cytochrome P450 {Nicotiana tabacum}GP|1237250|emb|CAA65580.1||X96784 cytochrome P450 {Nicotiana tab | TC81178 | BF051289 | cLEM22K1 | VIIIB3 | T2D6 | R19 | C4 | r4-c2 | r1-c3 |
| tryptophan synthase alpha 1-like protein {Arabidopsis thaliana}GP|3892048|gb|AAC78257.1|AAC78257|AC | TC81185 | BG643947 | cTOF33J9 | XXD4 | T5H6 | R19 | C8 | r3-c2 | r4-c4 |
| small zinc finger-like protein | TC81193 | BG129576 | cTOF25A17 | XIXG9 | T5N17 | R8 | C14 | r3-c2 | r4-c4 |
| MybSt1 {Solanum tuberosum} | TC81223 | AI484070 | cLED22J14 | IIIC7 | T1F13 | R12 | C6 | r1-c2 | r3-c3 |
| immediate-early salicylate-induced glucosyltransferase {Nicotiana tabacum}GP|1685005|gb|AAB36653.1| | TC81244 | AW441527 | cLEN17D17 | IXA10 | T3A19 | R6 | C1 | r2-c1 | r4-c3 |
| bZIP transcription factor {Nicotiana tabacum} | TC81272 | BG124528 | cTOF5H16 | XIH10 | T3P19 | R6 | C16 | r2-c1 | r4-c3 |
| aspartate aminotransferase, cytoplasmic {Daucus carota}SP|P28734|AATC_DAUCA ASPARTATE AMINOTRANSFER | TC81279 | BG123545 | cTOF2C6 | XXB5 | T5D10 | R15 | C4 | r3-c2 | r4-c4 |
| putative RING zinc finger protein; 53384-54880 {Arabidopsis thaliana}PIR|G96835|G96835 probable RIN | TC81280 | AW455343 | cLEX10C4 | XIIIA6 | T4A11 | R14 | C1 | r2-c2 | r1-c4 |
| similar to ATPases associated with various cellular activites (Pfam: AAA.hmm, score: 230.91) {Arabid | TC81288 | AW033821 | cLEC29N4 | IG12 | T1M23 | R2 | C13 | r1-c2 | r3-c3 |
| ASPARTATE AMINOTRANSFERASE, MITOCHONDRIAL PRECURSOR (EC 2.6.1.1) (TRANSAMINASE A).GP|531555|emb|CAA | TC81293 | AI486001 | cLED3P14 | XXIB7 | T6C13 | R12 | C3 | r3-c1 | r2-c4 |
| phosphoribosylanthranilate transferase {Arabidopsis thaliana} | TC81302 | BE459246 | cLEM6A14 | VB7 | T2C13 | R12 | C3 | r4-c2 | r1-c3 |

| | | | | | | | | | |
|---|---|---|---|---|---|---|---|---|---|
| D-ribulose-5-phosphate 3-epimerase {Oryza sativa} | TC81304 | AI896020 | cLEC13J3 | IC10 | T1E19 | R6 | C5 | r1-c2 | r3-c3 |
| malate dehydrogenase, glyoxysomal precursor {Citrullus vulgaris}EGAD\|130842\|139627 glyoxysomal mala | TC81324 | AW222290 | cLEN7H3 | IXD6 | T3G11 | R14 | C7 | r2-c1 | r4-c3 |
| glyceraldehyde 3-phosphate dehydrogenase, cytosolic {Petunia hybrida}SP\|P26520\|G3PC_PETHY GLYCERALD | TC81336 | BG127844 | cTOF18C20 | XIXB9 | T5D17 | R8 | C4 | r3-c2 | r4-c4 |
| CCAAT-binding transcription factor subunit A(CBF-A) {Arabidopsis thaliana}GP\|2244810\|emb\|CAB10233.1 | TC81341 | AI780026 | cLES9N8 | XIB10 | T3D19 | R6 | C4 | r2-c1 | r4-c3 |
| H+-transporting ATPase-like protein {Arabidopsis thaliana}GP\|7270157\|emb\|CAB79970.1\|\|AL161581 H+-tr | TC81342 | AW038933 | cLET10I17 | XXIF9 | T6K17 | R8 | C11 | r3-c1 | r2-c4 |
| ZF-HD homeobox protein {Flaveria bidentis} | TC81347 | BG125944 | cTOF10O6 | XVIIIG10 | T5M20 | R5 | C13 | r3-c2 | r4-c4 |
| PUTATIVE NADH-UBIQUINONE OXIDOREDUCTASE SUBUNIT B17.2 (EC 1.6.5.3) (EC 1.6.99.3) (COMPLEX I-B17.2) ( | TC81349 | BG643035 | cTOF26K20 | XXIC11 | T6E21 | R4 | C5 | r3-c1 | r2-c4 |
| SNF5, transcription regulatory protein homolog BSH {Arabidopsis thaliana} | TC81352 | AI894835 | cLEC6E3 | XXIH2 | T6O3 | R22 | C15 | r3-c1 | r2-c4 |
| putative glucosyltransferase {Arabidopsis thaliana}GP\|4309698\|gb\|AAD15482.1\|\|AC006266 putative gluc | TC81356 | BE436042 | cLEG30F1 | VIA2 | T2A4 | R21 | C1 | r4-c2 | r1-c3 |
| glutamine cyclotransferase precursor {Carica papaya}PIR\|T08168\|T08168 glutaminyl-peptide cyclotrans | TC81367 | BE459845 | cLEM8O15 | VIIIF10 | T2L20 | R5 | C12 | r4-c2 | r1-c3 |
| contains similarity to sugar transporters (Pfam: sugar_tr.hmm, score: 395.91) {Arabidopsis thaliana} | TC81373 | AW737195 | cTOD2G24 | XXIA3 | T6A5 | R20 | C1 | r3-c1 | r2-c4 |
| putative folylpolyglutamate synthetase {Oryza sativa} | TC81390 | AW033669 | cLEC30B12 | IH3 | T1O5 | R20 | C15 | r1-c2 | r3-c3 |
| S-adenosyl-L-methionine Mg-protoporphyrin IX methyltranserase {Nicotiana tabacum} | TC81391 | BG128901 | cTOF22P13 | XIXF3 | T5L5 | R20 | C12 | r3-c2 | r4-c4 |
| contains similarity to ATP synthase B/B' (Pfam: ATP-synt_B.hmm, score: 11.71) {Arabidopsis thaliana} | TC81399 | AW223278 | cLEN11G3 | VIIIG5 | T2N10 | R15 | C14 | r4-c2 | r1-c4 |
| Contains similarity to gb\|AJ006354 zinc finger protein (ZAC) from Homo sapiens. {Arabidopsis thalian | TC81419 | AW441698 | cLEN18K1 | IXB8 | T3C15 | R12 | C3 | r2-c1 | r4-c4 |
| dihydroflavonol 4-reductase-like {Arabidopsis thaliana} | TC81435 | AW622813 | cTOB1M3 | XVIIB1 | T5C1 | R24 | C3 | r3-c2 | r4-c4 |
| putative RING zinc finger protein {Arabidopsis thaliana} | TC81454 | AI488569 | cLED17F6 | IIIB2 | T1D3 | R22 | C4 | r1-c2 | r3-c3 |
| beta-amylase {Arabidopsis thaliana} | TC81481 | BG135937 | cLPP1I15 | XVE5 | T4J9 | R16 | C10 | r2-c2 | r1-c4 |

EP 1 454 993 A1

| | | | | | | | | | |
|---|---|---|---|---|---|---|---|---|---|
| zinc finger-like protein {Arabidopsis thaliana}PIR|T49033|T49033 zinc finger-like protein - Arabido | TC81513 | BG134694 | cTOE17L7 | XXH4 | T5P8 | R17 | C16 | r3-c2 | r4-c4 |
| putative methylmalonate semi-aldehyde dehydrogenase {Arabidopsis thaliana}PIR|H84514|H84514 hypothe | TC81537 | AW649038 | cLEI6J14 | VIIF5 | T2L9 | R16 | C12 | r4-c2 | r1-c3 |
| myb-related protein 340 - garden snapdragon | TC81538 | AI486576 | cLED6E18 | IVB6 | T1D12 | R13 | C4 | r1-c2 | r3-c3 |
| ISOCITRATE DEHYDROGENASE [NADP] (EC 1.1.1.42) (OXALOSUCCINATE DECARBOXYLASE) (IDH) (NADP+-SPECIFIC I | TC81566 | AW094166 | cLET27B21 | XIG5 | T3N9 | R16 | C14 | r2-c1 | r4-c3 |
| UTP-glucose glucosyltransferase-like protein {Arabidopsis thaliana}GP|4835225|emb|CAB42903.1||AL049 | TC81577 | AW649171 | cLEI7I4 | VIIF12 | T2L23 | R2 | C12 | r4-c2 | r1-c3 |
| flavanone 3beta-hydroxylase {Petunia x hybrida} | TC81579 | BE462481 | cTOA13M9 | XVID7 | T4H14 | R11 | C8 | r2-c2 | r1-c4 |
| pyruvate kinase {Arabidopsis thaliana} | TC81587 | BG134368 | cTOE16K9 | XVIIIC12 | T5E24 | R1 | C5 | r3-c2 | r4-c4 |
| serine hydroxymethyltransferase, mitochondrial precursor {Solanum tuberosum}SP|P50433|GLYM_SOLTU SE | TC81590 | BE432582 | cLEG8L10 | VIH11 | T2O22 | R3 | C15 | r4-c2 | r1-c3 |
| serine hydroxymethyltransferase, mitochondrial precursor {Solanum tuberosum}SP|P50433|GLYM_SOLTU SE | TC81591 | BE460493 | cLEG31H1 | VIA7 | T2A14 | R11 | C1 | r4-c2 | r1-c3 |
| biotin carboxylase subunit {Nicotiana tabacum}GP|870726|gb|AAC41659.1||L38260 biotin carboxylase su | TC81634 | AW222488 | cLEN8G14 | IVD4 | T1H8 | R17 | C8 | r1-c2 | r3-c3 |
| ornithine carbamoyltransferase {Pisum sativum}SP|Q43814|OTC_PEA ORNITHINE CARBAMOYLTRANSFERASE PREC | TC81652 | BG125394 | cTOF8B19 | XXG3 | T5N6 | R19 | C14 | r3-c2 | r4-c4 |
| phosphoenolpyruvate carboxylase kinase {Lycopersicon esculentum} | TC81676 | AW223421 | cLEN11J18 | VIIIG7 | T2N14 | R11 | C14 | r4-c2 | r1-c3 |
| UDP-glucose glucosyltransferase {Solanum tuberosum}GP|1857447|gb|AAB48444.1||U82367 UDP-glucose glu | TC81688 | BG133854 | cTOE14G24 | XVIF6 | T4L12 | R13 | C12 | r2-c2 | r1-c4 |
| Strong similarity to UDP-glucose glucosyltransferase from Arabidopsis thaliana gb|AB016819 and conta | TC81690 | AW033693 | cLEC29O17 | IH1 | T1O1 | R24 | C15 | r1-c2 | r3-c3 |
| transcription factor NF-Y, CCAAT-binding-like protein {Arabidopsis thaliana}PIR|T45874|T45874 trans | TC81698 | AW621652 | cLEX12N12 | XIIIB11 | T4C21 | R4 | C3 | r2-c2 | r1-c4 |
| malate dehydrogenase (NADP), chloroplast precursor | TC81700 | BE458361 | cLEM1J8 | XIXA6 | T5B11 | R14 | C2 | r3-c2 | r4-c4 |

EP 1 454 993 A1

| | | | | | | | | | |
|---|---|---|---|---|---|---|---|---|---|
| (NADp-mdh) {Pisum sativum}SP|P21528|MDHC_PEA MAL | | | | | | | | | |
| acyl-CoA:1-acylglycerol-3-phosphate acyltransferase {Arabidopsis thaliana} | TC81716 | BE353669 | cTOA19N6 | XVIE7 | T4J14 | R11 | C10 | r2-c2 | r1-c4 |
| VACUOLAR ATP SYNTHASE SUBUNIT G 2 (EC 3.6.1.34) (V-ATPASE G SUBUNIT 2) (VACUOLAR PROTON PUMP G SUBUN | TC81726 | AW094576 | cLET29A14 | XIG8 | T3N15 | R12 | C14 | r2-c1 | r4-c3 |
| MADS-box transcription factor jointless | TC81749 | AI489275 | cLED17L17 | IIIB3 | T1D5 | R20 | C4 | r1-c2 | r3-c3 |
| putative RING zinc finger protein; 27623-28978 {Arabidopsis thaliana}PIR|H96703|H96703 probable RIN | TC81762 | AW622667 | cLEX15P2 | XIIID3 | T4G5 | R20 | C7 | r2-c2 | r1-c4 |
| Strong similarity to MRP-like ABC transporter gb|U92650 from A. thaliana and canalicular multi-drug | TC81773 | BE450653 | cLEY14D7 | XIIIG4 | T4M7 | R18 | C13 | r2-c2 | r1-c4 |
| alanine aminotransferase {Arabidopsis thaliana} | TC81776 | AW033989 | cLEC29A19 | IG8 | T1M15 | R12 | C13 | r1-c2 | r3-c3 |
| starch synthase, isoform V {Vigna unguiculata} | TC81835 | AI489258 | cLED17F23 | IIIB1 | T1D1 | R24 | C4 | r1-c2 | r3-c3 |
| ribulosebisphosphate carboxylase large subunit | TC81850 | AI897282 | cLED26L23 | IIID11 | T1H21 | R4 | C8 | r1-c2 | r3-c3 |
| acetyl-coA dehydrogenase, putative {Arabidopsis thaliana} | TC81857 | AW737344 | cTOD3O23 | XIVB11 | T4C22 | R3 | C3 | r2-c2 | r1-c4 |
| MADS box transcription factor MADS1 {Capsicum annuum} | TC81862 | BE353915 | cTOD6P15 | XXIC6 | T6E11 | R14 | C5 | r3-c1 | r2-c4 |
| tyrosine aminotransferase-like protein {Arabidopsis thaliana} | TC81863 | AW928492 | cTOC1J9 | XVIID2 | T5G3 | R22 | C7 | r3-c2 | r4-c4 |
| putative RING-H2 zinc finger protein ATL6 {Arabidopsis thaliana} | TC81880 | AW622674 | cLEX15P18 | XIIID2 | T4G3 | R22 | C7 | r2-c2 | r1-c4 |
| dihydrodipicolinate synthase {Nicotiana tabacum}SP|Q42948|DAPA_TOBAC DIHYDRODIPICOLINATE SYNTHASE P | TC81883 | BG127974 | cTOF18L13 | XIXC2 | T5F3 | R22 | C6 | r3-c2 | r4-c4 |
| putative CTP synthase {Oryza sativa} | TC81887 | BE450785 | cLEY15I1 | XIIIH6 | T4O11 | R14 | C15 | r2-c2 | r1-c4 |
| transcription factor-like; similar to CH6 and COP9 complex subunit 6 {Arabidopsis thaliana} | TC81893 | AW034210 | cLEC33E16 | IIA1 | T1A2 | R23 | C1 | r1-c2 | r3-c3 |
| putative phosphoribosylanthranilate transferase {Arabidopsis thaliana}GP|7267861|emb|CAB78204.1||AL | TC81895 | AW029720 | cLEC28K17 | IG6 | T1M11 | R14 | C13 | r1-c2 | r3-c3 |
| starch phosphorylase (AA 1-966) {Solanum tuberosum} | TC81900 | AI486229 | cLED5N5 | IVE1 | T1J2 | R23 | C10 | r1-c2 | r3-c3 |
| contains similarity to cyclopropane fatty acid synthase~gene_id:MEE5.5 {Arabidopsis thaliana} | TC81904 | AW618853 | cLPT17E8 | XVH3 | T4P5 | R20 | C16 | r2-c2 | r1-c4 |

113

| | | | | | | | | | |
|---|---|---|---|---|---|---|---|---|---|
| PROBABLE VACUOLAR ATP SYNTHASE SUBUNIT F (EC 3.6.1.34) (V-ATPASE F SUBUNIT) (VACUOLAR PROTON PUMP F | TC81929 | AW621415 | cLEX11L22 | XIIIB6 | T4C11 | R14 | C3 | r2-c2 | r1-c4 |
| cytochrome P450-like protein {Arabidopsis thaliana}GP\|7270932\|emb\|CAB80611.1\|\|AL161595 cytochrome P | TC81940 | AW617528 | cLHT23L11 | XXIB11 | T6C21 | R4 | C3 | r3-c1 | r2-c4 |
| cytochrome P450-like protein {Arabidopsis thaliana}PIR\|T46196\|T46196 cytochrome P450-like protein - | TC81993 | AW617528 | cLPT11J23 | XVB9 | T4D17 | R8 | C4 | r2-c2 | r1-c4 |
| uroporphyrinogen decarboxylase {Arabidopsis thaliana} | TC81996 | BG124909 | cTOF7E17 | XXF7 | T5L14 | R11 | C12 | r3-c2 | r4-c4 |
| succinate dehydrogenase flavoprotein alpha subunit {Arabidopsis thaliana}GP\|8843734\|dbj\|BAA97282.1\| | TC82029 | AW218470 | cLEZ9G24 | XIVG11 | T4M22 | R3 | C13 | r2-c2 | r1-c4 |
| HD-Zip protein {Arabidopsis thaliana}GP\|3132474\|gb\|AAC16263.1\|\|AC003096 homeodomain transcription f | TC82042 | AW647780 | cLEI2C10 | VIID5 | T2H9 | R16 | C8 | r4-c2 | r1-c3 |
| phosphate transporter^^putative phosphate transporter^^inorganic phosphate transporter | TC82044 | AI776381 | cLER18E22 | IXH5 | T3O9 | R16 | C15 | r2-c1 | r4-c3 |
| heat stress transcription factor A3 {Lycopersicon peruvianum} | TC82048 | AW399021 | cLPT5B12 | XVIB1 | T4D2 | R23 | C4 | r2-c2 | r1-c4 |
| putative glucosyl transferase {Arabidopsis thaliana}PIR\|H84784\|H84784 probable glucosyl transferase | TC82051 | AI772146 | cLER1F22 | XA5 | T3A10 | R15 | C1 | r2-c1 | r4-c3 |
| contains similarity to chalcone-flavonone isomerase (chalcone isomerase)~gene_id:K18I23.7 {Arabidops | TC82093 | BG628492 | cLEL22G17 | XXIE9 | T6I17 | R8 | C9 | r3-c1 | r2-c4 |
| phosphoribosylanthranilate isomerase {Arabidopsis thaliana} | TC82095 | AW037478 | cLET1I13 | XIE8 | T3J15 | R12 | C10 | r2-c1 | r4-c3 |
| LIN6^^acid invertase | TC82103 | AI779579 | cLES8B13 | XIA11 | T3B21 | R4 | C2 | r2-c1 | r4-c3 |
| Contains similarity to ARI, RING finger protein gb\|X98309 from Drosophila melanogaster. ESTs gb\|T44 | TC82118 | AI484345 | cLES1O17 | XG5 | T3M10 | R15 | C13 | r2-c1 | r4-c3 |
| transcription factor-like protein {Arabidopsis thaliana}GP\|7576196\|emb\|CAB87947.1\|\|AL163912 transcr | TC82130 | BG643340 | cTOF27E22 | XXA1 | T5B2 | R23 | C2 | r3-c2 | r4-c4 |
| polyphenol oxidase precursor | TC82138 | AW624791 | cLEZ8M21 | XIVG1 | T4M2 | R23 | C13 | r2-c2 | r1-c4 |
| bHLH transcription factor GBOF-1 {Tulipa gesneriana} | TC82153 | AI490119 | cLED22C14 | IIIC6 | T1F11 | R14 | C6 | r1-c2 | r3-c3 |
| glycerol-3-phosphate dehydrogenase {Arabidopsis | TC82167 | AI773999 | cLER9I5 | XC11 | T3E22 | R3 | C5 | r2-c1 | r4-c3 |

114

| | | | | | | | | | |
|---|---|---|---|---|---|---|---|---|---|
| thaliana}PIR\|F84832\|F84832 glycerol-3-phosphate deh | | | | | | | | | |
| putative cytochrome P450 {Oryza sativa}GP\|11761117\|dbj\|BAB19107.1\|AP002839 putative cytochrome P45 | TC82169 | AW624950 | cLEZ9A23 | XIVG9 | T4M18 | R7 | C13 | r2-c2 | r1-c4 |
| transcription factor IIA small subunit {Arabidopsis thaliana}GP\|5051786\|emb\|CAB45079.1\|AL078637 tr | TC82195 | BG126942 | cTOF13L22 | XVIIIH11 | T5O22 | R3 | C15 | r3-c2 | r4-c4 |
| glucosyltransferase-like protein {Arabidopsis thaliana}GP\|7340661\|emb\|CAB82941.1\|AL162506 putative | TC82199 | BF051083 | cLEM21D1 5 | VIIIA3 | T2B6 | R19 | C2 | r4-c2 | r1-c3 |
| chalcone synthase-like protein {Arabidopsis thaliana}GP\|7270436\|emb\|CAB80202.1\|AL161586 chalcone s | TC82205 | AW623633 | cTOB11H1 | XVIG3 | T4N6 | R19 | C14 | r2-c2 | r1-c4 |
| sugar transporter-like protein {Arabidopsis thaliana} | TC82207 | AW041746 | cLET14I14 | XID2 | T3H3 | R22 | C8 | r2-c1 | r4-c3 |
| putative cytochrome P450 {Arabidopsis thaliana}GP\|13877669\|gb\|AAK43912.1\|AF370593_1\|A F370593 putati | TC82226 | AW651015 | cLEI15O17 | VIIC9 | T2F17 | R8 | C6 | r4-c2 | r1-c3 |
| putative zinc finger protein {Arabidopsis thaliana}GP\|7270045\|emb\|CAB79860.1\|AL161579 putative zin | TC82243 | AI781951 | cLES17L23 | XF4 | T3K8 | R17 | C11 | r2-c1 | r4-c3 |
| 2-oxoglutarate/malate translocator precursor {Spinacia oleracea}SP\|Q41364\|SOT1_SPIOL 2-OXOGLUTARATE | TC82252 | AW442880 | cLET42G24 | XXIA1 | T6A1 | R24 | C1 | r3-c1 | r2-c4 |
| NADH-dependent glutamate synthase {Arabidopsis thaliana} | TC82279 | AW035530 | cLEC39M7 | XXIE2 | T6I3 | R22 | C9 | r3-c1 | r2-c4 |
| anthocyanidin 3-O-glucosyltransferase {Petunia x hybrida} | TC82331 | BG630259 | cLEL33O12 | XXIF10 | T6K19 | R6 | C11 | r3-c1 | r2-c4 |
| 3-methylcrotonyl-CoA carboxylase non-biotinylated subunit {Arabidopsis thaliana}GP\|7021224\|gb\|AAF35 | TC82338 | BF098233 | cLEW26M6 | XIIG12 | T3N24 | R1 | C14 | r2-c1 | r4-c3 |
| Cytochrome P450-like protein {Arabidopsis thaliana}GP\|7270098\|emb\|CAB79912.1\|AL161580 Cytochrome P | TC82348 | AW738451 | cTOD7I20 | XVIIIA6 | T5A12 | R13 | C1 | r3-c2 | r4-c4 |
| transketolase 1 {Capsicum annuum}PIR\|T09541\|T09541 transketolase (EC 2.2.1.1) TKT1 precursor, chlor | TC82386 | BF097344 | cLEW20C1 8 | XIIG1 | T3N2 | R23 | C14 | r2-c1 | r4-c3 |
| HEAT SHOCK FACTOR PROTEIN 5 (HSF 5) (HEAT SHOCK TRANSCRIPTION FACTOR 5) (HSTF 5).GP\|6624614\|emb\|CAB | TC82389 | AW649996 | cLEI11K12 | IVH1 | T1P2 | R23 | C16 | r1-c2 | r3-c3 |

| Similar to Populus balsamifera subsp. trichocarpa X Populus deltoides vegetative storage protein. (L | TC82393 | BG627286 | cLEL16O9 | XXIC5 | T6E9 | R16 | C5 | r3-c1 | r2-c4 |
|---|---|---|---|---|---|---|---|---|---|
| putative enolase (2-phospho-D-glycerate hydroylase) {Arabidopsis thaliana}PIR\|G84697\|G84697 hypothe | TC82394 | AW441644 | cLEN17N4 | IXB3 | T3C5 | R20 | C3 | r2-c1 | r4-c3 |
| putative CCCH-type zinc finger protein {Arabidopsis thaliana}PIR\|D84581\|D84581 probable CCCH-type z | TC82395 | AI773737 | cLER8C11 | XC6 | T3E12 | R13 | C5 | r2-c1 | r4-c3 |
| 3-dehydroquinate synthase-like protein {Arabidopsis thaliana} | TC82414 | AI774955 | cLER13L4 | IXE12 | T3I23 | R2 | C9 | r2-c1 | r4-c3 |
| cytochrome P450-like protein {Arabidopsis thaliana}GP\|7270932\|emb\|CAB80611.1\|\|AL161595 cytochrome P | TC82416 | AW651396 | cLEI16D15 | XVIIE8 | T5I15 | R12 | C9 | r3-c2 | r4-c4 |
| isoflavone reductase homolog {Solanum tuberosum}SP\|P52578\|IFRH_SOLTU ISOFLAVONE REDUCTASE HOMOLOG ( | TC82426 | BG130065 | cTOF29O11 | XXB2 | T5D6 | R19 | C4 | r3-c2 | r4-c4 |
| putative pyrophosphate--fructose-6-phosphate 1-phosphotransferase {Arabidopsis thaliana}PIR\|B84613\| | TC82429 | BF051504 | cLEM23M13 | VIIIB11 | T2D22 | R3 | C4 | r4-c2 | r1-c3 |
| phosphoglycerate mutase {Solanum tuberosum} | TC82433 | BG643106 | cTOF26J11 | XIXH8 | T5P15 | R12 | C16 | r3-c2 | r4-c4 |
| 6-phosphogluconate dehydrogenase, putative; 13029-14489 {Arabidopsis thaliana} | TC82459 | BE450814 | cLEY15O9 | XIIIH10 | T4O19 | R6 | C15 | r2-c2 | r1-c4 |
| putative P-protein: chorismate mutase, prephenate dehydratase {Arabidopsis thaliana} | TC82472 | BF050999 | cLEM21C18 | VIIIA2 | T2B4 | R21 | C2 | r4-c2 | r1-c3 |
| putative arginine methyltransferase {Arabidopsis thaliana} | TC82475 | BF051059 | cLEM21O8 | VIIIA8 | T2B16 | R9 | C2 | r4-c2 | r1-c3 |
| P450 hydroxylase {Petunia x hybrida}PIR\|S32110\|S32110 cytochrome P450 PET-1 - garden petunia (fragm | TC82490 | AI489137 | cLED15E15 | IIIA1 | T1B1 | R24 | C2 | r1-c2 | r3-c3 |
| pathogenesis-related homeodomain protein (prhp) {Petroselinum crispum}SP\|P48786\|PRH_PETCR PATHOGENE | TC82493 | AW618573 | cLPT13P12 | XVG11 | T4N21 | R4 | C14 | r2-c2 | r1-c4 |
| ferredoxin--nitrite reductase {Nicotiana tabacum}GP\|19893\|emb\|CAA46940.1\|\|X66145 ferredoxin--nitrit | TC82500 | AI775854 | cLER16D16 | IXG4 | T3M7 | R18 | C13 | r2-c1 | r4-c3 |
| contains similarity to heat shock transcription factor~gene_id:MOB24.9 {Arabidopsis thaliana} | TC82508 | AW979619 | cLEW8I23 | XVIIIA8 | T5A16 | R9 | C1 | r3-c2 | r4-c4 |
| starch synthase {Ipomoea batatas} | TC82511 | AI899166 | cLED37O8 | XVIIC4 | T5E7 | R18 | C5 | r3-c2 | r4-c4 |
| alpha-glucosidase {Solanum tuberosum subsp. tuberosum} | TC82534 | AW648278 | cLEI4E5 | VIIE6 | T2J11 | R14 | C10 | r4-c2 | r1-c3 |

| Description | TC | Accession | Clone | | | | | | |
|---|---|---|---|---|---|---|---|---|---|
| similar to class I knotted-like homeodomain protein {LeT6 | TC82559 | AI490554 | cLED25E14 | IIID4 | T1H7 | R18 | C8 | r1-c2 | r3-c3 |
| putative internal rotenone-insensitive NADH dehydrogenase {Solanum tuberosum} | TC82565 | BG629691 | cLEL29K7 | XXIH1 | T6O1 | R24 | C15 | r3-c1 | r2-c4 |
| putative C3HC4-type RING zinc finger protein {Arabidopsis thaliana}GP|11908040|gb|AAG41449.1|AF3268 | TC82567 | AI778661 | cLES6C1 | XVIA6 | T4B12 | R13 | C2 | r2-c2 | r1-c4 |
| acetyl-CoA C-acetyltransferase {Arabidopsis thaliana} | TC82576 | BE459497 | cLEM7A3 | VIIIE7 | T2J14 | R11 | C10 | r4-c2 | r1-c3 |
| amidophosphoribosyltransferase {Arabidopsis thaliana} | TC82583 | AW154805 | cLEW1C23 | XIIE11 | T3J22 | R3 | C10 | r2-c1 | r4-c3 |
| putative anthocyanin 5-aromatic acyltransferase {Arabidopsis thaliana}PIR|G84823|G84823 probable an | TC82613 | AW092901 | cLET22L23 | XIIB6 | T3D12 | R13 | C4 | r2-c1 | r4-c3 |
| cystathionine beta-lyase {Solanum tuberosum} | TC82650 | BE461649 | cLEG39D17 | VIE7 | T2I14 | R11 | C9 | r4-c2 | r1-c3 |
| glutamine synthetase I {Medicago truncatula} | TC82659 | AI896662 | cLEC16M12 | ID10 | T1G19 | R6 | C7 | r1-c2 | r3-c3 |
| putative cytochrome P450; 1456-3294 {Arabidopsis thaliana}GP|10092278|gb|AAG12691.1|AC025814_15|AC O | TC82728 | AW622085 | cLEX14O19 | XIIIC9 | T4E17 | R8 | C5 | r2-c2 | r1-c4 |
| HOMEOBOX-LEUCINE ZIPPER PROTEIN HAT22 (HD-ZIP PROTEIN 22).GP|549887|gb|AAA56902.1||U09336 homeobox | TC82731 | AW038246 | cLET1H12 | XIE7 | T3J13 | R12 | C10 | r2-c1 | r4-c3 |
| transcription factor inhibitor I kappa B homolog {Arabidopsis thaliana}GP|1773295|gb|AAC49611.1||U7 | TC82775 | BG124935 | cTOF7K1 | XXF11 | T5L22 | R3 | C12 | r3-c2 | r4-c4 |
| putative glycerol-3-phosphate dehydrogenase {Arabidopsis thaliana} | TC82784 | AW219947 | cLEX6M3 | XIIIF2 | T4K3 | R22 | C11 | r2-c2 | r1-c4 |
| NADH dehydrogenase like protein {Arabidopsis thaliana}GP|7268946|emb|CAB81256.1||AL161555 NADH dehy | TC82792 | AW040558 | cLET7I22 | XIIC4 | T3F8 | R17 | C6 | r2-c1 | r4-c3 |
| zinc finger protein SHI-like {Arabidopsis thaliana}GP|4929803|gb|AAD34162.1|AF152555_1|AF152555 put | TC82801 | AW216650 | cLEC23D4 | IIF8 | T1K16 | R9 | C11 | r1-c2 | r3-c3 |
| inorganic phosphate transporter | TC82826 | AW621975 | cLEX13J24 | XIIIC4 | T4E7 | R18 | C5 | r2-c2 | r1-c4 |
| polyneuridine aldehyde esterase {Rauvolfia serpentina} | TC82834 | BE433359 | cLEG13E6 | VE4 | T2I7 | R18 | C9 | r4-c2 | r1-c3 |
| alpha-glucosidase {Solanum tuberosum subsp. tuberosum} | TC82868 | AI487222 | cLED6K9 | IVB10 | T1D20 | R5 | C4 | r1-c2 | r3-c3 |
| ABC transporter-like protein {Arabidopsis thaliana}GP|13899119|gb|AAK48981.1|AF370554_1|A | TC82872 | AW623019 | cTOB8A15 | XVIIB8 | T5C15 | R12 | C3 | r3-c2 | r4-c4 |

| | | | | | | | | | |
|---|---|---|---|---|---|---|---|---|---|
| F370554 AB | | | | | | | | | |
| heat shock factor protein hsf24 (heat shock transcription factor 24) (hstf 24) (heat stress transcri | TC82923 | BF097217 | cLEW19F16 | XIIE2 | T3J4 | R21 | C10 | r2-c1 | r4-c3 |
| sugar transporter like protein {Arabidopsis thaliana}GP\|2464913\|emb\|CAB16808.1\|Z99708 sugar transp | TC82942 | BG138983 | cLPP11N17 | XVD4 | T4H7 | R18 | C8 | r2-c2 | r1-c4 |
| cytochrome P450-like protein {Arabidopsis thaliana}PIR\|T47554\|T47554 cytochrome P450 homolog F8J2.1 | TC82954 | BE450630 | cLEY14M8 | XIIIG8 | T4M15 | R12 | C13 | r2-c2 | r1-c4 |
| TRYPTOPHAN SYNTHASE BETA CHAIN 2 PRECURSOR (EC 4.2.1.20).GP\|2792520\|gb\|AAB97087.1\|AF042320 tryptop | TC82960 | BE458808 | cLEM4L3 | VIIID9 | T2H18 | R7 | C8 | r4-c2 | r1-c3 |
| putative C3HC4-type RING zinc finger protein {Arabidopsis thaliana}PIR\|B84813\|B84813 probable RING | TC82965 | AW621441 | cLEX12A9 | XIIIB9 | T4C17 | R8 | C3 | r2-c2 | r1-c4 |
| Similar to gb\|Z84571 anthranilate N-hydroxycinnamoyl/benzoyltransferase from Dianthus caryophyllus. | TC82992 | BG133540 | cTOE13N21 | XIIF6 | T3L12 | R13 | C12 | r2-c1 | r4-c3 |
| ABC transporter homolog {Populus nigra} | TC83000 | AW033000 | cLEC19N6 | XVIIC9 | T5E17 | R8 | C5 | r3-c2 | r4-c4 |
| contains similarity to ABC transporter~gene_id:MAC9.4 {Arabidopsis thaliana} | TC83006 | BG141252 | cLPP20D10 | XVE6 | T4J11 | R14 | C10 | r2-c2 | r1-c4 |
| putative strictosidine synthase | TC83008 | BG128579 | cTOF21M10 | XIXE5 | T5J9 | R16 | C10 | r3-c2 | r4-c4 |
| phosphate/phosphoenolpyruvate translocator precursor {Nicotiana tabacum}GP\|1778145\|gb\|AAB40648.1\|U | TC83014 | BE460073 | cLEM8L2 | VIIIF6 | T2L12 | R13 | C12 | r4-c2 | r1-c3 |
| putative enolase; 31277-33713 {Arabidopsis thaliana}PIR\|B96768\|B96768 protein enolase F2P9.10 impo | TC83066 | AW648181 | cLEI3N19 | VIIE2 | T2J3 | R22 | C10 | r4-c2 | r1-c3 |
| diacylglycerol kinase {Lycopersicon esculentum} | TC83073 | AW223728 | cLEN12N22 | VIIIG11 | T2N22 | R3 | C14 | r4-c2 | r1-c3 |
| cytochrome P450 {Capsicum annuum} | TC83085 | AW219261 | cLEX3H20 | XIIID12 | T4G23 | R2 | C7 | r2-c2 | r1-c4 |
| contains similarity to RNA polymerase transcriptional regulation mediator~gene_id:MHC9.3 {Arabidopsi | TC83117 | AW218226 | cLEZ1O15 | XIVF9 | T4K18 | R7 | C11 | r2-c2 | r1-c4 |
| acetyl-CoA synthetase, putative; 45051-31547 {Arabidopsis thaliana}PIR\|D96595\|D96595 probable acety | TC83139 | AW647759 | cLEI2M11 | VIID7 | T2H13 | R12 | C8 | r4-c2 | r1-c3 |

| | | | | | | | | | |
|---|---|---|---|---|---|---|---|---|---|
| ABC transporter-like protein {Arabidopsis thaliana}PIR|T07717|T07717 probable ABC-type transport pr | TC83143 | AW038363 | cLET6E16 | XIIB12 | T3D24 | R1 | C4 | r2-c1 | r4-c3 |
| putative sugar transporter {Arabidopsis thaliana} | TC83157 | BG132543 | cTOE7P6 | XVIIIG2 | T5M4 | R21 | C13 | r3-c2 | r4-c4 |
| putative zinc finger protein {Arabidopsis thaliana}GP|7270045|emb|CAB79860.1||AL161579 putative zin | TC83165 | BE436795 | cLEG34A24 | VIB12 | T2C24 | R1 | C3 | r4-c2 | r1-c3 |
| CYTOCHROME P450 98A2 (EC 1.14.-.-).GP|2738998|gb|AAB94587.1||AF022458 CYP98A2p {Glycine max}PIR|T0 | TC83207 | BE436335 | cLEG32E11 | VIA12 | T2A24 | R1 | C1 | r4-c2 | r1-c3 |
| transcription factor, putative {Arabidopsis thaliana}PIR|E96612|E96612 probable transcription facto | TC83217 | BE458948 | cLEM5G9 | VIIID12 | T2H24 | R1 | C8 | r4-c2 | r1-c3 |
| auxin-induced basic helix-loop-helix transcription factor {Gossypium hirsutum} | TC83218 | BG130684 | cTOF31L2 | XXC6 | T5F12 | R13 | C6 | r3-c2 | r4-c4 |
| ABC transporter-like protein {Arabidopsis thaliana}GP|9964121|gb|AAG09829.1|AF287699_1|AF 287699 hal | TC83254 | BE434861 | cLEG24A21 | VG6 | T2M11 | R14 | C13 | r4-c2 | r1-c3 |
| bHLH transcription factor JAF13 {Petunia x hybrida} | TC83264 | BE433296 | cLEG13C11 | XVIIF11 | T5K21 | R4 | C11 | r3-c2 | r4-c4 |
| glycerol-3-phosphate dehydrogenase {Arabidopsis thaliana}PIR|F84832|F84832 glycerol-3-phosphate deh | TC83308 | BE431781 | cLEG3E11 | VIE11 | T2I22 | R3 | C9 | r4-c2 | r1-c3 |
| cytochrome p450 lxxia3 {Solanum melongena}SP|P37119|C713_SOLME CYTOCHROME P450 71A3 (EC 1.14.-.-) ( | TC83334 | BG631413 | cLEL7M4 | XXH7 | T5P14 | R11 | C16 | r3-c2 | r4-c4 |
| putative 6-phosphogluconolactonase {Arabidopsis thaliana} | TC83350 | AW622762 | cTOB5D8 | XVIIB4 | T5C7 | R18 | C3 | r3-c2 | r4-c4 |
| soluble starch (bacterial glycogen) synthase {Solanum tuberosum}SP|P93568|UGS2_SOLTU SOLUBLE GLYCOG | TC83359 | BE432874 | cLEG10I6 | VD4 | T2G7 | R18 | C7 | r4-c2 | r1-c3 |
| beta-amylase-like {Arabidopsis thaliana} | TC83371 | BE433215 | cLEG12B22 | VD10 | T2G19 | R6 | C7 | r4-c2 | r1-c3 |
| cytochrome P450 {Arabidopsis thaliana}GP|7268718|emb|CAB78925.1||AL161550 cytochrome P450 {Arabidop | TC83399 | AI896822 | cLEC23P7 | IF3 | T1K5 | R20 | C11 | r1-c2 | r3-c3 |
| fructokinase {Lycopersicon esculentum}GP|2102691|gb|AAB57733.1||U64817 | TC83425 | BG139714 | cLPP14C5 | XVD9 | T4H17 | R8 | C8 | r2-c2 | r1-c4 |

| Description | TC # | Accession | Clone | | | | | | |
|---|---|---|---|---|---|---|---|---|---|
| fructokinase {Lycopersicon e<br>putative citrate synthase {Arabidopsis thaliana} PIR\|C84858\|C84858 probable citrate synthase [import | TC83487 | BE434567 | cLEGI8A2 3 | VF7 | T2K13 | R12 | C11 | r4-c2 | r1-c3 |
| putative acetone-cyanohydrin lyase {Arabidopsis thaliana} PIR\|T01151\|T01151 probable acetone-cyanohy | TC83491 | AW219332 | cLEX4G11 | XIIIE5 | T4I9 | R16 | C9 | r2-c2 | r1-c4 |
| putative pyrophosphate-dependent phosphofructo-1-kinase {Arabidopsis thaliana} | TC83500 | AW738248 | cTOD6B4 | XVIIH12 | T5O23 | R2 | C15 | r3-c2 | r4-c4 |
| uroporphyrinogen decarboxylase {Arabidopsis thaliana} | TC83506 | BG129172 | cTOF23D1 9 | XIXF8 | T5L15 | R12 | C12 | r3-c2 | r4-c4 |
| Zn finger protein {Nicotiana tabacum} GP\|1360078\|emb\|CAA66601.1\|\|X97942 Zn finger protein {Nicotiana | TC83522 | AI489847 | cLED15I6 | IIIA5 | T1B9 | R16 | C2 | r1-c2 | r3-c3 |
| transcription factor WRKY6 {Arabidopsis thaliana} GP\|12658412\|gb\|AAK01128.1\|AF331713_1\|A F331713 tran | TC83553 | AW029692 | cLEC11I13 | IC3 | T1E5 | R20 | C5 | r1-c2 | r3-c3 |
| limonene cyclase like protein {Arabidopsis thaliana} GP\|2245029\|emb\|CAB10449.1\|\|Z97341 limonene cycl | TC83555 | AW932587 | cLEF49C20 | VA1 | T2A1 | R24 | C1 | r4-c2 | r1-c3 |
| GMP synthase; 61700-64653 {Arabidopsis thaliana} PIR\|E96661\|E96661 GMP synthase, 61700-64653 [import | TC83694 | AW616601 | cLHT11H8 | XIVH7 | T4O14 | R11 | C15 | r2-c2 | r1-c4 |
| beta-amylase {Glycine max} GP\|902938\|dbj\|BAA09462.1\|\|D50866 beta-amylase {Glycine max} | TC83696 | AW616937 | cLHT18H3 | XVA7 | T4B13 | R12 | C2 | r2-c2 | r1-c4 |
| pyruvate kinase (EC 2.7.1.40) A, chloroplast - common tobacco | TC83701 | BE459930 | cLEM8O6 | VIIIF11 | T2L22 | R3 | C12 | r4-c2 | r1-c3 |
| cytochrome p450-like protein {Arabidopsis thaliana} GP\|7270718\|emb\|CAB80401.1\|\|AL161591 cytochrome p | TC83712 | AW616143 | cLHT6I19 | XVC12 | T4F23 | R2 | C6 | r2-c2 | r1-c4 |
| Putative UDP-glucose glucosyltransferase {Arabidopsis thaliana} PIR\|H86356\|H86356 probable UDP-gluco | TC83719 | AW616197 | cLHT1O22 | XVA11 | T4B21 | R4 | C2 | r2-c2 | r1-c4 |
| Similar to gb\|Z84386 anthranilate N-hydroxycinnamoyl/benzoyltransferase from Dianthus caryophyllus. | TC83737 | AW650590 | cLEI13B9 | VIIB6 | T2D11 | R14 | C4 | r4-c2 | r1-c3 |
| Phosphoglycerate dehydrogenase-like protein {Arabidopsis | TC83740 | AW650696 | cLEI13H4 | VIIB11 | T2D21 | R4 | C4 | r4-c2 | r1-c3 |

EP 1 454 993 A1

120

| | | | | | | | | | |
|---|---|---|---|---|---|---|---|---|---|
| thaliana}GP\|7270370\|emb\|CAB80137.1\|\|AL1615 | | | | | | | | | |
| phosphoribosyl pyrophosphate synthase isozyme 4 {Spinacia oleracea} | TC83753 | AW623660 | cTOB11L23 | XVIG6 | T4N12 | R13 | C14 | r2-c2 | r1-c4 |
| putative dihydroflavonol reductase {Oryza sativa} | TC83761 | AW623558 | cTOB11G1 | XVIG2 | T4N4 | R21 | C14 | r2-c2 | r1-c4 |
| hexose transporter | TC83763 | AI776698 | cLER19B17 | IXH11 | T3O21 | R4 | C15 | r2-c1 | r4-c3 |
| tyrosine/dopa decarboxylase {Thalictrum flavum subsp. glaucum} | TC83804 | AW617134 | cLHT21B22 | XVA12 | T4B23 | R2 | C2 | r2-c2 | r1-c4 |
| Cytochrom P450-like protein {Arabidopsis thaliana}PIR\|T46159\|T46159 cytochrome P450-like protein - | TC83813 | AW617348 | cLHT22P7 | XVB2 | T4D3 | R22 | C4 | r2-c2 | r1-c4 |
| glycolate oxidase {Arabidopsis thaliana} | TC83832 | BE449563 | cLHT31P20 | XVC6 | T4F11 | R14 | C6 | r2-c2 | r1-c4 |
| alpha-glucan phosphorylase, h isozyme phosphorylase h) {Solanum tuberosum}SP\|P32811\|PHSH_SOLTU ALPH | TC83865 | AI775729 | cLER16K20 | IXG8 | T3M15 | R12 | C13 | r2-c1 | r4-c3 |
| CYTOCHROME P450 98A3 (EC 1.14.-.-).GP\|2623303\|gb\|AAB86449.1\|\|AC002409 putative cytochrome P450 {Ara | TC83866 | AI775624 | cLER16G13 | IXG5 | T3M9 | R16 | C13 | r2-c1 | r4-c3 |
| general negative transcription regulator-like {Arabidopsis thaliana} | TC83872 | BG139930 | cLPP15E1 | XVD11 | T4H21 | R4 | C8 | r2-c2 | r1-c4 |
| Dof zinc finger protein {Nicotiana tabacum}PIR\|T02203\|T02203 finger protein Dof - common tobacco (f | TC83881 | AI894749 | cLEC5D8 | IID11 | T1G22 | R3 | C7 | r1-c2 | r3-c3 |
| putative ABC transporter {Arabidopsis thaliana}GP\|4115931\|gb\|AAD03441.1\|\|AF118223 contains similari | TC83901 | AW930474 | cLEF42N1 | IVF11 | T1L22 | R3 | C12 | r1-c2 | r3-c3 |
| ornithine carbamoyltransferase; OCTase {Canavalia lineata}GP\|12003188\|gb\|AAG43481.1\|AF203688_1\|AF 20 | TC83905 | BG127833 | cTOF18A20 | XIXB7 | T5D13 | R12 | C4 | r3-c2 | r4-c4 |
| succinate dehydrogenase iron-protein subunit {Arabidopsis thaliana} | TC83937 | BG131824 | cTOE5K6 | XVIIIF10 | T5K20 | R5 | C11 | r3-c2 | r4-c4 |
| tyrosine aminotransferase {Arabidopsis thaliana} | TC83980 | AW737817 | cTOD4I20 | XVIIH3 | T5O5 | R20 | C15 | r3-c2 | r4-c4 |
| ferredoxin--nitrite reductase {Nicotiana tabacum}GP\|861067\|emb\|CAA46942.1\|\|X66147 ferredoxin--nitri | TC83986 | AI776320 | cLER18K1 | IXH9 | T3O17 | R8 | C15 | r2-c1 | r4-c3 |
| legumin-like protein {Arabidopsis thaliana}PIR\|H84687\|H84687 legumin-like protein [imported] - Arab | TC84014 | BE344407 | cLEY7I19 | XIVC10 | T4E20 | R5 | C5 | r2-c2 | r1-c4 |

121

| Description | TC | Accession | Col1 | Col2 | Col3 | Col4 | Col5 | Col6 | Col7 |
|---|---|---|---|---|---|---|---|---|---|
| MADS transcriptional factor; STMADS16 {Solanum tuberosum}PIR|T06995|T06995 probable MADS box transc | TC84038 | AW929235 | XVIIE7 | cTOC6J20 | T5I13 | R12 | C9 | r3-c2 | r4-c4 |
| amidophosphoribosyltransferase {Arabidopsis thaliana} | TC84044 | AI775377 | IXF9 | cLER15G24 | T3K17 | R8 | C11 | r2-c1 | r4-c3 |
| transketolase 1 {Capsicum annuum}PIR|T09541|T09541 transketolase (EC 2.2.1.1) TKT1 precursor, chlor | TC84048 | BF051161 | VIIIA4 | cLEM21D4 | T2B8 | R17 | C2 | r4-c2 | r1-c3 |
| UDP-GLUCOSE 4-EPIMERASE GEPI48 (EC 5.1.3.2) (GALACTOWALDENASE) (UDP- GALACTOSE 4-EPIMERASE) GP|3021 | TC84055 | AW221109 | IVD12 | cLEF12H9 | T1H24 | R1 | C8 | r1-c2 | r3-c3 |
| contains similarity to chorismate mutase-T and prephenate dehydrogenase~gene_id:MGG23.1 {Arabidopsis | TC84057 | BF051293 | VIIIB4 | cLEM22K9 | T2D8 | R17 | C4 | r4-c2 | r1-c3 |
| Strong similarity to gb|Z50851 HD-zip (athb-8) gene from Arabidopsis thaliana containing Homeobox PF | TC84068 | BF050867 | VIIH6 | cLEM19F10 | T2P11 | R14 | C16 | r4-c2 | r1-c3 |
| Is a member of the PF|00044 glyceraldehyde 3-phosphate dehydrogenase family. ESTs gb|T43985, gb|N38 | TC84078 | AW034065 | IIC4 | cLEC37K13 | T1E8 | R17 | C5 | r1-c2 | r3-c3 |
| putative hydroxymethylglutaryl-CoA lyase {Arabidopsis thaliana}PIR|T02655|T02655 hydroxymethylgluta | TC84085 | AW031371 | IID8 | cLEC40O17 | T1G16 | R9 | C7 | r1-c2 | r3-c3 |
| putative anthranilate N-hydroxycinnamoyl/benzoyltransferase {Arabidopsis thaliana}PIR|T00527|T00527 | TC84103 | AW217704 | XVIIE3 | cTOC6A19 | T5I5 | R20 | C9 | r3-c2 | r4-c4 |
| RING zinc finger protein-like {Arabidopsis thaliana} | TC84140 | AW649904 | VIIA10 | cLEI11E13 | T2B19 | R6 | C2 | r4-c2 | r1-c3 |
| CCAAT box binding factor/ transcription factor Hap2a {Arabidopsis thaliana}PIR|T49898|T49898 CCAAT | TC84198 | BF113081 | VIF12 | cLEG43E15 | T2K24 | R1 | C11 | r4-c2 | r1-c3 |
| MADS-box transcription factor FBP24 {Petunia x hybrida} | TC84232 | AI486443 | IVC6 | cLED8C18 | T1F12 | R13 | C6 | r1-c2 | r3-c3 |
| adenylosuccinate lyase-like protein; 104558-106845 {Arabidopsis thaliana}PIR|B86484|B86484 hypothet | TC84319 | BG134019 | XVIIIC10 | cTOE15E21 | T5E20 | R5 | C5 | r3-c2 | r4-c4 |
| pyruvate kinase-like protein {Arabidopsis thaliana}PIR|T47556|T47556 pyruvate kinase-like protein - | TC84320 | BG133998 | XVIIIC8 | cTOE15C1 | T5E16 | R9 | C5 | r3-c2 | r4-c4 |
| unnamed protein product {unidentified}GP|2462911|emb|CAB06081.1||Z83832 | TC84322 | BG133901 | XVIIIC3 | cTOE14B12 | T5E6 | R19 | C5 | r3-c2 | r4-c4 |

| UDP-glucose:sterol glucosyl | | | | | | | | |
|---|---|---|---|---|---|---|---|---|
| folylpolyglutamate synthase-like protein {Arabidopsis thaliana} | TC84330 | BG133283 | cTOE11N8 | XVIIIB11 | T5C22 | R3 | C3 | r3-c2 | r4-c4 |
| dihydroxy-acid dehydratase {Arabidopsis thaliana} | TC84340 | BG134687 | cTOE17J11 | XVIIID4 | T5G8 | R17 | C7 | r3-c2 | r4-c4 |
| CTP synthase like protein {Arabidopsis thaliana}GP|7268827|emb|CAB79032.1||AL161552 CTP synthase li | TC84368 | AW980043 | cLEW8A6 | XIIIH11 | T3P22 | R3 | C16 | r2-c1 | r4-c3 |
| Putative acyl-CoA:1-acylglycerol-3-phosphate acyltransferase {Arabidopsis thaliana}PIR|D96550|D965 | TC84467 | BG135571 | cTOE23A11 | XVIIIE7 | T5I14 | R11 | C9 | r3-c2 | r4-c4 |
| putative gluconokinase {Arabidopsis thaliana}PIR|C84544|C84544 probable gluconokinase [imported] - | TC84507 | AI773430 | cLER7C7 | XC5 | T3E10 | R15 | C5 | r2-c1 | r4-c3 |
| Strong similarity to F19I3.2 GP|3033375 putative berberine bridge enzyme from Arabidopsis thaliana B | TC84522 | AI772745 | cLER3P15 | XB9 | T3C18 | R7 | C3 | r2-c1 | r4-c3 |
| homeodomain-leucine zipper protein 57 {Glycine max} | TC84550 | AW398295 | cLPT2L23 | XVIA4 | T4B8 | R17 | C2 | r2-c2 | r1-c4 |
| transcription factor {Nicotiana tabacum} | TC84557 | BG642494 | cTOD11G2 | XVIIG1 | T5M1 | R24 | C13 | r3-c2 | r4-c4 |
| carbamoyl-phosphate synthetase small subunit {Arabidopsis thaliana} | TC84578 | AW738069 | cTOD5K3 | XVIIH10 | T5O19 | R6 | C15 | r3-c2 | r4-c4 |
| hexose transporter^^hexose transporter protein^^pathogenesis-related protein P4^^pathogenesis-related protein PR1a (P4) | TC71791 | BG134154 | cTOE15O14 | XVIIIC11 | T5E22 | R3 | C5 | r3-c2 | r4-c4 |
| 33kDa precursor protein of oxygen-evolving complex | TC71796 | BG128633 | cTOF21H21 | XIXE3 | T5J5 | R20 | C10 | r3-c2 | r4-c4 |
| ADP-glucose pyrophosphorylase small subunit | TC71797 | AI781653 | cLES16N17 | XF2 | T3K4 | R21 | C11 | r2-c1 | r4-c3 |
| glutamine synthetase | TC71798 | AI490202 | cLER1O9 | XA12 | T3A24 | R1 | C1 | r2-c1 | r4-c3 |
| cytochrome P450 like_TBP {Nicotiana tabacum}GP|1545805|dbj|BAA10929.1||D64052 cytochrome P450 like_ | TC71805 | BG138584 | cLPP10E22 | XVD2 | T4H3 | R22 | C8 | r2-c2 | r1-c4 |
| fructose-bisphosphate aldolase {Persea americana} | TC71818 | AI774641 | cLER12N18 | IXE9 | T3I17 | R8 | C9 | r2-c1 | r4-c3 |
| plastidic aldolase NPALDP1 {Nicotiana paniculata} | TC71821 | BE433579 | cLEG16G20 | VE11 | T2I21 | R4 | C9 | r4-c2 | r1-c3 |
| glutamate decarboxylase {Petunia hybrida}SP|Q07346|DCE_PETHY GLUTAMATE DECARBOXYLASE (EC 4.1.1.15) | TC71841 | AI776639 | cLER19E4 | XA1 | T3A2 | R23 | C1 | r2-c1 | r4-c3 |
| phenylalanine ammonia lyase | TC71847 | AW034774 | cLEC32C18 | IH7 | T1O13 | R12 | C15 | r1-c2 | r3-c3 |

| | | | | | | | | | |
|---|---|---|---|---|---|---|---|---|---|
| glutamate decarboxylase isozyme 1 {Nicotiana tabacum} | TC71868 | BG138366 | cLPP9O23 | XVF8 | T4L15 | R12 | C12 | r2-c2 | r1-c4 |
| plastidic aldolase {Nicotiana paniculata} | TC71875 | AI782836 | cLES20N20 | XG10 | T3M20 | R5 | C13 | r2-c1 | r4-c3 |
| fructose-bisphosphate aldolase {Persea americana} | TC71877 | AW650010 | cLEI11M24 | VA7 | T2A13 | R12 | C1 | r4-c2 | r1-c3 |
| ADENINE PHOSPHORIBOSYLTRANSFERASE 1 (EC 2.4.2.7) (APRT).GP|16164|emb|CAA41497.1||X58640 adenine ph | TC71878 | BG123164 | cTOF1E19 | XIXD1 | T5H1 | R24 | C8 | r3-c2 | r4-c4 |
| ribulose 1,5-bisphosphate carboxylase/oxygenase small subunit^^ribulose 1,5-bisphosphate carboxylase/oxyenase | TC71908 | AW217485 | cTOB1E19 | XVIIA9 | T5A17 | R8 | C1 | r3-c2 | r4-c4 |
| ribulose-1,5-bisphosphate carboxylase, small subunit precursor^^ribulose 1,5-bisphosphate carboxylase/oxygenase^^ribulose-1,5-bisphophate carboxylase/ oxygenase small subunit | TC71912 | AI782070 | cLES18C15 | XF7 | T3K14 | R11 | C11 | r2-c1 | r4-c3 |
| phosphoribosyl diphosphate synthase {Arabidopsis thaliana}GP|4512664|gb|AAD21718.1||AC006931 putati | TC71917 | AW442702 | cLEM8K21 | XIIA1 | T3B2 | R23 | C2 | r2-c1 | r4-c3 |
| glutamate dehydrogenase | TC71919 | AI896071 | cLEC13D4 | IC9 | T1E17 | R8 | C5 | r1-c2 | r3-c3 |
| ribulose bisphosphate carboxylase small subunit 1 precursor {Lycopersicon esculentum}SP|P08706|RBS1 | TC71926 | AI774466 | cLER12M6 | IXE8 | T3I15 | R12 | C9 | r2-c1 | r4-c3 |
| plastidic aldolase NPALDP1 {Nicotiana paniculata} | TC71931 | BE432437 | cLEG8C18 | VIH10 | T2O20 | R5 | C15 | r4-c2 | r1-c3 |
| spermine synthase 1 {Datura stramonium}SP|Q96556|SPE1_DATST SPERMIDINE SYNTHASE 1 (EC 2.5.1.16) (PU | TC71984 | AW622878 | cTOB5N16 | XVIIB6 | T5C11 | R14 | C3 | r3-c2 | r4-c4 |
| glutamine synthetase {Lycopersicon esculentum} | TC71994 | BF098111 | cLEW25D9 | XIIG8 | T3N16 | R9 | C14 | r2-c1 | r4-c3 |
| glutamate decarboxylase isozyme 1 {Nicotiana tabacum} | TC71998 | AW625743 | cLEZ16B12 | XIVD9 | T4G18 | R7 | C7 | r2-c2 | r1-c4 |
| beta-fructosidase | TC72004 | AI894871 | cLEC6K3 | IIE9 | T1I18 | R7 | C9 | r1-c2 | r3-c3 |
| acid invertase, AI {EC 3.2.1.26} [Lycopersicon esculentum=tomatoes, cv. Super First, fruits, Peptide, 636 aa]^^vacuolar invertase precursor^^beta-fructofuranosidase | TC72005 | BF051964 | cLEM24F2 | VIIIC4 | T2F8 | R17 | C6 | r4-c2 | r1-c3 |
| beta-fructofuranosidase precursor {Lycopersicon esculentum}SP|P29000|INVA_LYCES ACID BETA-FRUCTOFUR | TC72006 | BE431858 | cLEG4M7 | VIH2 | T2O4 | R21 | C15 | r4-c2 | r1-c3 |
| beta-fructofuranosidase precursor {Lycopersicon | TC72007 | BE432922 | cLEG10J17 | VD5 | T2G9 | R16 | C7 | r4-c2 | r1-c3 |

124

| Description | TC | Accession | Clone | | | R | C | | |
|---|---|---|---|---|---|---|---|---|---|
| esculentum}SP\|P29000\|INVA_LYCES ACID BETA-FRUCTOFUR enolase | TC72015 | BE460442 | cLEG29K14 | VH11 | T2O21 | R4 | C15 | r4-c2 | r1-c3 |
| threonine deaminase | TC72016 | BE459391 | cLEM6N9 | VIIIE6 | T2J12 | R13 | C10 | r4-c2 | r1-c3 |
| ubiquinol--cytochrome-c reductase (EC 1.10.2.2) Rieske iron-sulfur protein - potato | TC72021 | AW033888 | cLEC32O14 | IVG8 | T1N16 | R9 | C14 | r1-c2 | r3-c3 |
| polyphenol oxidase precursor | TC72054 | AW033927 | cLEC37G14 | IIC1 | T1E2 | R23 | C5 | r1-c2 | r3-c3 |
| polyphenol oxidase precursor | TC72055 | AW626331 | cLEZ19D8 | XIVF4 | T4K8 | R17 | C11 | r2-c2 | r1-c4 |
| polyphenol oxidase precursor | TC72056 | AI897921 | cLED3I15 | XVID1 | T4H2 | R23 | C8 | r2-c2 | r1-c4 |
| polyphenoloxidase, P2 [Lycopersicon esculentum=tomatoes, cv Tiny Tim LA154, flowers, Peptide Chloroplast, 587 aa]^polyphenol oxidase precursor | TC72057 | AI773100 | cLER5I14 | XVIB7 | T4D14 | R11 | C4 | r2-c2 | r1-c4 |
| vacuolar ATP synthase subunit b isoform 1 subunit) {Gossypium hirsutum}SP\|Q43432\|VAT1_GOSHI VACUOLA | TC72080 | AI487183 | cLED9M8 | IVD1 | T1H2 | R23 | C8 | r1-c2 | r3-c3 |
| cytochrome p450 lxxvia2 {Solanum melongena}SP\|P37122\|C762_SOLME CYTOCHROME P450 76A2 (EC 1.14.-.-) | TC72085 | AI895030 | cLEC6H17 | IIE8 | T1I16 | R9 | C9 | r1-c2 | r3-c3 |
| ethylene-responsive methionine synthase | TC72099 | AW429057 | cTOA1I20 | XVIE9 | T4J18 | R7 | C10 | r2-c2 | r1-c4 |
| cinnamic acid 4-hydroxylase {Capsicum annuum} | TC72100 | BE435434 | cLEG26P11 | VG11 | T2M21 | R4 | C13 | r4-c2 | r1-c3 |
| cinnamic acid 4-hydroxylase {Capsicum chinense} | TC72101 | BE435368 | cLEG26M11 | IB4 | T1C7 | R18 | C3 | r1-c2 | r3-c3 |
| glyceraldehyde 3-phosphate dehydrogenase b precursor, chloroplast {Pisum sativum} SP\|P12859\|G3PB_PEA | TC72118 | AW039016 | cLET12C15 | XIC7 | T3F13 | R12 | C6 | r2-c1 | r4-c3 |
| glyceraldehyde 3-phosphate dehydrogenase b precursor, chloroplast {Pisum sativum} SP\|P12859\|G3PB_PEA | TC72119 | AI775099 | cLER14D17 | IXF3 | T3K5 | R20 | C11 | r2-c1 | r4-c3 |
| 4-hydroxyphenylpyruvate dioxygenase {Solenostemon scutellarioides} | TC72120 | BE460965 | cLEG37G9 | VID10 | T2G20 | R5 | C7 | r4-c2 | r1-c3 |
| 4-hydroxyphenylpyruvate dioxygenase {Solenostemon scutellarioides} | TC72121 | AW035977 | cLEC33J10 | IIA3 | T1A6 | R19 | C1 | r1-c2 | r3-c3 |
| obtusifoliol 14-alpha-demethylase {Triticum aestivum}SP\|P93596\|CP51_WHEAT CYTOCHROME P450 51 (EC 1. | TC72126 | AI773859 | cLER8M8 | XVIIA12 | T5A23 | R2 | C1 | r3-c2 | r4-c4 |
| fructokinase 1 {Arabidopsis | TC72131 | BG139399 | cLPP13C23 | XVD6 | T4H11 | R14 | C8 | r2-c2 | r1-c4 |

| | | | | | | | | | |
|---|---|---|---|---|---|---|---|---|---|
| thaliana}GP\|13878053\|gb\|AAK44104.1\|AF370289_1\|AF370289 putative fructok | | | | | | | | | |
| beta-glucosidase {Arabidopsis thaliana} | TC72139 | AW651266 | cLEI16I11 | VIID1 | T2H1 | R24 | C8 | r4-c2 | r1-c3 |
| AP2 domain containing protein {Prunus armeniaca} | TC72156 | AW441232 | cLEN13E14 | VIIIH4 | T2P8 | R17 | C16 | r4-c2 | r1-c3 |
| leucine zipper-containing protein AT103 {Arabidopsis thaliana}PIR\|T47754\|T47754 leucine zipper-cont | TC72159 | BG124441 | cTOF5C10 | XXE10 | T5J20 | R5 | C10 | r3-c2 | r4-c4 |
| proline oxidase precursor {Arabidopsis thaliana} | TC72165 | cLED18A19 | cLED18A19 | IVB4 | T1D8 | R17 | C4 | r1-c2 | r3-c3 |
| homeobox | TC72179 | AW441945 | cLEN19I1 | IXB9 | T3C17 | R8 | C3 | r2-c1 | r4-c3 |
| cytosolic aconitase {Nicotiana tabacum} | TC72186 | AI775579 | cLER15P4 | IXG1 | T3M1 | R24 | C13 | r2-c1 | r4-c3 |
| cytosolic aconitase {Nicotiana tabacum} | TC72187 | AW040810 | cLET10D11 | XIC1 | T3F1 | R24 | C6 | r2-c1 | r4-c3 |
| hypothetical Cys-3-His zinc finger protein {Arabidopsis thaliana}GP\|6598933\|gb\|AAF18728.1\|AC018721_ | TC72194 | BE434577 | cLEG18C23 | VF8 | T2K15 | R12 | C11 | r4-c2 | r1-c3 |
| aminotransferase-like protein {Arabidopsis thaliana} | TC72199 | AW033908 | cLEC27G7 | IG1 | T1M1 | R24 | C13 | r1-c2 | r3-c3 |
| PROBABLE VACUOLAR ATP SYNTHASE SUBUNIT D 2 (EC 3.6.1.34) (V-ATPASE D SUBUNIT 2) (VACUOLAR PROTON PUM | TC72206 | BE436940 | cLEG34P7 | VIC5 | T2E10 | R15 | C5 | r4-c2 | r1-c3 |
| malate dehydrogenase, glyoxysomal precursor {Citrullus vulgaris}EGAD\|130842\|139627 glyoxysomal mala | TC72213 | BG123651 | cTOF2L11 | XXB8 | T5D16 | R9 | C4 | r3-c2 | r4-c4 |
| w-3 desaturase {Solanum tuberosum}PIR\|T07685\|T07685 omega-3 fatty acid desaturase (EC 1.14.99.-) - | TC72222 | BE436518 | cLEG33A5 | VIB5 | T2C10 | R15 | C3 | r4-c2 | r1-c3 |
| ATP synthase gamma subunit, mitochondrial precursor {Ipomoea batatas}SP\|P26360\|ATP3_IPOBA ATP SYNTH | TC72225 | BE459571 | cLEM7M13 | VIIIE12 | T2J24 | R1 | C10 | r4-c2 | r1-c3 |
| nucleoside diphosphate kinase | TC72228 | AW621401 | cLEX11J10 | XIIIB3 | T4C5 | R20 | C3 | r2-c2 | r1-c4 |
| glutamine synthetase | TC72235 | BG124516 | cTOF5D22 | XXE11 | T5J22 | R3 | C10 | r3-c2 | r4-c4 |
| sulfite reductase {Nicotiana tabacum}GP\|3721540\|dbj\|BAA33531.1\|\|D83583 Sulfite Reductase {Nicotiana | TC72279 | BE458560 | cLEM2D21 | VIIID1 | T2H2 | R23 | C8 | r4-c2 | r1-c3 |
| ALTERNATIVE OXIDASE 1 PRECURSOR (EC 1.-.--).GP\|558054\|gb\|AAC60576.1\|\|S71335 alternative oxidase, A | TC72280 | BG136312 | cLPP2C18 | XVE7 | T4J13 | R12 | C10 | r2-c2 | r1-c4 |
| homologous to glucosyltransferases | TC72286 | AI898951 | cLED36O17 | IIIH3 | T1P5 | R20 | C16 | r1-c2 | r3-c3 |
| CYTOCHROME P450 81E1 (EC 1.14.-.-) | TC72288 | AW034115 | cLEC33I16 | XVA10 | T4B19 | R6 | C2 | r2-c2 | r1-c4 |

126

| | | | | | | | | | |
|---|---|---|---|---|---|---|---|---|---|
| (ISOFLAVONE 2'-HYDROXYLASE) (P450 91A4) (CYP GE-3).GP|2443348|db | | | | | | | | | |
| UDP-GLUCOSE 4-EPIMERASE (EC 5.1.3.2) (GALACTOWALDENASE) (UDP- GALACTOSE 4-EPIMERASE).GP|8698725|gb| | TC72291 | AW219913 | cLEX6I21 | XIIIF1 | T4K1 | R24 | C11 | r2-c2 | r1-c4 |
| transaldolase | TC72292 | AW621229 | cLEX11O7 | XIIIB7 | T4C13 | R12 | C3 | r2-c2 | r1-c4 |
| transcription factor {Vicia faba}GP|2104681|emb|CAA66481.1||X97907 transcription factor {Vicia faba | TC72300 | BE435913 | cLEG30M14 | VIA4 | T2A8 | R17 | C1 | r4-c2 | r1-c3 |
| putative CONSTANS-like B-box zinc finger protein {Arabidopsis thaliana}PIR|A84720|A84720 hypothetic | TC72313 | BG129862 | cTOF28H9 | XXA10 | T5B20 | R5 | C2 | r3-c2 | r4-c4 |
| glucose-6-phosphate 1-dehydrogenase {Solanum tuberosum}SP|P37830|G6PD_SOLTU GLUCOSE-6-PHOSPHATE 1-D | TC72317 | AW929634 | cTOC9N19 | XIIIA4 | T4A7 | R18 | C1 | r2-c2 | r1-c4 |
| glucose-6-phosphate 1-dehydrogenase {Solanum tuberosum}SP|P37830|G6PD_SOLTU GLUCOSE-6-PHOSPHATE 1-D | TC72318 | AI894720 | cLEC5K16 | IIE1 | T1I2 | R23 | C9 | r1-c2 | r3-c3 |
| pyruvate kinase-like protein {Arabidopsis thaliana}PIR|T47556|T47556 pyruvate kinase-like protein - | TC72325 | BG135918 | cLPP1E21 | XVE4 | T4J7 | R18 | C10 | r2-c2 | r1-c4 |
| beta-amylase {Prunus armeniaca} | TC72330 | BG136584 | cLPP2H4 | VB8 | T2C15 | R12 | C3 | r4-c2 | r1-c3 |
| glucose-6-phosphate isomerase, cytosolic 1 (gpi) (phosphoglucose isomerase) (pgi) (phosphohexose iso | TC72335 | BG129054 | cTOF23M19 | XIXG2 | T5N3 | R22 | C14 | r3-c2 | r4-c4 |
| AP2 domain-containing transcription factor {Nicotiana tabacum} | TC72337 | AW221854 | cLEN4I21 | IXC11 | T3E21 | R4 | C5 | r2-c1 | r4-c3 |
| pyruvate dehydrogenase E1 beta subunit isoform 1 {Zea mays} | TC72349 | BE431827 | cLEG4E1 | VIG12 | T2M24 | R1 | C13 | r4-c2 | r1-c3 |
| fructose-1,6-bisphosphatase, cytosolic bisphosphate 1-phosphohydrolase) (fbpase) (cy-f1) {Solanum tu | TC72350 | BG127847 | cTOF18E4 | XIXB10 | T5D19 | R6 | C4 | r3-c2 | r4-c4 |
| fructose-1,6-bisphosphatase, cytosolic bisphosphate 1-phosphohydrolase) (fbpase) (cy-f1) {Solanum tu | TC72351 | BG123415 | cTOF1J18 | XIXD5 | T5H9 | R16 | C8 | r3-c2 | r4-c4 |
| isopentenyl diphosphate isomerase 1 {Nicotiana tabacum} | TC72352 | AW616688 | cLHT12K1 | XVA1 | T4B1 | R24 | C2 | r2-c2 | r1-c4 |
| putative glucose regulated repressor protein {Arabidopsis thaliana}PIR|A84649|A84649 probable gluco | TC72370 | AW455273 | cLEX10M20 | XIIIA9 | T4A17 | R8 | C1 | r2-c2 | r1-c4 |
| pyruvate dehydrogenase E1 alpha subunit {Arabidopsis | TC72372 | AW039459 | cLET10E22 | XIC2 | T3F3 | R22 | C6 | r2-c1 | r4-c3 |

EP 1 454 993 A1

| | | | | | | | | |
|---|---|---|---|---|---|---|---|---|
| thaliana} | | | | | | | | |
| PYROPHOSPHATE--FRUCTOSE 6-PHOSPHATE 1-PHOSPHOTRANSFERASE BETA SUBUNIT (EC 2.7.1.90) (PFP) (6-PHOSPHO | TC72375 | BF051072 | cLEM21B13 | VIIH12 | T2P23 | R2 | C16 | r4-c2 | r1-c3 |
| transketolase, chloroplast precursor {Solanum tuberosum}SP|Q43848|TKTC_SOLTU TRANSKETOLASE, CHLOROP | TC72376 | BG128738 | cTOF21N8 | VIIG5 | T2N9 | R16 | C14 | r4-c2 | r1-c3 |
| glutamyl-tRNA synthetase {Arabidopsis thaliana}PIR|T52043|T52043 probable glutamate--tRNA ligase (E | TC72377 | BE461031 | cLEG37E10 | VID9 | T2G18 | R7 | C7 | r4-c2 | r1-c3 |
| URIDYLATE KINASE (EC 2.7.4.-) (UK) (URIDINE MONOPHOSPHATE KINASE) (UMP KINASE) (UMP/CMP KINASE).GP| | TC72400 | BE458716 | cLEM3K4 | VIIID5 | T2H10 | R15 | C8 | r4-c2 | r1-c3 |
| asparagine synthetase {Triphysaria versicolor}GP|2429282|gb|AAD05034.1||AF014056 asparagine synthet | TC72402 | AI487482 | cLED11G10 | IIG10 | T1M20 | R5 | C13 | r1-c2 | r3-c3 |
| cytochrome p450 lxxii hydroxylase) (ge10h) {Catharanthus roseus}SP|Q05047|CP72_CATRO CYTOCHROME P45 | TC72403 | BE459772 | cLEM8C1 | VIIIF2 | T2L4 | R21 | C12 | r4-c2 | r1-c3 |
| cytochrome P450 {Arabidopsis thaliana} | TC72404 | AW650317 | cLEI12N11 | IIB10 | T1C20 | R5 | C3 | r1-c2 | r3-c3 |
| VACUOLAR ATP SYNTHASE SUBUNIT C (EC 3.6.1.34) (V-ATPASE C SUBUNIT) (VACUOLAR PROTON PUMP C SUBUNIT). | TC72410 | BG643163 | cTOF26D14 | XIIE10 | T3J20 | R5 | C10 | r2-c1 | r4-c3 |
| phosphoenolpyruvate carboxylase 2 | TC72426 | BE459547 | cLEM7I13 | VC3 | T2E5 | R20 | C5 | r4-c2 | r1-c3 |
| lipoxygenase^^loxc homologue | TC72430 | AW442155 | cLEN21F5 | IXC1 | T3E1 | R24 | C5 | r2-c1 | r4-c3 |
| lipoxygenase^^loxc homologue | TC72431 | AW738558 | cTOD7B10 | XVIIIA4 | T5A8 | R17 | C1 | r3-c2 | r4-c4 |
| succinyl-CoA synthetase, alpha subunit {Arabidopsis thaliana} | TC72435 | AW224006 | cLEN14M4 | XIIA6 | T3B12 | R13 | C2 | r2-c1 | r4-c3 |
| VACUOLAR ATP SYNTHASE SUBUNIT D (EC 3.6.1.34) (V-ATPASE D SUBUNIT) (VACUOLAR PROTON PUMP D SUBUNIT). | TC72437 | AW624445 | cTOB15F12 | XVIIA2 | T5A3 | R22 | C1 | r3-c2 | r4-c4 |
| NADPH-cytochrome P450 oxidoreductase (EC 1.-.-.-) - common tobacco | TC72444 | BE354227 | cTOD9L11 | IIF12 | T1F24 | R1 | C6 | r1-c2 | r3-c3 |
| ATP synthase beta subunit | TC72461 | BE449406 | cLHT31M12 | XVC3 | T4F5 | R20 | C6 | r2-c2 | r1-c4 |
| putative sulfite oxidase {Arabidopsis thaliana}GP|6513940|gb|AAF14844.1|AC011664_26|A | TC72463 | BE431431 | cLEG1G5 | VF11 | T2K21 | R4 | C11 | r4-c2 | r1-c3 |

| C011664 sulfit | | | | | | | | |
|---|---|---|---|---|---|---|---|---|---|
| putative homeodomain transcription factor {Arabidopsis thaliana}PIR\|F84565\|F84565 probable homeodom | TC72471 | BE436986 | cLEG35I11 | VIC6 | T2E12 | R13 | C5 | r4-c2 | r1-c3 |
| NADP-dependent glyceraldehyde-3-phosphate dehydrogenase (non-phosphorylating glyceraldehyde 3-phosph | TC72481 | AW616527 | cLHT11J5 | XIVH10 | T4O20 | R5 | C15 | r2-c2 | r1-c4 |
| WRKY transcription factor Nt-SubD48 {Nicotiana tabacum} | TC72483 | AI487657 | cLED13K15 | IIH8 | T1O16 | R9 | C15 | r1-c2 | r3-c3 |
| putative anthocyanin 5-aromatic acyltransferase {Arabidopsis thaliana}PIR\|G84823\|G84823 probable an | TC72484 | AW625642 | cLEZ16I6 | XIVE2 | T4I4 | R21 | C9 | r2-c2 | r1-c4 |
| pyruvate dehydrogenase E1 beta subunit isoform 2 {Zea mays} | TC72498 | AW096554 | cLET38N6 | XIH1 | T3P1 | R24 | C16 | r2-c1 | r4-c3 |
| S-adenosylmethionine decarboxylase {Nicotiana tabacum}PIR\|T01934\|T01934 adenosylmethionine decarbox | TC72506 | AW224365 | cLEN16L22 | IXA8 | T3A15 | R12 | C1 | r2-c1 | r4-c3 |
| NADH-UBIQUINONE OXIDOREDUCTASE 24 KDA SUBUNIT PRECURSOR (EC 1.6.5.3) (EC 1.6.99.3).GP\|7269018\|emb\|C | TC72512 | AI779124 | cLES7M17 | XIA10 | T3B19 | R6 | C2 | r2-c1 | r4-c3 |
| phosphate/phosphoenolpyruvate translocator precursor {Nicotiana tabacum}GP\|1778145\|gb\|AAB40648.1\|\|U | TC72515 | BG132441 | cTOE7J1 | XVIIG3 | T5M5 | R20 | C13 | r3-c2 | r4-c4 |
| putative glucosyltransferase {Arabidopsis thaliana}PIR\|H84870\|H84870 probable glucosyltransferase [ | TC72520 | AW035575 | cLEC39D16 | IIC11 | T1E22 | R3 | C5 | r1-c2 | r3-c3 |
| pyruvate dehydrogenase | TC72539 | BF096522 | cLEW12E7 | XIID8 | T3H16 | R9 | C8 | r2-c1 | r4-c3 |
| zinc finger transcription factor-like protein {Arabidopsis thaliana}PIR\|T49899\|T49899 zinc finger t | TC72540 | AW621257 | cLEX11K20 | XIIIB5 | T4C9 | R16 | C3 | r2-c2 | r1-c4 |
| flavanone 3-hydroxylase-like protein {Arabidopsis thaliana} | TC72542 | BG133370 | cTOE12M9 | XVIIIB12 | T5C24 | R1 | C3 | r3-c2 | r4-c4 |
| gamma-glutamylcysteine synthetase | TC72560 | BE432272 | cLEG7C8 | VIH6 | T2O12 | R13 | C15 | r4-c2 | r1-c3 |
| transcription initiation factor iib (tfiib) {Glycine max}SP\|P48513\|TF2B_SOYBN TRANSCRIPTION INITIAT | TC72566 | AI775379 | cLER15I4 | IXF10 | T3K19 | R6 | C11 | r2-c1 | r4-c3 |
| dihydroxy-acid dehydratase {Arabidopsis thaliana} | TC72576 | BE459380 | cLEM6L1 | VIIIE4 | T2J8 | R17 | C10 | r4-c2 | r1-c3 |
| arginine methyltransferase (pam1) {Arabidopsis thaliana}GP\|7269850\|emb\|CAB79709.1\|\|AL161575 arginin | TC72613 | AI486209 | cLED5L13 | IVA12 | T1B24 | R1 | C2 | r1-c2 | r3-c3 |
| N-hydroxycinnamoyl/benzoyltransferase {Ipomoea | TC72632 | AI777857 | cLES3C10 | XH2 | T3O4 | R21 | C15 | r2-c1 | r4-c3 |

| | | | | | | | | | |
|---|---|---|---|---|---|---|---|---|---|
| batatas} | | | | | | | | | |
| similar to ATPases associated with various cellular activites (Pfam: AAA.hmm, score: 230.91) {Arabid | TC72650 | AW035993 | cLEC33L22 | IIA5 | T1A10 | R15 | C1 | r1-c2 | r3-c3 |
| putative monosaccharide transporter 1 {Petunia x hybrida} | TC72655 | BG140550 | cLPP17H17 | XVE2 | T4J3 | R22 | C10 | r2-c2 | r1-c4 |
| 78 kDa glucose regulated protein homolog 5 precursor (grp 78-5) (immunoglobulin heavy subunit bindin | TC72660 | AI775845 | cLER16B20 | IXG3 | T3M5 | R20 | C13 | r2-c1 | r4-c3 |
| threonine synthase {Solanum tuberosum} | TC72666 | BG643425 | cTOF27J7 | XXA4 | T5B8 | R17 | C2 | r3-c2 | r4-c4 |
| CONSTANS-like B-box zinc finger protein-like {Arabidopsis thaliana} | TC72668 | AI775819 | cLER16N1 3 | IXG10 | T3M19 | R6 | C13 | r2-c1 | r4-c3 |
| Identical to ribose-phosphate pyrophosphokinase 2 (phosphoribosyl pyrophosphate synthetase 2) (PRSII | TC72677 | AW651192 | cLEI15J16 | VIIC7 | T2F13 | R12 | C6 | r4-c2 | r1-c3 |
| NADH dehydrogenase {Solanum tuberosum}GP\|668987\|emb\|CAA59063.1\|\|X84320 NADH dehydrogenase {Solanum | TC72678 | AW041573 | cLET10A15 | XIB11 | T3D21 | R4 | C4 | r2-c1 | r4-c3 |
| Similar to gb\|Z84386 anthranilate N-hydroxycinnamoyl/benzoyltransferase from Dianthus caryophyllus. | TC72703 | AW616976 | cLHT19A1 4 | XVA8 | T4B15 | R12 | C2 | r2-c2 | r1-c4 |
| putative H+-transporting ATPase {Oryza sativa} | TC72705 | AW094623 | cLET29G16 | IXA4 | T3A7 | R18 | C1 | r2-c1 | r4-c3 |
| Similar to Populus balsamifera subsp. trichocarpa X Populus deltoides vegetative storage protein. (L | TC72708 | AW929488 | cTOC9G1 | XVIIF7 | T5K13 | R12 | C11 | r3-c2 | r4-c4 |
| cytochrome P450-dependent fatty acid hydroxylase {Vicia sativa} | TC72710 | BE433735 | cLEG20P11 | VG5 | T2M9 | R16 | C13 | r4-c2 | r1-c3 |
| anthocyanidin 3-O-glucosyltransferase {Petunia x hybrida} | TC72713 | AI488786 | cLED18F12 | IIIB7 | T1D13 | R12 | C4 | r1-c2 | r3-c3 |
| cytochrome p450 lxxia4 {Solanum melongena}SP\|P37117\|C714_SOLME CYTOCHROME P450 71A4 (EC 1.14.-.-) ( | TC72718 | BE431818 | cLEG4A21 | VIG10 | T2M20 | R5 | C13 | r4-c2 | r1-c3 |
| cytochrome p450 lxxia4 {Solanum melongena}SP\|P37117\|C714_SOLME CYTOCHROME P450 71A4 (EC 1.14.-.-) ( | TC72719 | AI487522 | cLED11B9 | IIG8 | T1M16 | R9 | C13 | r1-c2 | r3-c3 |
| phosphoenolpyruvate carboxykinase {Flaveria pringlei} | TC72722 | BE436435 | cLEG32K2 | VIB1 | T2C2 | R23 | C3 | r4-c2 | r1-c3 |
| phosphoribosyl pyrophosphate synthase {Spinacia oleracea} | TC72727 | AW622719 | cTOB4I14 | XVIB5 | T4D10 | R15 | C4 | r2-c2 | r1-c4 |
| dehydroquinate dehydratase/shikimate:NADP oxidoreductase | TC72770 | BF051225 | cLEM21P8 | VIIIA9 | T2B18 | R7 | C2 | r4-c2 | r1-c3 |
| AP2 domain containing protein {Prunus armeniaca} | TC72775 | BG132603 | cTOE8E22 | XVIIIG3 | T5M6 | R19 | C13 | r3-c2 | r4-c4 |

130

| | | | | | | | | | |
|---|---|---|---|---|---|---|---|---|---|
| putative phosphate/phosphoenolpyruvate translocator {Arabidopsis thaliana} | TC72794 | BF051658 | cLEM23F10 | IIH2 | T1O4 | R21 | C15 | r1-c2 | r3-c3 |
| SNAP25A protein {Arabidopsis thaliana}GP|5731763|emb|CAB52582.1||X92419 SNAP25A protein {Arabidopsi | TC72817 | AW041561 | cLET10A17 | XIB12 | T3D23 | R2 | C4 | r2-c1 | r4-c3 |
| heat shock transcription factor like protein {Arabidopsis thaliana}GP|2244754|emb|CAB10177.1||Z9733 | TC72821 | AI897308 | cLED26B20 | IIID7 | T1H13 | R12 | C8 | r1-c2 | r3-c3 |
| ADP-glucose pyrophosphorylase large subunit 1 | TC72843 | BG126269 | cTOF11F18 | XVIIIG11 | T5M22 | R3 | C13 | r3-c2 | r4-c4 |
| putative methylmalonate semi-aldehyde dehydrogenase {Arabidopsis thaliana}PIR|H84514|H84514 hypothe | TC72868 | BG643765 | cTOF32D7 | XXC9 | T5F18 | R7 | C6 | r3-c2 | r4-c4 |
| flavonol 3-o-glucosyltransferase 6 {Manihot esculenta}SP|Q40288|UFO6_MANES FLAVONOL 3-O-GLUCOSYLTRA | TC72874 | AW031872 | cLEC38K2 | IIC9 | T1E18 | R7 | C5 | r1-c2 | r3-c3 |
| Identical to gene ZW10 from Arabidopsis thaliana gb|AB028195 and is a member of the Phosphoglycerate | TC72889 | AW091993 | cLET17D3 | XID9 | T3H17 | R8 | C8 | r2-c1 | r4-c3 |
| coproporphyrinogen iii oxidase precursor (coproporphyrinogenase) (coprogen oxidase) {Nicotiana tabac | TC72896 | BG125496 | cTOF8H12 | XXG4 | T5N8 | R17 | C14 | r3-c2 | r4-c4 |
| zinc finger protein-like {Arabidopsis thaliana}GP|5006473|gb|AAD37511.1|AF139098_1|AF139098 putativ | TC72898 | AI484751 | cLED3B15 | IIIH9 | T1P17 | R8 | C16 | r1-c2 | r3-c3 |
| caffeoyl-CoA O-methyltransferase {Nicotiana tabacum}GP|1103487|emb|CAA91228.1||Z56282 caffeoyl-CoA | TC72904 | BG133660 | cTOE13D5 | VIIE4 | T2J7 | R18 | C10 | r4-c2 | r1-c3 |
| phosphoglycerate mutase {Solanum tuberosum} | TC72922 | AW648021 | cLEI3M7 | VIIE1 | T2J1 | R24 | C10 | r4-c2 | r1-c3 |
| acetyl-CoA C-acetyltransferase {Arabidopsis thaliana} | TC72945 | AW217996 | cTOD6E1 | VA5 | T2A9 | R16 | C1 | r4-c2 | r1-c3 |
| acetyl-CoA C-acetyltransferase {Arabidopsis thaliana} | TC72946 | BG126595 | cTOF12F6 | XVIIIH4 | T5O8 | R17 | C15 | r3-c2 | r4-c4 |
| putative NADH-ubiquinone oxireductase {Arabidopsis thaliana}PIR|C84588|C84588 probable NADH-ubiquin | TC72951 | BE431573 | cLEG27G1 | VG12 | T2M23 | R2 | C13 | r4-c2 | r1-c3 |
| acetyl-CoA C-acetyltransferase {Arabidopsis thaliana} | TC72956 | AI485611 | cLED6J18 | IVB9 | T1D18 | R7 | C4 | r1-c2 | r3-c3 |
| malonyl-CoA:ACP transacylase {Perilla frutescens} | TC72961 | AW648980 | cLEI6N11 | XVG1 | T4N1 | R24 | C14 | r2-c2 | r1-c4 |
| N-carbamyl-L-amino acid amidohydrolase-like protein {Arabidopsis thaliana} | TC72977 | AW649291 | cLEI7D16 | VIIF8 | T2L15 | R12 | C12 | r4-c2 | r1-c3 |
| uracil phosphoribosyltransferase {Nicotiana tabacum}SP|P93394|UPP_TOBAC URACIL PHOSPHORIBOSYLTRANSF | TC72985 | BE458242 | cLEM1J11 | VIIH10 | T2P19 | R6 | C16 | r4-c2 | r1-c3 |
| heat stress transcription factor A3 {Lycopersicon | TC72992 | AW035854 | cLEC36E8 | IIB9 | T1C18 | R7 | C3 | r1-c2 | r3-c3 |

EP 1 454 993 A1

| | | | | | | | | | |
|---|---|---|---|---|---|---|---|---|---|
| peruvianum} | | | | | | | | | |
| invertase-like protein {Arabidopsis thaliana}GP|7270437|emb|CAB80203.1||AL161586 invertase-like pro | TC73000 | BF114092 | cLEY24H5 | IVG6 | T1N12 | R13 | C14 | r1-c2 | r3-c3 |
| contains similarity to acyl-CoA thioesterase~gene_id:K23F3.9 {Arabidopsis thaliana} | TC73001 | BE432689 | cLEG9J24 | VIIA6 | T2B11 | R14 | C2 | r4-c2 | r1-c3 |
| acetylornithine aminotransferase precursor {Alnus glutinosa}SP|O04866|ARGD_ALNGL ACETYLORNITHINE AM | TC73014 | AW650016 | cLEI11O22 | VIIA12 | T2B23 | R2 | C2 | r4-c2 | r1-c3 |
| glucose-6-phosphate isomerase {Spinacia oleracea}PIR|T09153|T09153 glucose-6-phosphate isomerase (E | TC73016 | AW030975 | cLEC5G17 | IA10 | T1A19 | R6 | C1 | r1-c2 | r3-c3 |
| hydroxypyruvate reductase {Bruguiera gymnorhiza} | TC73027 | AW040337 | cLET5K21 | XIIB11 | T3D22 | R3 | C4 | r2-c1 | r4-c3 |
| PROTEIN-L-ISOASPARTATE O-METHYLTRANSFERASE (EC 2.1.1.77) (PROTEIN-BETA-ASPARTATE METHYLTRANSFERASE) | TC73037 | AW623004 | cTOB7N21 | XVIIB7 | T5C13 | R12 | C3 | r3-c2 | r4-c4 |
| putative anthocyanidin-3-glucoside rhamnosyltransferase {Arabidopsis thaliana}PIR|D84614|D84614 hyp | TC73055 | BE450766 | cLEY15C5 | XIIIH1 | T4O1 | R24 | C15 | r2-c2 | r1-c4 |
| putative RING-H2 zinc finger protein ATL6 {Arabidopsis thaliana} | TC73059 | BF051368 | cLEM22N1 | VIIIB5 | T2D10 | R15 | C4 | r4-c2 | r1-c3 |
| adenosine kinase {Arabidopsis thaliana}GP|7378610|emb|CAB83286.1||AL162751 adenosine kinase-like pr | TC73160 | BF113600 | cLEY21J1 | XIVB9 | T4C18 | R7 | C3 | r2-c2 | r1-c4 |
| putative PHD-type zinc finger protein {Arabidopsis thaliana}PIR|A84437|A84437 probable PHD-type zin | TC73164 | AI775153 | cLER14N11 | IXF6 | T3K11 | R14 | C11 | r2-c1 | r4-c3 |
| quinolinate phosphoribosyltransferase {Nicotiana tabacum} | TC73169 | AI894647 | cLEC4L14 | XIIA9 | T3B18 | R7 | C2 | r2-c1 | r4-c3 |
| 5-enolpyruvylshikimate-3-phosphate synthase precursor (EC 2.5.1.19) | TC73179 | AW041710 | cLET12G6 | XIC8 | T3F15 | R12 | C6 | r2-c1 | r4-c3 |
| transcription factor | TC73183 | AI897672 | cLED30E23 | IIIF1 | T1L1 | R24 | C12 | r1-c2 | r3-c3 |
| Dof zinc finger protein {Arabidopsis thaliana}GP|9280230|dbj|BAB01720.1||AB023045 Dof zinc finger p | TC73204 | AI897222 | cLED27I9 | IIIE1 | T1J1 | R24 | C10 | r1-c2 | r3-c3 |
| aldose 1-epimerase-like protein {Arabidopsis thaliana}PIR|T07719|T07719 aldose 1-epimerase homolog | TC73210 | AW441440 | cLEN17M15 | IXB2 | T3C3 | R22 | C3 | r2-c1 | r4-c3 |

132

| | | | | | | | | | |
|---|---|---|---|---|---|---|---|---|---|
| glucosyl transferase {Nicotiana tabacum}GP|1805359|dbj|BAA19155.1||AB000623 glucosyl transferase {N | TC73225 | AI487283 | cLED10H2 1 | IIG3 | T1M6 | R19 | C13 | r1-c2 | r3-c3 |
| unnamed protein product {unidentified} □GP|2462931|emb|CAB06082.1||Z83833 UDP-glucose:sterol glucosyl | TC73255 | AI486456 | cLED8C24 | IVC7 | T1F14 | R11 | C6 | r1-c2 | r3-c3 |
| homogentisate 1,2-dioxygenase | TC73262 | AW737196 | cTOD2I2 | XIIA11 | T3B22 | R3 | C2 | r2-c1 | r4-c3 |
| putative aspartate aminotransferase; 38163-36256 {Arabidopsis thaliana}PIR|C96835|C96835 hypothetic | TC73280 | AI488655 | cLED18H1 6 | IIIB9 | T1D17 | R8 | C4 | r1-c2 | r3-c3 |
| ATP synthase alpha chain {Vigna radiata} | TC73289 | BG125450 | cTOF8N13 | VIE5 | T2I10 | R15 | C9 | r4-c2 | r1-c3 |
| MYB-like DNA-binding protein {Catharanthus roseus} | TC73317 | AW039424 | cLET12C14 | XIC6 | T3F11 | R14 | C6 | r2-c1 | r4-c3 |
| CYTOCHROME P450 90A1 (EC 1.14.-.-).GP|853719|emb|CAA60793.1||X87367 CYP90 protein {Arabidopsis thal | TC73326 | AW648875 | cLEI6O5 | VIIF7 | T2L13 | R12 | C12 | r4-c2 | r1-c3 |
| vsf-1^^transcription factor VSF-1 | TC73332 | BE460903 | cLEG36J22 | VID1 | T2G2 | R23 | C7 | r4-c2 | r1-c3 |
| ATP synthase alpha subunit {Nicotiana tabacum}SP|P00823|ATPA_TOBAC ATP SYNTHASE ALPHA CHAIN (EC 3.6 | TC73341 | AI898491 | cLED34M3 | IIIG10 | T1N19 | R6 | C14 | r1-c2 | r3-c3 |
| isoflavone reductase homolog {Solanum tuberosum}SP|P52578|IFRH_SOLTU ISOFLAVONE REDUCTASE HOMOLOG ( | TC73357 | BG124289 | cTOF4F20 | XXE3 | T5J6 | R19 | C10 | r3-c2 | r4-c4 |
| fumarase {Solanum tuberosum}GP|1488652|emb|CAA62817.1||X91615 fumarase {Solanum tuberosum}PIR|T073 | TC73367 | AW034595 | cLEC11N1 1 | IC4 | T1E7 | R18 | C5 | r1-c2 | r3-c3 |
| ornithine carbamoyltransferase; OCTase {Canavalia lineata}GP|12003188|gb|AAG43481.1|AF203688_1|AF 20 | TC73370 | AI486421 | cLED5L18 | IVB1 | T1D2 | R23 | C4 | r1-c2 | r3-c3 |
| N-glyceraldehyde-2-phosphotransferase-like {Arabidopsis thaliana} | TC73374 | BG129565 | cTOF24P2 | XXG12 | T5N24 | R1 | C14 | r3-c2 | r4-c4 |
| putative cinnamyl alcohol dehydrogenase {Malus x domestica}PIR|T16995|T16995 probable cinnamyl-alco | TC73375 | BE437155 | cLEG35N3 | VIC9 | T2E18 | R7 | C5 | r4-c2 | r1-c3 |
| putative anthocyanidin-3-glucoside rhamnosyltransferase {Arabidopsis thaliana}PIR|D84614|D84614 hyp | TC73422 | AW031678 | cLEC37H1 9 | IIC2 | T1E4 | R21 | C5 | r1-c2 | r3-c3 |
| serine/threonine-specific protein kinase NAK {Arabidopsis thaliana}PIR|T48250|T48250 serine/threoni | TC73426 | AI773080 | cLER5E14 | XB12 | T3C24 | R1 | C3 | r2-c1 | r4-c3 |

| | | | | | | | | | |
|---|---|---|---|---|---|---|---|---|---|
| putative threonine dehydratase/deaminase {Oryza sativa} | TC73457 | AI897298 | cLED26P9 | IIID12 | T1H23 | R2 | C8 | r1-c2 | r3-c3 |
| pyruvate dehydrogenase kinase {Arabidopsis thaliana} | TC73458 | AI774213 | cLER11N2 4 | IXE5 | T3I9 | R16 | C9 | r2-c1 | r4-c3 |
| Cytochrome P450-like protein {Arabidopsis thaliana}GP|7270098|emb|CAB79912.1||AL161580 Cytochrome P | TC73461 | AW932471 | cLEF48M2 2 | IVH12 | T1P24 | R1 | C16 | r1-c2 | r3-c3 |
| putative arginine methyltransferase {Arabidopsis thaliana} | TC73476 | AW647829 | cLEI2M24 | VIID8 | T2H15 | R12 | C8 | r4-c2 | r1-c3 |
| glutamine synthetase {Nicotiana tabacum}GP|1419094|emb|CAA65173.1||X95932 glutamine synthetase {Nic | TC73479 | BE450846 | cLEY15G2 0 | XIIIH4 | T4O7 | R18 | C15 | r2-c2 | r1-c4 |
| glucosyl transferase {Nicotiana tabacum}GP|1805359|dbj|BAA19155.1||AB000623 glucosyl transferase {N | TC73487 | BE436515 | cLEG32P19 | VIB3 | T2C6 | R19 | C3 | r4-c2 | r1-c3 |
| putative RING zinc finger protein; 36546-35989 {Arabidopsis thaliana}GP|12323975|gb|AAG51946.1|AC01 | TC73490 | AW223330 | cLEN11H5 | VIIIG6 | T2N12 | R13 | C14 | r4-c2 | r1-c3 |
| cytochrome P450 {Nicotiana tabacum} | TC73493 | BG126018 | cTOF10P11 | IIF6 | T1K12 | R13 | C11 | r1-c2 | r3-c3 |
| PHOSPHOGLUCOMUTASE, CYTOPLASMIC (EC 5.4.2.2) (GLUCOSE PHOSPHOMUTASE) (PGM).GP|8250624|emb|CAB93681. | TC73495 | BF050298 | cLEM17E1 2 | XVH5 | T4P9 | R16 | C16 | r2-c2 | r1-c4 |
| starch phosphorylase (AA 1-966) {Solanum tuberosum} | TC73506 | AW934093 | cLEF57H6 | VC6 | T2E11 | R14 | C5 | r4-c2 | r1-c3 |
| alpha-glucan phosphorylase, l isozyme 1 precursor (starch phosphorylase l-1) {Solanum tuberosum}SP| | TC73507 | AW618336 | cLPT12G14 | XVG3 | T4N5 | R20 | C14 | r2-c2 | r1-c4 |
| acetyl-coA dehydrogenase, putative {Arabidopsis thaliana} | TC73523 | BF113496 | cLEY21I7 | XIVB8 | T4C16 | R9 | C3 | r2-c2 | r1-c4 |
| l-asparaginase (l-asparagine amidohydrolase) {Arabidopsis thaliana} | TC73526 | AW031986 | cLEC34N1 | IIA11 | T1A22 | R3 | C1 | r1-c2 | r3-c3 |
| PROBABLE PHOSPHORIBOSYLFORMYLGLYCINAMIDINE SYNTHASE, CHLOROPLAST PRECURSOR (EC 6.3.5.3) (FGAM SYNTHA | TC73527 | AW223040 | cLEN10E2 | VIIIG2 | T2N4 | R21 | C14 | r4-c2 | r1-c3 |
| Putative phospholipid cytidylyltransferase {Oryza sativa} | TC73548 | AW221069 | cLEF3J23 | IVF7 | T1L14 | R11 | C12 | r1-c2 | r3-c3 |
| 2-oxoglutarate/malate translocator {Arabidopsis thaliana} | TC73585 | AW093605 | cLET25M1 4 | XIH6 | T3P11 | R14 | C16 | r2-c1 | r4-c3 |

134

| TC | Accession | Clone | X | T | R | C | | | | Description |
|---|---|---|---|---|---|---|---|---|---|---|
| TC73588 | AW617938 | cLPT11O16 | XVG2 | T4N3 | R22 | C14 | r2-c2 | r1-c4 | | putative ripening-related bZIP protein {Vitis vinifera} |
| TC73593 | BG129027 | cTOF23I9 | XIXF12 | T5L23 | R2 | C12 | r3-c2 | r4-c4 | | ethylene-responsive transcriptional coactivator |
| TC73595 | AI771994 | cLER1I10 | XA7 | T3A14 | R11 | C1 | r2-c1 | r4-c3 | | PHOSPHOGLUCOMUTASE, CYTOPLASMIC (EC 5.4.2.2) (GLUCOSE PHOSPHOMUTASE) (PGM).GP|8250624|emb|CAB93681. |
| TC73599 | AW034565 | cLEC12H20 | IC6 | T1E11 | R14 | C5 | r1-c2 | r3-c3 | | putative transcripton factor {Nostoc sp. PCC 7120} |
| TC73604 | BG136483 | cLPP3E13 | XVE10 | T4J19 | R6 | C10 | r2-c2 | r1-c4 | | microsomal oleate desaturase {Arachis ipaensis} |
| TC73607 | AW224394 | cLEW6P3 | XIIIH9 | T3P18 | R7 | C16 | r2-c1 | r4-c3 | | pyruvate dehydrogenase e1 component, alpha subunit precursor {Solanum tuberosum}SP|P52903|ODPA_SOLT |
| TC73615 | AW622780 | cTOB1E5 | XVIIA11 | T5A21 | R4 | C1 | r3-c2 | r4-c4 | | enoyl-ACP reductase {Nicotiana tabacum}GP|2204236|emb|CAA74176.1||Y13861 enoyl-ACP reductase {Nicot |
| TC73630 | AW623099 | cTOB8G2 | XVIIB10 | T5C19 | R6 | C3 | r3-c2 | r3-c3 | | putative dehydroquinase shikimate dehydrogenase {Arabidopsis thaliana} |
| TC73643 | AI898594 | cLED34B18 | IIIG4 | T1N7 | R18 | C14 | r1-c2 | r4-c4 | | myb-related transcription factor {Lycopersicon esculentum}PIR|T07393|T07393 myb-related transcripti |
| TC73646 | AW223726 | cLEN12N16 | VIIIG10 | T2N20 | R5 | C14 | r4-c2 | r1-c3 | | bZIP transcriptional activator RSG, putative {Arabidopsis thaliana}GP|12321383|gb|AAG50761.1|AC0791 |
| TC73652 | BF052197 | cLEM25L18 | XVG9 | T4N17 | R8 | C14 | r2-c2 | r1-c4 | | putative zinc finger protein {Oryza sativa} |
| TC73662 | AW223671 | cLEN12P17 | VD2 | T2G3 | R22 | C7 | r4-c2 | r1-c3 | | pyruvate kinase (EC 2.7.1.40) A, chloroplast - common tobacco |
| TC73678 | AI780193 | cLES11C1 | XD2 | T3G4 | R21 | C7 | r2-c1 | r4-c3 | | 6-phosphogluconate dehydrogenase, putative; 13029-14489 {Arabidopsis thaliana} |
| TC73679 | AW031533 | cLEC34F1 | XVIC12 | T4F24 | R1 | C6 | r2-c2 | r1-c4 | | zinc-finger protein, putative; 7043-7771 {Arabidopsis thaliana}PIR|H86450|H86450 probable zinc-fing |
| TC73702 | BG642898 | cTOF26A17 | XIXH3 | T5P5 | R20 | C16 | r3-c2 | r4-c4 | | ornithine aminotransferase {Arabidopsis thaliana} |
| TC73706 | BE433184 | cLEG12J15 | VID3 | T2G6 | R19 | C7 | r4-c2 | r1-c3 | | pyrophosphate-dependent phosphofructo-1-kinase {Arabidopsis thaliana}GP|7269478|emb|CAB79482.1||AL1 |
| TC73722 | BF113143 | cLEG43N9 | VIG6 | T2M12 | R13 | C13 | r4-c2 | r1-c3 | | sugar-phosphate isomerase-like protein {Arabidopsis thaliana}PIR|T47628|T47628 sugar-phosphate isom |
| TC73737 | AW624021 | cTOB13J12 | XVIH7 | T4P14 | R11 | C16 | r2-c2 | r1-c4 | | cytochrome P450, putative {Arabidopsis thaliana} |

| | | | | | | | | | |
|---|---|---|---|---|---|---|---|---|---|
| RING-H2 finger protein RHF2a {Arabidopsis thaliana}GP\|13374859\|emb\|CAC34493.1\|\|AL589883 RING-H2 fin | TC73753 | cLHT23O1 | cLHT23O1 | IG10 | T1M19 | R6 | C13 | r1-c2 | r3-c3 |
| Contains similarity to gb\|AF136530 transcriptional regulator from Zea mays. {Arabidopsis thaliana}P | TC73763 | BG124550 | cTOF5G18 | XXF1 | T5L2 | R23 | C12 | r3-c2 | r4-c4 |
| glucose-6-phosphate 1-dehydrogenase {Solanum tuberosum} | TC73842 | AW616687 | cLHT12I23 | XIVH12 | T4O24 | R1 | C15 | r2-c2 | r1-c4 |
| putative pyrophosphate--fructose-6-phosphate 1-phosphotransferase {Arabidopsis thaliana}PIR\|B84613\| | TC73845 | BE462789 | cTOA15B6 | XVID6 | T4H12 | R13 | C8 | r2-c2 | r1-c4 |
| putative glucosyltransferase {Arabidopsis thaliana}PIR\|E84680\|E84680 probable glucosyltransferase [ | TC73904 | AI485027 | cLED2F12 | IIIE8 | T1J15 | R12 | C10 | r1-c2 | r3-c3 |
| acetyl-CoA synthetase {Solanum tuberosum} | TC73927 | BG133933 | cTOE14H4 | XVIIIC5 | T5E10 | R15 | C5 | r3-c2 | r4-c4 |
| Knotted 1 (TKn1) | TC73929 | AW648827 | cLEI6C17 | VIIF2 | T2L3 | R22 | C12 | r4-c2 | r1-c3 |
| homeotic protein BEL1 homolog {Arabidopsis thaliana} | TC73945 | BG129460 | cTOF24F9 | XIXG6 | T5N11 | R14 | C14 | r3-c2 | r4-c4 |
| flavanone 3-hydroxylase-like protein {Arabidopsis thaliana} | TC73949 | AW218877 | cLEX1K4 | XIIID6 | T4G11 | R14 | C7 | r2-c2 | r1-c4 |
| putative cinnamoyl CoA reductase {Arabidopsis thaliana}PIR\|C84630\|C84630 probable cinnamoyl CoA red | TC73957 | BE460616 | cLEG33E6 | VIB8 | T2C16 | R9 | C3 | r4-c2 | r1-c3 |
| glucosyl transferase {Nicotiana tabacum}GP\|1805359\|dbj\|BAA19155.1\|\|AB000623 glucosyl transferase {N | TC73986 | AI898797 | cLED35N9 | IIIG12 | T1N23 | R2 | C14 | r1-c2 | r3-c3 |
| helicase-like transcription factor-like protein {Arabidopsis thaliana} | TC73997 | AW093171 | cLET23N4 | XIF4 | T3L7 | R18 | C12 | r2-c1 | r4-c3 |
| pyruvate dehydrogenase E1 beta subunit isoform 1 {Zea mays} | TC74002 | BE459681 | cLEM7B14 | VIIIE8 | T2J16 | R9 | C10 | r4-c2 | r1-c3 |
| fructose-6-phosphate 2-kinase/fructose-2,6-bisphosphatase {Solanum tuberosum}PIR\|T07016\|T07016 6-ph | TC74004 | AI896936 | cLEC24G15 | IIF10 | T1K20 | R5 | C11 | r1-c2 | r3-c3 |
| 1,4-alpha-glucan branching enzyme {Solanum tuberosum} □GP\|1621012\|emb\|CAA70038.1\|\|Y08786 1,4-alpha-gl | TC74010 | AI484710 | cLED3F9 | IVA1 | T1B2 | R23 | C2 | r1-c2 | r3-c3 |
| 76 kDa mitochondrial complex I subunit {Solanum tuberosum}SP\|Q43644\|NUAM_SOLTU NADH-UBIQUINONE OXID | TC74019 | AW096331 | cLET38C16 | XIG11 | T3N21 | R4 | C14 | r2-c1 | r4-c3 |
| Similar to ATP-citrate-lyase {Arabidopsis | TC74060 | BE463260 | cTOC12I8 | XVIIC7 | T5E13 | R12 | C5 | r3-c2 | r4-c4 |

| | | | | | | | | | |
|---|---|---|---|---|---|---|---|---|---|
| thaliana}PIR\|F96633\|F96633 hypothetical protein F8A5.32 [ | | | | | | | | | |
| putative anthocyanidin-3-glucoside rhamnosyltransferase {Arabidopsis thaliana}PIR\|D84614\|D84614 hyp | TC74086 | AW625949 | cLEZ17N5 | XIVE7 | T4I14 | R11 | C9 | r2-c2 | r1-c4 |
| floral homeotic protein pmads 2 {Petunia hybrida}SP\|Q07474\|MAD2_PETHY FLORAL HOMEOTIC PROTEIN PMADS | TC74087 | AW929900 | cTOC8C8 | XVIIE11 | T5I21 | R4 | C9 | r3-c2 | r4-c4 |
| CYTOCHROME P450 71D10 (EC 1.14.-.- ).GP\|2739000\|gb\|AAB94588.1\|\|AF022459 CYP71D10p {Glycine max}PIR\| | TC74103 | AW648815 | cLEI6A3 | XIIF5 | T3L10 | R15 | C12 | r2-c1 | r4-c3 |
| putative CONSTANS-like B-box zinc finger protein {Arabidopsis thaliana}PIR\|G84920\|G84920 hypothetic | TC74105 | AW617537 | cLHT23N13 | VB9 | T2C17 | R8 | C3 | r4-c2 | r1-c3 |
| Similar to gb\|X90982 phosphoenolpyruvate carboxylase (ppc1) from Solanum tuberosum. {Arabidopsis tha | TC74116 | AW738719 | cTOD8G6 | VB4 | T2C7 | R18 | C3 | r4-c2 | r1-c3 |
| putative lipoxygenase {Arabidopsis thaliana}PIR\|B96699\|B96699 probable lipoxygenase F12B7.11 [impor | TC74124 | AW651422 | cLEI16J7 | XVIIF6 | T5K11 | R14 | C11 | r3-c2 | r4-c4 |
| putative beta-amylase {Arabidopsis thaliana}GP\|5302810\|emb\|CAB46051.1\|\|Z97342 putative beta-amylase | TC74131 | BG643197 | cTOF26L2 | XIXH10 | T5P19 | R6 | C16 | r3-c2 | r4-c4 |
| UDP-glucose:protein transglucosylase {Solanum tuberosum} | TC74136 | AW738238 | cTOD5P3 | XVIIH11 | T5O21 | R4 | C15 | r3-c2 | r4-c4 |
| hydroxymethyltransferase {Arabidopsis thaliana}GP\|2244749\|emb\|CAB10172.1\|\|Z97335 hydroxymethyltrans | TC74141 | BE450641 | cLEY14O16 | XIIIG10 | T4M19 | R6 | C13 | r2-c2 | r1-c4 |
| hexose transporter {Nicotiana tabacum} | TC74146 | AW617271 | cLHT22B5 | IVF6 | T1L12 | R13 | C12 | r1-c2 | r3-c3 |
| alpha-glucosidase {Solanum tuberosum subsp. tuberosum} | TC74149 | AW651564 | cLEI17E11 | VIID4 | T2H7 | R18 | C8 | r4-c2 | r1-c3 |
| ATP synthase delta' subunit, mitochondrial precursor {Ipomoea batatas}SP\|Q40089\|ATP4_IPOBA ATP SYNT | TC74166 | AI774812 | cLER13M16 | IXF1 | T3K1 | R24 | C11 | r2-c1 | r4-c3 |
| knotted1-like homeobox protein {Malus domestica}SP\|O04136\|HKL3_MALDO HOMEOBOX PROTEIN KNOTTED-1 LIK | TC74178 | AW622767 | cTOB1A17 | XVIIA8 | T5A15 | R12 | C1 | r3-c2 | r4-c4 |
| GLYCINE DEHYDROGENASE [DECARBOXYLATING], MITOCHONDRIAL | TC74186 | BG126353 | cTOF12G5 | XVIIIH5 | T5O10 | R15 | C15 | r3-c2 | r4-c4 |

137

| | | | | | | | | | |
|---|---|---|---|---|---|---|---|---|---|
| PRECURSOR (EC 1.4.4.2) (GLYCINE DECARBOXYLASE | | | | | | | | | |
| contains similarity to CONSTANS homologs~gene_id:MIF21.14 {Arabidopsis thaliana} | TC74187 | BG127619 | cTOF17G8 | XIXB3 | T5D5 | R20 | C4 | r3-c2 | r4-c4 |
| chalcone synthase | TC74227 | AW617911 | cLPT11A16 | XXIF2 | T6K3 | R22 | C11 | r3-c1 | r2-c4 |
| ADP-glucose pyrophosphorylase large subunit | TC74234 | AW622851 | cTOB5H12 | XVIIB5 | T5C9 | R16 | C3 | r3-c2 | r4-c4 |
| aldose 1-epimerase-like protein {Arabidopsis thaliana} | TC74239 | BG133289 | cTOE11N2 2 | XVIIIB10 | T5C20 | R5 | C3 | r3-c2 | r4-c4 |
| 3-phoshoshikimate 1-carboxyvinyltransferase precursor (5-enolpyruvylshikimate-3-phosphate synthase) | TC74249 | AW650241 | cLEI12M14 | IA5 | T1A9 | R16 | C1 | r1-c2 | r3-c3 |
| CYTOCHROME P450 98A2 (EC 1.14.-.- ).GP\|2738998\|gb\|AAB94587.1\|\|AF022458 CYP98A2p {Glycine max}PIR\|T0 | TC74259 | BE450893 | cLEY15F17 | XIIIH3 | T4O5 | R20 | C15 | r2-c2 | r1-c4 |
| zinc finger protein {Oryza sativa}PIR\|JE0113\|JE0113 zinc-finger protein S3574 [imported] - rice | TC74290 | BG129164 | cTOF23D3 | XIXF9 | T5L17 | R8 | C12 | r3-c2 | r4-c4 |
| Chain A, Glycolate Oxidase (E.C.1.1.3.15) Mutant With Tyr 24 Replaced By Phe (Y24f)GP\|999543\|pdb\|1G | TC74300 | AI896203 | cLEC14K6 | IVG11 | T1N22 | R3 | C14 | r1-c2 | r3-c3 |
| RING finger-like protein {Arabidopsis thaliana}PIR\|T47605\|T47605 RING finger-like protein - Arabido | TC74308 | BE433585 | cLEG16I12 | VE12 | T2I23 | R2 | C9 | r4-c2 | r1-c3 |
| Contains similarity to acyl-CoA thioesterase from Streptomyces coelicolor A3(2) gb\|AL163641. EST gb | TC74331 | BE462429 | cTOA13A1 | XVID3 | T4H6 | R19 | C8 | r2-c2 | r1-c4 |
| putative bZIP transcription factor {Arabidopsis thaliana}PIR\|G84831\|G84831 probable bZIP transcript | TC74347 | AW224459 | cLEW6F7 | XVIIA5 | T5A9 | R16 | C1 | r3-c2 | r4-c4 |
| hyoscyamine 6-dioxygenase hydroxylase, putative {Arabidopsis thaliana}PIR\|G86472\|G86472 probable hy | TC74351 | BG629895 | cLEL30F24 | XXIH5 | T6O9 | R16 | C15 | r3-c1 | r2-c4 |
| leucine zipper transcription factor TGA2.1 {Nicotiana tabacum}SP\|O24160\|TG21_TOBAC TGACG-SEQUENCE S | TC74356 | BE459991 | cLEM8L3 | VIIIF7 | T2L14 | R11 | C12 | r4-c2 | r1-c3 |
| succinate dehydrogenase flavoprotein alpha subunit {Arabidopsis thaliana}GP\|8843734\|dbj\|BAA97282.1\| | TC74365 | BG130159 | cTOF29O1 6 | XXB4 | T5D8 | R17 | C4 | r3-c2 | r4-c4 |
| cytochrome P450 {Arabidopsis thaliana} | TC74397 | AW616398 | cLHT11A5 | XIVG12 | T4M24 | R1 | C13 | r2-c2 | r1-c4 |
| N-hydroxycinnamoyl/benzoyltransferase-like protein {Arabidopsis thaliana} | TC74426 | BE460983 | cLEG37K7 | VID11 | T2G22 | R3 | C7 | r4-c2 | r1-c3 |
| phosphoribosyl pyrophosphate synthase isozyme 4 {Spinacia oleracea} | TC74427 | AW224477 | cLEW6J7 | XXIG6 | T6M11 | R14 | C13 | r3-c1 | r2-c4 |

EP 1 454 993 A1

| | | | | | | | | | |
|---|---|---|---|---|---|---|---|---|---|
| (+)-DELTA-CADINENE SYNTHASE ISOZYME A (EC 4.6.1.11) (D-CADINENE SYNTHASE).GP\|1217956\|emb\|CAA65289.1 | TC74429 | AW030787 | cLEC22K16 | IE12 | T1I23 | R2 | C9 | r1-c2 | r3-c3 |
| contains similarity to apoptosis antagonizing transcription factor~gene_id:MFB13.10 {Arabidopsis tha | TC74441 | AI486615 | cLED6M5 | IVB12 | T1D24 | R1 | C4 | r1-c2 | r3-c3 |
| putative sugar transporter; member of major facilitative superfamily; integral membrane protein {Bet | TC74458 | AW738618 | cTOD7P2 | XIIE4 | T3J8 | R17 | C10 | r2-c1 | r4-c3 |
| 4-alpha-glucanotransferase precursor (disproportionating enzyme) (d-enzyme) {Solanum tuberosum}SP\|Q | TC74463 | AW737154 | cTOD2A10 | XVIIG4 | T5M7 | R18 | C13 | r3-c2 | r4-c4 |
| NADH glutamate dehydrogenase {Nicotiana plumbaginifolia}SP\|O04937\|DHEA_NICPL GLUTAMATE DEHYDROGENAS | TC74481 | BE458856 | cLEM4D24 | VIIID7 | T2H14 | R11 | C8 | r4-c2 | r1-c3 |
| bZIP protein {Arabidopsis thaliana}PIR\|T49227\|T49227 bZIP protein - Arabidopsis thaliana | TC74487 | BG135017 | cTOE21E23 | XVIIIE2 | T5I4 | R21 | C9 | r3-c2 | r4-c4 |
| zinc-finger protein, putative; 7043-7771 {Arabidopsis thaliana}PIR\|H86450\|H86450 probable zinc-fing | TC74525 | BF051436 | cLEM22N6 | VIIIB6 | T2D12 | R13 | C4 | r4-c2 | r1-c3 |
| cytochrome P450 {Helianthus tuberosus} | TC74527 | BG130949 | cTOE2A19 | XVIIIE11 | T5I22 | R3 | C9 | r3-c2 | r4-c4 |
| fructose-bisphosphate aldolase, cytoplasmic isozyme 1 {Pisum sativum}SP\|P46256\|ALF1_PEA FRUCTOSE-BI | TC74553 | AI781507 | cLES16A23 | XVG6 | T4N11 | R14 | C14 | r2-c2 | r1-c4 |
| transcriptional adaptor ADA2b {Arabidopsis thaliana} | TC74560 | AW648772 | cLEI5H16 | VIIE12 | T2J23 | R2 | C10 | r4-c2 | r1-c3 |
| putative oxalyl-CoA decarboxylase {Oryza sativa} | TC74622 | AW094043 | cLET27G5 | XXIE11 | T6I21 | R4 | C9 | r3-c1 | r2-c4 |
| putative UDP-N-acetylglucosamine--N-acetylmuramyl-(pentapeptide)-pyrophosphoryl-undecaprenol N-acety | TC74633 | BF112480 | cLEG41O24 | VIF6 | T2K12 | R13 | C11 | r4-c2 | r1-c3 |
| G-Box binding protein 2 {Catharanthus roseus} | TC74645 | AW648927 | cLEI6D11 | VIIF3 | T2L5 | R20 | C12 | r4-c2 | r1-c3 |
| putative indole-3-glycerol phosphate synthase {Arabidopsis thaliana}PIR\|B84457\|B84457 probable indo | TC74653 | AI490823 | cLEB3N3 | IB9 | T1C17 | R8 | C3 | r1-c2 | r3-c3 |
| contains similarity to nucleotide sugar epimerases {Arabidopsis thaliana}GP\|7267098\|emb\|CAB80769.1\| | TC74661 | BE433127 | cLEG12I22 | VD12 | T2G23 | R2 | C7 | r4-c2 | r1-c3 |
| contains similarity to ATPases associated with various cellular activities (Pfam: AAA.hmm, score: 15 | TC74673 | BE459682 | cLEM7B22 | VIIIE9 | T2J18 | R7 | C10 | r4-c2 | r1-c3 |
| putative dihydrolipoamide succinyltransferase {Arabidopsis thaliana}GP\|7269544\|emb\|CAB79546.1\|\|AL16 | TC74697 | BE459191 | cLEM5D12 | XVH12 | T4P23 | R2 | C16 | r2-c2 | r1-c4 |

| | | | | | | | | | |
|---|---|---|---|---|---|---|---|---|---|
| r4-c4 | r3-c2 | C7 | R6 | T5G19 | XVIID10 | cTOC4H22 | AW929135 | TC74751 | auxin-induced basic helix-loop-helix transcription factor, putative {Arabidopsis thaliana}GP|123213 |
| r4-c3 | r2-c1 | C14 | R19 | T3N6 | XIIG3 | cLEW23B2 | BF097801 | TC74758 | Myb-related transcription factor-like protein {Arabidopsis thaliana} |
| r1-c4 | r2-c2 | C7 | R24 | T4G1 | XIIID1 | cLEX15O5 | AW622435 | TC74779 | contains similarity to enolase-phosphatase~gene_id:K19P17.1 {Arabidopsis thaliana} |
| r3-c3 | r1-c2 | C13 | R20 | T1M5 | IG3 | cLEC28G7 | AW034743 | TC74804 | formyl transferase, putative {Arabidopsis thaliana}PIR|H96690|H96690 probable formyl transferase F2 |
| r4-c3 | r2-c1 | C15 | R5 | T3O20 | XH10 | cLES5F19 | AI778508 | TC74808 | immediate-early salicylate-induced glucosyltransferase {Nicotiana tabacum}GP|1685005|gb|AAB36653.1| |
| r4-c3 | r2-c1 | C1 | R20 | T3A5 | IXA3 | cLEN14P12 | AW224247 | TC74821 | putative para-aminobenzoate synthase and glutamine amidotransferase, a bifunctional enzyme {Arabidop} |
| r4-c3 | r2-c1 | C16 | R16 | T3P9 | XIIH5 | cLET39H6 | AW096689 | TC74846 | alpha-glucosidase {Solanum tuberosum subsp. tuberosum} |
| r1-c3 | r4-c2 | C12 | R1 | T2L24 | VIIIF12 | cLEN10A24 | AW223028 | TC74865 | MADS-box transcription factor FBP4 {Petunia x hybrida} |
| r4-c4 | r3-c2 | C10 | R9 | T5J16 | XXE8 | cTOF4N22 | BG124332 | TC74889 | NAD-dependent isocitrate dehydrogenase {Nicotiana tabacum} |
| r1-c4 | r2-c2 | C4 | R7 | T4D18 | XVIB9 | cLPT6F18 | AW399268 | TC74928 | cytochrome p450 lxxviia1 {Solanum melongena}SP|P37123|C771_SOLME CYTOCHROME P450 77A1 (EC 1.14.-.-) |
| r4-c3 | r2-c1 | C9 | R9 | T3I16 | XE8 | cLES16D9 | AI781599 | TC74935 | threonine deaminase {Nicotiana attenuata} |
| r4-c3 | r2-c1 | C7 | R7 | T3G18 | XD9 | cLES13E10 | AI780838 | TC74979 | putative fatty acid desaturase/cytochrome b5 fusion protein {Arabidopsis thaliana}PIR|A84900|A84900 |
| r4-c3 | r2-c1 | C8 | R7 | T3H18 | XIIID9 | cLEW13D18 | BF096665 | TC74988 | sugar transporter like protein {Arabidopsis thaliana}GP|2464913|emb|CAB16808.1||Z99708 sugar transp |
| r4-c3 | r2-c1 | C13 | R4 | T3M21 | IXG11 | cLER16O15 | AI775668 | TC75003 | putative UDP-glucose:glycoprotein glucosyltransferase; 101200-91134 {Arabidopsis thaliana}PIR|G9673 |
| r1-c4 | r2-c2 | C11 | R18 | T4K7 | XIIIF4 | cLEX6M9 | AW219953 | TC75014 | putative nucleotide-sugar transporter {Arabidopsis thaliana}PIR|E84509|E84509 probable vanadate res |
| r1-c4 | r2-c2 | C5 | R14 | T4E11 | XIIIC6 | cLEX13P4 | AW621996 | TC75032 | Putative UDP-glucose glucosyltransferase {Arabidopsis thaliana}PIR|H86356|H86356 probable UDP-gluco |
| r1-c3 | r4-c2 | C6 | R2 | T2F23 | VIIC12 | cLEI16F6 | AW651478 | TC75038 | RING-H2 finger protein RHF2a {Arabidopsis thaliana}GP|13374859|emb|CAC34493.1||AL589883 RING-H2 fin |

| Description | TC | Accession | cLEE | | | | | | |
|---|---|---|---|---|---|---|---|---|---|
| reticuline oxidase-like protein {Arabidopsis thaliana}GP|7268879|emb|CAB79083.1||AL161553 reticulin | TC75058 | AW036319 | cLEE1L12 | IVD7 | T1H14 | R11 | C8 | r1-c2 | r3-c3 |
| zinc finger protein-like {Arabidopsis thaliana} | TC75079 | BG128229 | cTOF19M22 | XIXC9 | T5F17 | R8 | C6 | r3-c2 | r4-c4 |
| contains similarity to ATPases associated with various cellular activities (Pfam: AAA.hmm, score: 15 | TC75096 | BG626509 | cLEL13B4 | XXIB9 | T6C17 | R8 | C3 | r3-c1 | r2-c4 |
| contains similarity to phosphoenolpyruvate synthase (ppsA) (GB:AE001056) {Arabidopsis thaliana}PIR| | TC75102 | AI778552 | cLES5N17 | XIAI | T3B1 | R24 | C2 | r2-c1 | r4-c3 |
| cytochrome P450 {Arabidopsis thaliana} | TC75118 | AW033748 | cLEC29K11 | IG11 | T1M21 | R4 | C13 | r1-c2 | r3-c3 |
| Similar to yeast general negative regulator of transcription subunit 1 {Arabidopsis thaliana}PIR|G8 | TC75146 | AW617698 | cLHT24C16 | XVB6 | T4D11 | R14 | C4 | r2-c2 | r1-c4 |
| putative cytochrome P450 {Oryza sativa}GP||11761120|dbj|BAB19110.1||AP002839 putative cytochrome P45 | TC75173 | BF112432 | cLEG41G20 | VIF3 | T2K6 | R19 | C11 | r4-c2 | r1-c3 |
| nitrite reductase {Capsicum annuum} | TC75187 | BE451369 | cLEY17P24 | XIVA7 | T4A14 | R11 | C1 | r2-c2 | r1-c4 |
| tryptophan synthase beta chain {Arabidopsis thaliana} | TC75190 | BG628235 | cLEL21F5 | XXIE6 | T6I11 | R14 | C9 | r3-c1 | r2-c4 |
| Contains a weak similarity to chalcone--flavonone isomerase from Pueraria lobata GP|Q43056 and conta | TC75202 | BE450563 | cLEY13N12 | XIIIG3 | T4M5 | R20 | C13 | r2-c2 | r1-c4 |
| similar to ATPases associated with various cellular activites (Pfam: AAA.hmm, score: 230.91) {Arabid | TC75211 | BE432530 | cLEG8L3 | VIH12 | T2O24 | R1 | C15 | r4-c2 | r1-c3 |
| UTP-glucose glucosyltransferase {Arabidopsis thaliana} | TC75218 | BG627392 | cLEL17E23 | XXID1 | T6G1 | R24 | C7 | r3-c1 | r2-c4 |
| PROBABLE VACUOLAR ATP SYNTHASE SUBUNIT H (EC 3.6.1.34) (V-ATPASE H SUBUNIT) (VACUOLAR PROTON PUMP H | TC75225 | AW399611 | cLPT8MI | XVIC6 | T4F12 | R13 | C6 | r2-c2 | r1-c4 |
| flavanone 3-hydroxylase-like protein {Arabidopsis thaliana} | TC75238 | BG136323 | cLPP2E20 | XVE8 | T4J15 | R12 | C10 | r2-c2 | r1-c4 |
| isoflavone reductase-like protein {Arabidopsis thaliana}GP|7270404|emb|CAB80171.1||AL161585 isoflav | TC75240 | BG127518 | cTOF17H7 | XIXB4 | T5D7 | R18 | C4 | r3-c2 | r4-c4 |
| cystathionine beta-lyase {Solanum tuberosum} | TC75245 | AW442491 | cLET41E21 | XIH12 | T3P23 | R2 | C16 | r2-c1 | r4-c3 |
| putative acyl-CoA synthetase; 62297-59022 {Arabidopsis thaliana}PIR|D96805|D96805 probable acyl-CoA | TC75253 | BF051331 | cLEM22D11 | VIIIA11 | T2B22 | R3 | C2 | r4-c2 | r1-c3 |
| PHOSPHOGLUCOMUTASE, CHLOROPLAST PRECURSOR (EC 5.4.2.2) (GLUCOSE | TC75272 | AI484186 | cLER1G13 | XA6 | T3A12 | R13 | C1 | r2-c1 | r4-c3 |

| | | | | | | | | | |
|---|---|---|---|---|---|---|---|---|---|
| PHOSPHOMUTASE) (PGM).GP\|8250622\|emb | | | | | | | | | |
| putative cytochrome P450 {Solanum chacoense}SP\|P93531\|C7D7_SOLCH CYTOCHROME P450 71D7 (EC 1.14.-.-) | TC75279 | AW616142 | cLHT6I17 | XVC11 | T4F21 | R4 | C6 | r2-c2 | r1-c4 |
| hydroxymethyltransferase {Arabidopsis thaliana}GP\|2244749\|emb\|CAB10172.1\|\|Z97335 hydroxymethyltrans | TC75281 | AW220206 | cLEX9B18 | XIIIF8 | T4K15 | R12 | C11 | r2-c2 | r1-c4 |
| cytochrome P450, putative {Arabidopsis thaliana}PIR\|F86441\|F86441 probable cytochrome P450 [importe | TC75284 | AI773792 | cLER8M23 | XC9 | T3E18 | R7 | C5 | r2-c1 | r4-c3 |
| HD-Zip protein {Arabidopsis thaliana}GP\|3132474\|gb\|AAC16263.1\|\|AC003096 homeodomain transcription f | TC75348 | BG128856 | cTOF22F23 | XIXE11 | T5J21 | R4 | C10 | r3-c2 | r4-c4 |
| putative bZIP transcription factor {Arabidopsis thaliana}PIR\|G84831\|G84831 probable bZIP transcript | TC75368 | BF098455 | cLEW27E14 | XIIH3 | T3P6 | R19 | C16 | r2-c1 | r4-c3 |
| putative phosphate/phosphoenolpyruvate translocator protein {Arabidopsis thaliana}PIR\|D84649\|D84649 | TC75371 | AW617774 | cLHT24B5 | XVB5 | T4D9 | R16 | C4 | r2-c2 | r1-c4 |
| myb-like protein {Arabidopsis thaliana}PIR\|T48253\|T48253 myb-like protein - Arabidopsis thaliana | TC75389 | AW224023 | cLEN14B1 | VIIIH12 | T2P24 | R1 | C16 | r4-c2 | r1-c3 |
| putative glucosyltransferase {Arabidopsis thaliana}PIR\|H84786\|H84786 probable glucosyltransferase [ | TC75393 | BE463368 | cTOC12J20 | XVIIC8 | T5E15 | R12 | C5 | r3-c2 | r4-c4 |
| tyrosine decarboxylase {Papaver somniferum}SP\|P54771\|TYD5_PAPSO TYROSINE/DOPA DECARBOXYLASE 5 [INCL | TC75404 | AW398822 | cLPT5E15 | XVIB2 | T4D4 | R21 | C4 | r2-c2 | r1-c4 |
| Similar to gb\|U44028 transcription factor CKC from Arabidopsis thaliana and contains two PF\|00847 AP | TC75426 | GAA824963 | CT247 | XVIC10 | T4F20 | R5 | C6 | r2-c2 | r1-c4 |
| bZIP transcription factor 6 {Phaseolus vulgaris} | TC75472 | AW933325 | cLEF52N9 | VB6 | T2C11 | R14 | C3 | r4-c2 | r1-c3 |
| HEAT SHOCK FACTOR PROTEIN 7 (HSF 7) (HEAT SHOCK TRANSCRIPTION FACTOR 7) (HSTF 7).GP\|4539457\|emb\|CAB | TC75476 | AW931781 | cLEF46E16 | IVG10 | T1N20 | R5 | C14 | r1-c2 | r3-c3 |
| phosphoenolpyruvate carboxylase 1 {Gossypium hirsutum}GP\|2266947\|gb\|AAB80714.1\|\|AF008939 phosphoeno | TC75495 | AW738428 | cTOD7E6 | XVIIIA5 | T5A10 | R15 | C1 | r3-c2 | r4-c4 |
| CYP82C1p {Glycine max}PIR\|T05942\|T05942 cytochrome P450 82C1 - soybean | TC75545 | AW039537 | cLET14I9 | VC12 | T2E23 | R2 | C5 | r4-c2 | r1-c3 |

| | | | | | | | | | |
|---|---|---|---|---|---|---|---|---|---|
| glucose-6-phosphate isomerase {Spinacia oleracea}PIR\|T09153\|T09153 glucose-6-phosphate isomerase (E | TC75577 | AW621176 | cLEX11E13 | XIIIB1 | T4C1 | R24 | C3 | r2-c2 | r1-c4 |
| cytochrome P450 {Solanum tuberosum} | TC75602 | BF176446 | cLEZ20B13 | XIVF11 | T4K22 | R3 | C11 | r2-c2 | r1-c4 |
| acetyl-CoA synthetase-like protein {Arabidopsis thaliana} | TC75606 | BG630353 | cLEL35F12 | XXIIA4 | T6A8 | R17 | C1 | r3-c1 | r2-c4 |
| Similar to ribokinase {Arabidopsis thaliana}PIR\|F86307\|F86307 hypothetical protein AAD50017.1 [impo | TC75622 | AW933452 | cLEF55G23 | VB11 | T2C21 | R4 | C3 | r4-c2 | r1-c3 |
| 3-phosphoshikimate 1-carboxyvinyltransferase precursor (5-enolpyruvylshikimate-3-phosphate synthase) | TC75646 | AI487775 | cLED10P21 | IIG4 | T1M8 | R17 | C13 | r1-c2 | r3-c3 |
| TRYPTOPHAN SYNTHASE BETA CHAIN 2 PRECURSOR (EC 4.2.1.20).GP\|2792520\|gb\|AAB97087.1\|\|AF042320 tryptop | TC75671 | AW160221 | cLPT1G17 | XVH6 | T4P11 | R14 | C16 | r2-c2 | r1-c4 |
| cinnamoyl CoA reductase-like protein {Arabidopsis thaliana}PIR\|T48643\|T48643 cinnamoyl CoA reductas | TC75675 | AW039131 | cLET8J13 | XIIC11 | T3F22 | R3 | C6 | r2-c1 | r4-c3 |
| nucleotide diphosphate kinase Ia {Arabidopsis thaliana}GP\|6065740\|emb\|CAB58230.1\|\|AJ012758 nucleoti | TC75705 | BG124009 | cTOF3J14 | XXD10 | T5H20 | R5 | C8 | r3-c2 | r4-c4 |
| diacylglycerol kinase ATDGK1 homolog {Arabidopsis thaliana}GP\|6562306\|emb\|CAB62604.1\|\|AL133421 diac | TC75708 | AW737503 | cTOD3M20 | XVIIG12 | T5M23 | R2 | C13 | r3-c2 | r4-c4 |
| adenosine kinase {Arabidopsis thaliana}GP\|7378610\|emb\|CAB83286.1\|\|AL162751 adenosine kinase-like pr | TC75729 | BG129426 | cTOF24K12 | XIXG8 | T5N15 | R12 | C14 | r3-c2 | r4-c4 |
| bZIP protein {Arabidopsis thaliana}PIR\|T49227\|T49227 bZIP protein - Arabidopsis thaliana | TC75747 | BG135017 | cTOE21E23 | XVIA7 | T4B14 | R11 | C2 | r2-c2 | r1-c4 |
| transcription factor like protein {Arabidopsis thaliana}GP\|2244999\|emb\|CAB10419.1\|\|Z97341 transcrip | TC75876 | AI484089 | cLED25G21 | IIID6 | T1H11 | R14 | C8 | r1-c2 | r3-c3 |
| putative RING zinc finger protein {Arabidopsis thaliana}GP\|6682260\|gb\|AAF23312.1\|AC016661_37\|AC0166 | TC75892 | AW931852 | cLEF46D13 | IVG9 | T1N18 | R7 | C14 | r1-c2 | r3-c3 |
| polyneuridine aldehyde esterase, putative; 10297-12282 {Arabidopsis thaliana} | TC75906 | BG643086 | cTOF26F13 | XIXH6 | T5P11 | R14 | C16 | r3-c2 | r4-c4 |
| glucose 6 phosphate/phosphate translocator-like protein | TC75908 | AI772836 | cLER4C12 | XB11 | T3C22 | R23 | C2 | r2-c1 | r4-c3 |

| | | | | | | | | | |
|---|---|---|---|---|---|---|---|---|---|
| {Arabidopsis thaliana}PIR\|T51467\|T51467 glu | | | | | | | | | |
| leucine zipper transcription factor {Solanum tuberosum}GP\|575418\|emb\|CAA57894.1\|\|X82544 leucine zip | TC75947 | AW219193 | cLEX3E16 | XIIID11 | T4G21 | R4 | C7 | r2-c2 | r1-c4 |
| zinc finger protein-like {Arabidopsis thaliana} | TC75949 | AW221908 | cLEN6G5 | IXD3 | T3G5 | R20 | C7 | r2-c1 | r4-c3 |
| Strong similarity to the TATA binding protein-associated factor from A. thaliana gb\|Y13673. ESTs gb | TC76006 | AI490486 | cLED20L11 | IIIC2 | T1F3 | R22 | C6 | r1-c2 | r3-c3 |
| contains similarity to transcription regulator~gene_id:MRG7.19 {Arabidopsis thaliana} | TC76014 | AI896705 | cLEC22E15 | IE10 | T1I19 | R6 | C9 | r1-c2 | r3-c3 |
| putative beta-amylase {Oryza sativa}GP\|13489165\|gb\|AAK27799.1\|AC022457_2\|AC 022457 putative beta-amy | TC76034 | AI774687 | cLER13G1 | IXE11 | T3I21 | R4 | C9 | r2-c1 | r4-c3 |
| aspartate carbamoyltransferase-poj | TC76045 | | | | | | | | |
| phosphate/phosphoenolpyruvate translocator protein-like {Arabidopsis thaliana} | TC76052 | BF112541 | cLEG41L23 | VIF4 | T2K8 | R17 | C11 | r4- c2 | r1-c3 |
| bZIP transcriptional activator RSG {Nicotiana tabacum} | TC76055 | AI895167 | cLEC6P24 | IIE12 | T1I24 | R1 | C9 | r1-c2 | r3-c3 |
| soluble starch (bacterial glycogen) synthase {Solanum tuberosum}SP\|P93568\|UGS2_SOLTU SOLUBLE GLYCOG | TC76060 | BF113316 | cLEG44E24 | VIG8 | T2M16 | R9 | C13 | r4-c2 | r1-c3 |
| RING zinc finger protein-like {Arabidopsis thaliana} | TC76080 | AW220356 | cLEX10N2 3 | XIIIA10 | T4A19 | R6 | C1 | r2-c2 | r1-c4 |
| lipoxygenase {Zantedeschia aethiopica} | TC76090 | AW216452 | cLEC85F21 | IIF5 | T1K10 | R15 | C11 | r1-c2 | r3-c3 |
| 13-lipoxygenase {Solanum tuberosum}GP\|1495802\|emb\|CAA65268.1\|\|X96405 13-lipoxygenase {Solanum tuber | TC76097 | AI780011 | cLES9J20 | XIB8 | T3D15 | R12 | C4 | r2-c1 | r4-c3 |
| phosphoribosylanthranilate isomerase {Arabidopsis thaliana} | TC76109 | AW441506 | cLEN17O1 8 | IXB4 | T3C7 | R18 | C3 | r2-c1 | r4-c3 |
| glycine hydroxymethyltransferase (EC 2.1.2.1)-like protein {Arabidopsis thaliana}GP\|7270156\|emb\|CAB | TC76133 | AI782607 | cLES20I19 | XG7 | T3M14 | R11 | C13 | r2-c1 | r4-c3 |
| aldose 1-epimerase-like protein {Arabidopsis thaliana} | TC76138 | AW218619 | cLEZ10I24 | XIVC12 | T4E24 | R1 | C5 | r2-c2 | r1-c4 |
| Contains a bZIP transcription factor PF\|00170 domain. ESTs gb\|R30400, gb\|AA650964, gb\|AI994521 come | TC76171 | AI778643 | cLES5P4 | XIA2 | T3B3 | R22 | C2 | r2-c1 | r4-c3 |
| branched-chain alpha-keto acid decarboxylase E1 beta subunit {Arabidopsis thaliana}PIR\|D96597\|D9659 | TC76174 | BG123833 | cTOF3E12 | XXD8 | T5H16 | R9 | C8 | r3-c2 | r4-c4 |
| HYDROXYMETHYLGLUTARYL-COA SYNTHASE (EC 4.1.3.5) (HMG-COA SYNTHASE) (3-HYDROXY-3-METHYLGLUTARYL COENZ | TC76206 | AI897702 | cLED30M1 | XIIH12 | T3P24 | R1 | C16 | r2-c1 | r4-c3 |

144

| | | | | | | | | |
|---|---|---|---|---|---|---|---|---|---|
| PROBABLE (S)-2-HYDROXY-ACID OXIDASE, PEROXISOMAL 2 (EC 1.1.3.15) (GLYCOLATE OXIDASE 2) (GOX 2) (SHOR | TC76210 | AI484731 | cLED3B19 | IIIH10 | T1P19 | R6 | C16 | r1-c2 | r3-c3 |
| glycolate oxidase | TC76211 | AW040933 | cLET7O20 | XIIC7 | T3F14 | R11 | C6 | r2-c1 | r4-c3 |
| s-adenosylmethionine decarboxylase proenzyme (induced stolen tip protein tub13) {Solanum tuberosum} | TC76213 | BE450610 | cLEY14G24 | XIIIG6 | T4M11 | R14 | C13 | r2-c2 | r1-c4 |
| S-adenosyl-L-methionine synthetase | TC76214 | AW650285 | cLEI12H5 | VIIB2 | T2D3 | R22 | C4 | r4-c2 | r1-c3 |
| lipoxygenase | TC76225 | AW441863 | cLEN18D8 | IXB7 | T3C13 | R12 | C3 | r2-c1 | r4-c3 |
| lipoxygenase (LOX) | TC76226 | AW222683 | cLEN9C23 | IXD11 | T3G21 | R4 | C7 | r2-c1 | r4-c3 |
| Contains PF|00249 Myb-like DNA-binding domain. EST gb|Z18152 comes from this gene. {Arabidopsis tha | TC76257 | BE435655 | cLEG28A4 | VH4 | T2O7 | R18 | C15 | r4-c2 | r1-c3 |
| UDP-glucose pyrophosphorylase precursor {Solanum tuberosum}PIR|JX0128|XNPOU UTP--glucose-1-phosphat | TC76266 | BF050633 | cLEM19E21 | VIIH5 | T2P9 | R16 | C16 | r4-c2 | r1-c3 |
| hydroxycinnamoyl-CoA:tyramine N-(hydroxycinnamoyl)transferase {Capsicum annuum} | TC76267 | AI894433 | cLEC4A12 | IID9 | T1G18 | R7 | C7 | r1-c2 | r3-c3 |
| hydroxycinnamoyl-CoA:tyramine N-(hydroxycinnamoyl)transferase {Capsicum annuum} | TC76268 | AW034593 | cLEC11P7 | IC5 | T1E9 | R16 | C5 | r1-c2 | r3-c3 |
| hydroxycinnamoyl-CoA:tyramine N-(hydroxycinnamoyl)transferase {Capsicum annuum} | TC76269 | AI779165 | cLES7G2 | XIA7 | T3B13 | R12 | C2 | r2-c1 | r4-c3 |
| hydroxycinnamoyl-CoA:tyramine N-(hydroxycinnamoyl)transferase {Capsicum annuum} | TC76270 | AI775716 | cLER16I14 | IXG7 | T3M13 | R12 | C13 | r2-c1 | r4-c3 |
| tyramine hydroxycinnamoyltransferase {Nicotiana tabacum} | TC76271 | AW626294 | cLEZ19G14 | XIVF5 | T4K10 | R15 | C11 | r2-c2 | r1-c4 |
| tyramine hydroxycinnamoyltransferase {Nicotiana tabacum} | TC76272 | AI775105 | cLER14F7 | XIIA10 | T3B20 | R5 | C2 | r2-c1 | r4-c3 |
| putative transcription factor BTF3 (RNA polymerase B transcription factor 3); 26343-27201 {Arabidops | TC76286 | BG129308 | cTOF23N20 | XIXG4 | T5N7 | R18 | C14 | r3-c2 | r4-c4 |
| putative transcription factor BTF3 (RNA polymerase B transcription factor 3); 26343-27201 {Arabidops | TC76287 | AW031103 | cLEC13K9 | ID2 | T1G3 | R22 | C7 | r1-c2 | r3-c3 |
| chloroplast triose phosphate translocator precursor (ctpt) (e29) {Solanum tuberosum}SP|P29463|CPTR | TC76288 | BG127183 | cTOF14N1 | XIXA4 | T5B7 | R18 | C2 | r3-c2 | r4-c4 |
| chloroplast triose phosphate translocator precursor (ctpt) (e29) {Solanum tuberosum}SP|P29463|CPTR | TC76289 | AW093406 | cLET24D14 | XIF10 | T3L19 | R6 | C12 | r2-c1 | r4-c3 |
| anthocyanin 5-O-glucosyltransferase {Petunia x hybrida} | TC76292 | AI779099 | cLES7I9 | XIA9 | T3B17 | R8 | C2 | r2-c1 | r4-c3 |
| phosphoglycerate kinase, cytosolic {Nicotiana | TC76300 | BG128272 | cTOF19H1 | XIXC7 | T5F13 | R12 | C6 | r3-c2 | r4-c4 |

145

| | | | | | | | | | |
|---|---|---|---|---|---|---|---|---|---|
| tabacum}SP\|Q42962\|PGKY_TOBAC PHOSPHOGLYCERATE KINASE, | | | 3 | | | | | | |
| homeobox 1 protein^^class II knotted-like homeodomain protein | TC76313 | BE436744 | cLEG34E19 | VIC1 | T2E2 | R23 | C5 | r4-c2 | r1-c3 |
| chorismate synthase 1 | TC76324 | AW626279 | cLEZ19C2 | XIVF3 | T4K6 | R19 | C11 | r2-c2 | r1-c4 |
| chorismate synthase 1 precursor 3-phosphate phospholyase 1) {Lycopersicon esculentum}SP\|Q42884\|ARC1 | TC76325 | BG643072 | cTOF26D9 | XIXH4 | T5P7 | R18 | C16 | r3-c2 | r4-c4 |
| chorismate synthase 1 precursor 3-phosphate phospholyase 1) {Lycopersicon esculentum}SP\|Q42884\|ARC1 | TC76326 | AI488071 | cLED20E15 | IIIB12 | T1D23 | R2 | C4 | r1-c2 | r3-c3 |
| cytosolic NADP-malic enzyme^^malate dehydrogenase | TC76336 | AW649636 | cLEI8P8 | VIIG4 | T2N7 | R18 | C14 | r4-c2 | r1-c3 |
| ATP synthase gamma subunit, chloroplast precursor {Nicotiana tabacum}SP\|P29790\|ATPG_TOBAC ATP SYNTH | TC76348 | AI775755 | cLER16B11 | IXG2 | T3M3 | R22 | C13 | r2-c1 | r4-c3 |
| zinc-finger protein {Petunia x hybrida}GP\|439493\|dbj\|BAA05079.1\|\|D26086 zinc-finger protein {Petuni | TC76350 | BE435119 | cLEG25I19 | VG8 | T2M15 | R12 | C13 | r4-c2 | r1-c3 |
| fructokinase | TC76354 | BE459351 | cLEM6F5 | VIIIE3 | T2J6 | R19 | C10 | r4-c2 | r1-c3 |
| ATP synthase beta subunit, mitochondrial precursor {Nicotiana plumbaginifolia}SP\|P17614\|ATP2_NICPL | TC76359 | BE354286 | cTOD9H12 | XVIIIB7 | T5C14 | R11 | C3 | r3-c2 | r4-c4 |
| spermidine synthase | TC76360 | AW222198 | cLEN7C24 | IVG3 | T1N6 | R19 | C14 | r1-c2 | r3-c3 |
| cDNA~Strawberry pyruvate decarboxylase {Fragaria x ananassa}GP\|10121330\|gb\|AAG13131.1\|AF193791_1\|A F | TC76372 | AI896062 | cLEC13B10 | IC7 | T1E13 | R12 | C5 | r1-c2 | r3-c3 |
| oxoglutarate malate translocator {Solanum tuberosum}GP\|1486472\|emb\|CAA68164.1\|\|X99853 oxoglutarate | TC76379 | BG134301 | cLET20P12 | XIF2 | T3L3 | R22 | C12 | r2-c1 | r4-c3 |
| aspartate aminotransferase glyoxysomal isozyme AAT1 precursor {Glycine max}PIR\|T06136\|T06136 aspart | TC76380 | AW029827 | cLEC15H12 | XIIB1 | T3D2 | R23 | C4 | r2-c1 | r4-c3 |
| putative bZIP DNA-binding protein {Capsicum chinense} | TC76385 | BG126538 | cTOF12J15 | XVIIIH7 | T5O14 | R11 | C15 | r3-c2 | r4-c4 |
| ATP synthase B' subunit precursor {Spinacia oleracea}SP\|P31853\|ATPX_SPIOL ATP SYNTHASE B' CHAIN PRE | TC76386 | BG123713 | cTOF2J20 | XXB7 | T5D14 | R11 | C4 | r3-c2 | r4-c4 |
| ATP synthase beta chain precursor (subunit II) {Arabidopsis | TC76387 | AI775445 | cLER15F23 | XXIH11 | T6O21 | R4 | C15 | r3-c1 | r2-c4 |

146

EP 1 454 993 A1

| | | | | | | | | |
|---|---|---|---|---|---|---|---|---|
| thaliana}GP|2864617|emb|CAA16964.1||AL0 | | | | | | | | |
| homeodomain-leucine zipper protein 57 {Glycine max} | TC76389 | AI485338 | cLED7K14 | IVC5 | T1F10 | R15 | C6 | r1-c2 | r3-c3 |
| VACUOLAR ATP SYNTHASE SUBUNIT G 1 (EC 3.6.1.34) (V-ATPASE G SUBUNIT 1) (VACUOLAR PROTON PUMP G SUBUN | TC76393 | AW222172 | cLEN7O7 | IVD11 | T1H22 | R3 | C8 | r1-c2 | r3-c3 |
| cytochrome p450 lxxii hydroxylase) (ge10h) {Catharanthus roseus}SP|Q05047|CP72_CATRO CYTOCHROME P45 | TC76404 | AW030204 | cLEC19P18 | ID12 | T1G23 | R2 | C7 | r1-c2 | r3-c3 |
| nucleoside diphosphate kinase {Pisum sativum} | TC76406 | AW626159 | cLEZ18D16 | XIVE9 | T4I18 | R7 | C9 | r2-c2 | r1-c4 |
| proline oxidase precursor {Arabidopsis thaliana} | TC76411 | AW034201 | cLEC33G2 | IIA2 | T1A4 | R21 | C1 | r1-c2 | r3-c3 |
| UDP-glucose dehydrogenase {Glycine max}SP|Q96558|UGDH_SOYBN UDP-GLUCOSE 6-DEHYDROGENASE (EC 1.1.1.2 | TC76434 | AW648929 | cLEI6D15 | VIIF4 | T2L7 | R18 | C12 | r4-c2 | r1-c3 |
| UDP-glucose dehydrogenase {Arabidopsis thaliana} | TC76435 | BE353739 | cTOD5J6 | IIF7 | T1K14 | R11 | C11 | r1-c2 | r3-c3 |
| UDP-glucose dehydrogenase-like protein {Arabidopsis thaliana}PIR|T51527|T51527 UDP-glucose dehydrog | TC76436 | AW626165 | cLEZ18F6 | XIVE11 | T4I22 | R3 | C9 | r2-c2 | r1-c4 |
| lipoxygenase {Solanum tuberosum}GP|1407705|gb|AAB67865.1||U60202 lipoxygenase {Solanum tuberosum}P | TC76460 | AW030773 | cLEC25F7 | IF8 | T1K15 | R12 | C11 | r1-c2 | r3-c3 |
| 3-ketoacyl-CoA thiolase {Arabidopsis thaliana}GP|2981618|dbj|BAA25249.1||AB008855 3-ketoacyl-CoA th | TC76465 | AW037869 | cLET3G1 | XVIIIB6 | T5C12 | R13 | C3 | r3-c2 | r4-c4 |
| alanine aminotransferase {Arabidopsis thaliana}GP|12325273|gb|AAG52580.1|AC016529_11| AC016529 putat | TC76487 | AW033411 | cLEC28J11 | IG5 | T1M9 | R16 | C13 | r1-c2 | r3-c3 |
| malate synthase, glyoxysomal {Cucumis sativus}SP|P08216|MASY_CUCSA MALATE SYNTHASE, GLYOXYSOMAL (EC | TC76489 | AW651326 | cLEI16E8 | VIIC11 | T2F21 | R4 | C6 | r4-c2 | r1-c3 |
| putative 6-phosphogluconolactonase {Arabidopsis thaliana} | TC76497 | BG133253 | cTOE11H1 2 | XVIIIB9 | T5C18 | R7 | C3 | r3-c2 | r4-c4 |
| 6-phosphogluconolactonase-like protein {Arabidopsis thaliana} | TC76498 | AW219335 | cLEX4G17 | XIIIE6 | T4I11 | R14 | C9 | r2-c2 | r1-c4 |
| putative cinnamyl alcohol dehydrogenase {Malus x domestica}PIR|T16995|T16995 probable cinnamyl-alco | TC76501 | AW096395 | cLET38O18 | XIH2 | T3P3 | R22 | C16 | r2-c1 | r4-c3 |
| pyrophosphate--fructose 6-phosphate 1-phosphotransferase alpha subunit (pfp) (6-phosphofructokinase | TC76514 | BF097042 | cLEW19M9 | XIIE9 | T3J18 | R7 | C10 | r2-c1 | r4-c3 |

EP 1 454 993 A1

| | | | | | | | | | |
|---|---|---|---|---|---|---|---|---|---|
| pyrophosphate--fructose 6-phosphate 1-phosphotransferase alpha subunit (pfp) (6-phosphofructokinase | TC76515 | BE460061 | cLEM8H22 | VIIIF4 | T2L8 | R17 | C12 | r4-c2 | r1-c3 |
| triosephosphate isomerase chloroplast precursor {Spinacia oleracea}SP\|P48496\|TPIC_SPIOL TRIOSEPHOSP | TC76528 | BG133344 | cTOE12G21 | XIIB4 | T3D8 | R17 | C4 | r2-c1 | r4-c3 |
| MALATE DEHYDROGENASE [NADP], CHLOROPLAST PRECURSOR (EC 1.1.1.82) (NADP-MDH).GP\|2827076\|gb\|AAB99753. | TC76554 | BG129853 | cTOF28F11 | XXA9 | T5B18 | R7 | C2 | r3-c2 | r4-c4 |
| succinyl-CoA-ligase beta subunit {Arabidopsis thaliana}GP\|6598664\|gb\|AAD25643.2\|AC007109_1\|AC007109 | TC76558 | BG123463 | cTOF2C9 | XVIF7 | T4L14 | R11 | C12 | r2-c2 | r1-c4 |
| caffeoyl-CoA O-methyltransferase 5 {Nicotiana tabacum}GP\|1679853\|emb\|CAB05369.1\|\|Z82982 caffeoyl-Co | TC76559 | BE450770 | cLEY15E1 | XIIIH2 | T4O3 | R22 | C15 | r2-c2 | r1-c4 |
| fructokinase, putative {Arabidopsis thaliana}GP\|12324405\|gb\|AAG52172.1\|AC020665_17\|AC020665 fructok | TC76564 | BE460923 | cLEG36N20 | VID4 | T2G8 | R17 | C7 | r4-c2 | r1-c3 |
| transcription factor TEIL {Nicotiana tabacum} | TC76569 | BF098555 | cLEW27J14 | XIIH6 | T3P12 | R13 | C16 | r2-c1 | r4-c3 |
| putative phospholipid cytidylyltransferase {Arabidopsis thaliana}PIR\|H84807\|H84807 probable phospho | TC76594 | AW031599 | cLEC34I4 | IIA9 | T1A18 | R7 | C1 | r1-c2 | r3-c3 |
| S-adenosylmethionine:2-demethylmenaquinone methyltransferase-like protein {Arabidopsis thaliana} | TC76601 | BG642944 | cTOF26K3 | XIXH9 | T5P17 | R8 | C16 | r3-c2 | r4-c4 |
| fructose-1,6-bisphosphatase precursor {Solanum tuberosum} | TC76605 | AW091821 | cLET16D8 | XID6 | T3H11 | R14 | C8 | r2-c1 | r4-c3 |
| Similar to gb\|D86180 phosphoribosylanthranilate transferase from Pisum sativum and contains 2 PF\|001 | TC76611 | AW092411 | cLET20K18 | XIE12 | T3J23 | R2 | C10 | r2-c1 | r4-c3 |
| VACUOLAR ATP SYNTHASE 16 KDA PROTEOLIPID SUBUNIT (EC 3.6.1.34) (V- ATPASE 16 KDA PROTEOLIPID SUBUNIT | TC76632 | BG128625 | cTOF21F21 | XIXE2 | T5J3 | R22 | C10 | r3-c2 | r4-c4 |
| glutamate 1-semialdehyde 2,1-aminomutase | TC76649 | AW039253 | cLET8F12 | XIIC8 | T3F16 | R9 | C6 | r2-c1 | r4-c3 |
| FRUCTOSE-1,6-BISPHOSPHATASE, CHLOROPLAST PRECURSOR (EC 3.1.3.11) (D-FRUCTOSE-1,6-BISPHOSPHATE 1-PHOS | TC76659 | BG128167 | cTOF19A20 | XIXC3 | T5F5 | R20 | C6 | r3-c2 | r4-c4 |
| Similar to transaldolase {Arabidopsis thaliana}PIR\|D86257\|D86257 hypothetical protein [imported] - | TC76672 | BG130627 | cTOF31N1 | XXC7 | T5F14 | R11 | C6 | r3-c2 | r4-c4 |

148

| | | | | | | | | | |
|---|---|---|---|---|---|---|---|---|---|
| CYP94A1 {Vicia sativa}PIR\|T08014\|T08014 cytochrome P450 CYP94A1 - spring vetch | TC76678 | AW626091 | cLEZ18E2 | XIVE10 | T4I20 | R5 | C9 | r2-c2 | r1-c4 |
| 2-isopropylmalate synthase {Lycopersicon pennellii}SP\|O04973\|LU1A_LYCPN 2-ISOPROPYLMALATE SYNTHASE | TC76694 | BF051054 | cLEM21M20 | VIIIA7 | T2B14 | R11 | C2 | r4-c2 | r1-c3 |
| putative cinnamoyl-CoA reductase {Arabidopsis thaliana}PIR\|D84747\|D84747 probable cinnamoyl-CoA red | TC76707 | BG130999 | cTOE2K3 | XVIIIF2 | T5K4 | R21 | C11 | r3-c2 | r4-c4 |
| putative nucleotide-sugar dehydratase {Arabidopsis thaliana}PIR\|T00419\|T00419 dTDP-glucose 4-6-dehy | TC76708 | AW218947 | cLEX2K19 | XIIID9 | T4G17 | R8 | C7 | r2-c2 | r1-c4 |
| zinc finger protein {Arabidopsis thaliana} | TC76725 | BG125170 | cTOF7H24 | XXF9 | T5L18 | R7 | C12 | r3-c2 | r4-c4 |
| transcription factor Hap5a-like protein {Arabidopsis thaliana} | TC76772 | BE460825 | cLEG36J5 | VID2 | T2G4 | R21 | C7 | r4-c2 | r1-c3 |
| cytochrome c oxidase subunit 6b-1 {Oryza sativa}GP\|9967277\|dbj\|BAB12338.1\|\|AB047975 cytochrome c ox | TC76784 | AW218304 | cLEZ6K17 | XIVG4 | T4M8 | R17 | C13 | r2-c2 | r1-c4 |
| beta-fructofuranosidase (invertase)^^beta-fructosidase^^beta fructosidase | TC76785 | AI486242 | cLED5P21 | IVB3 | T1D6 | R19 | C4 | r1-c2 | r3-c3 |
| ATP synthase delta subunit, chloroplast precursor {Nicotiana tabacum}SP\|P32980\|ATPD_TOBAC ATP SYNTH | TC76808 | AI776862 | cLER20E5 | XVIH4 | T4P8 | R17 | C16 | r2-c2 | r1-c4 |
| aminotransferase-like protein {Arabidopsis thaliana} | TC76819 | BF052130 | cLEM25N13 | VIIIC11 | T2F22 | R3 | C6 | r4-c2 | r1-c3 |
| contains similarity to C2H2-type zinc finger protein~gene_id:MOK16.6 {Arabidopsis thaliana} | TC76821 | BE459738 | cLEM7L20 | VIIIE11 | T2J22 | R3 | C10 | r4-c2 | r1-c3 |
| NADH-cytochrome b5 reductase {Arabidopsis thaliana}GP\|4240118\|dbj\|BAA74838.1\|\|AB007800 NADH-cytochr | TC76827 | AW626229 | cLEZ19A19 | XIVF1 | T4K2 | R23 | C11 | r2-c2 | r1-c4 |
| lipoxygenase | TC76842 | AW035972 | cLEC33J22 | IIA4 | T1A8 | R17 | C1 | r1-c2 | r3-c3 |
| Similar to dTDP-D-glucose 4,6-dehydratase {Arabidopsis thaliana}PIR\|C96814\|C96814 hypothetical prot | TC76851 | AW929920 | cTOC8G22 | XVIIF1 | T5K1 | R24 | C11 | r3-c2 | r4-c4 |
| UDP-glucose:salicylic acid glucosyltransferase {Nicotiana tabacum} | TC76866 | BE431755 | cLEG1L22 | VF12 | T2K23 | R2 | C11 | r4-c2 | r1-c3 |
| putative cytochrome P450 {Arabidopsis thaliana}GP\|13877669\|gb\|AAK43912.1\|AF370593_1\|A F370593 putati | TC76887 | AW221681 | cLEN3H3 | IXC7 | T3E13 | R12 | C5 | r2-c1 | r4-c3 |

| | | | | | | | | | |
|---|---|---|---|---|---|---|---|---|---|
| contains similarity to shikimate kinase precursor~gene_id:MDJ14.24 {Arabidopsis thaliana} | TC76889 | BG128905 | cTOF22P21 | XIXF4 | T5L7 | R18 | C12 | r3-c2 | r4-c4 |
| contains similarity to RING zinc finger protein~gene_id:MBD2.14 {Arabidopsis thaliana} | TC76902 | AW625867 | cLEZ17K21 | XIVE4 | T4I8 | R17 | C9 | r2-c2 | r1-c4 |
| alpha-glucan phosphorylase, h isozyme phosphorylase h) {Solanum tuberosum}SP|P32811|PHSH_SOLTU ALPH | TC76936 | BG129281 | cTOF23H24 | XVG4 | T4N7 | R18 | C14 | r2-c2 | r1-c4 |
| CTP:phosphocholine cytidylyltransferase {Brassica napus}GP|1416514|dbj|BAA09644.1||D63168 CTP:phosp | TC76939 | AI484842 | cLED2N18 | IIIE10 | T1J19 | R6 | C10 | r1-c2 | r3-c3 |
| citrate synthase {Nicotiana tabacum}EGAD|126596|143593 citrate synthase {Nicotiana tabacum}GP|2300 | TC76947 | AI778988 | cLES6D20 | XIIA12 | T3B24 | R1 | C2 | r2-c1 | r4-c3 |
| nucleoside diphosphate kinase II precursor {Spinacia oleracea}SP|Q01402|NDK2_SPIOL NUCLEOSIDE DIPHO | TC76957 | BG128139 | cTOF19K9 | XIXC8 | T5F15 | R12 | C6 | r3-c2 | r4-c4 |
| delta 1-pyrroline-5-carboxylate synthetase | TC76960 | BG130959 | cTOE2C17 | XVIIIE12 | T5I24 | R1 | C9 | r3-c2 | r4-c4 |
| ferritin subunit cowpea2 precursor {Vigna unguiculata}PIR|T08124|T08124 ferritin 2 precursor - cowp | TC76962 | BG134968 | cTOE20L1 | XVIIIE1 | T5I2 | R23 | C9 | r3-c2 | r4-c4 |
| tomato invertase inhibitor | TC76975 | AI782237 | cLES18F9 | XF8 | T3K16 | R9 | C11 | r2-c1 | r4-c3 |
| chalcone--flavanone isomerase a {Petunia hybrida}SP|P11650|CFIA_PETHY CHALCONE--FLAVONONE ISOMERASE | TC76987 | BG126620 | cTOF12L6 | XVIIIH8 | T5O16 | R9 | C15 | r3-c2 | r4-c4 |
| 3-ketoacyl-CoA thiolase {Arabidopsis thaliana}GP|2981618|dbj|BAA25249.1||AB008855 3-ketoacyl-CoA th | TC76988 | AI775821 | cLER16N17 | VC4 | T2E7 | R18 | C5 | r4-c2 | r1-c3 |
| chalcone synthase | TC77000 | BE435825 | cLEG29O5 | VH12 | T2O23 | R2 | C15 | r4-c2 | r1-c3 |
| contains similarity to diaminopimelate decarboxylase~gene_id:MLN21.17 {Arabidopsis thaliana} | TC77005 | AW928611 | cTOC2D23 | XVIID3 | T5G5 | R20 | C7 | r3-c2 | r4-c4 |
| glyceraldehyde 3-phosphate dehydrogenase | TC77013 | AW647921 | cLEI2J10 | VIID6 | T2H11 | R14 | C8 | r4-c2 | r1-c3 |
| aspartate aminotransferase {Medicago sativa}□GP|777387|gb|AAB46611.1||L25335 aspartate aminotransfer | TC77015 | BG127416 | cTOF16M22 | XVIID6 | T5G11 | R14 | C7 | r3-c2 | r4-c4 |
| Contains similarity to a putative 6-phosphogluconolactonase T1G12.6 GP|6553917 from | TC77016 | AW219887 | cLEX6G21 | XIIIE11 | T4I21 | R4 | C9 | r2-c2 | r1-c4 |

150

| Arabidopsis thal | | | | | | | | | |
|---|---|---|---|---|---|---|---|---|---|
| WRKY transcription factor Nt-SubD48 {Nicotiana tabacum} | TC77024 | AW034258 | cLEC38A11 | XVIG11 | T4N22 | R3 | C14 | r2-c2 | r1-c4 |
| glutamate decarboxylase isozyme 4 {Nicotiana tabacum} | TC77052 | AW030295 | cLEC18C17 | ID11 | T1G21 | R4 | C7 | r1-c2 | r3-c3 |
| D-3-PHOSPHOGLYCERATE DEHYDROGENASE PRECURSOR (EC 1.1.1.95) (PGDH).GP\|2189964\|dbj\|BAA20405.1\|\|AB0032 | TC77066 | BE451433 | cLEY18P1 | XIVA11 | T4A22 | R3 | C1 | r2-c2 | r1-c4 |
| putative glucosyl transferase {Arabidopsis thaliana}PIR\|H84784\|H84784 probable glucosyl transferase | TC77071 | AW737341 | cTOD3O15 | VIE2 | T2I4 | R21 | C9 | r4-c2 | r1-c3 |
| 11S globulin precursor {Sesamum indicum} | TC77083 | BF051303 | cLEM22M13 | IVD3 | T1H6 | R19 | C8 | r1-c2 | r3-c3 |
| Putative ABC transporter {Arabidopsis thaliana}PIR\|H96622\|H96622 probable ABC transporter F23H11.19 | TC77089 | AI490083 | cLED19M7 | IIIB11 | T1D21 | R4 | C4 | r1-c2 | r3-c3 |
| putative fatty acid desaturase {Arabidopsis thaliana}GP\|4325341\|gb\|AAD17340.1\|\|AF128393 similar to | TC77096 | AW038672 | cLET2D1 | XIG10 | T3N19 | R6 | C14 | r2-c1 | r4-c3 |
| S-adenosyl-L-methionine:salicylic acid carboxyl methyltransferase {Stephanotis floribunda} | TC77118 | BE459581 | cLEM7O9 | VIIIF1 | T2L2 | R23 | C12 | r4-c2 | r1-c3 |
| putative hydroxymethylglutaryl-CoA lyase protein {Arabidopsis thaliana}GP\|13194812\|gb\|AAK15568.1\|AF | TC77127 | AW217746 | cTOC6G1 | XVIIE6 | T5I11 | R14 | C9 | r3-c2 | r4-c4 |
| PYRROLINE-5-CARBOXYLATE REDUCTASE (EC 1.5.1.2) (P5CR) (P5C REDUCTASE).GP\|1928962\|gb\|AAC14482.1\|\|U92 | TC77132 | BF051120 | cLEM21L1 | VIIIA6 | T2B12 | R13 | C2 | r4-c2 | r1-c3 |
| putative transcription factor {Oryza sativa}GP\|12328532\|dbj\|BAB21190.1\|\|AP002909 putative transcrip | TC77133 | AW944929 | cTOB12J3 | XVIG10 | T4N20 | R5 | C14 | r2-c2 | r1-c4 |
| S-adenosylmethionine:2-demethylmenaquinone methyltransferase-like {Arabidopsis thaliana} | TC77161 | BG127962 | cTOF18H11 | XIXB11 | T5D21 | R4 | C4 | r3-c2 | r4-c4 |
| UDP rhamnose: anthocyanidin-3-glucoside rhamnosyltransferase {Petunia x hybrida}PIR\|S36655\|S36655 U | TC77166 | BE432345 | cLEG7H11 | VIH7 | T2O14 | R11 | C15 | r4-c2 | r1-c3 |
| phosphoenolpyruvate carboxylase kinase | TC77168 | BE431605 | cLEG27M9 | VH3 | T2O5 | R20 | C15 | r4-c2 | r1-c3 |
| putative aspartate aminotransferase {Arabidopsis | TC77175 | BG123217 | cTOF1O17 | XIXD9 | T5H17 | R8 | C8 | r3-c2 | r4-c4 |

EP 1 454 993 A1

| | | | | | | | | | |
|---|---|---|---|---|---|---|---|---|---|
| thaliana}PIR|E84610|E84610 probable aspartate amin | | | | | | | | | |
| ADP-glucose pyrophosphorylase large subunit | TC77179 | BE459469 | cLEM6L16 | VIIIE5 | T2J10 | R15 | C10 | r4-c2 | r1-c3 |
| threonine synthase {Solanum tuberosum} | TC77196 | AW091608 | cLET15L20 | XID5 | T3H9 | R16 | C8 | r2-c1 | r4-c3 |
| tryptophan synthase beta chain {Arabidopsis thaliana} | TC77225 | BF051762 | cLEM24I23 | VIIIC6 | T2F12 | R13 | C6 | r4-c2 | r1-c3 |
| putative C3HC4-type RING zinc finger/ankyrin protein {Arabidopsis thaliana}PIR|E84689|E84689 probab | TC77229 | AW929405 | cTOC8B24 | IVG12 | T1N24 | R1 | C14 | r1-c2 | r3-c3 |
| UMP/CMP kinase like protein {Arabidopsis thaliana}GP|7269379|emb|CAB81339.1||AL161563 UMP/CMP kinas | TC77240 | BF050903 | cLEM19L18 | VIIH7 | T2P13 | R12 | C16 | r4-c2 | r1-c3 |
| PROBABLE UDP-GLUCOSE 4-EPIMERASE AT4G23920 (EC 5.1.3.2) (GALACTOWALDENASE) (UDP-GALACTOSE 4-EPIMERAS | TC77242 | AW738215 | cTOD5J9 | XVIIH9 | T5O17 | R8 | C15 | r3-c2 | r4-c4 |
| putative zinc finger protein {Oryza sativa} | TC77251 | AW737484 | cTOD3I18 | XVIIG9 | T5M17 | R8 | C13 | r3-c2 | r4-c4 |
| fructose-1,6-biphosphatase | TC77264 | BG131255 | cTOE3E20 | XVIIIF5 | T5K10 | R15 | C11 | r3-c2 | r4-c4 |
| shikimate kinase precursor | TC77265 | AI773666 | cLER7F8 | IVH5 | T1P10 | R15 | C16 | r1-c2 | r3-c3 |
| shikimate kinase precursor {Lycopersicon esculentum}SP|Q00497|AROK_LYCES SHIKIMATE KINASE PRECURSOR | TC77266 | BE433355 | cLEG13C20 | VE3 | T2I5 | R20 | C9 | r4-c2 | r1-c3 |
| putative sugar transporter | TC77279 | AI489511 | cLED16M9 | IIIA10 | T1B19 | R6 | C2 | r1-c2 | r3-c3 |
| S-adenosyl-L-methionine:salicylic acid carboxyl methyltransferase {Atropa belladonna} | TC77297 | AW929283 | cTOC7K17 | XVIIE10 | T5I19 | R6 | C9 | r3-c2 | r4-c4 |
| G-box binding protein | TC77305 | AI487557 | cLED9N21 | IVD2 | T1H4 | R21 | C8 | r1-c2 | r3-c3 |
| unnamed protein product {unidentified}GP|6683619|dbj|BAA89269.1||AB025250 ATP phosphoribosyl transf | TC77310 | BE460937 | cLEG37A9 | VID5 | T2G10 | R15 | C7 | r4-c2 | r1-c3 |
| putative NADH dehydrogenase (ubiquinone oxidoreductase) {Arabidopsis thaliana}PIR|T02486|T02486 hyp | TC77318 | BG643872 | cTOF33I9 | XXD2 | T5H4 | R21 | C8 | r3-c2 | r4-c4 |
| contains similarity to transcription regulator~gene_id:MRG7.19 {Arabidopsis thaliana} | TC77334 | AI896178 | cLEC14E22 | IB12 | T1C23 | R2 | C3 | r1-c2 | r3-c3 |
| G-box binding protein | TC77337 | BG129706 | cTOF27L18 | XXA5 | T5B10 | R15 | C2 | r3-c2 | r4-c4 |
| NADH dehydrogenase {Solanum tuberosum}SP|P80269|NUIM_SOLTU NADH-UBIQUINONE OXIDOREDUCTASE 23 KDA SU | TC77343 | BG128821 | cTOF22M16 | XIXF2 | T5L3 | R22 | C12 | r3-c2 | r4-c4 |
| phosphatidylserine decarboxylase {Arabidopsis thaliana} | TC77346 | BE434333 | cLEG16I23 | VF1 | T2K1 | R24 | C11 | r4-c2 | r1-c3 |
| bHLH transcription factor JAF13 {Petunia x hybrida} | TC77374 | AI489467 | cLED16J1 | IIH4 | T1O8 | R17 | C15 | r1-c2 | r3-c3 |

| Description | TC | Accession | Clone | | | | | | |
|---|---|---|---|---|---|---|---|---|---|
| nitrite reductase {Capsicum annuum} | TC77375 | BF098124 | cLEW25H15 | XIIG9 | T3N18 | R7 | C14 | r2-c1 | r4-c3 |
| putative aminotransferase; 101422-99564 {Arabidopsis thaliana}PIR|D96806|D96806 probable aminotrans | TC77383 | AI777500 | cLES1L18 | XXIC1 | T6E1 | R24 | C5 | r3-c1 | r2-c4 |
| VACUOLAR ATP SYNTHASE 16 KDA PROTEOLIPID SUBUNIT (EC 3.6.1.34) (V- ATPASE 16 KDA PROTEOLIPID SUBUNIT | TC77384 | AW092235 | cLET18D11 | XID11 | T3H21 | R4 | C8 | r2-c1 | r4-c3 |
| cytochrome c oxidase subunit Vb precursor-like protein {Arabidopsis thaliana} | TC77386 | BE436938 | cLEG34P1 | VIC4 | T2E8 | R17 | C5 | r4-c2 | r1-c3 |
| PROBABLE RIBOSE 5-PHOSPHATE ISOMERASE (EC 5.3.1.6) (PHOSPHORIBOISOMERASE).GP|4262236|gb|AAD14529.1| | TC77390 | AI775880 | cLER16H24 | IXG6 | T3M11 | R14 | C13 | r2-c1 | r4-c3 |
| phosphoenolpyruvate carboxylase 1 | TC77403 | AI781606 | cLES16D23 | IVF4 | T1L8 | R17 | C12 | r1-c2 | r3-c3 |
| nitrite reductase {Capsicum annuum}; | TC77408 | BF113958 | cLEY23O22 | XIVC1 | T4E2 | R23 | C5 | r2-c2 | r1-c4 |
| hypothetical protein {Arabidopsis thaliana}GP|3281856|emb|CAA19751.1||AL031004 Transcription factor | TC77438 | BG130561 | cTOF31B5 | XXC2 | T5F4 | R21 | C6 | r3-c2 | r4-c4 |
| putative glucose-6-phosphate/phosphate-tranlocat or {Oryza sativa} | TC77448 | AI895217 | cLEC7I15 | IIF1 | T1K2 | R23 | C11 | r1-c2 | r3-c3 |
| uroporphyrinogen decarboxylase precursor {Nicotiana tabacum}SP|Q42967|DCUP_TOBAC UROPORPHYRINOGEN D | TC77455 | AI778274 | cLES4D24 | XVID2 | T4H4 | R21 | C8 | r2-c2 | r1-c4 |
| putative strictosidine synthase; 35901-37889 {Arabidopsis thaliana}PIR|A96768|A96768 protein strict | TC77457 | BE449584 | cLHT32E19 | XVC7 | T4F13 | R12 | C6 | r2-c2 | r1-c4 |
| putative aminotransferase {Arabidopsis thaliana} | TC77480 | AI489286 | cLED17E18 | IIIA12 | T1B23 | R2 | C2 | r1-c2 | r3-c3 |
| TRANSCRIPTION INITIATION FACTOR TFIID 100 KDA SUBUNIT (TAFII-100) (TAFII100).GP|1932938|gb|AAC51215 | TC77486 | BE432492 | cLEG8O16 | VIIA2 | T2B3 | R22 | C2 | r4-c2 | r1-c3 |
| putative glucosyl transferase {Arabidopsis thaliana}PIR|C84784|C84784 probable glucosyl transferase | TC77491 | AI485737 | cLED4G20 | IVA5 | T1B10 | R15 | C2 | r1-c2 | r3-c3 |
| glucose-6-phosphate 1-dehydrogenase {Solanum tuberosum}SP|P37830|G6PD_SOLTU GLUCOSE-6-PHOSPHATE 1-D | TC77502 | BE459880 | cLEM8E24 | IVG2 | T1N4 | R21 | C14 | r1-c2 | r3-c3 |

153

| | | | | | | | | | |
|---|---|---|---|---|---|---|---|---|---|
| omega-6 fatty acid desaturase {Sesamum indicum} | TC77526 | AW031750 | cLEC21B10 | IE8 | T1I15 | R12 | C9 | r1-c2 | r3-c3 |
| putative C3HC4-type RING zinc finger protein {Arabidopsis thaliana}PIR\|T02413\|T02413 probable RING | TC77528 | AW738118 | cTOD5E12 | XVIIH8 | T5O15 | R12 | C15 | r3-c2 | r4-c4 |
| Alfin-1 {Medicago sativa}PIR\|T09646\|T09646 probable zinc finger protein - alfalfa (fragment) | TC77531 | BG643537 | cTOF31G7 | XXC5 | T5F10 | R15 | C6 | r3-c2 | r4-c4 |
| putative ABC transporter; 66585-65723 {Arabidopsis thaliana}PIR\|C96702\|C96702 probable ABC transpor | TC77538 | AI775901 | cLER16L22 | IXG9 | T3M17 | R8 | C13 | r2-c1 | r4-c3 |
| phosphoribosyl pyrophosphate synthase isozyme 3 {Spinacia oleracea} | TC77539 | AI781720 | cLES16L10 | XE12 | T3I24 | R1 | C9 | r2-c1 | r4-c3 |
| TRYPTOPHAN SYNTHASE BETA CHAIN 2 PRECURSOR (EC 4.2.1.20).GP\|2792520\|gb\|AAB97087.1\|\|AF042320 tryptop | TC77555 | AI488512 | cLED23N20 | IIIC10 | T1F19 | R6 | C6 | r1-c2 | r3-c3 |
| indole-3-glycerol phosphate synthase {Arabidopsis thaliana} | TC77561 | AW618611 | cLPT14I1 | XVG7 | T4N13 | R12 | C14 | r2-c2 | r1-c4 |
| cytochrome P450 {Solanum tuberosum} | TC77562 | AI896104 | cLEC13J4 | IC11 | T1E21 | R4 | C5 | r1-c2 | r3-c3 |
| acetyl-CoA carboxylase {Medicago sativa}GP\|495725\|gb\|AAB42144.1\|\|L25042 acetyl-CoA carboxylase {Med | TC77572 | BE433724 | cLEG20L23 | VG4 | T2M7 | R18 | C13 | r4-c2 | r1-c3 |
| 3-isopropylmalate dehydratase, small subunit {Arabidopsis thaliana}PIR\|H84861\|H84861 3-isopropylmal | TC77576 | AW649879 | cLEI9P8 | VIIG8 | T2N15 | R12 | C14 | r4-c2 | r1-c3 |
| MADS-box transcription factor FBP22 {Petunia x hybrida} | TC77597 | AW623098 | cTOB8E22 | XVIIB9 | T5C17 | R8 | C3 | r3-c2 | r4-c4 |
| probable UDP-glucuronosyltransferase (EC 2.4.1.-) - garden pea | TC77599 | AI897234 | cLED27K11 | IIIE2 | T1J3 | R22 | C10 | r1-c2 | r3-c3 |
| NADH dehydrogenase {Solanum tuberosum}GP\|639834\|emb\|CAA58823.1\|\|X83999 NADH dehydrogenase {Solanum | TC77602 | AW929834 | cTOC5H20 | XVIIE2 | T5I3 | R22 | C9 | r3-c2 | r4-c4 |
| phosphoglucomutase-like protein {Arabidopsis thaliana}PIR\|T51457\|T51457 phosphoglucomutase-like pro | TC77603 | BG135437 | cTOE22H5 | XVIIIE5 | T5I10 | R15 | C9 | r3-c2 | r4-c4 |
| biotin-binding protein^^biotin-containing subunit of methylcrotonyl-CoA carboxylase | TC77606 | BF112416 | cLEG41E6 | VIF2 | T2K4 | R21 | C11 | r4-c2 | r1-c3 |
| ATP:citrate lyase {Capsicum annuum} | TC77626 | AW617219 | cLHT22E7 | XIIIB12 | T4C23 | R2 | C3 | r2-c2 | r1-c4 |
| putative cytochrome P450 {Solanum | TC77648 | AI771126 | cLED28H1 | IIIE3 | T1J5 | R20 | C10 | r1-c2 | r3-c3 |

154

| Description | TC | Accession | Clone | Roman | T | R | C | Map1 | Map2 |
|---|---|---|---|---|---|---|---|---|---|
| chacoense}SP|P93530|C7D6_SOLCH CYTOCHROME P450 71D6 (EC 1.14.-.-) | | | 3 | | | | | | |
| cytochrome c oxidase subunit 6b {Oryza sativa}GP|9967162|dbj|BAB12275.1||AB047923 cytochrome c oxid | TC77660 | AI484719 | cLED3L3 | IVA2 | T1B4 | R21 | C2 | r1-c2 | r3-c3 |
| glycerol-3-phosphate acyltransferase {Cucumis sativus}SP|Q39639|PLSB_CUCSA GLYCEROL-3-PHOSPHATE ACY | TC77666 | AW623199 | cTOB9G15 | XVIIB12 | T5C23 | R2 | C3 | r3-c2 | r4-c4 |
| S-adenosyl-L-methionine:salicylic acid carboxyl methyltransferase-like protein {Arabidopsis thaliana | TC77684 | AW738171 | cTOD5O22 | XVIIIB5 | T5C10 | R15 | C3 | r3-c2 | r4-c4 |
| 3-deoxy-D-arabino-heptulosonate 7-phosphate synthase {Morinda citrifolia} | TC77693 | AW738296 | cTOD6J18 | XVIIIA3 | T5A6 | R19 | C1 | r3-c2 | r4-c4 |
| serine hydroxymethyltransferase, mitochondrial precursor (serine methylase) (glycine hydroxymethyltr | TC77724 | BE460801 | cLEG36D13 | VIC10 | T2E20 | R5 | C5 | r4-c2 | r1-c3 |
| putative sugar transporter {Arabidopsis thaliana} | TC77728 | AW622974 | cTOB7H5 | IVG4 | T1N8 | R17 | C14 | r1-c2 | r3-c3 |
| alpha-glucan phosphorylase, l isozyme 2 precursor (starch phosphorylase l-2) {Solanum tuberosum}SP| | TC77734 | AW738276 | cTOD6F22 | IA1 | T1A1 | R24 | C1 | r1-c2 | r3-c3 |
| HB2 homeodomain protein {Populus tremula x Populus tremuloides} | TC77747 | AW618062 | cLPT11L16 | XVF11 | T4L21 | R4 | C12 | r2-c2 | r1-c4 |
| Strong similarity to gb|U61231 cytochrome P450 from Arabidopsis thaliana and is a member of the PF|0 | TC77757 | AI484951 | cLED2F4 | IIIE9 | T1J17 | R8 | C10 | r1-c2 | r3-c3 |
| succinyl-CoA synthetase, alpha subunit {Arabidopsis thaliana} | TC77763 | BG125244 | cTOF8E15 | XVIF9 | T4L18 | R7 | C12 | r2-c2 | r1-c4 |
| NADH dehydrogenase {Solanum tuberosum}GP|639834|emb|CAA58823.1||X83999 NADH dehydrogenase {Solanum | TC77792 | AI778324 | cLES4P12 | XH9 | T3O18 | R7 | C15 | r2-c1 | r4-c3 |
| glucose-6-phosphate/phosphate-translocator precursor {Pisum sativum}PIR|T06254|T06254 glucose-6-pho | TC77793 | BE450870 | cLEY15O8 | XIIIH9 | T4O17 | R8 | C15 | r2-c2 | r1-c4 |
| pyrophosphate--fructose 6-phosphate 1-phosphotransferase alpha subunit (pfp) (6-phosphofructokinase | TC77808 | BE434021 | cLEG13H5 | XVIH1 | T4P2 | R23 | C16 | r2-c2 | r1-c4 |
| adenosine kinase {Arabidopsis thaliana}GP|7378610|emb|CAB83286.1||AL162751 adenosine kinase-like pr | TC77810 | AW615848 | cTOA17I8 | XVID10 | T4H20 | R5 | C8 | r2-c2 | r1-c4 |
| sucrose transporter {Lycopersicon esculentum} | TC77814 | BF050231 | cLEM17E17 | VIIG10 | T2N19 | R6 | C14 | r4-c2 | r1-c3 |
| transketolase 1 {Capsicum | TC77838 | AW442085 | cLEN21E6 | IXB12 | T3C23 | R2 | C3 | r2-c1 | r4-c3 |

| Description | TC# | Accession | Clone | | | | | | |
|---|---|---|---|---|---|---|---|---|---|
| annuum}PIR|T09541|T09541 transketolase (EC 2.2.1.1) TKT1 precursor, chlor | | | | | | | | | |
| transcription factor inhibitor I kappa B homolog {Arabidopsis thaliana} GP|7773295|gb|AAC49611.1||U7 | TC77842 | BE432768 | cLEG10A24 | VH2 | T2O3 | R22 | C15 | r4-c2 | r1-c3 |
| aspartate-semialdehyde dehydrogenase precursor {Arabidopsis thaliana} | TC77844 | BG124618 | cTOF5F3 | XXH3 | T5P6 | R19 | C16 | r3-c2 | r4-c4 |
| putative flavonol 3-O-glucosyltransferase {Arabidopsis thaliana} PIR|F84618|F84618 probable flavonol | TC77847 | AW091930 | cLET17G11 | VIIF1 | T2L1 | R24 | C12 | r4-c2 | r1-c3 |
| ornithine carbamoyltransferase; OCTase {Canavalia lineata} GP|12003188|gb|AAG43481.1|AF203688_1|AF 20 | TC77862 | BG130753 | cTOEIM7 | XVIIID11 | T5G22 | R3 | C7 | r3-c2 | r4-c4 |
| NADH-dependent glutamate synthase {Medicago sativa} | TC77867 | BE449812 | cLEY14O21 | XIIIG12 | T4M23 | R2 | C13 | r2-c2 | r1-c4 |
| contains similarity to transcription regulator~gene_id:MRG7.19 {Arabidopsis thaliana} | TC77873 | BE436142 | cLEG30J20 | VIA3 | T2A6 | R19 | C1 | r4-c2 | r1-c3 |
| contains similarity to C2H2-type zinc finger protein~gene_id:MOK16.6 {Arabidopsis thaliana} | TC77881 | AW648552 | cLEI4P14 | VC8 | T2E15 | R12 | C5 | r4-c2 | r1-c3 |
| putative zinc finger protein {Oryza sativa} | TC77907 | AW094020 | cLET27A3 | XVIIE9 | T5I17 | R8 | C9 | r3-c2 | r4-c4 |
| putative hydroxymethyltransferase; 49598-47322 {Arabidopsis thaliana} PIR|F86484|F86484 probable hyd | TC77914 | AW030791 | cLEC22A6 | XXIIA2 | T6A4 | R21 | C1 | r3-c1 | r2-c4 |
| deoxyuridine triphosphatase, dUTPase, P18 {EC 3.6.1.23} [tomatoes, Tint Tim cultivar LA154, Peptide, 169 aa] | TC77923 | AI486505 | cLED6E4 | IVB7 | T1D14 | R11 | C4 | r1-c2 | r3-c3 |
| cytochrome P450, putative {Arabidopsis thaliana} PIR|F86441|F86441 probable cytochrome P450 importe | TC77959 | AI773114 | cLER5K20 | XC1 | T3E2 | R23 | C5 | r2-c1 | r4-c3 |
| NAD-malate dehydrogenase {Nicotiana tabacum} | TC77971 | BE435129 | cLEG25M1 | XVIIC12 | T5E23 | R2 | C5 | r3-c2 | r4-c4 |
| aspartate aminotransferase {Panicum miliaceum} GP|20597|emb|CAA45022.1||X63428 aspartate aminotransf | TC77973 | AI898706 | cLED35I22 | IIIG11 | T1N21 | R4 | C14 | r1-c2 | r3-c3 |
| phosphoenolpyruvate carboxylase {Nicotiana tabacum} SP|P27154|CAPP_TOBAC PHOSPHOENOLPYRUVATE CARBOXY | TC77975 | BF114018 | cLEY23H10 | XIVB12 | T4C24 | R1 | C3 | r2-c2 | r1-c4 |
| Contains similarity to DNA-binding protein MYB1 from Petroselinum crispum GP|7488946 and contains MY | TC77983 | BE450376 | cLEY13G13 | XIIIG2 | T4M3 | R22 | C13 | r2-c2 | r1-c4 |

| | | | | | | | | |
|---|---|---|---|---|---|---|---|---|---|
| phosphatidylinositol 4-kinase {Solanum tuberosum} | TC78010 | AI771897 | cLED38E12 | IIIH5 | T1P9 | R16 | C16 | r1-c2 | r3-c3 |
| L-allo-threonine aldolase homolog F22O13.11 - Arabidopsis thaliana | TC78023 | BE437087 | cLEG35M18 | XIF12 | T3L23 | R2 | C12 | r2-c1 | r4-c3 |
| argininosuccinate synthase-like protein {Arabidopsis thaliana}GP\|7269334\|emb\|CAB79393.1\|AL161562 a | TC78033 | AW029695 | cLEC11G5 | IC2 | T1E3 | R22 | C5 | r1-c2 | r3-c3 |
| hyoscyamine 6-dioxygenase hydroxylase, putative {Arabidopsis thaliana}PIR\|G86472\|G86472 probable hy | TC78043 | BE434473 | cLEG17M15 | VF6 | T2K11 | R14 | C11 | r4-c2 | r1-c3 |
| SNF2 subfamily global transcription activator {Arabidopsis thaliana}PIR\|G84897\|G84897 hypothetical | TC78051 | BG127884 | cTOF18K14 | XIXC1 | T5F1 | R24 | C6 | r3-c2 | r4-c4 |
| CYTOCHROME P450 98A2 (EC 1.14.-.-).GP\|2738998\|gb\|AAB94587.1\|AF022458 CYP98A2p {Glycine max}PIR\|T0 | TC78082 | AI895234 | cLEC7M3 | IIF3 | T1K6 | R19 | C11 | r1-c2 | r3-c3 |
| branched-chain amino acid aminotransferase {Solanum tuberosum} | TC78133 | AW034526 | cLEC24K18 | IF7 | T1K13 | R12 | C11 | r1-c2 | r3-c3 |
| geranylgeranyl pyrophosphate synthase-related protein {Arabidopsis thaliana}GP\|7270829\|emb\|CAB80510 | TC78147 | BE436393 | cLEG32A20 | XIH4 | T3P7 | R18 | C16 | r2-c1 | r4-c3 |
| putative phosphatidylserine decarboxylase {Arabidopsis thaliana}GP\|7269448\|emb\|CAB79452.1\|AL161564 | TC78160 | BF112654 | cLEG42C5 | VIF7 | T2K14 | R11 | C11 | r4-c2 | r1-c3 |
| cytochrome P450 {Catharanthus roseus}PIR\|T09999\|T09999 cytochrome P450 - Madagascar periwinkle | TC78161 | AW035889 | cLEC35F7 | IIB3 | T1C6 | R19 | C3 | r1-c2 | r3-c3 |
| CYTOCHROME P450 71A9 (EC 1.14.-.-) (P450 CP1).GP\|3334659\|emb\|CAA71513.1\|Y10489 putative cytochrome | TC78170 | AW031676 | cLEC37B20 | IIB12 | T1C24 | R1 | C3 | r1-c2 | r3-c3 |
| bA554C12.1 (RBX1 or ROC1 (ring-box or ring finger protein 1)) {Homo sapiens}GP\|4769004\|gb\|AAD29715. | TC78191 | AW092264 | cLET18J1 | XID12 | T3H23 | R2 | C8 | r2-c1 | r4-c3 |
| alpha amylase precursor {Cuscuta reflexa}GP\|458456\|gb\|AAA16513.1\|U06754 alpha amylase precursor {C | TC78197 | BE460877 | cLEG36F6 | VIC11 | T2E22 | R3 | C5 | r4-c2 | r1-c3 |
| putative strictosidine synthase-like {Arabidopsis thaliana} | TC78210 | BG628308 | cLEL21K15 | XXIE5 | T6I9 | R16 | C9 | r3-c1 | r2-c4 |
| malate dehydrogenase, mitochondrial precursor {Citrullus vulgaris}EGAD\|148462\|158380 hypothetical p | TC78217 | BG134615 | cTOE16N4 | XVIIID2 | T5G4 | R21 | C7 | r3-c2 | r4-c4 |
| flavanone 3-hydroxylase {Citrus sinensis} | TC78218 | BG628122 | cLEL20G15 | XXIE3 | T6I5 | R20 | C9 | r3-c1 | r2-c4 |
| Contains similarity to gb\|Y13720 Hap2a transcription | TC78236 | BF051108 | cLEM21H2 | VIIIA5 | T2B10 | R15 | C2 | r4-c2 | r1-c3 |

| | | | | | | | | | |
|---|---|---|---|---|---|---|---|---|---|
| factor from Arabidopsis thaliana.PIR\|A86430\|A8 | | | 1 | | | | | | |
| GALACTOKINASE (EC 2.7.1.6) (GALACTOSE KINASE).GP\|12322687\|gb\|AAG51339.1\|AC020580_19 \|AC020580 galact | TC78245 | AW624648 | cTOB16F12 | XVIIA6 | T5A11 | R14 | C1 | r3-c2 | r4-c4 |
| DIACYLGLYCEROL KINASE 1 (EC 2.7.1.107) (DIGLYCERIDE KINASE) (DGK 1) (DAG KINASE 1).GP\|1374772\|dbj\|B | TC78269 | AI486282 | cLED8G16 | IVC8 | T1F16 | R9 | C6 | r1-c2 | r3-c3 |
| myb-related transcription factor LBM1 {Nicotiana tabacum} | TC78270 | AW030476 | cLEC11D1 0 | IIB5 | T1C10 | R15 | C3 | r1-c2 | r3-c3 |
| transcription factor {Vicia faba}GP\|2104679\|emb\|CAA66480.1\|\|X97906 transcription factor {Vicia faba | TC78324 | BG126414 | cTOF12A2 4 | XVIIIH1 | T5O2 | R23 | C15 | r3-c2 | r4-c4 |
| alpha-glucan phosphorylase, h isozyme phosphorylase h) {Solanum tuberosum}SP\|P32811\|PHSH_SOLTU ALPH | TC78327 | AW616770 | cLHT12L6 | XVA2 | T4B3 | R22 | C2 | r2-c2 | r1-c4 |
| isocitrate dehydrogenase (NAD+) {Solanum tuberosum} | TC78346 | BG644139 | cTOF34O1 7 | XXD7 | T5H14 | R11 | C8 | r3-c2 | r4-c4 |
| NADH dehydrogenase (ubiquinone) (EC 1.6.5.3) chain nad9 - wheat mitochondrion | TC78368 | AI485504 | cLED7A14 | IVC3 | T1F6 | R19 | C6 | r1-c2 | r3-c3 |
| Zn finger protein {Nicotiana tabacum}GP\|1360086\|emb\|CAA66605.1\|\|X97946 Zn finger protein {Nicotiana | TC78369 | BF097661 | cLEW23I9 | XIIG5 | T3N10 | R15 | C14 | r2-c1 | r4-c3 |
| flavonoid 3',5'-hydroxylase-like; cytochrome P450 {Arabidopsis thaliana} | TC78380 | AW623747 | cTOB13C1 3 | XVIG5 | T4N10 | R15 | C14 | r2-c2 | r1-c4 |
| geranylgeranyl pyrophosphate synthetase precursor (ggpp synthetase) (dimethylallyltransferase {Capsi | TC78381 | BE434535 | cLEG17J7 | VIIA7 | T2B13 | R12 | C2 | r4-c2 | r1-c3 |
| MYB-like DNA-binding domain protein {Gossypium hirsutum}PIR\|T09745\|T09745 myb-related protein - upl | TC78385 | AI488744 | cLED13H1 5 | IIH6 | T1O12 | R13 | C15 | r1-c2 | r3-c3 |
| homeobox 2 protein | TC78390 | AI897002 | cLED26E23 | IIID9 | T1H17 | R8 | C8 | r1-c2 | r3-c3 |
| Contains similarity to dTPD-D-glucose-4,6-dehydratase from Sphingomonas sp.S88 gb\|U51197 and contain | TC78391 | AI486284 | cLED8I4 | IVC9 | T1F18 | R7 | C6 | r1-c2 | r3-c3 |
| fructokinase | TC78393 | BF098243 | cLEW26O6 | XIIH2 | T3P4 | R21 | C16 | r2-c1 | r4-c3 |
| fructose-6-phosphate 2-kinase/fructose-2,6-bisphosphatase {Solanum tuberosum}PIR\|T07016\|T07016 6-ph | TC78403 | AI896831 | cLEC23B12 | IF1 | T1K1 | R24 | C11 | r1-c2 | r3-c3 |
| cytochrome P450 {Nicotiana tabacum}GP\|1171579\|emb\|CAA64635.1\|\|X95342 | TC78431 | AW616606 | cLHT11J2 | XIVH9 | T4O18 | R7 | C15 | r2-c2 | r1-c4 |

| | | | | | | | | | |
|---|---|---|---|---|---|---|---|---|---|
| cytochrome P450 {Nicotiana tab | | | | | | | | | |
| MADS-box transcription factor FBP21 {Petunia x hybrida} | TC78439 | AW039132 | cLET8H21 | XIIC9 | T3F18 | R7 | C6 | r2-c1 | r4-c3 |
| 76 kDa mitochondrial complex I subunit {Solanum tuberosum}SP|Q43644|NUAM_SOLTU NADH-UBIQUINONE OXID | TC78448 | BE460354 | cLEG28D24 | VH5 | T2O9 | R16 | C15 | r4-c2 | r1-c3 |
| contains similarity to SNF2/RAD54 family (RAD26 subfamily) transcription-repair coupling factor~gene | TC78453 | AW615869 | cTOA17M20 | XVIE2 | T4J4 | R21 | C10 | r2-c2 | r1-c4 |
| homeodomain protein {Malus x domestica} | TC78458 | AW031305 | cLEC35K11 | IIB7 | T1C14 | R11 | C3 | r1-c2 | r3-c3 |
| transcription regulator Sir2-like protein {Arabidopsis thaliana}GP|12006420|gb|AAG44850.1|AF283757 | TC78492 | AW441312 | cLEN15I15 | IXA5 | T3A9 | R16 | C1 | r2-c1 | r4-c3 |
| pyrophosphate-dependent phosphofructokinase beta subunit {Citrus x paradisi} | TC78498 | AI896796 | cLEC23J17 | IF2 | T1K3 | R22 | C11 | r1-c2 | r3-c3 |
| CYP83D1p {Glycine max}PIR|T05940|T05940 cytochrome P450 83D1p - soybean (fragment) | TC78508 | BG125130 | cTOF7B4 | XXF5 | T5L10 | R15 | C12 | r3-c2 | r4-c4 |
| Putative UDP-glucose:sterol glucosyltransferase {Arabidopsis thaliana}PIR|D96499|D96499 probable UD | TC78531 | BG128011 | cTOF18B24 | XIXB8 | T5D15 | R12 | C4 | r3-c2 | r4-c4 |
| contains similarity to cyclopropane fatty acid synthase~gene_id:MEE5.5 {Arabidopsis thaliana} | TC78544 | AW399730 | cLPT8J6 | XVIC4 | T4F8 | R17 | C6 | r2-c2 | r1-c4 |
| zinc finger and C2 domain protein {Arabidopsis thaliana} | TC78550 | AW039744 | cLET13K10 | XIC11 | T3F21 | R4 | C6 | r2-c1 | r4-c3 |
| cinnamoyl CoA reductase-like protein {Arabidopsis thaliana}PIR|T48643|T48643 cinnamoyl CoA reductas | TC78570 | BE433500 | cLEG15E22 | VE7 | T2I13 | R12 | C9 | r4-c2 | r1-c3 |
| tyrosine aminotransferase-like protein {Arabidopsis thaliana} | TC78598 | BF096944 | cLEW18G10 | XIIE1 | T3J2 | R23 | C10 | r2-c1 | r4-c3 |
| omega-3 fatty acid desaturase, endoplasmic reticulum {Nicotiana tabacum}SP|P48626|FD3E_TOBAC OMEGA- | TC78627 | BG629236 | cLEL2K2 | XXIG3 | T6M5 | R20 | C13 | r3-c1 | r2-c4 |
| anthranilate phosphoribosyltransferase-like protein {Arabidopsis thaliana}PIR|T46010|T46010 anthran | TC78632 | AW650876 | cLEI14P19 | VIIC4 | T2F7 | R18 | C6 | r4-c2 | r1-c3 |
| putative folylpolyglutamate synthetase {Oryza sativa} | TC78676 | BE441141 | cLEM6E13 | VIIIE2 | T2J4 | R21 | C10 | r4-c2 | r1-c3 |
| transcription factor IIA large subunit {Arabidopsis thaliana}PIR|T51333|T51333 transcription factor | TC78698 | AI894579 | cLEC4L15 | IID10 | T1G20 | R5 | C7 | r1-c2 | r3-c3 |
| putative alpha-amylase; 60344-64829 {Arabidopsis thaliana}PIR|E96720|E96720 probable alpha-amylase | TC78700 | BE436615 | cLEG33F11 | VIB9 | T2C18 | R7 | C3 | r4-c2 | r1-c3 |

| polyneuridine aldehyde esterase {Rauvolfia serpentina} | TC78712 | BG126704 | cTOF13K13 | XVIIIH9 | T5O18 | R7 | C15 | r3-c2 | r4-c4 |
|---|---|---|---|---|---|---|---|---|---|
| 2S seed albumin-1 large subunit [Lycopersicon esculentum] | TC78715 | AW036308 | cLEE1G12 | IVD5 | T1H10 | R15 | C8 | r1-c2 | r3-c3 |
| Strong similarity to F19I3.8 GP|3033381 putative UDP-galactose-4-epimerase from Arabidopsis thaliana | TC78747 | AW623155 | cTOB8J19 | XVIIB11 | T5C21 | R4 | C3 | r3-c2 | r4-c4 |
| Similar to gb|AF135422 GDP-mannose pyrophosphorylase A (GMPPA) from Homo sapiens. ESTs gb|AA712990, | TC78749 | AW094226 | cLET27N17 | XXID12 | T6G23 | R2 | C7 | r3-c1 | r2-c4 |
| heat stress transcription factor 8 | TC78884 | AW223123 | cLEN10H3 | VIIIG3 | T2N6 | R19 | C14 | r4-c2 | r1-c3 |
| pyruvate kinase, cytosolic isozyme {Nicotiana tabacum}SP|Q42954|KPYC_TOBAC PYRUVATE KINASE, CYTOSOL | TC78918 | AI897691 | cLED30K3 | IIIF6 | T1L11 | R14 | C12 | r1-c2 | r3-c3 |
| 2-oxoglutarate/malate translocator precursor-like protein {Arabidopsis thaliana}PIR|T49900|T49900 2 | TC78921 | BG125571 | cTOF9G3 | XVIIH6 | T5O11 | R14 | C15 | r3-c2 | r4-c4 |
| cytochrome P450 {Solanum tuberosum} | TC78950 | BG131258 | cTOE3G12 | XVIIIF6 | T5K12 | R13 | C11 | r3-c2 | r4-c4 |
| CYTOCHROME P450 97B2 (EC 1.14.-.-).GP|2738996|gb|AAB94586.1||AF022457 CYP97B2p {Glycine max}PIR|T0 | TC78953 | AI482691 | cLEB1L15 | IA7 | T1A13 | R12 | C1 | r1-c2 | r3-c3 |
| alpha-glucosidase {Solanum tuberosum}PIR|T07391|T07391 probable alpha-glucosidase (EC 3.2.1.20) - p | TC78967 | BE435693 | cLEG28I14 | VH7 | T2O13 | R12 | C15 | r4-c2 | r1-c3 |
| threonine synthase {Solanum tuberosum} | TC78978 | AW092379 | cLET20E24 | XIE10 | T3J19 | R6 | C10 | r2-c1 | r4-c3 |
| NADH dehydrogenase subunit | TC79000 | AI775474 | cLER15L15 | IXF11 | T3K21 | R4 | C11 | r2-c1 | r4-c3 |
| RING-H2 finger protein RHF2a {Arabidopsis thaliana}GP|13374859|emb|CAC34493.1||AL589883 RING-H2 fin | TC79012 | BG134178 | cTOE15F13 | VIIIC8 | T2F16 | R9 | C6 | r4-c2 | r1-c3 |
| putative C3HC4-type RING zinc finger/ankyrin protein {Arabidopsis thaliana}PIR|E84689|E84689 probab | TC79013 | AI483217 | cLEB8I19 | IIB8 | T1C16 | R9 | C3 | r1-c2 | r3-c3 |
| Dof zinc finger protein {Solanum tuberosum} | TC79103 | AW622638 | cLEX15J2 | XIIIC12 | T4E23 | R2 | C5 | r2-c2 | r1-c4 |
| glucose acyltransferase {Lycopersicon pennellii} | TC79131 | AW399149 | cLPT6K12 | XVIB11 | T4D22 | R3 | C4 | r2-c2 | r1-c4 |
| tyrosine decarboxylase {Arabidopsis thaliana} | TC79134 | AW217489 | cTOB1E23 | XVIIA10 | T5A19 | R6 | C1 | r3-c2 | r4-c4 |
| aldose-1-epimerase-like protein {Nicotiana tabacum}PIR|T01933|T01933 probable aldose 1-epimerase (E | TC79135 | BE450681 | cLEY14N1 | XIIIG9 | T4M17 | R8 | C13 | r2-c2 | r1-c4 |
| putative ABC transporter {Arabidopsis thaliana}GP|4115931|gb|AAD03441.1||AF118223 | TC79147 | AW932006 | cLEF47A5 | IVH3 | T1P6 | R19 | C16 | r1-c2 | r3-c3 |

| Description | TC | Accession | Clone | | | | | | |
|---|---|---|---|---|---|---|---|---|---|
| contains similari | | | | | | | | | |
| polyneuridine aldehyde esterase {Rauvolfia serpentina} | TC79199 | AW621893 | cLEX13L15 | XIIIC5 | T4E9 | R16 | C5 | r2-c2 | r1-c4 |
| glycogen (starch) synthase precursor {Solanum tuberosum}SP|Q00775|UGST_SOLTU GRANULE-BOUND GLYCOGEN | TC79234 | AW399814 | cLPT10L12 | XVF9 | T4L17 | R8 | C12 | r2-c2 | r1-c4 |
| alpha glucosidase-like protein {Arabidopsis thaliana} | TC79238 | AW223788 | cLEN13L15 | VIIIH7 | T2P14 | R11 | C16 | r4-c2 | r1-c3 |
| transcription factor TEIL {Nicotiana tabacum} | TC79239 | BF112869 | cLEG42L9 | VIF9 | T2K18 | R7 | C11 | r4-c2 | r1-c3 |
| neutral invertase, putative {Arabidopsis thaliana}GP|12324537|gb|AAG52223.1|AC021665_6|A C021665 put | TC79243 | BG129477 | cTOF24J3 | XIXG7 | T5N13 | R12 | C14 | r3-c2 | r4-c4 |
| putative alpha-amylase; 60344-64829 {Arabidopsis thaliana}PIR|E96720|E96720 probable alpha-amylase | TC79253 | BE461271 | cLEG38E23 | VIE1 | T2I2 | R23 | C9 | r4-c2 | r1-c3 |
| HOMEOBOX-LEUCINE ZIPPER PROTEIN HAT7 (HD-ZIP PROTEIN 7) (HD-ZIP PROTEIN ATHB-3).GP|54989|gb|AAA569 | TC79277 | BF098451 | cLEW27E6 | XIIH4 | T3P8 | R17 | C16 | r2-c1 | r4-c3 |
| Similar to UTP-glucose glucosyltransferases {Arabidopsis thaliana}PIR|G86144|G86144 hypothetical pr | TC79292 | AI779058 | cLES7A11 | XIA6 | T3B11 | R14 | C2 | r2-c1 | r4-c3 |
| CYP83D1p {Glycine max}PIR|T05940|T05940 cytochrome P450 83D1p - soybean (fragment) | TC79302 | AI895521 | cLEC9E21 | IIG2 | T1M4 | R21 | C13 | r1-c2 | r3-c3 |
| mas-binding factor MBF2=transcription factor TGA1a homolog {Solanum tuberosum=potatoes, root, Peptid | TC79327 | BF114202 | cLEY25L20 | XIVC3 | T4E6 | R19 | C5 | r2-c2 | r1-c4 |
| cytochrome P450 {Arabidopsis thaliana} | TC79360 | AI898424 | cLED3318 | IIIG3 | T1N5 | R20 | C14 | r1-c2 | r3-c3 |
| invertase inhibitor homolog {Nicotiana tabacum}PIR|T03396|T03396 invertase inhibitor homolog - comm | TC79368 | AW621436 | cLEX11P22 | XIIIB8 | T4C15 | R20 | C3 | r2-c2 C | r1-c4 |
| putative cytochrome P450 {Arabidopsis thaliana}GP|13877661|gb|AAK43908.1|AF370589_1|A F370589 putati | TC79382 | BE432605 | cLEG9B5 | VIIA4 | T2B7 | R18 | C2 | r4-c2 | r1-c3 |
| triosephosphate isomerase, cytosolic {Coptis japonica}SP|P21820|TPIS_COPJA TRIOSEPHOSPHATE ISOMERAS | TC79385 | AI898957 | cLED36B9 | IIIH1 | T1P1 | R24 | C16 | r1-c2 | r3-c3 |
| putative ABC transporter; 60211-54925 {Arabidopsis thaliana}PIR|E96742|E96742 probable ABC transpor | TC79386 | AI488366 | cLED21K4 | IIIC5 | T1F9 | R16 | C6 | r1-c2 | r3-c3 |
| tyrosine aminotransferase-like protein {Arabidopsis thaliana} | TC79388 | AW221912 | cLED19D8 | IXD2 | T3G3 | R22 | C7 | r2-c1 | r4-c3 |
| fructose-6-phosphate 2-kinase/fructose-2,6- | TC79403 | AW651075 | cLEI15M10 | VIIC8 | T2F15 | R12 | C6 | r4-c2 | r1-c3 |

| bisphosphatase {Solanum tuberosum}PIR\|T07016\|T07016 6-ph | | | | | | | | | |
|---|---|---|---|---|---|---|---|---|---|
| putative glycerol-3-phosphate dehydrogenase {Arabidopsis thaliana} | TC79406 | AW219948 | cLEX6M3 | XIIIF3 | T4K5 | R20 | C11 | r2-c2 | r1-c4 |
| putative cytochrome P450 {Oryza sativa} | TC79461 | AW219566 | cLEX4P4 | XIIIE9 | T4I17 | R8 | C9 | r2-c2 | r1-c4 |
| cytochrome P450 {Arabidopsis thaliana} | TC79463 | AW160267 | cLPT1K21 | XVH10 | T4P19 | R6 | C16 | r2-c2 | r1-c4 |
| anthranilate synthase alpha subunit {Catharanthus roseus} | TC79471 | AW218352 | cLEZ7H6 | XIVG5 | T4M10 | R15 | C13 | r2-c2 | r1-c4 |
| homeodomain protein {Malus x domestica} | TC79485 | AW737981 | cTOD4J4 | XVIIH4 | T5O7 | R18 | C15 | r3-c2 | r4-c4 |
| ferredoxin--nitrite reductase (EC 1.7.7.1) nir-3 - common tobacco (fragment) | TC79488 | AI776122 | cLER17B5 | IXH1 | T3O1 | R24 | C15 | r2-c1 | r4-c3 |
| putative sugar transporter {Arabidopsis thaliana} | TC79538 | AW979980 | cLEW10M1 7 | XIID4 | T3H8 | R17 | C8 | r2-c1 | r4-c3 |
| transcription factor-like protein {Arabidopsis thaliana} | TC79544 | AW624235 | cTOB15G6 | XVIIA3 | T5A5 | R20 | C1 | r3-c2 | r4-c4 |
| Similar to UTP-glucose glucosyltransferases {Arabidopsis thaliana}PIR\|G86144\|G86144 hypothetical pr | TC79554 | AW979370 | cLEW1O16 | XIIF11 | T3L22 | R3 | C12 | r2-c1 | r4-c3 |
| RING finger protein {Arabidopsis thaliana}GP\|4689366\|gb\|AAD27870.1\|AF134155_1\|AF 134155 RING finger | TC79564 | AA824862 | CT149 | XVIC9 | T4F18 | R7 | C6 | r2-c2 | r1-c4 |
| hexokinase | TC79603 | AI487152 | cLED9E12 | XXIIA10 | T6A20 | R5 | C1 | r3-c1 | r2-c4 |
| starch-branching enzyme-like protein {Arabidopsis thaliana} | TC79638 | BG128764 | cTOF22C16 | XIXE7 | T5J13 | R12 | C10 | r3-c2 | r4-c4 |
| formyltransferase purU homolog {Arabidopsis thaliana}GP\|2245095\|emb\|CAB10517.1\|\|Z97343 formyltransf | TC79642 | AW399253 | cLPT6B20 | XVIB6 | T4D12 | R13 | C4 | r2-c2 | r1-c4 |
| putative heat shock transcription factor {Arabidopsis thaliana}PIR\|T02609\|T02609 probable heat shoc | TC79646 | AI637387 | DB#357 | XXG11 | T5N22 | R3 | C14 | r3-c2 | r4-c4 |
| Identical to A. thaliana Myb-like protein (gb\|D58424). {Arabidopsis thaliana}PIR\|F86231\|F86231 hypo | TC79650 | AW625194 | cLEZ11M1 2 | XIVD2 | T4G4 | R21 | C7 | r2-c2 | r1-c4 |
| nucleoside diphosphate kinase {Pisum sativum} | TC79659 | AW219287 | cLEX3P2 | XIIIE4 | T4I7 | R18 | C9 | r2-c2 | r1-c4 |
| putative C3HC4-type RING zinc finger/ankyrin protein {Arabidopsis thaliana}PIR\|E84689\|E84689 probab | TC79668 | AW651244 | cLEI16E1 | VIIC10 | T2F19 | R6 | C6 | r4-c2 | r1-c3 |
| isopentenyl diphosphate isomerase 1 {Nicotiana tabacum} | TC79669 | AI899441 | cLES11G14 | XD3 | T3G6 | R19 | C7 | r2-c1 | r4-c3 |
| vacuolar ATP synthase catalytic subunit a kDa subunit) {Daucus carota}SP\|P09469\|VATA_DAUCA | TC79686 | BG133536 | cTOE13N1 3 | XVIIIC2 | T5E4 | R21 | C5 | r3-c2 | r4-c4 |

EP 1 454 993 A1

| | | | | | | | | | |
|---|---|---|---|---|---|---|---|---|---|
| VACUOLAR zinc-finger-like protein {Arabidopsis thaliana}PIR|T45654|T45654 zinc-finger-like protein - Arabido | TC79692 | AI780412 | cLES11N11 | XD5 | T3G10 | R15 | C7 | r2-c1 | r4-c3 |
| phaseolin G-box binding protein PG1 {Phaseolus vulgaris}GP|1142619|gb|AAB00686.1||U18348 phaseolin | TC79707 | AW622187 | cLEX14B17 | XIIIC7 | T4E13 | R12 | C5 | r2-c2 | r1-c4 |
| ALTERNATIVE OXIDASE 1 PRECURSOR (EC 1.-.-.),GP|558054|gb|AAC60576.1||S71335 alternative oxidase, A | TC79757 | BE449757 | cLEY14E21 | XIIIG5 | T4M9 | R16 | C13 | r2-c2 | r1-c4 |
| dTDP-glucose 4-6-dehydratase-like protein {Arabidopsis thaliana}PIR|T45892|T45892 dTDP-glucose 4-6- | TC79798 | AW035334 | cLEC40J6 | IID7 | T1G14 | R11 | C7 | r1-c2 | r3-c3 |
| flavonol 3-o-glucosyltransferase 6 {Manihot esculenta}SP|Q40288|UFO6_MANES FLAVONOL 3-O-GLUCOSYLTRA | TC79809 | AW219975 | cLEX6O3 | XIIIF6 | T4K11 | R14 | C11 | r2-c2 | r1-c4 |
| soluble starch (bacterial glycogen) synthase {Solanum tuberosum}SP|P93568|UGS2_SOLTU SOLUBLE GLYCOG | TC79837 | AW398573 | cLPT2F24 | XVIA3 | T4B6 | R19 | C2 | r2-c2 | r1-c4 |
| lipoxygenase {Lycopersicon esculentum}GP|1654138|gb|AAB65766.1||U37839 lipoxygenase {Lycopersicon e | TC79855 | BG627392 | cLEL17E23 | XXIC10 | T6E19 | R6 | C5 | r3-c1 | r2-c4 |
| alpha-glucosidase {Solanum tuberosum}PIR|T07391|T07391 probable alpha-glucosidase (EC 3.2.1.20) - p | TC79865 | AW442724 | cLET41J16 | XIIA2 | T3B4 | R21 | C2 | r2-c1 | r4-c3 |
| putative tyrosine decarboxylase {Arabidopsis thaliana}PIR|A84588|A84588 probable tyrosine decarboxy | TC79868 | AI898611 | cLED34F18 | IIIG7 | T1N13 | R12 | C14 | r1-c2 | r3-c3 |
| CYTOCHROME P450 83B1 (EC 1.14.-.-).GP|3164126|dbj|BAA28531.1||D78598 cytochrome P450 monooxygenase | TC79908 | AW038144 | cLET1B18 | XIE5 | T3J9 | R16 | C10 | r2-c1 | r4-c3 |
| lipoxygenase {Solanum tuberosum}GP|1117793|gb|AAD09202.1||U24232 lipoxygenase {Solanum tuberosum}P | TC79919 | BG128802 | cTOF22I24 | XIXF1 | T5L1 | R24 | C12 | r3-c2 | r4-c4 |
| putative ABC transporter; 73228-76244 {Arabidopsis thaliana} | TC79941 | AW442122 | cLEN21M4 | IXC2 | T3E3 | R22 | C5 | r2-c1 | r4-c3 |
| cytochrome p450 lxxviia2 {Solanum | TC79994 | AI779498 | cLES8M17 | XIB2 | T3D3 | R22 | C4 | r2-c1 | r4-c3 |

163

| | | | | | | | | | |
|---|---|---|---|---|---|---|---|---|---|
| melongena}SP\|P37124\|C772_SOLME CYTOCHROME P450 77A2 (EC 1.14.-.-) | | | | | | | | | |
| ARF GAP-like zinc finger-containing protein ZiGA4 {Arabidopsis thaliana} | TC80002 | AW399699 | cLPT8B12 | XVIC2 | T4F4 | R21 | C6 | r2-c2 | r1-c4 |
| auxin-induced basic helix-loop-helix transcription factor, putative {Arabidopsis thaliana}GP\|123213 | TC80007 | BG127646 | cTOF17M2 | XIXB6 | T5D11 | R14 | C4 | r3-c2 | r4-c4 |
| contains similarity to limonene cyclase~gene_id:K15O15.2 {Arabidopsis thaliana} | TC80009 | AW442145 | cLEN21D5 | IXB11 | T3C21 | R4 | C3 | r2-c1 | r4-c3 |
| CYTOCHROME P450 90A1 (EC 1.14.-.-).GP\|853719\|emb\|CAA60793.1\|\|X87367 CYP90 protein {Arabidopsis thal | TC80029 | BG631306 | cLEL7D21 | XXH9 | T5P18 | R7 | C16 | r3-c2 | r4-c4 |
| CYTOCHROME P450 84A1 (FERULATE-5-HYDROXYLASE) (EC 1.14.-.-) (F5H).GP\|1488255\|gb\|AAC49389.1\|\|U38416 | TC80030 | AI780540 | cLES12C18 | XD7 | T3G14 | R11 | C7 | r2-c1 | r4-c3 |
| transcription factor RUSH-1alpha isolog; 18684-24052 {Arabidopsis thaliana}PIR\|A86245\|A86245 hypoth | TC80033 | BG630066 | cLEL31A17 | XXIH7 | T6O13 | R12 | C15 | r3-c1 | r2-c4 |
| FLAVONOID 3',5'-HYDROXYLASE (EC 1.14.-.-) (F3'5'H) (CYTOCHROME P450 75A4).GP\|1620009\|dbj\|BAA12735.1 | TC80085 | AW030933 | cLEC5M11 | IIE2 | T1I4 | R21 | C9 | r1-c2 | r3-c3 |
| 76 kDa mitochondrial complex I subunit {Solanum tuberosum}SP\|Q43644\|NUAM_SOLTU NADH-UBIQUINONE OXID | TC80110 | BG642432 | cTOD11I5 | XVH2 | T4P3 | R22 | C16 | r2-c2 | r1-c4 |
| ABC transporter homolog {Populus nigra} | TC80115 | BF096365 | cLEW11G12 | XIID5 | T3H10 | R15 | C8 | r2-c1 | r4-c3 |
| Contains similarity to 12S seed storage globulin precursor GP\|134919.  ESTs gb\|T13642, gb\|T21684 and | TC80151 | BG628122 | cLEL20G15 | XXIE4 | T6I7 | R18 | C9 | r3-c1 | r2-c4 |
| decarboxylase like protein {Arabidopsis thaliana}GP\|2245025\|emb\|CAB10445.1\|\|Z97341 decarboxylase li | TC80187 | BE451597 | cLEY20L7 | XIVB6 | T4C12 | R13 | C3 | r2-c2 | r1-c4 |
| aldose-1-epimerase-like protein {Nicotiana tabacum}PIR\|T01933\|T01933 probable aldose 1-epimerase (E | TC80210 | AI775211 | cLER14J20 | IXF5 | T3K9 | R16 | C11 | r2-c1 | r4-c3 |
| ATP synthase alpha subunit, mitochondrial {Nicotiana plumbaginifolia}SP\|P05495\|ATP0_NICPL ATP SYNTH | TC80243 | AW929036 | cTOC3L1 | XVIID7 | T5G13 | R12 | C7 | r3-c2 | r4-c4 |
| cytochrome p450 lxxia2 {Solanum melongena}SP\|P37118\|C712_SOLME CYTOCHROME | TC80252 | AW034502 | cLEC24A10 | IF4 | T1K7 | R18 | C11 | r1-c2 | r3-c3 |

EP 1 454 993 A1

| | | | | | | | | | |
|---|---|---|---|---|---|---|---|---|---|
| P450 71A2 (EC 1.14.-.-) ( | | | | | | | | | |
| CYTOCHROME P450 71A22 (EC 1.14.-.-).GP\|4678357\|emb\|CAB41167.1\|\|AL049659 cytochrome P450-like protei | TC80253 | AW032176 | cLEC20M3 | IE4 | T1I7 | R18 | C9 | r1-c2 | r3-c3 |
| putative dehydroquinase shikimate dehydrogenase {Arabidopsis thaliana} | TC80290 | AI779843 | cLES9G4 | XIB7 | T3D13 | R12 | C4 | r2-c1 | r4-c3 |
| homogentisate 1,2-dioxygenase {Lycopersicon esculentum} | TC80293 | AW092200 | cLET17L20 | IB3 | T1C5 | R20 | C3 | r1-c2 | r3-c3 |
| general negative transcription regulator-like {Arabidopsis thaliana} | TC80302 | AW399344 | cLPT7O15 | XVIC1 | T4F2 | R23 | C6 | r2-c2 | r1-c4 |
| putative transcriptional co-activator {Arabidopsis thaliana}GP\|3513735\|gb\|AAC33951.1\|\|AF080118 cont | TC80320 | AI779030 | cLES6L18 | XIA5 | T3B9 | R16 | C2 | r2-c1 | r4-c3 |
| putative cytochrome P450 | TC80358 | BG131093 | cTOE2F5 | XVIIIF1 | T5K2 | R23 | C11 | r3-c2 | r4-c4 |
| phospho-2-dehydro-3-deoxyheptonate aldolase | TC80361 | AI897267 | cLED26J15 | IIID10 | T1H19 | R6 | C8 | r1-c2 | r3-c3 |
| glyceraldehyde 3-phosphate dehydrogenase a precursor, chloroplast {Nicotiana tabacum}SP\|P09043\|G3PA | TC80364 | AW428960 | cTOA2K2 | XVIE10 | T4J20 | R5 | C10 | r2-c2 | r1-c4 |
| aminomethyltransferase precursor system t protein) {Solanum tuberosum}SP\|P54260\|GCST_SOLTU AMINOMET | TC80365 | BG127639 | cTOF17K6 | XIXB5 | T5D9 | R16 | C4 | r3-c2 | r4-c4 |
| glyceraldehyde 3-phosphate dehydrogenase a precursor, chloroplast {Nicotiana tabacum}SP\|P09043\|G3PA | TC80366 | BE434460 | cLEG17I17 | VF5 | T2K9 | R16 | C11 | r4-c2 | r1-c3 |
| hydroxymethyltransferase {Arabidopsis thaliana}GP\|2244749\|emb\|CAB10172.1\|\|Z97335 hydroxymethyltrans | TC80391 | BG135712 | cTOE23K18 | XVIIIE9 | T5I18 | R7 | C9 | r3-c2 | r4-c4 |
| hydroxymethyltransferase {Arabidopsis thaliana}GP\|2244749\|emb\|CAB10172.1\|\|Z97335 hydroxymethyltrans | TC80394 | BG130817 | cTOE1I22 | XVIIID10 | T5G20 | R5 | C7 | r3-c2 | r4-c4 |
| putative fructose-bisphosphate aldolase, plastidic form {Arabidopsis thaliana}GP\|11762176\|gb\|AAG403 | TC80401 | AI895082 | cLEC6B14 | IIE4 | T1I8 | R17 | C9 | r1-c2 | r3-c3 |
| ATP synthase delta' subunit, mitochondrial precursor {Ipomoea batatas}SP\|Q40089\|ATP4_IPOBA ATP SYNT | TC80407 | BE450930 | cLEY15P13 | XIIIH11 | T4O21 | R4 | C15 | r2-c2 | r1-c4 |
| glyceraldehyde 3-phosphate dehydrogenase | TC80408 | AI775098 | cLER14D11 | IXF2 | T3K3 | R22 | C11 | r2-c1 | r4-c3 |
| S-adenosyl-L-methionine synthetase | TC80422 | AW221689 | cLEN3H21 | IXC6 | T3E11 | R14 | C5 | r2-c1 | r4-c3 |
| S-adenosyl-L-methionine synthetase | TC80423 | BG123796 | cTOF3K21 | XXE1 | T5J2 | R23 | C10 | r3-c2 | r4-c4 |
| S-adenosylmethionine synthase 3 {Lycopersicon | TC80424 | BF051914 | cLEM24J5 | VIIIC7 | T2F14 | R11 | C6 | r4-c2 | r1-c3 |

EP 1 454 993 A1

| | | | | | | | | | |
|---|---|---|---|---|---|---|---|---|---|
| esculentum}SP\|P43282\|METM_LYCES S-ADENOSYLMETHIONINE | | | | | | | | | |
| cystathionine gamma-synthase isoform 1 {Solanum tuberosum} | TC80427 | BE433564 | cLEG16E12 | VC11 | T2E21 | R4 | C5 | r4-c2 | r1-c3 |
| acetyl-CoA acyltransferase {Cucumis sativus}GP\|393707\|emb\|CAA47926.1\|\|X67696 acetyl-CoA_acyltransf | TC80431 | AW648018 | cLEN15N9 | VIID12 | T2H23 | R2 | C8 | r4-c2 | r1-c3 |
| xylose isomerase {Hordeum vulgare}SP\|Q40082\|XYLA_HORVU XYLOSE ISOMERASE (EC 5.3.1.5).GP\|1296809\|em | TC80432 | AW928693 | cTOC2F20 | XVIID4 | T5G7 | R18 | C7 | r3-c2 | r4-c4 |
| cytochrome P450 {Arabidopsis thaliana} | TC80453 | AI484770 | cLED3B5 | IIIH11 | T1P21 | R4 | C16 | r1-c2 | r3-c3 |
| Contains a PF\|00175 Oxidoreductase FAD/NADH-binding domain. ESTs gb\|H76345 and gb\|AA651465 come fro | TC80456 | BE434351 | cLEG16M23 | VF3 | T2K5 | R20 | C11 | r4-c2 | r1-c3 |
| caffeoyl-coenzymeA O-methyltransferase {Nicotiana tabacum}GP\|1574946\|gb\|AAC49913.1\|\|U38612 caffeoyl | TC80468 | AW040773 | cLET10L1 | XIC4 | T3F7 | R18 | C6 | r2-c1 | r4-c3 |
| homologous to GATA-binding transcription factors {Arabidopsis thaliana}GP\|7288001\|emb\|CAB81839.1\|\|A | TC80486 | AW929937 | cTOC8K12 | XVIIF2 | T5K3 | R22 | C11 | r3-c2 | r4-c4 |
| NADP-malic enzyme^^malate dehydrogenase | TC80499 | AW616604 | cLHT11H20 | XIVH6 | T4O12 | R13 | C15 | r2-c2 | r1-c4 |
| MADS box transcription factor-like {Arabidopsis thaliana} | TC80500 | BG123322 | cTOF1H5 | XIXD4 | T5H7 | R18 | C8 | r3-c2 | r4-c4 |
| triosephosphate isomerase, cytosolic {Petunia hybrida}SP\|P48495\|TPIS_PETHY TRIOSEPHOSPHATE ISOMERAS | TC80531 | BG125992 | cTOF10J13 | XXID10 | T6G19 | R6 | C7 | r3-c1 | r2-c4 |
| putative homeodomain transcription factor {Arabidopsis thaliana}PIR\|H84774\|H84774 probable homeodom | TC80540 | AI490360 | cLED15E8 | IIIA3 | T1B5 | R20 | C2 | r1-c2 | r3-c3 |
| malate dehydrogenase {Nicotiana tabacum} | TC80550 | AI775216 | cLER14L14 | XIIB3 | T3D6 | R19 | C4 | r2-c1 | r4-c3 |
| bZIP DNA-binding protein | TC80553 | BF051949 | cLEM24B20 | VIIIC1 | T2F2 | R23 | C6 | r4-c2 | r1-c3 |
| lysine-ketoglutarate reductase/saccharopine dehydrogenase bifunctional enzyme {Arabidopsis thaliana} | TC80556 | AI894874 | cLEC6K9 | IIE10 | T1I20 | R5 | C9 | r1-c2 | r3-c3 |
| phosphoglycerate kinase precursor {Solanum tuberosum}PIR\|T07014\|T07014 phosphoglycerate kinase (EC | TC80567 | AI774923 | cLER13F4 | XID3 | T3H5 | R20 | C8 | r2-c1 | r4-c3 |

| | | | | | | | | | |
|---|---|---|---|---|---|---|---|---|---|
| proton pump interactor {Arabidopsis thaliana}GP|7269604|emb|CAB81400.1||AL161571 proton pump intera | TC80570 | AW441201 | cLEN4D17 | IXC8 | T3E15 | R12 | C5 | r2-c1 | r4-c3 |
| malate dehydrogenase {Glycine max} | TC80572 | BF098428 | cLEW27A2 | IB2 | T1C3 | R22 | C3 | r1-c2 | r3-c3 |
| NADH-ubiquinone oxidoreductase 20 kDa subunit precursor {Solanum tuberosum}SP|Q43844|NUKM_SOLTU NAD | TC80576 | BE436586 | cLEG33O5 | VIB11 | T2C22 | R3 | C3 | r4-c2 | r1-c3 |
| MADS-box transcription factor FBP5 {Petunia x hybrida} | TC80582 | BE461808 | cLEG40E23 | VIE12 | T2I24 | R1 | C9 | r4-c2 | r1-c3 |
| serine hydroxymethyltransferase, mitochondrial precursor {Solanum tuberosum}SP|P50433|GLYM_SOLTU SE | TC80593 | AI776896 | cLER20K5 | XB3 | T3C6 | R19 | C3 | r2-c1 | r4-c3 |
| serine hydroxymethyltransferase, mitochondrial precursor {Solanum tuberosum}SP|P50433|GLYM_SOLTU SE | TC80594 | AW040297 | cLET5K17 | XIIB10 | T3D20 | R5 | C4 | r2-c1 | r4-c3 |
| homeodomain protein | TC80595 | AI487520 | cLED11B1 | IIG6 | T1M12 | R13 | C13 | r1-c2 | r3-c3 |
| homeodomain leucine-zipper protein ATHB13 {Arabidopsis thaliana}GP|12325190|gb|AAG52541.1|AC013289_ | TC80599 | AI486971 | cLED6P3 | IVC1 | T1F2 | R23 | C6 | r1-c2 | r3-c3 |
| spermidine synthase {Arabidopsis thaliana} | TC80606 | BE433027 | cLEG11N5 | VD8 | T2G15 | R12 | C7 | r4-c2 | r1-c3 |
| putative ATP synthase {Arabidopsis thaliana}PIR|B84606|B84606 probable ATP synthase [imported] - Ar | TC80612 | AW649042 | cLEI6J24 | VIIF6 | T2L11 | R14 | C12 | r4-c2 | r1-c3 |
| dTDP-glucose 4-6-dehydratases-like protein {Arabidopsis thaliana}PIR|T45701|T45701 dTDP-glucose 4-6 | TC80616 | BE461694 | cLEG39N23 | VIE10 | T2I20 | R5 | C9 | r4-c2 | r1-c3 |
| homology to pyroxidal-5'-phosphate-dependant glutamate decarboxylases; putative start codon | TC80620 | BE434007 | cLEG13D11 | VID12 | T2G24 | R1 | C7 | r4-c2 | r1-c3 |
| glutamate decarboxylase {Lycopersicon esculentum}SP|P54767|DCE_LYCES GLUTAMATE DECARBOXYLASE (EC 4. | TC80621 | BE460614 | cLEG33E2 | VIB7 | T2C14 | R11 | C3 | r4-c2 | r1-c3 |
| osmotic stress-induced zinc-finger protein {Nicotiana tabacum}PIR|T01985|T01985 zinc-finger protein | TC80630 | AW030858 | cLEC21H24 | IE9 | T1I17 | R8 | C9 | r1-c2 | r3-c3 |
| ferritin subunit cowpea2 precursor {Vigna unguiculata}PIR|T08124|T08124 ferritin 2 precursor - cowp | TC80669 | BF050399 | cLEM17N17 | XIIA4 | T3B8 | R17 | C2 | r2-c1 | r4-c3 |
| contains similarity to NADH dehydrogenase chain CI- | TC80670 | AW625698 | cLEZ16F15 | XIVD12 | T4G24 | R1 | C7 | r2-c2 | r1-c4 |

167

EP 1 454 993 A1

| | | | | | | | | | |
|---|---|---|---|---|---|---|---|---|---|
| 18~gene_id:K9I9.16 {Arabidopsis thaliana} | | | | | | | | | |
| succinate dehydrogenase | TC80679 | BG136628 | cLPP3O11 | XVE11 | T4J21 | R4 | C10 | r2-c2 | r1-c4 |
| putative glycine decarboxylase p-protein | TC80681 | AW096471 | cLET38N21 | XVID4 | T4H8 | R17 | C8 | r2-c2 | r1-c4 |
| S-ADENOSYLMETHIONINE DECARBOXYLASE PROENZYME (EC 4.1.1.50) (ADOMETDC) (SAMDC).GP\|1498080\|gb\|AAC0461 | TC80692 | BG138411 | cLPP9K14 | XVF7 | T4L13 | R12 | C12 | r2-c2 | r1-c4 |
| CONSTANS-like protein 2 {Malus x domestica} | TC80693 | AI484848 | cLED2N21 | IIIE11 | T1J21 | R4 | C10 | r1-c2 | r3-c3 |
| pyruvate kinase (EC 2.7.1.40), cytosolic - potato | TC80694 | AW219226 | cLEX3K18 | XIIIE1 | T4I1 | R24 | C9 | r2-c2 | r1-c4 |
| ATP synthase delta subunit, mitochondrial precursor (oligomycin sensitivity conferral protein) (oscp | TC80699 | BG643528 | cTOF31E5 | XXC3 | T5F6 | R19 | C6 | r3-c2 | r4-c4 |
| Tetrafunctional protein of glyoxysomal fatty acid beta-oxidation {Brassica napus}PIR\|T08017\|T08017 | TC80702 | AI774151 | cLER10D2 2 | IXE4 | T3I7 | R18 | C9 | r2-c1 | r4-c3 |
| PROBABLE VACUOLAR ATP SYNTHASE SUBUNIT H (EC 3.6.1.34) (V-ATPASE H SUBUNIT) (VACUOLAR PROTON PUMP H | TC80710 | AW441330 | cLEN15M1 7 | IXA6 | T3A11 | R14 | C1 | r2-c1 | r4-c3 |
| Strong similarity to gb\|L34684 inosine monophosphate dehydrogenase (IMPDH) from Arabidopsis thaliana | TC80721 | AW737443 | cTOD2P4 | XVIIG6 | T5M11 | R14 | C13 | r3-c2 | r4-c4 |
| phosphate/phosphoenolpyruvate translocator-like protein {Arabidopsis thaliana} | TC80730 | BG138390 | cLPP9C20 | XVF6 | T4L11 | R14 | C12 | r2-c2 | r1-c4 |
| 3-isopropylmalate dehydrogenase precursor dehydrogenase) (imdh) (3-ipm-dh) {Solanum tuberosum}SP\|P2 | TC80800 | BE344489 | cLEY2O13 | XIVC7 | T4E14 | R11 | C5 | r2-c2 | r1-c4 |
| sucrose transporter | TC80801 | AI782568 | cLES20C5 | XIIA3 | T3B6 | R19 | C2 | r2-c1 | r4-c3 |
| citrate synthase, glyoxysomal precursor {Cucurbita maxima}SP\|P49299\|CYSZ_CUCMA CITRATE SYNTHASE, GL | TC80803 | BE437000 | cLEG35K1 5 | VIC7 | T2E14 | R11 | C5 | r4-c2 | r1-c3 |
| UDP-glucose:protein transglucosylase {Solanum tuberosum} | TC80818 | BG125578 | cTOF9G19 | XXG8 | T5N16 | R9 | C14 | r3-c2 | r4-c4 |
| delta-12 fatty acid desaturase {Borago officinalis} | TC80824 | BG138370 | cLPP9A16 | XVF5 | T4L9 | R16 | C12 | r2-c2 | r1-c4 |
| enoyl-ACP reductase {Petunia x hybrida} | TC80834 | BE459984 | cLEM8J9 | VIIIF5 | T2L10 | R15 | C12 | r4-c2 | r1-c3 |
| Similar to acyl carrier protein, mitochondrial precursor (ACP) NADH-ubiquinone oxidoreductase 9.6 KD | TC80836 | AI485151 | cLED7G23 | IVC4 | T1F8 | R17 | C6 | r1-c2 | r3-c3 |
| omega-3 fatty acid desaturase, endoplasmic reticulum {Nicotiana tabacum}SP\|P48626\|FD3E_TOBAC OMEGA- | TC80843 | BG123845 | cTOF3G12 | XXD9 | T5H18 | R7 | C8 | r3-c2 | r4-c4 |
| UDP-glucose glucosyltransferase {Arabidopsis | TC80847 | AW625604 | cLEZ15P21 | XIVD8 | T4G16 | R9 | C7 | r2-c2 | r1-c4 |

168

thaliana}GP|9392679|gb|AAF87256.1|AC068562_3|AC 068562

| Description | TC | Accession | cName | | T | R | C | | |
|---|---|---|---|---|---|---|---|---|---|
| isocitrate dehydrogenase (NADP+) {Solanum tuberosum}PIR|T07402|T07402 probable isocitrate dehydroge | TC80851 | AI895979 | cLEC13B9 | VIF11 | T2K22 | R3 | C11 | r4-c2 | r1-c3 |
| zinc finger protein {Pisum sativum}PIR|T48868|T48868 zinc finger protein [imported] - garden pea | TC80860 | BG136538 | cLPP2N17 | XVE9 | T4J17 | R8 | C10 | r2-c2 | r1-c4 |
| putative ABC transporter ATPase; 10053-12032 {Arabidopsis thaliana} | TC80867 | BG125731 | cTOF9J23 | XXG10 | T5N20 | R5 | C14 | r3-c2 | r4-c4 |
| transcriptional regulator, putative; 35498-34111 {Arabidopsis thaliana}PIR|H96576|H96576 hypothetic | TC80874 | BE435682 | cLEG28G1 2 | VH6 | T2O11 | R14 | C15 | r4-c2 | r1-c3 |
| legumin-like protein {Arabidopsis thaliana}PIR|H84687|H84687 legumin-like protein [imported] - Arab | TC80885 | AW218154 | cLEZ1G23 | XIVF7 | T4K14 | R11 | C11 | r2-c2 | r1-c4 |
| ATP:citrate lyase {Capsicum annuum} | TC80889 | BG128839 | cTOF22D3 | XIXE9 | T5J17 | R8 | C10 | r3-c2 | r4-c4 |
| hyoscyamine 6-dioxygenase hydroxylase) {Hyoscyamus niger}SP|P24397|HY6H_HYONI HYOSCYAMINE 6-DIOXYGE | TC80893 | AW649940 | cLEI11OI | VIIA11 | T2B21 | R4 | C2 | r4-c2 | r1-c3 |
| RING-H2 finger protein RHF2a {Arabidopsis thaliana}GP|13374859|emb|CAC34493.1||AL589883 RING-H2 fin | TC80911 | AW441998 | cLEN19D1 1 | VIH5 | T2O10 | R15 | C15 | r4-c2 | r1-c3 |
| putative GDP-mannose pyrophosphorylase; 64911-67597 {Arabidopsis thaliana}PIR|G96778|G96778 hypothe | TC80933 | AW929477 | cTOC9C23 | XVIIF4 | T5K7 | R18 | C11 | r3-c2 | r4-c4 |
| glucosyltransferase-like protein {Arabidopsis thaliana} | TC80941 | AI898616 | cLED34H6 | IIIG8 | T1N15 | R12 | C14 | r1-c2 | r3-c3 |
| putative caffeoyl-CoA O-methyltransferase {Arabidopsis thaliana} | TC80956 | BE459185 | cLEM5B22 | VIIID10 | T2H20 | R5 | C8 | r4-c2 | r1-c4 |
| fatty acid elongase-like protein (cer2-like) {Arabidopsis thaliana}GP|7268088|emb|CAB78426.1||AL161 | TC80962 | BG125978 | cTOF10H3 | XE2 | T3I4 | R21 | C9 | r2-c1 | r4-c3 |
| TOM (target of myb1)-like protein {Arabidopsis thaliana}PIR|T51543|T51543 TOM (target of myb1)-like | TC80976 | AW616929 | cLHT18D2 3 | XVA5 | T4B9 | R16 | C2 | r2-c2 | r1-c4 |
| heat shock transcription factor-like protein {Arabidopsis thaliana} | TC80978 | AI489721 | cLED15A9 | IIH11 | T1O22 | R3 | C15 | r1-c2 | r3-c3 |
| Similar to ATP-citrate-lyase {Arabidopsis thaliana}PIR|F86227|F86227 hypothetical protein [imported] | TC80979 | AI771253 | cLED28P7 | IIIE5 | T1J9 | R16 | C10 | r1-c2 | r3-c3 |

| | | | | | | | | |
|---|---|---|---|---|---|---|---|---|---|
| putative NADH-ubiquinone oxireductase {Arabidopsis thaliana}PIR\|C84588\|C84588 probable NADH-ubiquin | TC80984 | BF098069 | cLEW25M9 | XIIG10 | T3N20 | R5 | C14 | r2-c1 | r4-c3 |
| putative deoxycytidylate deaminase {Cicer arietinum} | TC80988 | AI772030 | cLER1O18 | XA11 | T3A22 | R3 | C1 | r2-c1 | r4-c3 |
| putative RING finger protein; 84236-82024 {Arabidopsis thaliana}PIR\|A96829\|A96829 probable RING fin | TC81001 | BE461509 | cLEG39A11 | VIE6 | T2I12 | R13 | C9 | r4-c2 | r1-c3 |
| **putative RING finger protein; 84236-82024 {Arabidopsis thaliana}PIR\|A96829\|A96829 probable RING fin** | TC81002 | AW648088 | cLEI3K12 | VIID10 | T2H19 | R6 | C8 | r4-c2 | r1-c3 |
| RING finger-like protein {Arabidopsis thaliana}PIR\|T47595\|T47595 RING finger protein T12E18.50 - Ar | TC81013 | AI489945 | cLED14K18 | IIH10 | T1O20 | R5 | C15 | r1-c2 | r3-c3 |
| mevalonate diphosphate decarboxylase {Arabidopsis thaliana}GP\|3250736\|emb\|CAA76803.1\|\|Y17593 mevalo | TC81015 | BF051253 | cLEM22C23 | VIIIA10 | T2B20 | R5 | C2 | r4-c2 | r1-c3 |
| aspartate aminotransferase {Oryza sativa}PIR\|JC5125\|JC5125 aspartate transaminase (EC 2.6.1.1) prec | TC81020 | BE432751 | cLEG10K11 | VD6 | T2G11 | R14 | C7 | r4-c2 | r1-c3 |
| NADP-dependent isocitrate dehydrogenase-like protein | TC81025 | AW651062 | cLEI15I20 | VIIC6 | T2F11 | R14 | C6 | r4-c2 | r1-c3 |
| Strong similarity to F19I3.8 GP\|3033381 putative UDP-galactose-4-epimerase from Arabidopsis thaliana | TC81034 | AW033767 | cLEC29E1 | XXIE10 | T6I19 | R6 | C9 | r3-c1 | r2-c4 |
| glucosyltransferase-like protein {Arabidopsis thaliana} | TC81042 | AW039906 | cLET13B22 | IA4 | T1A7 | R18 | C1 | r1-c2 | r3-c3 |
| omega-6 fatty acid desaturase, chloroplast precursor {Brassica napus}SP\|P48627\|FD6C_BRANA OMEGA-6 F | TC81045 | AW399600 | cLPT8K1 | XVIC5 | T4F10 | R15 | C6 | r2-c2 | r1-c4 |
| acyl-ACP thioesterase {Garcinia mangostana} | TC81091 | AI489140 | cLED15I7 | XVIIH5 | T5O9 | R16 | C15 | r3-c2 | r4-c4 |
| ATP synthase a subunit precursor {Nicotiana tabacum}SP\|P06288\|ATPI_TOBAC ATP SYNTHASE A CHAIN PRECU | TC81098 | AI898457 | cLED34E17 | IIIG6 | T1N11 | R14 | C14 | r1-c2 | r3-c3 |
| 3-isopropylmalate dehydrogenase precursor dehydrogenase) (imdh) (3-ipm-dh) {Brassica napus}SP\|P2910 | TC81104 | AW928771 | cTOC3E9 | VD7 | T2G13 | R12 | C7 | r4-c2 | r1-c3 |
| anthranilate N-benzoyltransferase {Arabidopsis thaliana} | TC81105 | AW625953 | cLEZ17P13 | XIVE8 | T4I16 | R9 | C9 | r2-c2 | r1-c4 |
| cytochrome c oxidase subunit Vb precursor-like protein {Arabidopsis thaliana} | TC81111 | AW039677 | cLET13C21 | XIC9 | T3F17 | R8 | C6 | r2-c1 | r4-c3 |
| UMP synthase {Nicotiana plumbaginifolia} | TC81117 | BG130029 | cTOF29I5 | XVIG8 | T4N16 | R9 | C14 | r2-c2 | r1-c4 |

EP 1 454 993 A1

| | | | | | | | | | |
|---|---|---|---|---|---|---|---|---|---|
| anthocyanin 5-O-glucosyltransferase {Petunia x hybrida} | TC81118 | BG135580 | cTOE23C9 | XVIIIE8 | T5I16 | R9 | C9 | r3-c2 | r4-c4 |
| uridine kinase-like protein {Arabidopsis thaliana} | TC81123 | AW928947 | cTOC4E6 | XVIC8 | T4F16 | R9 | C6 | r2-c2 | r1-c4 |
| putative homeodomain transcription factor {Arabidopsis thaliana}PIR|H84774|H84774 probable homeodom | TC81124 | BG129304 | cTOF23N12 | XIXG3 | T5N5 | R20 | C14 | r3-c2 | r4-c4 |
| glucose-regulated protein 78 | TC81129 | AW650486 | cLEI13M5 | XVIIH2 | T5O3 | R22 | C15 | r3-c2 | r4-c4 |
| contains similarity to acyl-CoA thioesterase~gene_id:K23F3.9 {Arabidopsis thaliana} | TC81143 | AI486923 | cLED6D7 | IVB5 | T1D10 | R15 | C4 | r1-c2 | r3-c3 |
| HOMEOBOX-LEUCINE ZIPPER PROTEIN HAT5 (HD-ZIP PROTEIN 5) (HD-ZIP PROTEIN ATHB-1).□GP|16329|emb|CAA416 | TC81154 | AW623618 | cTOB11B23 | XVIF11 | T4L22 | R3 | C12 | r2-c2 | r1-c4 |
| phosphoenolpyruvate carboxylase 1 {Gossypium hirsutum}GP|2266947|gb|AAB80714.1||AF008939 phosphoeno | TC81155 | BG626017 | cPP1O21 | XXIA5 | T6A9 | R16 | C1 | r3-c1 | r2-c4 |
| transcription factor CRC {Arabidopsis thaliana}GP|12325076|gb|AAG52485.1|AC018364_3|AC018364 transc | TC81170 | AI485831 | cLED4O9 | IVA9 | T1B18 | R7 | C2 | r1-c2 | r3-c3 |
| putative glucose regulated repressor protein {Arabidopsis thaliana}PIR|A84649|A84649 probable gluco | TC81176 | BE433829 | cLEG7F16 | XIIF3 | T3L6 | R19 | C12 | r2-c1 | r4-c3 |
| cytochrome P450 {Nicotiana tabacum}GP|1237250|emb|CAA65580.1||X96784 cytochrome P450 {Nicotiana tab | TC81178 | AW737552 | cTOD3H13 | XVIG9 | T4N18 | R7 | C14 | r2-c2 | r1-c4 |
| tryptophan synthase alpha 1-like protein {Arabidopsis thaliana}GP|3892048|gb|AAC78257.1|AAC78257|AC | TC81185 | BE460501 | cLEG31J1 | VIA9 | T2A18 | R7 | C1 | r4-c2 | r1-c3 |
| small zinc finger-like protein | TC81193 | BG127265 | cTOF14N18 | XIXA5 | T5B9 | R16 | C2 | r3-c2 | r4-c4 |
| MybSt1 {Solanum tuberosum} | TC81223 | AW091869 | cLET16L16 | XID7 | T3H13 | R12 | C8 | r2-c1 | r4-c3 |
| immediate-early salicylate-induced glucosyltransferase {Nicotiana tabacum}GP|1685005|gb|AAB36653.1| | TC81244 | BE434667 | cLEG20E17 | VG2 | T2M3 | R22 | C13 | r4-c2 | r1-c3 |
| bZIP transcription factor {Nicotiana tabacum} | TC81272 | AW738717 | cTOD8G2 | XVIIIB1 | T5C2 | R23 | C3 | r3-c2 | r4-c4 |
| aspartate aminotransferase, cytoplasmic {Daucus carota}SP|P28734|AATC_DAUCA ASPARTATE AMINOTRANSFER | TC81279 | BF052193 | cLEM25L10 | VIIIC10 | T2F20 | R5 | C6 | r4-c2 | r1-c3 |
| putative RING zinc finger protein; 53384-54880 {Arabidopsis thaliana}PIR|G96835|G96835 probable RIN | TC81280 | AI896388 | cLEC15M17 | ID8 | T1G15 | R12 | C7 | r1-c2 | r3-c3 |

171

| | | | | | | | | | |
|---|---|---|---|---|---|---|---|---|---|
| similar to ATPases associated with various cellular activites (Pfam: AAA.hmm, score: 230.91) {Arabid | TC81288 | AI775557 | cLER15L4 | IXF12 | T3K23 | R2 | C11 | r2-c1 | r4-c3 |
| ASPARTATE AMINOTRANSFERASE, MITOCHONDRIAL PRECURSOR (EC 2.6.1.1) (TRANSAMINASE A).GP\|531555\|emb\|CAA | TC81293 | BG125276 | cTOF8K11 | XXG5 | T5N10 | R15 | C14 | r3-c2 | r4-c4 |
| phosphoribosylanthranilate transferase {Arabidopsis thaliana} | TC81302 | BE460055 | cLEM8H10 | VIIIF3 | T2L6 | R19 | C12 | r4-c2 | r1-c3 |
| D-ribulose-5-phosphate 3-epimerase {Oryza sativa} | TC81304 | AW223738 | cLEN13B9 | VIIIH2 | T2P4 | R21 | C16 | r4-c2 | r1-c3 |
| malate dehydrogenase, glyoxysomal precursor {Citrullus vulgaris}EGAD\|130842\|139627 glyoxysomal mala | TC81324 | AW647654 | cLEI10F3 | VIIA9 | T2B17 | R8 | C2 | r4-c2 | r1-c3 |
| glyceraldehyde 3-phosphate dehydrogenase, cytosolic {Petunia hybrida}SP\|P26520\|G3PC_PETHY GLYCERALD | TC81336 | BG135725 | cTOE23M22 | XVG10 | T4N19 | R6 | C14 | r2-c2 | r1-c4 |
| CCAAT-binding transcription factor subunit A(CBF-A) {Arabidopsis thaliana}GP\|2244810\|emb\|CAB10233.1 | TC81341 | AI782351 | cLES18L2 | IVG7 | T1N14 | R11 | C14 | r1-c2 | r3-c3 |
| H+-transporting ATPase-like protein {Arabidopsis thaliana}GP\|7270157\|emb\|CAB79970.1\|AL161581 H+-tr | TC81342 | AW650530 | cLEI13E8 | VIIB9 | T2D17 | R8 | C4 | r4-c2 | r1-c3 |
| ZF-HD homeobox protein {Flaveria bidentis} | TC81347 | BG643218 | cTOF26N24 | XIXH12 | T5P23 | R2 | C16 | r3-c2 | r4-c4 |
| PUTATIVE NADH-UBIQUINONE OXIDOREDUCTASE SUBUNIT B17.2 (EC 1.6.5.3) (EC 1.6.99.3) (COMPLEX I-B17.2) ( | TC81349 | AW038388 | cLET5I16 | XIIB9 | T3D18 | R7 | C4 | r2-c1 | r4-c3 |
| SNF5, transcription regulatory protein homolog BSH {Arabidopsis thaliana} | TC81352 | AI898634 | cLED34L6 | IIIG9 | T1N17 | R8 | C14 | r1-c2 | r3-c3 |
| putative glucosyltransferase {Arabidopsis thaliana}GP\|4309698\|gb\|AAD15482.1\|AC006266 putative gluc | TC81356 | AW648029 | cLEI3O7 | VIIE3 | T2J5 | R20 | C10 | r4-c2 | r1-c3 |
| glutamine cyclotransferase precursor {Carica papaya}PIR\|T08168\|T08168 glutaminyl-peptide cyclotrans | TC81367 | AI897111 | cLED26K16 | XIVA8 | T4A16 | R9 | C1 | r2-c2 | r1-c4 |
| contains similarity to sugar transporters (Pfam: sugar_tr.hmm, score: 395.91) {Arabidopsis thaliana} | TC81373 | BG137973 | cLPP7N18 | XVF3 | T4L5 | R20 | C12 | r2-c2 | r1-c4 |
| putative folylpolyglutamate synthetase {Oryza sativa} | TC81390 | BE433834 | cLEG7H4 | VIH8 | T2O16 | R9 | C15 | r4-c2 | r1-c3 |
| S-adenosyl-L-methionine Mg-protoporphyrin IX methyltranserase {Nicotiana tabacum} | TC81391 | BG126969 | cTOF14A13 | XVIIIH12 | T5O24 | R1 | C15 | r3-c2 | r4-c4 |

172

| | | | | | | | | | |
|---|---|---|---|---|---|---|---|---|---|
| contains similarity to ATP synthase B/B' (Pfam: ATP-synt_B.hmm, score: 11.71) {Arabidopsis thaliana} | TC81399 | BF051056 | cLEM21O2 | XVH11 | T4P21 | R4 | C16 | r2-c2 | r1-c4 |
| Contains similarity to gb\|AJ006354 zinc finger protein (ZAC) from Homo sapiens. {Arabidopsis thalian | TC81419 | AW442254 | cLEN22G16 | IXC4 | T3E7 | R18 | C5 | r2-c1 | r4-c3 |
| dihydroflavonol 4-reductase-like {Arabidopsis thaliana} | TC81435 | BE435324 | cLEG26A9 | VC5 | T2E9 | R16 | C5 | r4-c2 | r1-c3 |
| putative RING zinc finger protein {Arabidopsis thaliana} | TC81454 | AI488438 | cLED21N23 | XVIIG7 | T5M13 | R12 | C13 | r3-c2 | r4-c4 |
| beta-amylase {Arabidopsis thaliana} | TC81481 | AW650286 | cLEI12H7 | XXH12 | T5P24 | R1 | C16 | r3-c2 | r4-c4 |
| zinc finger-like protein {Arabidopsis thaliana}PIR\|T49033\|T49033 zinc finger-like protein - Arabido | TC81513 | BG130666 | cTOF31H2 | XIVG2 | T4M4 | R21 | C13 | r2-c2 | r1-c4 |
| putative methylmalonate semi-aldehyde dehydrogenase {Arabidopsis thaliana}PIR\|H84514\|H84514 hypothe | TC81537 | AW625535 | cLEZ15D3 | XIVD6 | T4G12 | R13 | C7 | r2-c2 | r1-c4 |
| myb-related protein 340 - garden snapdragon | TC81538 | AI897784 | cLED30L23 | IIIF8 | T1L15 | R12 | C12 | r1-c2 | r3-c3 |
| ISOCITRATE DEHYDROGENASE [NADP] (EC 1.1.1.42) (OXALOSUCCINATE DECARBOXYLASE) (IDH) (NADP+-SPECIFIC I | TC81566 | BE432917 | cLEG10H23 | XVID12 | T4H24 | R1 | C8 | r2-c2 | r1-c4 |
| UTP-glucose glucosyltransferase-like protein {Arabidopsis thaliana}GP\|4835225\|emb\|CAB42903.1\|\|AL049 | TC81577 | BF113450 | cLEG44N2 | VIG9 | T2M18 | R7 | C13 | r4-c2 | r1-c3 |
| flavanone 3beta-hydroxylase {Petunia x hybrida} | TC81579 | BE449565 | cLHT32A3 | XVIE12 | T4J24 | R1 | C10 | r2-c2 | r1-c4 |
| pyruvate kinase {Arabidopsis thaliana} | TC81587 | BE353438 | cTOA19M23 | XVIE6 | T4J12 | R13 | C10 | r2-c2 | r1-c4 |
| serine hydroxymethyltransferase, mitochondrial precursor {Solanum tuberosum}SP\|P50433\|GLYM_SOLTU SE | TC81590 | BF098334 | cLEW26O3 | XIIH1 | T3P2 | R23 | C16 | r2-c1 | r4-c3 |
| serine hydroxymethyltransferase, mitochondrial precursor {Solanum tuberosum}SP\|P50433\|GLYM_SOLTU SE | TC81591 | BE431621 | cLEG30A5 | VIA1 | T2A2 | R23 | C1 | r4-c2 | r1-c3 |
| biotin carboxylase subunit {Nicotiana tabacum}GP\|870726\|gb\|AAC41659.1\|\|L38260 biotin carboxylase su | TC81634 | AI483969 | cLED23C19 | IIIC9 | T1F17 | R8 | C6 | r1-c2 | r3-c3 |
| ornithine carbamoyltransferase {Pisum sativum}SP\|Q43814\|OTC_PEA ORNITHINE CARBAMOYLTRANSFERASE PREC | TC81652 | AW093198 | cLET24A15 | XIF5 | T3L9 | R16 | C12 | r2-c1 | r4-c3 |
| phosphoenolpyruvate carboxylase kinase {Lycopersicon esculentum} | TC81676 | AW442172 | cLEN21B14 | IXB10 | T3C19 | R6 | C3 | r2-c1 | r4-c3 |

EP 1 454 993 A1

174

| | | | | | | | | | |
|---|---|---|---|---|---|---|---|---|---|
| UDP-glucose glucosyltransferase {Solanum tuberosum}GP\|1857447\|gb\|AAB48444.1\|\|U82367 UDP-glucose glu | TC81688 | AW217408 | cTOA6A11 | XVIF3 | T4L6 | R19 | C12 | r2-c2 | r1-c4 |
| Strong similarity to UDP-glucose glucosyltransferase from Arabidopsis thaliana gb\|AB016819 and conta | TC81690 | AW033397 | cLEC30E24 | IH4 | T1O7 | R18 | C15 | r1-c2 | r3-c3 |
| transcription factor NF-Y, CCAAT-binding-like protein {Arabidopsis thaliana}PIR\|T45874\|T45874 trans | TC81698 | AW738727 | cTOD8G22 | XVIIIB2 | T5C4 | R21 | C3 | r3-c2 | r4-c4 |
| malate dehydrogenase (NADP), chloroplast precursor (NADp-mdh) {Pisum sativum}SP\|P21528\|MDHC_PEA MAL | TC81700 | BE432974 | cLEG11O15 | VD9 | T2G17 | R8 | C7 | r4-c2 | r1-c3 |
| acyl-CoA:1-acylglycerol-3-phosphate acyltransferase {Arabidopsis thaliana} | TC81716 | AW944886 | cTOB12O24 | XVIH2 | T4P4 | R21 | C16 | r2-c2 | r1-c4 |
| VACUOLAR ATP SYNTHASE SUBUNIT G 2 (EC 3.6.1.34) (V-ATPASE G SUBUNIT 2) (VACUOLAR PROTON PUMP G SUBUN | TC81726 | BG131668 | cTOE4L10 | XVIIIF8 | T5K16 | R9 | C11 | r3-c2 | r4-c4 |
| MADS-box transcription factor jointless | TC81749 | AI895411 | cLEC7P7 | IIF4 | T1K8 | R17 | C11 | r1-c2 | r3-c3 |
| putative RING zinc finger protein; 27623-28978 {Arabidopsis thaliana}PIR\|H96703\|H96703 probable RIN | TC81762 | AI782511 | cLES19H15 | XF12 | T3K24 | R1 | C11 | r2-c1 | r4-c3 |
| Strong similarity to MRP-like ABC transporter gb\|U92650 from A. thaliana and canalicular multi-drug | TC81773 | AI489515 | cLED16O3 | IIIA11 | T1B21 | R4 | C2 | r1-c2 | r3-c3 |
| alanine aminotransferase {Arabidopsis thaliana} | TC81776 | BE433936 | cLEG9A20 | VIIA3 | T2B5 | R20 | C2 | r4-c2 | r1-c3 |
| starch synthase, isoform V {Vigna unguiculata} | TC81835 | BF051825 | cLEM24H9 | VIIIC5 | T2F10 | R15 | C6 | r4-c2 | r1-c3 |
| ribulosebisphosphate carboxylase large subunit | TC81850 | AI486088 | cLED5M17 | IVB2 | T1D4 | R21 | C4 | r1-c2 | r3-c3 |
| acetyl-coA dehydrogenase, putative {Arabidopsis thaliana} | TC81857 | AW031602 | cLEC34G18 | IIA8 | T1A16 | R9 | C1 | r1-c2 | r3-c3 |
| MADS box transcription factor MADS1 {Capsicum annuum} | TC81862 | BE436905 | cLEG34H13 | VIC2 | T2E4 | R21 | C5 | r4-c2 | r1-c3 |
| tyrosine aminotransferase-like protein {Arabidopsis thaliana} | TC81863 | AW928458 | cTOC1D7 | XVIID1 | T5G1 | R24 | C7 | r3-c2 | r4-c4 |
| putative RING-H2 zinc finger protein ATL6 {Arabidopsis thaliana} | TC81880 | AW223116 | cLEN10F7 | XIIIE3 | T4I5 | R20 | C9 | r2-c2 | r1-c4 |
| dihydrodipicolinate synthase {Nicotiana tabacum}SP\|Q42948\|DAPA_TOBAC DIHYDRODIPICOLINATE SYNTHASE P | TC81883 | AW623776 | cTOB13I5 | XVIH6 | T4P12 | R13 | C16 | r2-c2 | r1-c4 |
| putative CTP synthase {Oryza sativa} | TC81887 | AW035658 | cLEC34D4 | IIA7 | T1A14 | R11 | C1 | r1-c2 | r3-c3 |
| transcription factor-like; similar to CH6 and COP9 | TC81893 | BE353981 | cTOD8B20 | XVIIIA11 | T5A22 | R3 | C1 | r3-c2 | r4-c4 |

| | | | | | | | | | |
|---|---|---|---|---|---|---|---|---|---|
| complex subunit 6 {Arabidopsis thaliana} | | | | | | | | | |
| putative phosphoribosylanthranilate transferase {Arabidopsis thaliana}GP\|7267861\|emb\|CAB78204.1\|\|AL | TC81895 | BF051397 | cLEM22D24 | VIIIA12 | T2B24 | R1 | C2 | r4-c2 | r1-c3 |
| starch phosphorylase (AA 1-966) {Solanum tuberosum} | TC81900 | BF097142 | cLEW19F23 | XIIE3 | T3J6 | R19 | C10 | r2-c1 | r4-c3 |
| contains similarity to cyclopropane fatty acid synthase~gene_id:MEE5.5 {Arabidopsis thaliana} | TC81904 | AW616028 | cTOA17L12 | XVID11 | T4H22 | R3 | C8 | r2-c2 | r1-c4 |
| PROBABLE VACUOLAR ATP SYNTHASE SUBUNIT F (EC 3.6.1.34) (V-ATPASE F SUBUNIT) (VACUOLAR PROTON PUMP F | TC81929 | BG134700 | cTOE17L19 | XVIIID6 | T5G12 | R13 | C7 | r3-c2 | r4-c4 |
| cytochrome P450-like protein {Arabidopsis thaliana}GP\|7270932\|emb\|CAB80611.1\|\|AL161595 cytochrome P | TC81940 | BE433060 | cLEG12G7 | VD11 | T2G21 | R4 | C7 | r4-c2 | r1-c3 |
| cytochrome P450-like protein {Arabidopsis thaliana}PIR\|T46196\|T46196 cytochrome P450-like protein - | TC81993 | AW399577 | cLPT8E17 | XVIC3 | T4F6 | R19 | C6 | r2-c2 | r1-c4 |
| uroporphyrinogen decarboxylase {Arabidopsis thaliana} | TC81996 | BG128715 | cTOF21H22 | XIXE4 | T5J7 | R18 | C10 | r3-c2 | r4-c4 |
| succinate dehydrogenase flavoprotein alpha subunit {Arabidopsis thaliana}GP\|8843734\|dbj\|BAA97282.1\| | TC82029 | BE434365 | cLEG16B16 | VE9 | T2I17 | R8 | C9 | r4-c2 | r1-c3 |
| HD-Zip protein {Arabidopsis thaliana}GP\|3132474\|gb\|AAC16263.1\|\|AC003096 homeodomain transcription f | TC82042 | BG132703 | cTOE8N13 | XVIIIG5 | T5M10 | R15 | C13 | r3-c2 | r4-c4 |
| phosphate transporter^^putative phosphate transporter^^inorganic phosphate transporter | TC82044 | AI781884 | cLES17O18 | XF6 | T3K12 | R13 | C11 | r2-c1 | r4-c3 |
| heat stress transcription factor A3 {Lycopersicon peruvianum} | TC82048 | AW034881 | cLEC32C19 | IH8 | T1O15 | R12 | C15 | r1-c2 | r3-c3 |
| putative glucosyl transferase {Arabidopsis thaliana}PIR\|H84784\|H84784 probable glucosyl transferase | TC82051 | AW650919 | cLEI14J16 | XVIF5 | T4L10 | R15 | C12 | r2-c2 | r1-c4 |
| contains similarity to chalcone-flavonone isomerase (chalcone isomerase)~gene_id:K18I23.7 {Arabidops | TC82093 | BE435759 | cLEG28N15 | VH9 | T2O17 | R8 | C15 | r4-c2 | r1-c3 |
| phosphoribosylanthranilate isomerase {Arabidopsis thaliana} | TC82095 | BE435421 | cLEG26N3 | VG10 | T2M19 | R6 | C13 | r4-c2 | r1-c3 |
| LIN6^^acid invertase | TC82103 | AW040329 | cLET5E19 | XIIB7 | T3D14 | R11 | C4 | r2-c1 | r4-c3 |
| Contains similarity to ARI, RING finger protein | TC82118 | AW624814 | cLEZ8C12 | XIVG6 | T4M12 | R13 | C13 | r2-c2 | r1-c4 |

| | | | | | | | | | |
|---|---|---|---|---|---|---|---|---|---|
| gb|X98309 from Drosophila melanogaster. ESTs gb|T44 | | | | | | | | | |
| transcription factor-like protein {Arabidopsis thaliana}GP|7576196|emb|CAB87947.1||AL163912 transcr | TC82130 | AW039518 | cLET14M13 | XID8 | T3H15 | R12 | C8 | r2-c1 | r4-c3 |
| polyphenol oxidase precursor | TC82138 | BF098557 | cLEW27J20 | XIIH7 | T3P14 | R11 | C16 | r2-c1 | r4-c3 |
| bHLH transcription factor GBOF-1 {Tulipa gesneriana} | TC82153 | BF050210 | cLEM17A5 | VIIG9 | T2N17 | R8 | C14 | r4-c2 | r1-c3 |
| glycerol-3-phosphate dehydrogenase {Arabidopsis thaliana}PIR|F84832|F84832 glycerol-3-phosphate deh | TC82167 | BF050313 | cLEM17G22 | VIE3 | T2I6 | R19 | C9 | r4-c2 | r1-c3 |
| putative cytochrome P450 {Oryza sativa}GP|11761117|dbj|BAB19107.1||AP002839 putative cytochrome P45 | TC82169 | AI488390 | cLED21I12 | IVA3 | T1B6 | R19 | C2 | r1-c2 | r3-c3 |
| transcription factor IIA small subunit {Arabidopsis thaliana}GP|5051786|emb|CAB45079.1||AL078637 tr | TC82195 | BG132223 | cTOE6J10 | XVIIIG1 | T5M2 | R23 | C13 | r3-c2 | r4-c4 |
| glucosyltransferase-like protein {Arabidopsis thaliana}GP|7340661|emb|CAB82941.1||AL162506 putative | TC82199 | AW623225 | cTOB9O19 | XVIIC2 | T5E3 | R22 | C5 | r3-c2 | r4-c4 |
| chalcone synthase-like protein {Arabidopsis thaliana}GP|7270436|emb|CAB80202.1||AL161586 chalcone s | TC82205 | AW944832 | cTOB12G4 | XVIIA1 | T5A1 | R24 | C1 | r3-c2 | r4-c4 |
| sugar transporter-like protein {Arabidopsis thaliana} | TC82207 | BG123226 | cTOF1A20 | XIXC10 | T5F19 | R6 | C6 | r3-c2 | r4-c4 |
| putative cytochrome P450 {Arabidopsis thaliana}GP|13877669|gb|AAK43912.1|AF370593_1|AF370593 putati | TC82226 | AI898212 | cLED32E24 | IIIG1 | T1N1 | R24 | C14 | r1-c2 | r3-c3 |
| putative zinc finger protein {Arabidopsis thaliana}GP|7270045|emb|CAB79860.1||AL161579 putative zin | TC82243 | AW032181 | cLEC20K13 | IE2 | T1I3 | R22 | C9 | r1-c2 | r3-c3 |
| 2-oxoglutarate/malate translocator precursor {Spinacia oleracea}SP|Q41364|SOT1_SPIOL 2-OXOGLUTARATE | TC82252 | BE437181 | cLEG1G13 | VF10 | T2K19 | R6 | C11 | r4-c2 | r1-c3 |
| NADH-dependent glutamate synthase {Arabidopsis thaliana} | TC82279 | AW032148 | cLEC38H15 | IIC7 | T1E14 | R11 | C5 | r1-c2 | r3-c3 |
| anthocyanidin 3-O-glucosyltransferase {Petunia x hybrida} | TC82331 | BG628982 | cLEL24M4 | XXIA2 | T6A3 | R22 | C1 | r3-c1 | r2-c4 |
| 3-methylcrotonyl-CoA carboxylase non-biotinylated subunit {Arabidopsis thaliana}GP|7021224|gb|AAF35 | TC82338 | AW649104 | cLEI7I13 | VIIF11 | T2L21 | R4 | C12 | r4-c2 | r1-c3 |
| Cytochrome P450-like protein {Arabidopsis | TC82348 | AW033276 | cLEC28N5 | IG7 | T1M13 | R12 | C13 | r1-c2 | r3-c3 |

| | | | | | | | | | |
|---|---|---|---|---|---|---|---|---|---|
| thaliana}GP\|7270098\|emb\|CAB79912.1\|\|AL161580 Cytochrome P | | | | | | | | | |
| transketolase 1 {Capsicum annuum}PIR\|T09541\|T09541 transketolase (EC 2.2.1.1) TKT1 precursor, chlor | TC82386 | AW035937 | cLEC37J10 | IIC3 | T1E6 | R19 | C5 | r1-c2 | r3-c3 |
| HEAT SHOCK FACTOR PROTEIN 5 (HSF 5) (HEAT SHOCK TRANSCRIPTION FACTOR 5) (HSTF 5).GP\|6624614\|emb\|CAB | TC82389 | BE459508 | cLEM7C5 | VIIIE10 | T2J20 | R5 | C10 | r4-c2 | r1-c3 |
| Similar to Populus balsamifera subsp. trichocarpa X Populus deltoides vegetative storage protein. (L | TC82393 | BG626572 | cLEL13F1 | XXIB8 | T6C15 | R12 | C3 | r3-c1 | r2-c4 |
| putative enolase (2-phospho-D-glycerate hydroylase) {Arabidopsis thaliana}PIR\|G84697\|G84697 hypothe | TC82394 | BF112854 | cLEG42J1 | VIF8 | T2K16 | R9 | C11 | r4-c2 | r1-c3 |
| putative CCCH-type zinc finger protein {Arabidopsis thaliana}PIR\|D84581\|D84581 probable CCCH-type z | TC82395 | AW649384 | cLEI8G9 | IVH9 | T1P18 | R7 | C16 | r1-c2 | r3-c3 |
| 3-dehydroquinate synthase-like protein {Arabidopsis thaliana} | TC82414 | BE353857 | cTOD6D17 | XVIIIA1 | T5A2 | R23 | C1 | r3-c2 | r4-c4 |
| cytochrome P450-like protein {Arabidopsis thaliana}GP\|7270932\|emb\|CAB80611.1\|\|AL161595 cytochrome P | TC82416 | AW738712 | cTOD8E14 | XVIIIA12 | T5A24 | R1 | C1 | r3-c2 | r4-c4 |
| isoflavone reductase homolog {Solanum tuberosum}SP\|P52578\|IFRH_SOLTU ISOFLAVONE REDUCTASE HOMOLOG ( | TC82426 | AI897693 | cLED30K7 | IIIF7 | T1L13 | R12 | C12 | r1-c2 | r3-c3 |
| putative pyrophosphate--fructose-6-phosphate 1-phosphotransferase {Arabidopsis thaliana}PIR\|B84613\| | TC82429 | AW929062 | cTOC3P9 | XIIIE2 | T4I3 | R22 | C9 | r2-c2 | r1-c4 |
| phosphoglycerate mutase {Solanum tuberosum} | TC82433 | AI777247 | cLER20D8 | XVIIF12 | T5K23 | R2 | C11 | r3-c2 | r4-c4 |
| 6-phosphogluconate dehydrogenase, putative; 13029-14489 {Arabidopsis thaliana} | TC82459 | BG125863 | cTOF10K5 | XVIIIG8 | T5M16 | R9 | C13 | r3-c2 | r4-c4 |
| putative P-protein: chorismate mutase, prephenate dehydratase {Arabidopsis thaliana} | TC82472 | BG134235 | cTOE15D14 | XVIIIC9 | T5E18 | R7 | C5 | r3-c2 | r4-c4 |
| putative arginine methyltransferase {Arabidopsis thaliana} | TC82475 | BF050261 | cLEM17M11 | XIVA2 | T4A4 | R21 | C1 | r2-c2 | r1-c4 |
| P450 hydroxylase {Petunia x hybrida}PIR\|S32110\|S32110 cytochrome P450 PET-1 - garden petunia (fragm | TC82490 | AW035596 | cLEC39N22 | IID2 | T1G4 | R21 | C7 | r1-c2 | r3-c3 |
| pathogenesis-related homeodomain protein (prhp) {Petroselinum crispum}SP\|P48786\|PRH_PETCR PATHOGENE | TC82493 | BG135032 | cTOE21I7 | XVIIIE3 | T5I6 | R19 | C9 | r3-c2 | r4-c4 |

| | | | | | | | | |
|---|---|---|---|---|---|---|---|---|
| ferredoxin--nitrite reductase {Nicotiana tabacum}GP\|19893\|emb\|CAA46940.1\|\|X66145 ferredoxin--nitrit | TC82500 | AW648617 | cLEI5J9 | XVIA10 | T4B20 | R5 | C2 | r2-c2 | r1-c4 |
| contains similarity to heat shock transcription factor~gene_id:MOB24.9 {Arabidopsis thaliana} | TC82508 | AW649243 | cLEI7H17 | VIIF10 | T2L19 | R6 | C12 | r4-c2 | r1-c3 |
| starch synthase {Ipomoea batatas} | TC82511 | BG630199 | cLEL33H7 | XXIH10 | T6O19 | R6 | C15 | r3-c1 | r2-c4 |
| alpha-glucosidase {Solanum tuberosum subsp. tuberosum} | TC82534 | AW623694 | cTOB11D18 | XVIG1 | T4N2 | R23 | C14 | r2-c2 | r1-c4 |
| similar to class I knotted-like homeodomain protein (LeT6 | TC82559 | AW035887 | cLEC35D19 | IIB2 | T1C4 | R21 | C3 | r1-c2 | r3-c3 |
| putative internal rotenone-insensitive NADH dehydrogenase {Solanum tuberosum} | TC82565 | AI773904 | cLER8F23 | XC7 | T3E14 | R11 | C5 | r2-c1 | r4-c3 |
| putative C3HC4-type RING zinc finger protein {Arabidopsis thaliana}GP\|11908040\|gb\|AAG41449.1\|AF3268 | TC82567 | AI780031 | cLES9N18 | XIB9 | T3D17 | R8 | C4 | r2-c1 | r4-c3 |
| acetyl-CoA C-acetyltransferase {Arabidopsis thaliana} | TC82576 | BE458863 | cLEM4F14 | VIIID8 | T2H16 | R9 | C8 | r4-c2 | r1-c3 |
| amidophosphoribosyltransferase {Arabidopsis thaliana} | TC82583 | BE451488 | cLEY18N18 | XIH11 | T3P21 | R4 | C16 | r2-c1 | r4-c3 |
| putative anthocyanin 5-aromatic acyltransferase {Arabidopsis thaliana}PIR\|G84823\|G84823 probable an | TC82613 | AI775065 | cLER14O11 | IXF7 | T3K13 | R12 | C11 | r2-c1 | r4-c3 |
| cystathionine beta-lyase {Solanum tuberosum} | TC82650 | AI487273 | cLED11M11 | IIG12 | T1M24 | R1 | C13 | r1-c2 | r3-c3 |
| glutamine synthetase I {Medicago truncatula} | TC82659 | BG129068 cTOF23A10 | cTOF23A10 | XIXF6 | T5L11 | R14 | C12 | r3-c2 | r4-c4 |
| putative cytochrome P450; 1456-3294 {Arabidopsis thaliana}GP\|10092278\|gb\|AAG12691.1\|AC025814_15\| AC0 | TC82728 | BF097238 | cLEW19J16 | XIIE7 | T3J14 | R11 | C10 | r2-c1 | r4-c3 |
| HOMEOBOX-LEUCINE ZIPPER PROTEIN HAT22 (HD-ZIP PROTEIN 22).GP\|549887\|gb\|AAA56902.1\|\|U09336 homeobox | TC82731 | AW222687 | cLEN9E11 | IXD12 | T3G23 | R2 | C7 | r2-c1 | r4-c3 |
| transcription factor inhibitor I kappa B homolog {Arabidopsis thaliana}GP\|1773295\|gb\|AAC49611.1\|\|U7 | TC82775 | AW160235 | cLPT1I9 | XVH8 | T4P15 | R12 | C16 | r2-c2 | r1-c4 |
| putative glycerol-3-phosphate dehydrogenase {Arabidopsis thaliana} | TC82784 | BE354321 | cTOD9P8 | XVIIIB8 | T5C16 | R9 | C3 | r3-c2 | r4-c4 |
| NADH dehydrogenase like protein {Arabidopsis thaliana}GP\|7268946\|emb\|CAB81256.1\|\|AL161555 | TC82792 | AW738592 | cTOD7J8 | XVIIIA7 | T5A14 | R11 | C1 | r3-c2 | r4-c4 |

178

| | | | | | | | | | |
|---|---|---|---|---|---|---|---|---|---|
| NADH dehy | | | | | | | | | |
| zinc finger protein SHI-like {Arabidopsis thaliana}GP\|4929803\|gb\|AAD34162.1\|AF152555_1\|AF 152555 put | TC82801 | AW034970 | cLEC31N2 0 | IH6 | T1O11 | R14 | C15 | r1-c2 | r3-c3 |
| inorganic phosphate transporter | TC82826 | AW622613 | cLEX15D8 | XIIIC10 | T4E19 | R6 | C5 | r2-c2 | r1-c4 |
| polyneuridine aldehyde esterase {Rauvolfia serpentina} | TC82834 | AI772823 | cLER4A2 | XB10 | T3C20 | R5 | C3 | r2-c1 | r4-c3 |
| alpha-glucosidase {Solanum tuberosum subsp. tuberosum} | TC82868 | AI487472 | cLED11M2 | IIH1 | T1O2 | R23 | C15 | r1-c2 | r3-c3 |
| ABC transporter-like protein {Arabidopsis thaliana}GP\|13899119\|gb\|AAK48981.1\|AF370554_1\|A F370554 AB | TC82872 | BF097895 | cLEW24O1 7 | XVC5 | T4F9 | R16 | C6 | r2-c2 | r1-c4 |
| heat shock factor protein hsf24 (heat shock transcription factor 24) (hstf 24) (heat stress transcri | TC82923 | BG134658 | cTOE17D2 1 | XVIIID3 | T5G6 | R19 | C7 | r3-c2 | r4-c4 |
| sugar transporter like protein {Arabidopsis thaliana}GP\|2464913\|emb\|CAB16808.1\|\|Z99708 sugar transp | TC82942 | BG137839 | cLPP7D3 | XVF2 | T4L3 | R22 | C12 | r2-c2 | r1-c4 |
| cytochrome P450-like protein {Arabidopsis thaliana}PIR\|T47554\|T47554 cytochrome P450 homolog F8J2.1 | TC82954 | AW621777 | cLEX13E10 | XIIIC1 | T4E1 | R24 | C5 | r2-c2 | r1-c4 |
| TRYPTOPHAN SYNTHASE BETA CHAIN 2 PRECURSOR (EC 4.2.1.20).GP\|2792520\|gb\|AAB97087.1\|\|AF042320 tryptop | TC82960 | AW650635 | cLEI13J17 | VIIB12 | T2D23 | R2 | C4 | r4-c2 | r1-c3 |
| putative C3HC4-type RING zinc finger protein {Arabidopsis thaliana}PIR\|B84813\|B84813 probable RING | TC82965 | AW034233 | cLEC32P10 | IH11 | T1O21 | R4 | C15 | r1-c2 | r3-c3 |
| Similar to gb\|Z84571 anthranilate N-hydroxycinnamoyl/benzoyltransferase from Dianthus caryophyllus. | TC82992 | AW154873 | cLEW1M9 | XIIF9 | T3L18 | R7 | C12 | r2-c1 | r4-c3 |
| ABC transporter homolog {Populus nigra} | TC83000 | AW035260 | cLEC34N2 | IIA12 | T1A24 | R1 | C1 | r1-c2 | r3-c3 |
| contains similarity to ABC transporter~gene_id:MAC9.4 {Arabidopsis thaliana} | TC83006 | BG140588 | cLPP17P7 | XVE3 | T4J5 | R20 | C10 | r2-c2 | r1-c4 |
| putative strictosidine synthase | TC83008 | BG627604 | cLEL18I5 | XXID6 | T6G11 | R14 | C7 | r3-c1 | r2-c4 |
| phosphate/phosphoenolpyruvate translocator precursor {Nicotiana tabacum}GP\|1778145\|gb\|AAB40648.1\|\|U | TC83014 | AW040539 | cLET6N24 | XIIC1 | T3F2 | R23 | C6 | r2-c1 | r4-c3 |
| putative enolase; 31277-33713 {Arabidopsis thaliana}PIR\|B96768\|B96768 protein enolase F2P9.10 | TC83066 | AI488919 | cLED18H1 5 | IIIB8 | T1D15 | R12 | C4 | r1-c2 | r3-c3 |

179

| Description | TC | Accession | Clone | | | | | | | |
|---|---|---|---|---|---|---|---|---|---|---|
| diacylglycerol kinase {Lycopersicon esculentum} | TC83073 | AW616611 | cLHT11J12 | XIVH8 | T4O16 | R9 | C15 | r2-c2 | r1-c4 | |
| cytochrome P450 {Capsicum annuum} | TC83085 | BF097766 | cLEW23F1 1 | XIIG4 | T3N8 | R17 | C14 | r2-c1 | r4-c3 | |
| contains similarity to RNA polymerase transcriptional regulation mediator~gene_id:MHC9.3 {Arabidopsi | TC83117 | BG131064 | cTOE2M8 | XVIIIF4 | T5K8 | R17 | C11 | r3-c2 | r4-c4 | |
| acetyl-CoA synthetase, putative; 45051-31547 {Arabidopsis thaliana}PIR|D96595|D96595 probable acety | TC83139 | BG124837 | cTOF6J17 | XXF3 | T5L6 | R19 | C12 | r3-c2 | r4-c4 | |
| ABC transporter-like protein {Arabidopsis thaliana}PIR|T07717|T07717 probable ABC-type transport pr | TC83143 | AW035264 | cLEC34L14 | IIA10 | T1A20 | R5 | C1 | r1-c2 | r3-c3 | |
| putative sugar transporter {Arabidopsis thaliana} | TC83157 | AW032681 | cLEC25N4 | IF12 | T1K23 | R2 | C11 | r1-c2 | r3-c3 | |
| putative zinc finger protein {Arabidopsis thaliana}GP|7270045|emb|CAB79860.1||AL161579 putative zin | TC83165 | BE432483 | cLEG8M16 | VIIA1 | T2B1 | R24 | C2 | r4-c2 | r1-c3 | |
| CYTOCHROME P450 98A2 (EC 1.14.-.-).GP|2738998|gb|AAB94587.1||AF022458 CYP98A2p {Glycine max}PIR|T0 | TC83207 | BE432077 | cLEG5C20 | VIH3 | T2O6 | R19 | C15 | r4-c2 | r1-c3 | |
| transcription factor, putative {Arabidopsis thaliana}PIR|E96612|E96612 probable transcription facto | TC83217 | BF051437 | cLEM22N8 | VIIIB7 | T2D14 | R11 | C4 | r4-c2 | r1-c3 | |
| auxin-induced basic helix-loop-helix transcription factor {Gossypium hirsutum} | TC83218 | BG129020 | cTOF23G1 5 | XIXF10 | T5L19 | R6 | C12 | r3-c2 | r4-c4 | |
| ABC transporter-like protein {Arabidopsis thaliana}GP|9964121|gb|AAG09829.1|AF287699_1|AF287699 hal | TC83254 | BE460242 | cLEG27H1 1 | VH1 | T2O1 | R24 | C15 | r4-c2 | r1-c3 | |
| bHLH transcription factor JAF13 {Petunia x hybrida} | TC83264 | BE435307 | cLEG25N1 4 | VG9 | T2M17 | R8 | C13 | r4-c2 | r1-c3 | |
| glycerol-3-phosphate dehydrogenase {Arabidopsis thaliana}PIR|F84832|F84832 glycerol-3-phosphate deh | TC83308 | AI778474 | cLESSM24 | XH12 | T3O24 | R1 | C15 | r2-c1 | r4-c3 | |
| cytochrome p450 lxxia3 {Solanum melongena}SP|P37119|C713_SOLME CYTOCHROME P450 71A3 (EC 1.14.-.-) ( | TC83334 | BG734604 | cLEL12A1 | XXIB4 | T6C7 | R18 | C3 | r3-c1 | r2-c4 | |
| putative 6-phosphogluconolactonase {Arabidopsis thaliana} | TC83350 | BE451183 | cLEY16L2 | XIG4 | T3N7 | R18 | C14 | r2-c1 | r4-c3 | |
| soluble starch (bacterial glycogen) synthase {Solanum | TC83359 | BE434446 | cLEG17G1 | VF4 | T2K7 | R18 | C11 | r4-c2 | r1-c3 | |

180

| | | | | | | | | | |
|---|---|---|---|---|---|---|---|---|---|
| tuberosum}SP\|P93568\|UGS2_SOLTU SOLUBLE GLYCOG | | | | | | | | | |
| beta-amylase-like {Arabidopsis thaliana} | TC83371 | AW648284 | cLEI4E17 | VIIE5 | T2J9 | R16 | C10 | r4-c2 | r1-c3 |
| cytochrome P450 {Arabidopsis thaliana}GP\|7268718\|emb\|CAB78925.1\|\|AL161550 cytochrome P450 {Arabidop | TC83399 | AW031490 | cLEC40I14 | IID6 | T1G12 | R13 | C7 | r1-c2 | r3-c3 |
| fructokinase {Lycopersicon esculentum}GP\|2102691\|gb\|AAB57733.1\|\|U64817 fructokinase {Lycopersicon e | TC83425 | BG139723 | cLPP14E5 | XVD10 | T4H19 | R6 | C8 | r2-c2 | r1-c4 |
| putative citrate synthase {Arabidopsis thaliana}PIR\|C84858\|C84858 probable citrate synthase [import | TC83487 | AW037360 | cLET1C16 | XIE6 | T3J11 | R14 | C10 | r2-c1 | r4-c3 |
| putative acetone-cyanohydrin lyase {Arabidopsis thaliana}PIR\|T01151\|T01151 probable acetone-cyanohy | TC83491 | BE450272 | cLEY12P15 | XIIIG1 | T4M1 | R24 | C13 | r2-c2 | r1-c4 |
| putative pyrophosphate-dependent phosphofructo-1-kinase {Arabidopsis thaliana} | TC83500 | AW616742 | cLHT12F16 | XIIF8 | T3L16 | R9 | C12 | r2-c1 | r4-c3 |
| uroporphyrinogen decarboxylase {Arabidopsis thaliana} | TC83506 | AI491153 | cLEB3I24 | IB5 | T1C9 | R16 | C3 | r1-c2 | r3-c3 |
| Zn finger protein {Nicotiana tabacum}GP\|1360078\|emb\|CAA66601.1\|\|X97942 Zn finger protein {Nicotiana | TC83522 | AI490285 | cLED24N8 | IIID2 | T1H3 | R22 | C8 | r1-c2 | r3-c3 |
| transcription factor WRKY6 {Arabidopsis thaliana}GP\|12658412\|gb\|AAK01128.1\|AF331713_1\|A F331713 tran | TC83553 | AW648696 | cLEI5A6 | VIIE10 | T2J19 | R6 | C10 | r4-c2 | r1-c3 |
| limonene cyclase like protein {Arabidopsis thaliana}GP\|2245029\|emb\|CAB10449.1\|\|Z97341 limonene cycl | TC83555 | AI488634 | cLED17P22 | IIIB5 | T1D9 | R16 | C4 | r1-c2 | r3-c3 |
| GMP synthase; 61700-64653 {Arabidopsis thaliana}PIR\|E96661\|E96661 GMP synthase, 61700-64653 [import | TC83694 | AW616602 | cLHT11H10 | XIVH4 | T4O8 | R17 | C15 | r2-c2 | r1-c4 |
| beta-amylase {Glycine max}GP\|902938\|dbj\|BAA09462.1\|\|D50866 beta-amylase {Glycine max} | TC83696 | BG125052 | cTOF7D1 | XXF6 | T5L12 | R13 | C12 | r3-c2 | r4-c4 |
| pyruvate kinase (EC 2.7.1.40) A, chloroplast - common tobacco | TC83701 | AI780946 | cLES13J24 | XD10 | T3G20 | R5 | C7 | r2-c1 | r4-c3 |
| cytochrome p450-like protein {Arabidopsis thaliana}GP\|7270718\|emb\|CAB80401.1\|\|AL161591 cytochrome p | TC83712 | AW617482 | cLHT23O11 | XVB3 | T4D5 | R20 | C4 | r2-c2 | r1-c4 |

181

| | | | | | | | | |
|---|---|---|---|---|---|---|---|---|---|
| Putative UDP-glucose glucosyltransferase {Arabidopsis thaliana}PIR\|H86356\|H86356 probable UDP-gluco | TC83719 | AI482910 | cLEB1J8 | IA6 | T1A11 | R14 | C1 | r1-c2 | r3-c3 |
| Similar to gb\|Z84386 anthranilate N-hydroxycinnamoyl/benzoyltransferase from Dianthus caryophyllus. | TC83737 | AW616931 | cLHT18F9 | XVA6 | T4B11 | R14 | C2 | r2-c2 | r1-c4 |
| Phosphoglycerate dehydrogenase-like protein {Arabidopsis thaliana}GP\|7270370\|emb\|CAB80137.1\|AL1615 | TC83740 | AW650818 | cLEI14F1 | VIIC2 | T2F3 | R22 | C6 | r4-c2 | r1-c3 |
| phosphoribosyl pyrophosphate synthase isozyme 4 {Spinacia oleracea} | TC83753 | AW623664 | cTOB11N7 | XVIG7 | T4N14 | R11 | C14 | r2-c2 | r1-c4 |
| putative dihydroflavonol reductase {Oryza sativa} | TC83761 | AW624036 | cTOB13L22 | XVIH9 | T4P18 | R7 | C16 | r2-c2 | r1-c4 |
| hexose transporter | TC83763 | | | | | | | | |
| tyrosine/dopa decarboxylase {Thalictrum flavum subsp. glaucum} | TC83804 | BE449262 | cLHT31C18 | XVB12 | T4D23 | R2 | C4 | r2- c2 | r1-c4 |
| Cytochrom P450-like protein {Arabidopsis thaliana}PIR\|T46159\|T46159 cytochrome P450-like protein - | TC83813 | BF096935 | cLEW18E10 | XIID12 | T3H24 | R1 | C8 | r2-c1 | r4-c3 |
| glycolate oxidase {Arabidopsis thaliana} | TC83832 | BE449243 | cLHT31O9 | XVC4 | T4F7 | R18 | C6 | r2-c2 | r1-c4 |
| alpha-glucan phosphorylase, h isozyme phosphorylase h) {Solanum tuberosum}SP\|P32811\|PHSH_SOLTU ALPH | TC83865 | BG734793 | cLEL16K20 | XXID7 | T6G13 | R12 | C7 | r3-c1 | r2-c4 |
| CYTOCHROME P450 98A3 (EC 1.14.-.-).GP\|2623303\|gb\|AAB86449.1\|AC002409 putative cytochrome P450 {Ara | TC83866 | AI779624 | cLES8L1 | XIB1 | T3D1 | R24 | C4 | r2-c1 | r4-c3 |
| general negative transcription regulator-like {Arabidopsis thaliana} | TC83872 | BG140293 | cLPP16F4 | XVE1 | T4J1 | R24 | C10 | r2-c2 | r1-c4 |
| Dof zinc finger protein {Nicotiana tabacum}PIR\|T02203\|T02203 finger protein Dof - common tobacco (f | TC83881 | AI487752 | cLED11A23 | IIG5 | T1M10 | R15 | C13 | r1-c2 | r3-c3 |
| putative ABC transporter {Arabidopsis thaliana}GP\|4115931\|gb\|AAD03441.1\|AF118223 contains similari | TC83901 | AI779502 | cLES8O5 | XIB6 | T3D11 | R14 | C4 | r2-c1 | r4-c3 |
| ornithine carbamoyltransferase; OCTase {Canavalia lineata}GP\|12003188\|gb\|AAG43481.1\|AF203688_1\|AF 20 | TC83905 | AI778009 | cLES3P1 | XH4 | T3O8 | R17 | C15 | r2-c1 | r4-c3 |
| succinate dehydrogenase iron-protein subunit | TC83937 | AW032637 | cLEC20L4 | IE3 | T1I5 | R20 | C9 | r1-c2 | r3-c3 |

| {Arabidopsis thaliana} | | | | | | | | | |
|---|---|---|---|---|---|---|---|---|---|
| tyrosine aminotransferase {Arabidopsis thaliana} | TC83980 | AW219060 | cLEX2J17 | XIIID8 | T4G15 | R12 | C7 | r2-c2 | r1-c4 |
| ferredoxin--nitrite reductase {Nicotiana tabacum}GP|861067|emb|CAA46942.1||X66147 ferredoxin--nitri | TC83986 | AI776535 | cLER18J6 | IXH8 | T3O15 | R12 | C15 | r2-c1 | r4-c3 |
| legumin-like protein {Arabidopsis thaliana}PIR|H84687|H84687 legumin-like protein [imported] - Arab | TC84014 | AW622321 | cLEX14L6 | XIIIC8 | T4E15 | R12 | C5 | r2-c2 | r1-c4 |
| MADS transcriptional factor; STMADS16 {Solanum tuberosum}PIR|T06995|T06995 probable MADS box transc | TC84038 | AI482813 | cLEB3L11 | IB6 | T1C11 | R14 | C3 | r1-c2 | r3-c3 |
| amidophosphoribosyltransferase {Arabidopsis thaliana} | TC84044 | AW154806 | cLEW1C23 | XIIE11 | T3J22 | R3 | C10 | r2-c1 | r4-c3 |
| transketolase 1 {Capsicum annuum}PIR|T09541|T09541 transketolase (EC 2.2.1.1) TKT1 precursor, chlor | TC84048 | BE450953 | cLEY15H2 4 | XIIIH5 | T4O9 | R16 | C15 | r2-c2 | r1-c4 |
| UDP-GLUCOSE 4-EPIMERASE GEPI48 (EC 5.1.3.2) (GALACTOWALDENASE) (UDP- GALACTOSE 4-EPIMERASE).GP|3021 | TC84055 | AI484890 | cLED3N7 | IVA4 | T1B8 | R17 | C2 | r1-c2 | r3-c3 |
| contains similarity to chorismate mutase-T and prephenate dehydrogenase~gene_id:MGG23.1 {Arabidopsis | TC84057 | AW616637 | cLHT11N2 2 | XIVH11 | T4O22 | R3 | C15 | r2-c2 | r1-c4 |
| Strong similarity to gb|Z50851 HD-zip (athb-8) gene from Arabidopsis thaliana containing Homeobox PF | TC84068 | AI781675 | cLES16B20 | XE6 | T3I12 | R13 | C9 | r2-c1 | r4-c3 |
| Is a member of the PF|00044 glyceraldehyde 3-phosphate dehydrogenase family. ESTs gb|T43985, gb|N38 | TC84078 | AW930283 | cLEF41H8 | IVF3 | T1L6 | R19 | C12 | r1-c2 | r3-c3 |
| putative hydroxymethylglutaryl-CoA lyase {Arabidopsis thaliana}PIR|T02655|T02655 hydroxymethylgluta | TC84085 | AW650521 | cLEI13C14 | VIIB7 | T2D13 | R12 | C4 | r4-c2 | r1-c3 |
| putative anthranilate N-hydroxycinnamoyl/benzoyltransferase {Arabidopsis thaliana}PIR|T00527|T00527 | TC84103 | AW217721 | cTOC6C19 | XVIIE5 | T5I9 | R16 | C9 | r3-c2 | r4-c4 |
| RING zinc finger protein-like {Arabidopsis thaliana} | TC84140 | AW222126 | cLEN7C15 | IXD4 | T3G7 | R18 | C7 | r2-c1 | r4-c3 |
| CCAAT box binding factor/ transcription factor Hap2a {Arabidopsis thaliana}PIR|T49898|T49898 CCAAT | TC84198 | AW030886 | cLEC15K2 | ID7 | T1G13 | R12 | C7 | r1-c2 | r3-c3 |
| MADS-box transcription factor FBP24 {Petunia x hybrida} | TC84232 | AI899235 | cLED37P16 | IIIH4 | T1P7 | R18 | C16 | r1-c2 | r3-c3 |

| Description | ID | Accession | Clone | | | | | | |
|---|---|---|---|---|---|---|---|---|---|
| adenylosuccinate lyase-like protein; 104558-106845 {Arabidopsis thaliana}PIR\|B86484\|B86484 hypothet | TC84319 | BF097158 | cLEW19J11 | XIIE6 | T3J12 | R13 | C10 | r2-c1 | r4-c3 |
| pyruvate kinase-like protein {Arabidopsis thaliana}PIR\|T47556\|T47556 pyruvate kinase-like protein - | TC84320 | AW037759 | cLET3L18 | XIH9 | T3P17 | R8 | C16 | r2-c1 | r4-c3 |
| unnamed protein product {unidentified}GP\|2462911\|emb\|CAB06081.1\|\|Z83832 UDP-glucose:sterol glucosyl | TC84322 | AW040240 | cLET19N8 | XIE3 | T3J5 | R20 | C10 | r2-c1 | r4-c3 |
| folylpolyglutamate synthase-like protein {Arabidopsis thaliana} | TC84330 | AI782088 | cLES18G17 | XF9 | T3K18 | R7 | C11 | r2-c1 | r4-c3 |
| dihydroxy-acid dehydratase {Arabidopsis thaliana} | TC84340 | AI898180 | cLED31P20 | IIIF11 | T1L21 | R4 | C12 | r1-c2 | r3-c3 |
| CTP synthase like protein {Arabidopsis thaliana}GP\|7268827\|emb\|CAB79032.1\|\|AL161552 CTP synthase li | TC84368 | AW979649 | cLEW8A16 | XIIIH10 | T3P20 | R5 | C16 | r2-c1 | r4-c3 |
| Putative acyl-CoA:1-acylglycerol-3-phosphate acyltransferase {Arabidopsis thaliana}PIR\|D96550\|D965 | TC84467 | AA824736 | CT041 | XVIC7 | T4F14 | R11 | C6 | r2-c2 | r1-c4 |
| putative gluconokinase {Arabidopsis thaliana}PIR\|C84544\|C84544 probable gluconokinase [imported] - | TC84507 | AI781650 | cLES16N11 | XF1 | T3K2 | R23 | C11 | r2-c1 | r4-c3 |
| Strong similarity to F19I3.2 GP\|3033375 putative berberine bridge enzyme from Arabidopsis thaliana B | TC84522 | AI491197 | cLEB1G24 | IA3 | T1A5 | R20 | C1 | r1-c2 | r3-c3 |
| homeodomain-leucine zipper protein 57 {Glycine max} | TC84550 | AW617989 | cLPT11L19 | XVF12 | T4L23 | R2 | C12 | r2-c2 | r1-c4 |
| transcription factor {Nicotiana tabacum} | TC84557 | AW933661 | cLEF55M14 | VB12 | T2C23 | R2 | C3 | r4-c2 | r1-c3 |
| carbamoyl-phosphate synthetase small subunit {Arabidopsis thaliana} | TC84578 | AW092546 | cLET20F12 | XIE11 | T3J21 | R4 | C10 | r2-c1 | r4-c3 |
| UDP glucose dehydrogenase | 108 | | | A 1 | T6B1 | R24 | C2 | r3-c1 | r2-c4 |
| GDP mannose pyrophosphorylase | 110 | | | A 2 | T6B3 | R22 | C2 | r3-c1 | r2-c4 |
| β galactosidase | 112 | | | A 3 | T6B5 | R20 | C2 | r3-c1 | r2-c4 |
| Sucrose phosphate synthase | 116 | | | A 4 | T6B7 | R18 | C2 | r3-c1 | r2-c4 |
| Laccase | 122 | | | A 5 | T6B9 | R16 | C2 | r3-c1 | r2-c4 |
| Oxoglutarate malate translocator | 130 | | | A 6 | T6B11 | R14 | C2 | r3-c1 | r2-c4 |
| Uncoupling protein | 136 | | | A 7 | T6B13 | R12 | C2 | r3-c1 | r2-c4 |
| unknown funktion | 167 | | | A 8 | T6B15 | R12 | C2 | r3-c1 | r2-c4 |
| unknown funktion | 168 | | | A 9 | T6B17 | R8 | C2 | r3-c1 | r2-c4 |
| beta-amylase | 169 | | | A 10 | T6B19 | R6 | C2 | r3-c1 | r2-c4 |

| | | | | | | | | | |
|---|---|---|---|---|---|---|---|---|---|
| alpha-amylase 1 | 170 | | | A 11 | T6B21 | R4 | C2 | r3-c1 | r2-c4 |
| alpha-amylase 2 | 171 | | | A 12 | T6B23 | R2 | C2 | r3-c1 | r2-c4 |
| alpha-glucosidase 11 | 172 | | | B 1 | T6D1 | R24 | C4 | r3-c1 | r2-c4 |
| alpha-glucosidase 19 | 173 | | | B 2 | T6D3 | R22 | C4 | r3-c1 | r2-c4 |
| Branching enzyme | 174 | | | B 3 | T6D5 | R20 | C4 | r3-c1 | r2-c4 |
| ATPase | 195 | | | B 4 | T6D7 | R18 | C4 | r3-c1 | r2-c4 |
| Adenylate kinase | 204 | | | B 5 | T6D9 | R16 | C4 | r3-c1 | r2-c4 |
| UMP-synthase | 211 | | | B 6 | T6D11 | R14 | C4 | r3-c1 | r2-c4 |
| Major intrinsic protein 1 | 218 | | | B 7 | T6D13 | R12 | C4 | r3-c1 | r2-c4 |
| Major intrinsic protein 2 | 226 | | | B 8 | T6D15 | R12 | C4 | r3-c1 | r2-c4 |
| Phosphate transporter 1 | 229 | | | B 9 | T6D17 | R8 | C4 | r3-c1 | r2-c4 |
| Phosphate transporter 2 | 230 | | | B 10 | T6D19 | R6 | C4 | r3-c1 | r2-c4 |
| Phosphate transporter 3 | 231 | | | B 11 | T6D21 | R4 | C4 | r3-c1 | r2-c4 |
| Disproporting enzyme | 239 | | | B 12 | T6D23 | R2 | C4 | r3-c1 | r2-c4 |
| Branching enzyme | 261 | | | C 1 | T6F1 | R24 | C6 | r3-c1 | r2-c4 |
| Starch phosphorylase, plastidic | 265 | | | C 2 | T6F3 | R22 | C6 | r3-c1 | r2-c4 |
| Starch phosphorylase, cytosolic | 266 | | | C 3 | T6F5 | R20 | C6 | r3-c1 | r2-c4 |
| Granule bound starch synthase | 270 | | | C 4 | T6F7 | R18 | C6 | r3-c1 | r2-c4 |
| Root-phosphate-translocator | 272 | | | C 5 | T6F9 | R16 | C6 | r3-c1 | r2-c4 |
| Potato-brittle-transporter1 | 274 | | | C 6 | T6F11 | R14 | C6 | r3-c1 | r2-c4 |
| Pyruvate kinase, cytosolic | 300 | | | C 7 | T6F13 | R12 | C6 | r3-c1 | r2-c4 |
| Ppi:fru-6-P-phosphotransferase | 301 | | | C 8 | T6F15 | R12 | C6 | r3-c1 | r2-c4 |
| Tonoplasts bound pyrophosphatase | 302 | | | C 9 | T6F17 | R8 | C6 | r3-c1 | r2-c4 |
| Invertase, vacuolar | 303 | | | C 10 | T6F19 | R6 | C6 | r3-c1 | r2-c4 |
| | 358 | | | C 11 | T6F21 | R4 | C6 | r3-c1 | r2-c4 |
| | 359 | | | C 12 | T6F23 | R2 | C6 | r3-c1 | r2-c4 |
| | 360 | | | D 1 | T6H1 | R24 | C8 | r3-c1 | r2-c4 |
| | 362 | | | D 2 | T6H3 | R22 | C8 | r3-c1 | r2-c4 |
| | 364 | | | D 3 | T6H5 | R20 | C8 | r3-c1 | r2-c4 |
| | 378 | | | D 4 | T6H7 | R18 | C8 | r3-c1 | r2-c4 |
| Glycogene synthase kinase | 526 | | | D 5 | T6H9 | R16 | C8 | r3-c1 | r2-c4 |
| Amylogenine | 533 | | | D 6 | T6H11 | R14 | C8 | r3-c1 | r2-c4 |
| Starch synthase I | 534 | | | D 7 | T6H13 | R12 | C8 | r3-c1 | r2-c4 |
| Starch synthase II | 535 | | | D 8 | T6H15 | R12 | C8 | r3-c1 | r2-c4 |
| Phosphoglycerate mutase | 541 | | | D 9 | T6H17 | R8 | C8 | r3-c1 | r2-c4 |
| Hexokinase | 595 | | | D 10 | T6H19 | R6 | C8 | r3-c1 | r2-c4 |

| | | | | D 11 | T6H21 | R4 | C8 | r3-c1 | r2-c4 |
|---|---|---|---|---|---|---|---|---|---|
| Phosphoglucomutase | 612 | | | D 12 | T6H23 | R2 | C8 | r3-c1 | r2-c4 |
| Phosphoglucomutase | 615 | | | E 1 | T6J1 | R24 | C10 | r3-c1 | r2-c4 |
| Putative 14-3-3, unknown function | 1054 | | | E 2 | T6J3 | R22 | C10 | r3-c1 | r2-c4 |
| Putative 14-3-3, unknown function | 1055 | | | E 3 | T6J5 | R20 | C10 | r3-c1 | r2-c4 |
| Acid phosphate | 1378 | | | E 4 | T6J7 | R18 | C10 | r3-c1 | r2-c4 |
| unknown funktion | 1379 | | | E 5 | T6J9 | R16 | C10 | r3-c1 | r2-c4 |
| | 1480 | | | E 6 | T6J11 | R14 | C10 | r3-c1 | r2-c4 |
| | 1480 | | | E 7 | T6J13 | R12 | C10 | r3-c1 | r2-c4 |
| | 1513 | | | E 8 | T6J15 | R12 | C10 | r3-c1 | r2-c4 |
| beta-amylase | 1681 | | | E 9 | T6J17 | R8 | C10 | r3-c1 | r2-c4 |
| unknown funktion | 1685 | | | E 10 | T6J19 | R6 | C10 | r3-c1 | r2-c4 |
| Putativ starch degrading | 1735 | | | E 11 | T6J21 | R4 | C10 | r3-c1 | r2-c4 |
| Putativ starch degrading | 1737 | | | E 12 | T6J23 | R2 | C10 | r3-c1 | r2-c4 |
| ATP-sulfurylase, plastidic | 1823 | | | F 1 | T6L1 | R24 | C12 | r3-c1 | r2-c4 |
| ATP-sulfurylase, cytosolic | 1824 | | | F 2 | T6L3 | R22 | C12 | r3-c1 | r2-c4 |
| R1 protein | 1894 | | | F 3 | T6L5 | R20 | C12 | r3-c1 | r2-c4 |
| KST#8-1 | 1907 | | | F 4 | T6L7 | R18 | C12 | r3-c1 | r2-c4 |
| ICDH-1 | 1908 | | | F 5 | T6L9 | R16 | C12 | r3-c1 | r2-c4 |
| StIPS-1 | 1909 | | | F 6 | T6L11 | R14 | C12 | r3-c1 | r2-c4 |
| Mips | 1910 | | | F 7 | T6L13 | R12 | C12 | r3-c1 | r2-c4 |
| Malat-Enzym | 1911 | | | F 8 | T6L15 | R12 | C12 | r3-c1 | r2-c4 |
| Aconitase | 1937 | | | F 9 | T6L17 | R8 | C12 | r3-c1 | r2-c4 |
| | 1939 | | | F 10 | T6L19 | R6 | C12 | r3-c1 | r2-c4 |
| | 1940 | | | F 11 | T6L21 | R4 | C12 | r3-c1 | r2-c4 |
| | 1941 | | | F 12 | T6L23 | R2 | C12 | r3-c1 | r2-c4 |
| | 1942 | | | G 1 | T6N1 | R24 | C14 | r3-c1 | r2-c4 |
| | 1943 | | | G 2 | T6N3 | R22 | C14 | r3-c1 | r2-c4 |
| unknown funktion | 1944 | | | G 3 | T6N5 | R20 | C14 | r3-c1 | r2-c4 |
| 60S ribosomal protein L31 | 1945 | | | G 4 | T6N7 | R18 | C14 | r3-c1 | r2-c4 |
| Putative Lysopholipase | 1946 | | | G 5 | T6N9 | R16 | C14 | r3-c1 | r2-c4 |
| unknown funktion | 1947 | | | G 6 | T6N11 | R14 | C14 | r3-c1 | r2-c4 |
| unknown funktion | 1948 | | | G 7 | T6N13 | R12 | C14 | r3-c1 | r2-c4 |
| unknown funktion | 1949 | | | G 8 | T6N15 | R12 | C14 | r3-c1 | r2-c4 |
| 3-phoshoshikimate 1-carboxyvinyltransferase | 1951 | | | G 9 | T6N17 | R8 | C14 | r3-c1 | r2-c4 |
| homology to an unknown protein of Arabidopsis | 1952 | | | G 10 | T6N19 | R6 | C14 | r3-c1 | r2-c4 |

| | | | | | | | |
|---|---|---|---|---|---|---|---|
| Ribisco small chain 2A precursor | 1953 | G 11 | T6N21 | R4 | C14 | r3-c1 | r2-c4 |
| Isoamylase | 1956 | G 12 | T6N23 | R2 | C14 | r3-c1 | r2-c4 |
| Fructokinase | 1957 | H 1 | T6P1 | R24 | C16 | r3-c1 | r2-c4 |
| stearoyl-acyl carrier prot. | 1958 | H 2 | T6P3 | R22 | C16 | r3-c1 | r2-c4 |
| β-tubulin | 1959 | H 3 | T6P5 | R20 | C16 | r3-c1 | r2-c4 |
| S-adenosylmethionin decarboxylase | 1960 | H 4 | T6P7 | R18 | C16 | r3-c1 | r2-c4 |
| glucosidase | 1961 | H 5 | T6P9 | R16 | C16 | r3-c1 | r2-c4 |
| tuberisation induced gene (DC 10) | 1962 | H 6 | T6P11 | R14 | C16 | r3-c1 | r2-c4 |
| tuberisation induced gene (DC 8) | 1963 | H 7 | T6P13 | R12 | C16 | r3-c1 | r2-c4 |
| tuberisation-related gene | 1964 | H 8 | T6P15 | R12 | C16 | r3-c1 | r2-c4 |
| NtSUT1 | 1979 | H 9 | T6P17 | R8 | C16 | r3-c1 | r2-c4 |
| shaggy kinase | 1980 | H 10 | T6P19 | R6 | C16 | r3-c1 | r2-c4 |
| bZIP DANN binding protein | 1981 | H 11 | T6P21 | R4 | C16 | r3-c1 | r2-c4 |
| pollen specific ascorbat oxidase | 1982 | H 12 | T6P23 | R2 | C16 | r3-c1 | r2-c4 |

**Claims**

1.  A method for determining the function of a gene comprising

    (a) determining the amount of transcript for each of a set of candidate genes in two or more samples from an organism, wherein the samples correspond to different phenotypic and/or genotypic states of said organism;
    (b) determining the amount of each of a plurality of metabolites present in two or more samples corresponding to the same states as in (a);

(c) analysing by suitable mathematical methods the data obtained in steps (a) and (b) in order to identify a transcript and at least one metabolite the amounts of which significantly correlate in the different states,

said transcript corresponding to a gene having a function that influences the amount of said metabolite(s) in said organism.

2.  The method of claim 1, wherein the analysis in step (c) comprises the steps

    (i) determining transcripts the amount of which differs significantly between the samples of (a); and
    (ii) determining metabolites the amount of which differs significantly between the samples of (b);

    wherein the data obtained for the transcripts and the metabolites determined in steps (i) and (ii), respectively, are analysed by suitable mathematical methods in order to identify said transcript and metabolite(s) the amounts of which significantly correlate in the different states.

3.  The method of claim 1 or 2, wherein said gene encodes an enzyme, a regulatory protein, a transport protein or a transcription factor.

4.  The method of any one of claims 1 to 3, wherein a set of at least 100 candidate genes is used in step (a).

5.  The method of any one of claims 1 to 4, wherein probes that are homologous with respect to said organism are used for determining the amount of transcripts.

6.  The method of any one of claims of 1 to 5, wherein the different phenotypic and/or genotypic states are different developmental stages, taxonomic units, wild-type and mutant or transgenic organisms, infected and uninfected states, diseased and healthy states or different stages of a pathogenicity.

7.  The method of any one of claims 1 to 6, wherein the amount of transcripts and the amount of metabolites is each determined from the same sample.

8.  The method of any one of claims 1 to 7, wherein the metabolites comprise sugars, sugars alcohols, organic acids, amino acids, ascorbate, tocopherol, fatty acids, vitamins and/or polyamines.

9.  The method of any one of claims 1 to 8, wherein at least 50 metabolites are determined in step (b).

10. A method for identifying a gene which is capable of modifying the amount of a metabolite in an organism comprising steps (a) to (c) of the method of any one of claims 1 to 9, wherein said transcript identified in step (c) corresponds to a gene being capable of modifying the amount of said metabolite(s) identified in step (c).

11. A method for identifying a metabolite which is capable of modifying the amount of a transcript in an organism comprising steps (a) to (c) of the method of any one of claims 1 to 9, wherein a metabolite identified in step (c) is a candidate for a metabolite being capable of modifying the amount of said transcript identified in step (c).

12. Use of a gene the function of which has been determined by the method of any one of claims 1 to 9, of a nucleic acid molecule comprising the coding sequence of said gene or a fragment or derivative of said coding sequence showing said function or of a polypeptide encoded by said gene or nucleic acid molecule for applying said function.

13. Use of a gene identified by the method claim 10, of a nucleic acid molecule comprising the coding sequence of said gene or a fragment or derivative of said coding sequence which is capable of modifying the amount of a metabolite in an organism or of a polypeptide encoded by said gene or nucleic acid molecule for modifying the amount of a metabolite in an organism.

14. Use of a metabolite identified by the method claim 11 for modifying the amount of a transcript in an organism.

Figure 1

**Figure 2**

| | C1 | C2 | C3 | C4 | C5 | C6 | C7 | C8 | C9 | C10 | C11 | C12 | C13 | C14 | C15 | C16 |
|---|---|---|---|---|---|---|---|---|---|---|---|---|---|---|---|---|
| R1 | A24 | B24 | C24 | D24 | E24 | F24 | G24 | H24 | I24 | J24 | K24 | L24 | M24 | N24 | O24 | P24 |
| R2 | A23 | B23 | C23 | D23 | E23 | F23 | G23 | H23 | I23 | J23 | K23 | L23 | M23 | N23 | O23 | P23 |
| R3 | A22 | B22 | C22 | D22 | E22 | F22 | G22 | H22 | I22 | J22 | K22 | L22 | M22 | N22 | O22 | P22 |
| R4 | A21 | B21 | C21 | D21 | E21 | F21 | G21 | H21 | I21 | J21 | K21 | L21 | M21 | N21 | O21 | P21 |
| R5 | A20 | B20 | C20 | D20 | E20 | F20 | G20 | H20 | I20 | J20 | K20 | L20 | M20 | N20 | O20 | P20 |
| R6 | A19 | B19 | C19 | D19 | E19 | F19 | G19 | H19 | I19 | J19 | K19 | L19 | M19 | N19 | O19 | P19 |
| R7 | A18 | B18 | C18 | D18 | E18 | F18 | G18 | H18 | I18 | J18 | K18 | L18 | M18 | N18 | O18 | P18 |
| R8 | A17 | B17 | C17 | D17 | E17 | F17 | G17 | H17 | I17 | J17 | K17 | L17 | M17 | N17 | O17 | P17 |
| R9 | A16 | B16 | C16 | D16 | E16 | F16 | G16 | H16 | I16 | J16 | K16 | L16 | M16 | N16 | O16 | P16 |
| R10 | A15 | B15 | C15 | D15 | E15 | F15 | G15 | H15 | I15 | J15 | K15 | L15 | M15 | N15 | O15 | P15 |
| R11 | A14 | B14 | C14 | D14 | E14 | F14 | G14 | H14 | I14 | J14 | K14 | L14 | M14 | N14 | O14 | P14 |
| R12 | A13 | B13 | C13 | D13 | E13 | F13 | G13 | H13 | I13 | J13 | K13 | L13 | M13 | N13 | O13 | P13 |
| R13 | A12 | B12 | C12 | D12 | E12 | F12 | G12 | H12 | I12 | J12 | K12 | L12 | M12 | N12 | O12 | P12 |
| R14 | A11 | B11 | C11 | D11 | E11 | F11 | G11 | H11 | I11 | J11 | K11 | L11 | M11 | N11 | O11 | P11 |
| R15 | A10 | B10 | C10 | D10 | E10 | F10 | G10 | H10 | I10 | J10 | K10 | L10 | M10 | N10 | O10 | P10 |
| R16 | A9 | B9 | C9 | D9 | E9 | F9 | G9 | H9 | I9 | J9 | K9 | L9 | M9 | N9 | O9 | P9 |
| R17 | A8 | B8 | C8 | D8 | E8 | F8 | G8 | H8 | I8 | J8 | K8 | L8 | M8 | N8 | O8 | P8 |
| R18 | A7 | B7 | C7 | D7 | E7 | F7 | G7 | H7 | I7 | J7 | K7 | L7 | M7 | N7 | O7 | P7 |
| R19 | A6 | B6 | C6 | D6 | E6 | F6 | G6 | H6 | I6 | J6 | K6 | L6 | M6 | N6 | O6 | P6 |
| R20 | A5 | B5 | C5 | D5 | E5 | F5 | G5 | H5 | I5 | J5 | K5 | L5 | M5 | N5 | O5 | P5 |
| R21 | A4 | B4 | C4 | D4 | E4 | F4 | G4 | H4 | I4 | J4 | K4 | L4 | M4 | N4 | O4 | P4 |
| R22 | A3 | B3 | C3 | D3 | E3 | F3 | G3 | H3 | I3 | J3 | K3 | L3 | M3 | N3 | O3 | P3 |
| R23 | A2 | B2 | C2 | D2 | E2 | F2 | G2 | H2 | I2 | J2 | K2 | L2 | M2 | N2 | O2 | P2 |
| R24 | A1 | B1 | C1 | D1 | E1 | F1 | G1 | H1 | I1 | J1 | K1 | L1 | M1 | N1 | O1 | P1 |

A

| r1-c1 | r1-c2 | r1-c3 | r1-c4 |
|---|---|---|---|
| r2-c1 | r2-c2 | r2-c3 | r2-c4 |
| r3-c1 | r3-c2 | r3-c3 | r3-c4 |
| r4-c1 | r4-c2 | r4-c3 | r4-c4 |

B

C

Figure 3

**European Patent**
**Office**

## PARTIAL EUROPEAN SEARCH REPORT

**Application Number**

which under Rule 45 of the European Patent Convention EP 03 00 4838
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | FIEHN O ET AL: "Deciphering metabolic networks" EUROPEAN JOURNAL OF BIOCHEMISTRY 2003 UNITED KINGDOM, vol. 270, no. 4, February 2003 (2003-02), pages 579-588, XP002253430 ISSN: 0014-2956 * figure 3 * | 1-11 | C12Q1/68 G01N33/483 G01N30/72 |
| X | MAY GD: "An integrated approach to MEDICAGO functional genomics" RECENT ADVANCES IN PHYTOCHEMISTRY, vol. 36, 2002, pages 179-195, XP008021427 * figure 11.1 * | 1-11 | |
| E | WO 03 058238 A (MAX PLANCK GESELLSCHAFT) 17 July 2003 (2003-07-17) * page 5, line 1 - page 7, line 1 * | 1 | |
| | -/-- | | |

| | |
|---|---|
| | TECHNICAL FIELDS SEARCHED (Int.Cl.7) |
| | G01N C12Q |

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 4 September 2003 | Gunster, M |

**European Patent Office**

**INCOMPLETE SEARCH SHEET C**

Application Number

EP 03 00 4838

Claim(s) not searched:
    12-14

Reason for the limitation of the search:

Present claims 12-14 relate to the use of genes or metabolites defined by reference to a desirable characteristic or property, namely that their function can be determined by performing the method of claims 1-11

The claims cover all genes and metabolites having this characteristic or property, whereas the application provides support within the meaning of Article 84 EPC and disclosure within the meaning of Article 83 EPC only for the genes and metabolites of Table 3. In the present case, the claims so lack support, and the application so lacks disclosure, that a meaningful search over the whole of the claimed scope is impossible. Independent of the above reasoning, the claims also lack clarity (Article 84 EPC). An attempt is made to define the genes or metabolites by reference to a result to be achieved. Again, this lack of clarity in the present case is such as to render a meaningful search over the whole of the claimed scope impossible.

Furthermore, the use of genes or metabolites as defined in claims 12-14 is for carrying out a certain function of the gene or metabolite, which function can allegedly determined by the methods of claims 1-11. However, the methods of claims 1-11 do not determine the function of genes or metabolites at all. The methods of claims 1-11 merely show that the altered expression of a number of genes coincides(or correlates) with an alteration in the levels of certain metabolites. From this correlation it can in no way be deducted that there is a causal relationship between the expression levels of said genes and the levels of said metabolites. Thus, there is no support or disclosure in the description as to whether the genes or metabolites can actually perform the intended function.

Consequently, claims 12-14 so lack clarity, support and disclosure in the sense of Articles 83 and 84 EPC that a search of claim 12-14 is impossible.

## DOCUMENTS CONSIDERED TO BE RELEVANT

**European Patent Office**

**PARTIAL EUROPEAN SEARCH REPORT**

**Application Number**

EP 03 00 4838

| Category | Citation of document with indication, where appropriate, of relevant passages | Relevant to claim |
|---|---|---|
| X | PHELPS TOMMY J ET AL: "Metabolomics and microarrays for improved understanding of phenotypic characteristics controlled by both genomics and environmental constraints." CURRENT OPINION IN BIOTECHNOLOGY, vol. 13, no. 1, February 2002 (2002-02), pages 20-24, XP002253431 ISSN: 0958-1669 * abstract * * page 20, column 2, paragraph 2 * * page 23, column 1, paragraph 3 * --- | 1-11 |
| X | SUMNER L W ET AL: "Plant metabolomics: large-scale phytochemistry in the functional genomics era" PHYTOCHEMISTRY, PERGAMON PRESS, GB, vol. 62, no. 6, available online, 8 February 2003 (2003-02-08), pages 817-836, XP004408969 ISSN: 0031-9422 * page 829, column 2, line 38 - line 40; figure 1 * --- | 1-11 |
| A | RAAMSDONK L M ET AL: "A functional genomics strategy that uses metabolome data to reveal the phenotype of silent mutations." NATURE BIOTECHNOLOGY. UNITED STATES JAN 2001, vol. 19, no. 1, January 2001 (2001-01), pages 45-50, XP002253432 ISSN: 1087-0156 * the whole document * --- | 1-11 |

-/--

**CLASSIFICATION OF THE APPLICATION (Int.Cl.7)**

**TECHNICAL FIELDS SEARCHED (Int.Cl.7)**

EPO FORM 1503 03.82 (P04C10)

European Patent  PARTIAL EUROPEAN SEARCH REPORT  Application Number

Office  EP 03 00 4838

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| D,A | TER KUILE A B H ET AL: "Transcriptome meets metabolome: hierarchical and metabolic regulation of the glycolytic pathway" FEBS LETTERS, ELSEVIER SCIENCE PUBLISHERS, AMSTERDAM, NL, vol. 500, no. 3, 6 July 2001 (2001-07-06), pages 169-171, XP004251401 ISSN: 0014-5793 * the whole document * | 1-11 | |
| A | AHARONI A ET AL: "Identification of the SAAT gene involved in strawberry flavor biogenesis by use of DNA microarrays." THE PLANT CELL. UNITED STATES MAY 2000, vol. 12, no. 5, May 2000 (2000-05), pages 647-661, XP002253433 ISSN: 1040-4651 * figures 4,5 * | 1-11 | **TECHNICAL FIELDS SEARCHED** (Int.Cl.7) |
| A | OLIVER DAVID J ET AL: "Functional genomics: high-throughput mRNA, protein, and metabolite analyses." METABOLIC ENGINEERING. UNITED STATES JAN 2002, vol. 4, no. 1, January 2002 (2002-01), pages 98-106, XP002253434 ISSN: 1096-7176 * page 105, column 1 * | 1-11 | |
| A | AHARONI ASAPH ET AL: "DNA microarrays for functional plant genomics." PLANT MOLECULAR BIOLOGY, vol. 48, no. 1-2, January 2002 (2002-01), pages 99-118, XP002253435 January, 2002 ISSN: 0167-4412 * page 115, column 2, paragraph 3 * | 1-11 | |

-/--

EPO FORM 1503 03.82 (P04C10)

European Patent
Office

**PARTIAL EUROPEAN SEARCH REPORT**

Application Number

EP 03 00 4838

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| D,A | FIEHN OLIVER: "Metabolomics: The link between genotypes and phenotypes." PLANT MOLECULAR BIOLOGY, vol. 48, no. 1-2, January 2002 (2002-01), pages 155-171, XP002253436 January, 2002 ISSN: 0167-4412 * the whole document * | 1-11 | |
| A | DE LA FUENTE A ET AL: "Metabolic control in integrated biochemical systems" EUROPEAN JOURNAL OF BIOCHEMISTRY 2002 UNITED KINGDOM, vol. 269, no. 18, 2002, pages 4399-4408, XP002253437 ISSN: 0014-2956 * the whole document * | 1-11 | TECHNICAL FIELDS SEARCHED (Int.Cl.7) |
| A | MENDES P: "Bioinformatics and computational biology for plant functional genomics" RECENT ADVANCES IN PHYTOCHEMISTRY, vol. 36, 2002, pages 1-13, XP008021534 * the whole document * | 1-11 | |

EPO FORM 1503 03.82 (P04C10)

## ANNEX TO THE EUROPEAN SEARCH REPORT
## ON EUROPEAN PATENT APPLICATION NO.

EP 03 00 4838

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2003

| Patent document cited in search report | Publication date | Patent family member(s) | Publication date |
|---|---|---|---|
| WO 03058238 A | 17-07-2003 | EP 1327883 A2 | 16-07-2003 |
| | | WO 03058238 A1 | 17-07-2003 |

EPO FORM P0459

For more details about this annex : see Official Journal of the European Patent Office, No. 12/82